# EUROPEAN PATENT APPLICATION

(11) **EP 1 491 535 A1**
(43) Date of publication of application: **29.12.2004**
(21) Application number: 03710257.1
(22) Date of filing: 06.03.2003
(51) Int. Cl.: C07D 261/08, C07D 413/04, C07D 413/12, C07D 417/04, C07D 417/12, C07D 413/06, A01N 43/80, A01N 47/12, A01N 47/18, A01N 47/22, A01N 47/28, A01N 47/30, A01N 47/32, A01N 47/36, A01N 47/38

(54) **SUBSTITUTED ISOXAZOLE ALKYLAMINE DERIVATIVE AND AGRI- AND HORTICULTURAL FUNGICIDE**

(30) Priority: 07.03.2002 JP 2002061835
(71) Applicant: SDS Biotech K.K., Tokyo 105-0014 (JP)
(72) Inventor: SHIMOZONO, N., c/o Tsukuba Lab., SDS Biotech K.K., Tsukuba-shi, Ibara 300-2646 (JP); WADA, H., c/o Tsukuba Lab., SDS Biotech K.K., Tsukuba-shi, Ibara 300-2646 (JP)
(74) Representative: Strehl Schübel-Hopf & Partner
(86) International application number: PCT/JP2003/002632
(87) International publication number: WO 2003/074501

(57) **Abstract**

An agri-horticultural fungicide containing a substituted isoxazole alkylamine derivative represented by the formula (I) as an active ingredient exhibits no crossreaction with known drug resistant fungi and exerts a sufficient activity for preventing plant disease damages : 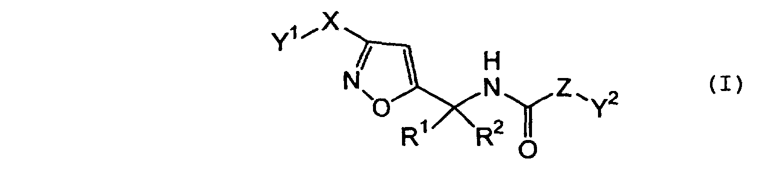 wherein R¹ and R² each represent a hydrogen atom, a (substituted) alkyl, or the like; X is a single bond, alkylene, or the like; Y¹ represents a (substituted) lower alkyl, (substituted) cycloalkyl, (substituted) phenyl, (substituted) heteroaryl, or the like; Y² represents a (substituted) cycloalkyl, (substituted) phenyl, (substituted) heteroaryl, or the like; and Z represents a (substituted) alkylene, -O-((substituted)alkylene)-, -NR-((substituted)alkylene)- or the like.

## Description

### TECHNICAL FIELD

The present invention relates to:
1) a novel substituted isoxazole derivative represented by the formula (I): 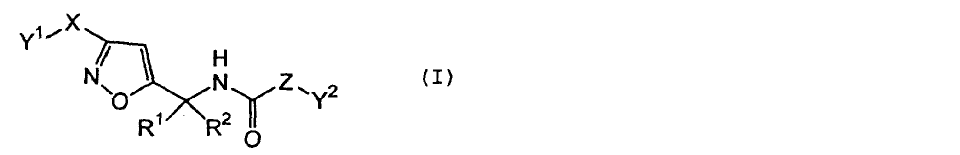 (symbols in the formula are as described later); and
2) an agri-horticultural fungicide containing a substituted isoxazole derivative represented by the formula (IA): 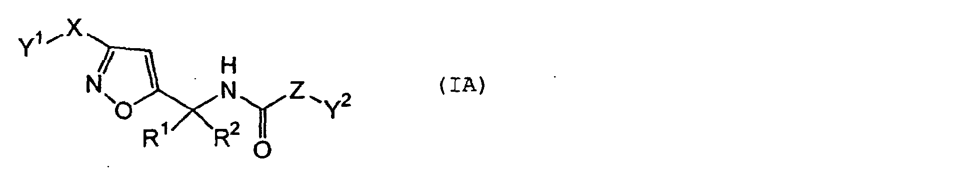 (symbols in the formula are as described later).

### BACKGROUND ART

1) JP 9-509951 A, International Publication WO 98/28282, US Patent No. 5,859,257, British Patent No. 1,067,203, and European Patent No. 277,791 disclose compounds of substituted isoxazole alkylamine derivatives, but have no description about fungicidal activity.
2) In J. C. S. Perkin Trans. I, 16, 1694-1696 (1976), a spirooxazoline ring formation reaction is performed using the following compounds (a) and (b) as starting materials, but there is no description about fungicidal activity of the compounds (a) and (b). 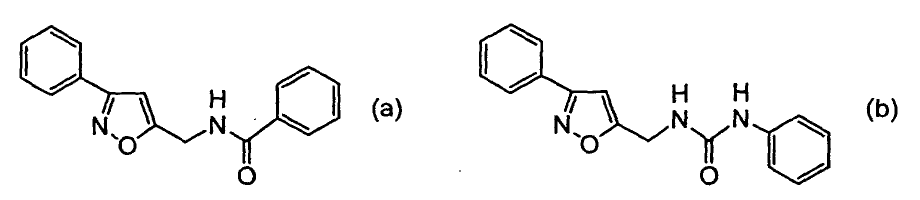
3) JP 52-154525 A shows that a compound represented by the following general formula (A): 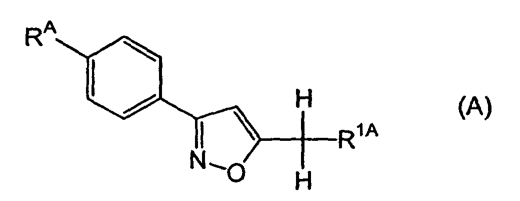 (wherein R^{A} represents a chloro, bromo, fluoro, or trifluoromethyl group or a hydrogen atom, and R^{1A} represents a bromo, chloro, isothiocyanate, or amino group, amino-hydrochloride or hydrobromide, -NHCSNH(C1 to C3)alkyl, -NHCOO(C1 to C3)alkyl, or 3,5,7-triaza-1-azonia adamantyl chloride or bromide) serves as fungicide to exhibit disease control activity particularly against tomato late blight, bean powdery mildew, cucumber anthracnose, rice blast, wheat leaf spot, grape gray mold, apple scab, grape downy mildew, and sugar beet leaf spot. However, the activity as the fungicide of the compound described in the specification of this patent document is not sufficient.

In cultivation of agricultural or horticultural crops, various disease damages which seriously affect productive efficiency have been known, and examples of the vital disease damages include: rice blast and sheath blight in rice cultivation; and powdery mildew, downy mildew, and gray mold in cultivation of fruits, vegetables, and grain crops.

Various fungicides have been used against the disease damages caused by those plant pathogenic fungi. However, some fungicides known publicly and used conventionally are not effective enough any longer because fungi resistant to each of the fungicides occur owing to long-continued use of the fungicides, or some of the fungicides can exhibit sufficient fungicidal effects only when used in combination with other drugs. Therefore, in the industrial field of agricultural or horticultural cultivation, a novel compound for preventing/treating plant disease damages, with a chemical skeleton different from conventional drugs, which exerts a sufficient preventive effect, has been long demanded. The invention is to solve problems involved in the prior art mentioned above. An object of the invention is to provide a novel drug which, owing to its chemical skeleton different from those of conventionally known drugs exerts a sufficient activity for preventing plant disease damage without cross-reacting with resistant fungi.

### DISCLOSURE OF THE INVENTION

In order to accomplish the above object, the inventors of the present invention have synthesized novel substituted isoxazole alkylamine derivatives whose activity for preventing plant disease damages has not been known, and they have intensively studied on the activity for preventing plant disease damages and usefulness of the derivatives. As a result, the inventors have found that a substituted isoxazole alkylamine derivative represented by the formula (I) or the formula (IA) described below has an excellent plant pathogenic fungus control effect without giving a harmful effect to plants.

That is, the present invention relates to an agent for preventing plant diseases which is for use in the agri-horticultural field, comprising as active ingredient a substituted isoxazole alkylamine derivative represented by the following general formula (I) or a substituted isoxazole alkylamine derivative represented by the following general formula (IA).

A substituted isoxazole alkylamine derivative denoted by the formula (I): 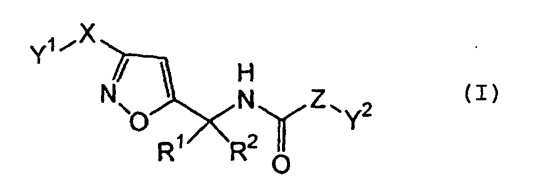 wherein R¹ and R² may be the same or different, and each represent a hydrogen atom, a lower alkyl group which may be substituted, a lower alkenyl group, a lower alkynyl group, a cycloalkyl group which may be substituted, a lower alkoxy group, a lower alkoxycarbonyl group, a lower alkylthio group, a halogen atom, a hydroxyl group, a carboxyl group, or a cyano group, or R¹ and R² may together form a cycloalkyl group which may be substituted;
X represents the following formula (1), (2), or (3): 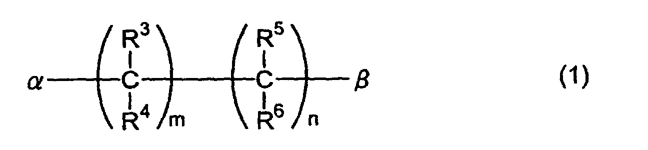 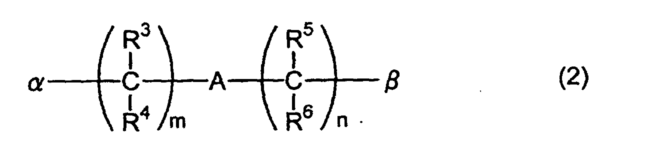 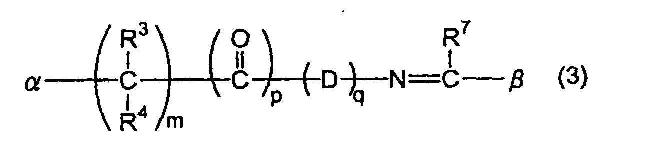 (wherein R³, R⁴, R⁵, and R⁶ may be the same or different, and each represent a hydrogen atom, a lower alkyl group which may be substituted, a lower alkenyl group, a lower alkynyl group, a cycloalkyl group which may be substituted, a lower alkoxy group, a lower alkoxycarbonyl group, a lower alkylthio group, an amino group, a lower alkylamino group, a di(lower alkyl)amino group, a halogen atom, a hydroxyl group, or a cyano group, or each pair of R³ and R⁴, and R⁵ and R⁶ may together form a cycloalkyl group which may be substituted;
A represents an oxygen atom, a sulfur atom, an -S(O)- group, an -S(O)₂- group, an -NR¹²- group (wherein R¹² represents a hydrogen atom or a lower alkyl group), a carbonyl group, an -NH-CO- group, a -CO-NH- group, a -C=C- group, an -NH-CO-NH- group, an -O-CONH- group, an -HC=N- group or a group represented by the formula below 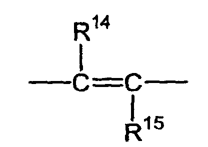 (wherein R¹⁴ and R¹⁵ each represent a hydrogen atom or a lower alkyl group);
m and n each represent 0 or an integer of 1 to 3;
p and q each represent 0 or 1;
R⁷ represents a hydrogen atom or a lower alkyl group; and
D represents an oxygen atom or an -NH- group, wherein α binds to a Y¹ side, and β binds to an isoxazole moiety);
Y¹ represents a lower alkyl group which may be substituted, a lower alkenyl group which may be substituted, a lower alkynyl group which may be substituted, a cycloalkyl group which may be substituted, a lower cyclo alkenyl group which may be substituted, a phenyl group which may be substituted, a naphthyl group which may be substituted, a heteroaryl group which may be substituted, an aliphatic hetero ring which may be substituted, or the following formula (4): 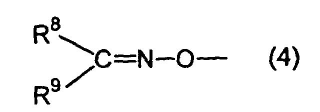 (wherein R⁸ and R⁹ each represent a hydrogen atom, a lower alkyl group which may be substituted, or a phenyl group which may be substituted, or R⁸ and R⁹ may together form a cycloalkyl group which may be substituted);
Y² represents a lower alkenyl group which may be substituted, a lower alkynyl group which may be substituted, a cycloalkyl group which may be substituted, a lower cyclo alkenyl group which may be substituted, a phenyl group which may be substituted, a naphthyl group which may be substituted, a heteroaryl group which may be substituted, or an aliphatic hetero ring which may be substituted;
Z represents a group denoted by the following formula (5) or (6): 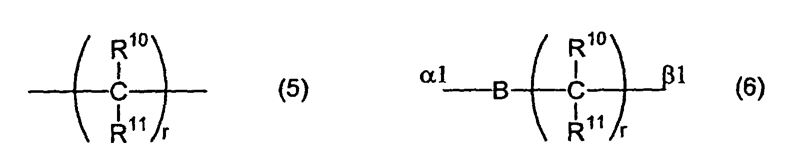 (wherein R¹⁰ and R¹¹ may be the same or different, and each represent a hydrogen atom, a lower alkyl group which may be substituted, a cycloalkyl group which may be substituted, a lower alkoxy group, a lower alkoxycarbonyl group, a lower alkylthio group, an amino group, a lower alkylamino group, a di(lower alkyl)amino group, a halogen atom, a hydroxyl group, or a cyano group, or R¹⁰ and R¹¹ may together form a cycloalkyl group which may be substituted;
r represents 0 or an integer of 1 to 3; and
B represents an oxygen atom, a sulfur atom, or an -NR¹³- group wherein R¹³ represents a hydrogen atom or a lower alkyl group), wherein any one of α1 and β1 may be bound to a Y² side;
provided that the following (1) to (3) are excluded:
(1) a compound in which X is a single bond (i.e. the case that each of m and n is 0 in the formula (1)) and Y¹ represents a 4-hydroxy-3,5-di-tert-butylphenyl group;
(2) a compound in which R¹ and R² represent hydrogen atoms, X is a single bond (i.e. the case that each of m and n is 0 in the formula (1)), and both Y¹ and Y² represent unsubstituted phenyl groups (2-1) when Z is a single bond (i.e. the case that r is 0 in the formula (5)) or (2-2) when Z is an NH group (i.e. the case that r=0 and B represents an -NH- group in the formula (6)); and
(3) a compound in which R¹ and R² represent hydrogen atoms, Z is a single bond (i.e. the case that r is 0 in the formula (5)), and Y² represents a 4-hydroxycinnolin-3-yl group which may be substituted.

A substituted isoxazole alkylamine derivative represented by the formula (IA): 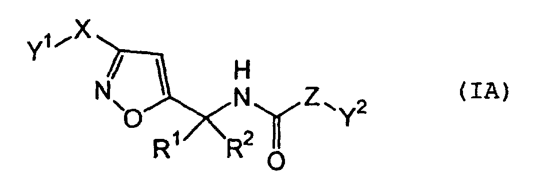 wherein R¹ and R² may be the same or different, and each represent a hydrogen atom, a lower alkyl group which may be substituted, a lower alkenyl group, a lower alkynyl group, a cycloalkyl group which may be substituted, a lower alkoxy group, a lower alkoxycarbonyl group, a lower alkylthio group, a halogen atom, a hydroxyl group, a carboxyl group or a cyano group, or R¹ and R² may together form a cycloalkyl group which may be substituted;
X represents the following formula (1), (2) or (3): 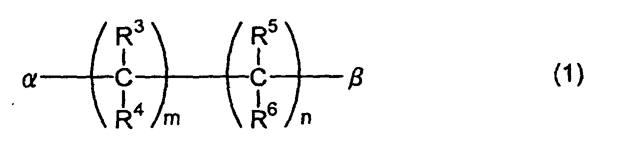 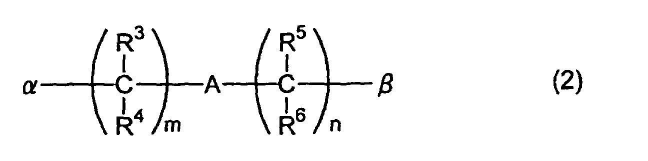 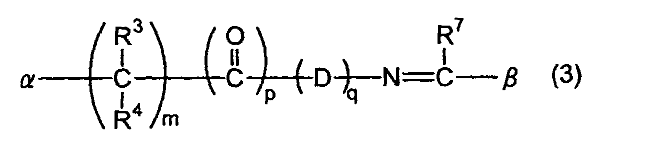 (wherein R³, R⁴, R⁵, and R⁶ may be the same or different, and each represent a hydrogen atom, a lower alkyl group which may be substituted, a lower alkenyl group; a lower alkynyl group, a cycloalkyl group which may be substituted, a lower alkoxy group, a lower alkoxycarbonyl group, a lower alkylthio group, an amino group, a lower alkylamino group, a di(lower alkyl)amino group, a halogen atom, a hydroxyl group, or a cyano group, or each pair of R³ and R⁴, and R⁵ and R⁶ may together form a cycloalkyl group which may be substituted;
A represents an oxygen atom, a sulfur atom, an -S(O)- group, an -S(O)₂- group, an -NR¹²- group (wherein R¹² represents a hydrogen atom or a lower alkyl group), a carbonyl group, an -NH-CO- group, a -CO-NH- group, a -C≡C- group, an -NH-CO-NH- group, an -O-CONH- group, an -HC=N- group, or
a 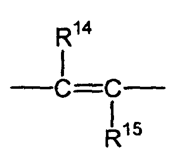 group
(wherein R¹⁴ and R¹⁵ each represent a hydrogen atom or a lower alkyl group);
m and n each represent 0 or an integer of 1 to 3;
p and q each represent 0 or 1;
R⁷ represents a hydrogen atom or a lower alkyl group; and
D represents an oxygen atom or an -NH- group,
α binds to a Y¹ side, and β binds to an isoxazole moiety);
Y¹ represents a lower alkyl group which may be substituted, a lower alkenyl group which may be substituted, a lower alkynyl group which may be substituted, a cycloalkyl group which may be substituted, a lower cyclo alkenyl group which may be substituted, a phenyl group which may be substituted, a naphthyl group which may be substituted, a heteroaryl group which may be substituted, an aliphatic hetero ring which may be substituted, or the following formula (4): 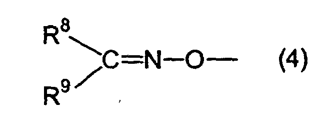 (wherein R⁸ and R⁹ each represent a hydrogen atom, a lower alkyl group which may be substituted, or a phenyl group which may be substituted, or R⁸ and R⁹ may together form a cycloalkyl group which may be substituted);
Y² represents a lower alkenyl group which may be substituted, a lower alkynyl group which may be substituted, a cycloalkyl group which may be substituted, a lower cyclo alkenyl group which may be substituted, a phenyl group which may be substituted, a naphthyl group which may be substituted, a heteroaryl group which may be substituted, or an aliphatic hetero ring which may be substituted;
Z represents a group represented by the following formula (5) or (6): 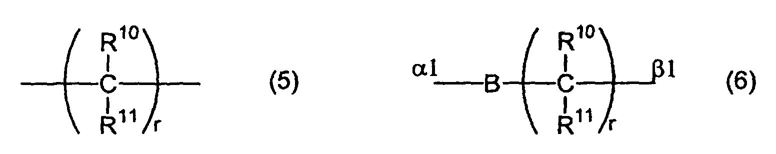 (wherein R¹⁰ and R¹¹ may be the same or different, and each represent a hydrogen atom, a lower alkyl group which may be substituted, a cycloalkyl group which may be substituted, a lower alkoxy group, a lower alkoxycarbonyl group, a lower alkylthio group, an amino group, a lower alkylamino group, a di(lower alkyl)amino group, a halogen atom, a hydroxyl group, or a cyano group, or R¹⁰ and R¹¹ may together form a cycloalkyl group which may be substituted;
r represents 0 or an integer of 1 to 3; and
B represents an oxygen atom, a sulfur atom, or an -NR¹³- group wherein R¹³ represents a hydrogen atom or a lower alkyl group), wherein any one of α1 and β1 may be bound to a Y² side.

### [Compounds of the Present Invention]

In the formula (I) or (IA), lower alkyl group refers to an alkyl group having 1 to 6 carbon atoms such as methyl, ethyl, propyl, butyl, pentyl, and hexyl groups, and isomer groups thereof.

The lower alkenyl group refers to an alkenyl group having 1 to 3 double bonds and 2 to 6 carbon atoms such as vinyl, propenyl, butenyl, butadienyl, and hexatrienyl groups, and isomer groups thereof.

The lower alkynyl group refers to an alkynyl group having 1 to 3 triple bonds and 2 to 6 carbon atoms such as an ethynyl, propynyl, pentynyl, and hexynyl group, and isomer groups thereof.

The cycloalkyl group refers to a cycloalkyl group having 3 to 8 carbon atoms such as cyclopropyl, cyclobutyl, cyclopentyl, cyclohexyl, cycloheptyl, and cyclooctyl groups.

The cycloalkenyl group refers to a cycloalkenyl group having 3 to 8 carbon atoms such as cyclopropynyl, cyclobutenyl, cyclopentenyl, cyclohexenyl, cycloheptenyl, and cyclooctenyl groups, and isomer groups thereof.

The lower alkoxy group refers to an alkoxy group having 1 to 6 carbon atoms such as a methyloxy group, an ethyloxy group, a propyloxy group, a butyloxy group, a pentyloxy group, a hexyloxy group, and isomer groups thereof.

The lower alkoxycarbonyl group refers to an alkoxycarbonyl group having 1 to 6 carbon atoms such as a methyloxycarbonyl group, an ethyloxycarbonyl group, a propyloxycarbonyl group, a butyloxycarbonyl group, a pentyloxycarbonyl group, a hexyloxycarbonyl group, and isomer groups thereof.

The lower alkylthio group refers to an alkylthio group having 1 to 6 carbon atoms such as a methylthio group, an ethylthio group, a propylthio group, a butylthio group, a pentylthio group, a hexylthio group, and isomer groups thereof.

The halogen atom refers to a fluorine atom, a chlorine atom, a bromine atom, or an iodine atom.

The heteroaryl group refers to an aromatic monocyclic or bicyclic hetero ring having 1 to 3 hetero atoms including an oxygen atom, a sulfur atom, and a nitrogen atom as a ring forming atom. Examples thereof include pyrrole, pyrazole, imidazole, triazole, pyridine, pyrimidine, triazine, furan, thiophene, thiazole, oxazole, isoxazole, isothiazole, benzothiazole, benzoxazole, indazole, indole, quinoline, purine, and pteridine rings.

The aliphatic hetero ring refers to a 5 to 6-membered aliphatic hetero ring having 1 to 3 hetero atoms including an oxygen atom, a sulfur atom, and a nitrogen atom as a ring forming atom. Examples thereof include a pyrrolidine ring, a piperidine ring, a piperazine ring, a tetrahydropyran ring, a tetrahydrofuran ring, a morpholine ring, and dioxane.

A lower alkyl group which may be substituted, a lower alkenyl group which may be substituted, a lower alkynyl group which may be substituted, a cycloalkyl group which may be substituted, a cycloalkenyl group which may be substituted, lower alkoxy group which may be substituted, or an aliphatic hetero ring which may be substituted may be substituted with one or more substituents selected from: a lower alkoxy group; a lower alkoxycarbonyl group; a halogen atom; a cyano group; a nitro group; a phenyl group which may be substituted; a lower acyl group; and a lower acyloxy group.

A phenyl group which may be substituted, a phenyloxy group which may be substituted, a naphthyl group which may be substituted, or a heteroaryl group which may be substituted may be substituted with one or more substituents selected from: a lower alkyl group which may be substituted with a halogen atom or a lower alkoxy group; a lower alkenyl group which may be substituted; a lower alkynyl group which may be substituted; a lower alkoxy group which may be substituted with a halogen atom and like; a lower alkylthio group; a lower alkanesulfinyl group; a lower alkanesulfonyl group; an R¹⁶-NHCO- group; an R¹⁶-CONH- group (wherein, R¹⁶ represents a hydrogen atom or a lower alkyl group); an R¹⁷-O-NHCO- group (wherein, R¹⁷ represents a lower alkyl group); an R¹⁸-CO- group (wherein, R¹⁸ represents a hydrocarbon group (for example, a lower alkyl group and a phenyl group) which may be substituted with a halogen atom and like); a lower alkoxycarbonyl group; a carboxyl group; a cycloalkyl group which may be substituted; an amino group; a lower alkylamino group; a lower dialkylamino group; an R¹⁹O-N=C(R²⁰)- group (wherein, R¹⁹ represents a hydrogen atom or a lower alkyl group, and R²⁰ represents a hydrogen atom, a lower alkyl group, or an amino group); an aliphatic heterocyclo ring group; a lower alkylcarbonylhydrazino group; a lower alkyloxycarbonylhydrazino group; a formyl group; an H₂NN=C(R²¹)- group; an R²²(O)CHNN=C(R²¹)- group (wherein, R²¹ and R²² each represent a hydrogen atom or a lower alkyl group); a hydroxyl group; a phenyl group which may be substituted; a phenyloxy group which may be substituted; a naphthyl group which may be substituted; a heteroaryl group which may be substituted; a halogen atom; a cyano group; and a nitro group. Further, adjacent substituents together may form a cyclic alkyl group which may be substituted or a cyclic ether group which may be substituted.

A preferable X may be provided as follows. In the above-mentioned formula (1), m and n are each 0 or 1, more specifically m = n = 0 (single bond), m + n = 1 (a methylene group which may be substituted), and m = n = 1 (an ethylene group which may be substituted). In the above-mentioned formula (2), m and n are preferably each 0 or 1, more preferably m = n = 0 (A), m = 1 and n = 0, and m = 0 and n = 1, even more preferably m = n = 0 (A), and m = 0 and n = 1. In the above-mentioned formula (3), preferable examples of the -(CO)p-(D)q- include a single bond, an -O- group, and a -CONH- group.

A preferable Z is a group provided in the above-mentioned formulae (5) and (6), where r is 0 or 1. More preferable examples of Z include a single bond, a -CH(R^{10a})-group (where, R^{10a} represents a hydrogen atom, a C1 to C3 alkyl group, a halogen atom, or a cyano group), an -O- group, an -NH- group, an -N(CH₃)- group, an -O-C(R^{11a})₂- group, a -C(R^{11a})₂-O- group, and an -NH-C(R^{11a})₂- group (where, R^{11a} independently represents a hydrogen atom or C1 to C3 alkyl group).

Preferable examples of Y¹ include: a lower alkyl group which may be substituted; a cycloalkyl group which may be substituted; a phenyl group which may be substituted; and a heteroaryl group which may be substituted.

Preferable examples of Y² include: a cycloalkyl group which may be substituted; a phenyl group which may be substituted; a naphthyl group which may be substituted; and a heteroaryl group which may be substituted. More Preferred examples of Y² include: a cycloalkyl group which may be substituted; a phenyl group which may be substituted; and a naphthyl group which may be substituted.

Examples of the compound represented by the formula (I) or (IA) are preferably those represented below.
[1] A substituted isoxazole alkylamine derivative, in which R¹ and R² each independently represent a hydrogen atom or a lower alkyl group which may be substituted.
[2] The substituted isoxazole alkylamine derivative according to [1], in which Z is an oxygen atom (when r is 0 and B is an oxygen atom in the formula (6)), and Y² represents a phenyl group which may be substituted, a naphthyl group which may be substituted, or a cycloalkyl group which may be substituted.
[3] The substituted isoxazole alkylamine derivative according to [1], in which Z represents an -NR¹³- group (wherein R¹³ represents a hydrogen atom or a lower alkyl group) (when r is 0 and B is an -NR¹³- group) and Y² represents a phenyl group which may be substituted, a naphthyl group which may be substituted, or a cycloalkyl group which may be substituted.
[4] The substituted isoxazole alkylamine derivative according to [1], in which X and Z are single bonds (when m and n are 0 in the formula (1) and r is 0 in the formula (5)) and Y² represents a phenyl group which may be substituted, a naphthyl group which may be substituted, or a cycloalkyl group which may be substituted.
[5] The substituted isoxazole alkylamine derivative according to [1], in which X represents a group represented by the formula (1) wherein R³, R⁴, R⁵, and R⁶ may be the same or different and each represent a hydrogen atom, a lower alkyl group which may be substituted, a lower alkoxy group, a lower alkoxycarbonyl group, a halogen atom, or a cyano group, Y² represents a phenyl group which may be substituted, a naphthyl group which may be substituted, or a cycloalkyl group which may be substituted, and m + n is 1, 2, or 3.
[6] The substituted isoxazole alkylamine derivative according to [1], in which X represents a group represented by the formula (2) wherein R³, R⁴, R⁵, and R⁶ may be the same or different and each represent a hydrogen atom, a lower alkyl group which may be substituted, a lower alkoxy group, a lower alkoxycarbonyl group, a halogen atom, or a cyano group, Y² represents a phenyl group which may be substituted, a naphthyl group which may be substituted, or a cycloalkyl group which may be substituted, n is 1 or 2, and m + n is 1, 2, or 3.
[7] The substituted isoxazole alkylamine derivative according to [1], in which X represents a group represented by the formula (2) wherein R³, R⁴, R⁵, and R⁶ may be the same or different and each represent a hydrogen atom, a lower alkyl group which may be substituted, a lower alkoxy group, a lower alkoxycarbonyl group, a halogen atom, or a cyano group, Y² represents a phenyl group which may be substituted, a naphthyl group which may be substituted, or a cycloalkyl group which may be substituted, n is 0, and m is 0, 1, or 2.
[8] The substituted isoxazole alkylamine derivative according to [1], in which X represents a group denoted by the formula (3) wherein R³ and R⁴ may be the same or different and each represent a hydrogen atom, a lower alkyl group which may be substituted, a lower alkoxy group, a halogen atom, or a cyano group, m represents 0, 1, or 2, and Y² represents a phenyl group which may be substituted, a naphthyl group which may be substituted, or a cycloalkyl group which may be substituted.
[9] The substituted isoxazole alkylamine derivative according to [1], in which Y¹ represents a methyl group substituted with a halogen atom, and Z is a single bond (when m and n are 0 in the formula (1)).

Next, representative examples of the compounds of the invention represented by the formula (I) are shown in Tables 1-1 to 4-8, but the compounds of the invention are not limited thereto (the same number is used as Compound Number throughout the specification.).

In the table, Me represents a methyl group, Et represents an ethyl group, n-Pr represents a normal propyl group, i-Pr represents an isopropyl group, n-Bu represents a normal butyl group, i-Bu represents an isobutyl group, t-Bu represents a tertiary butyl group, c-Bu represents a cyclobutyl group, c-Pen represents a cyclopentyl group, c-Hex represents a cyclohexyl group, Ac represents an acetyl group, and Ph represents a phenyl group.

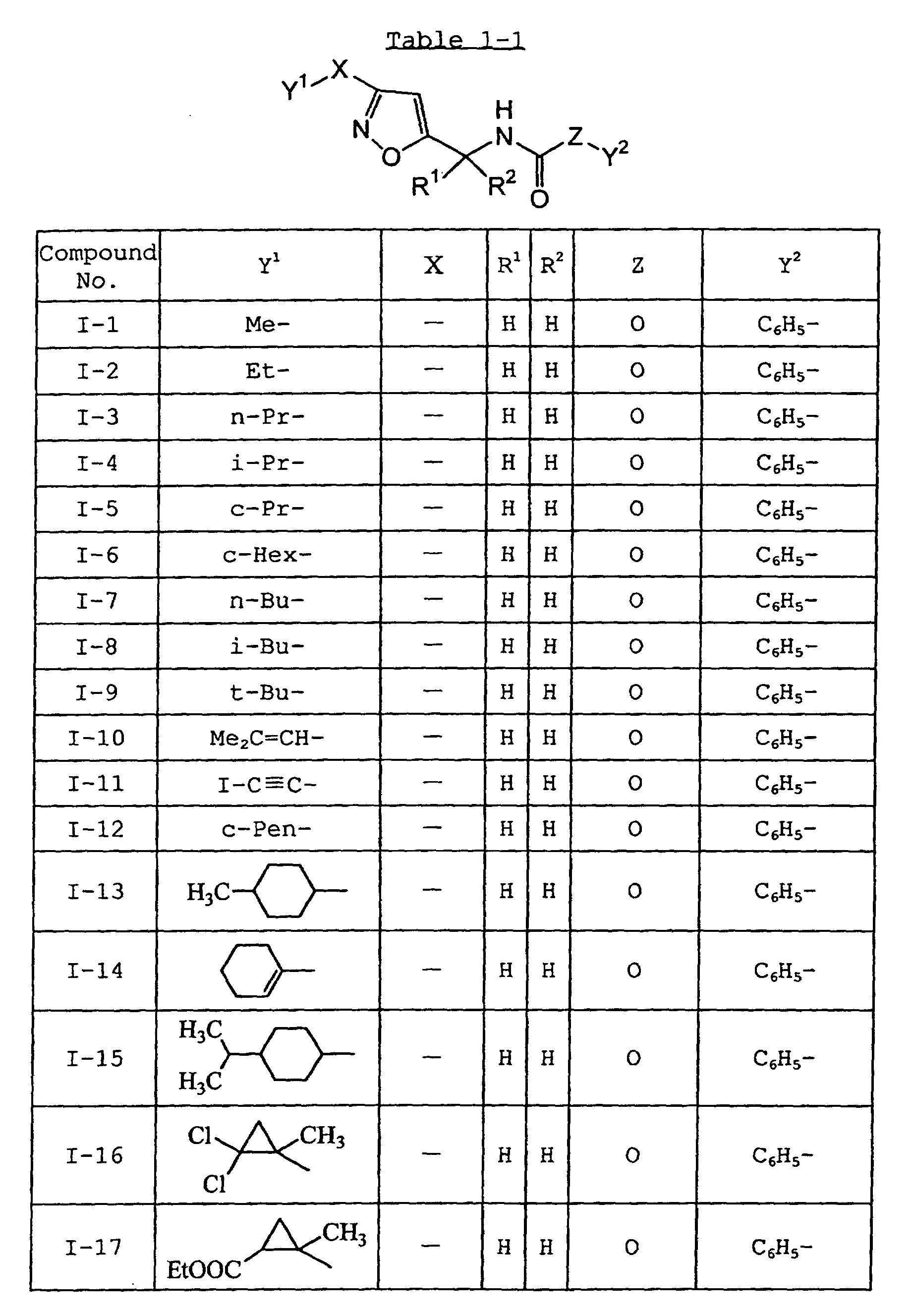

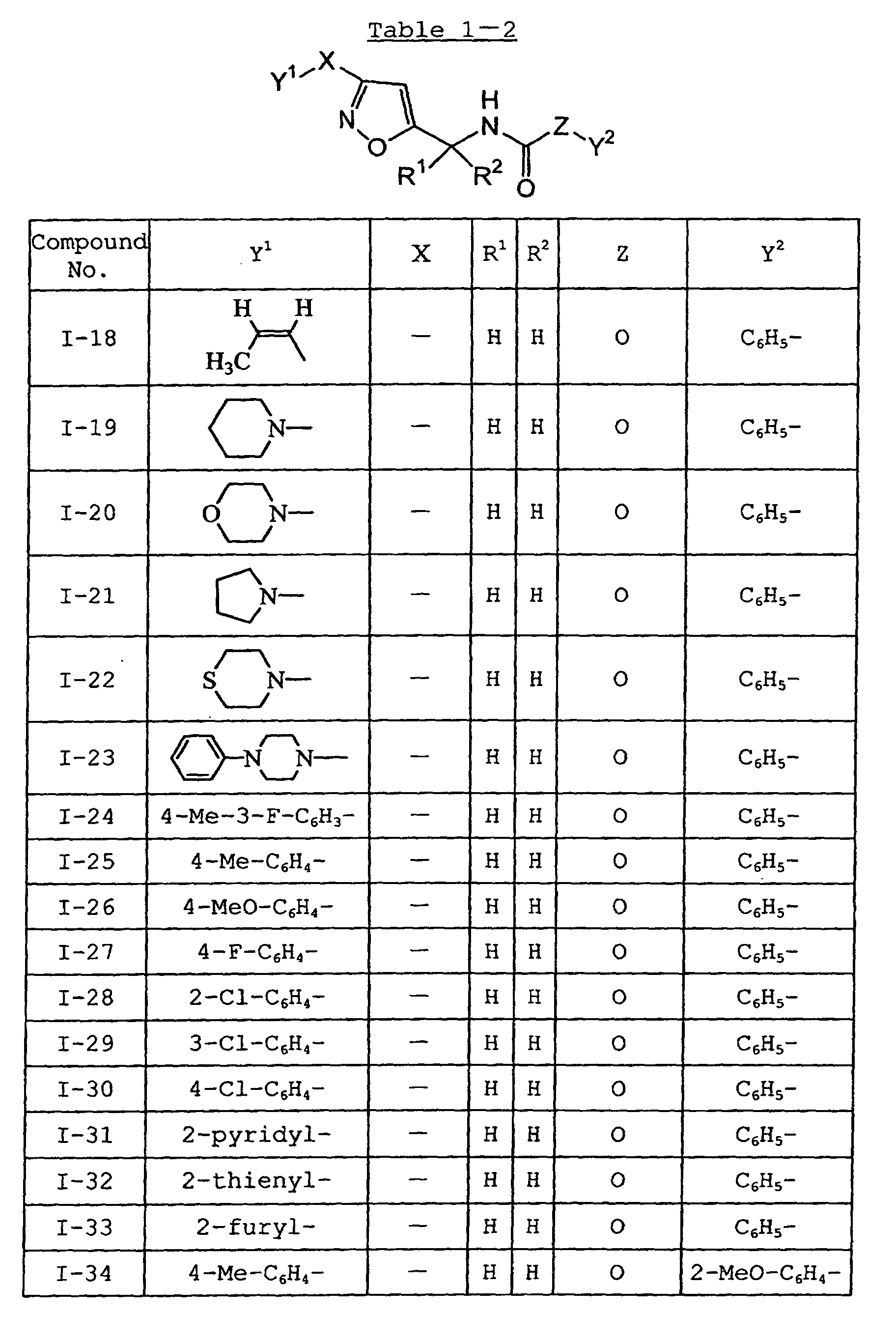

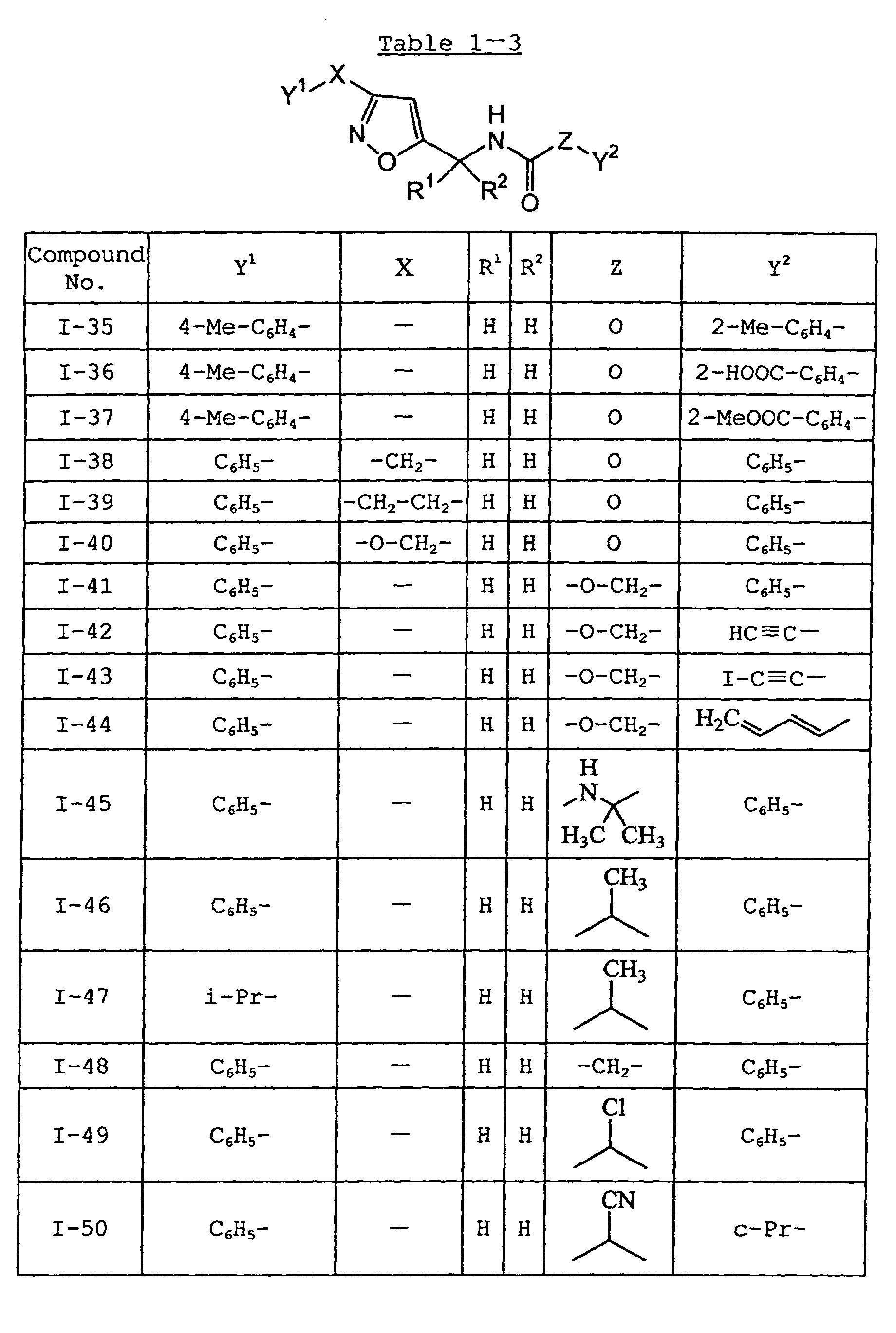

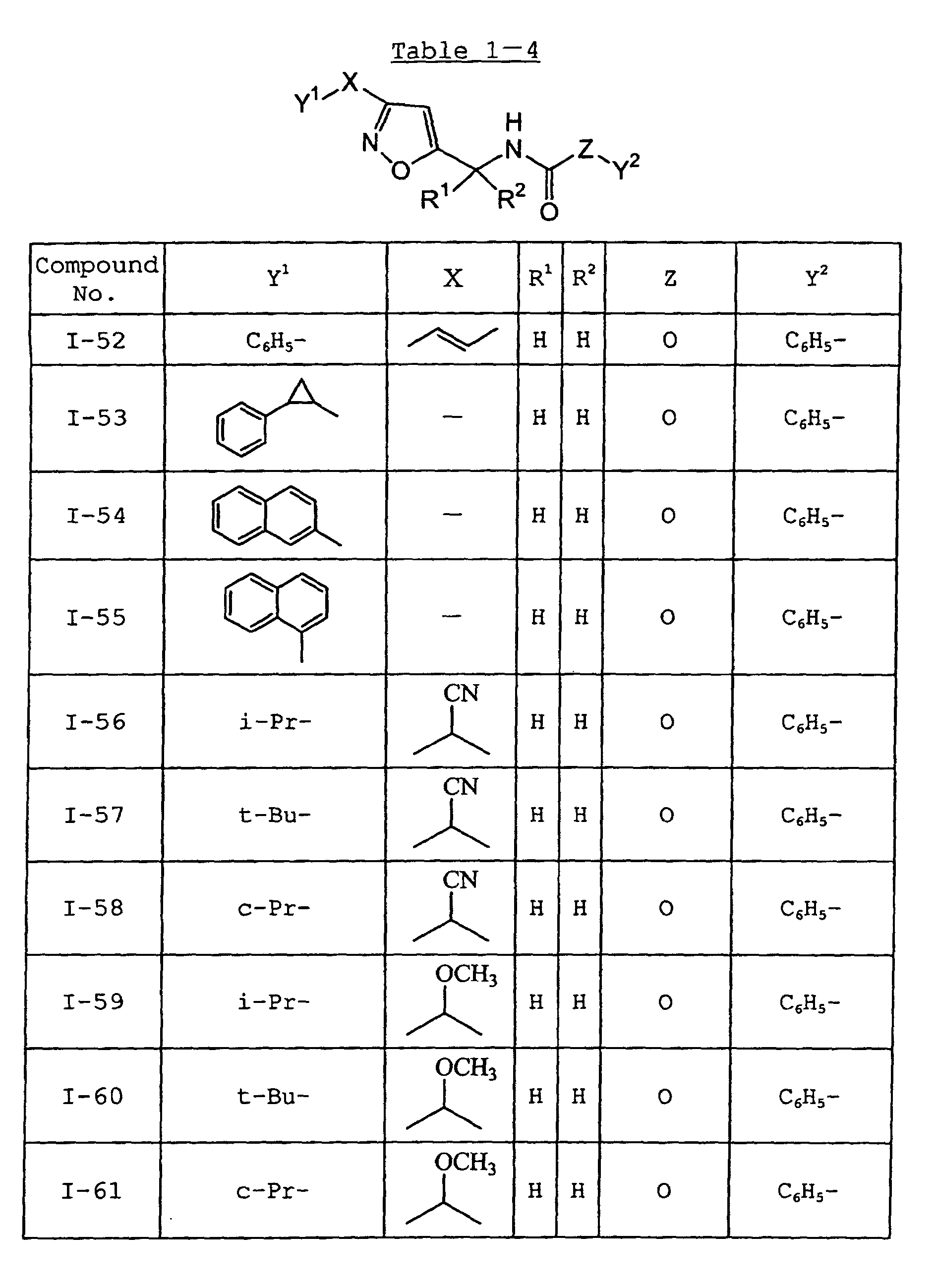

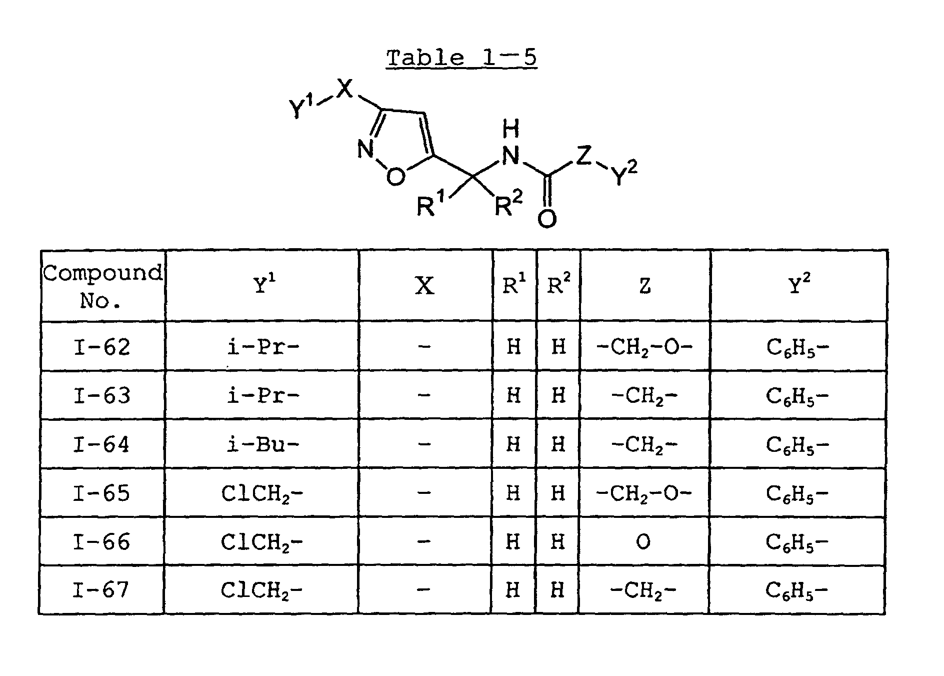

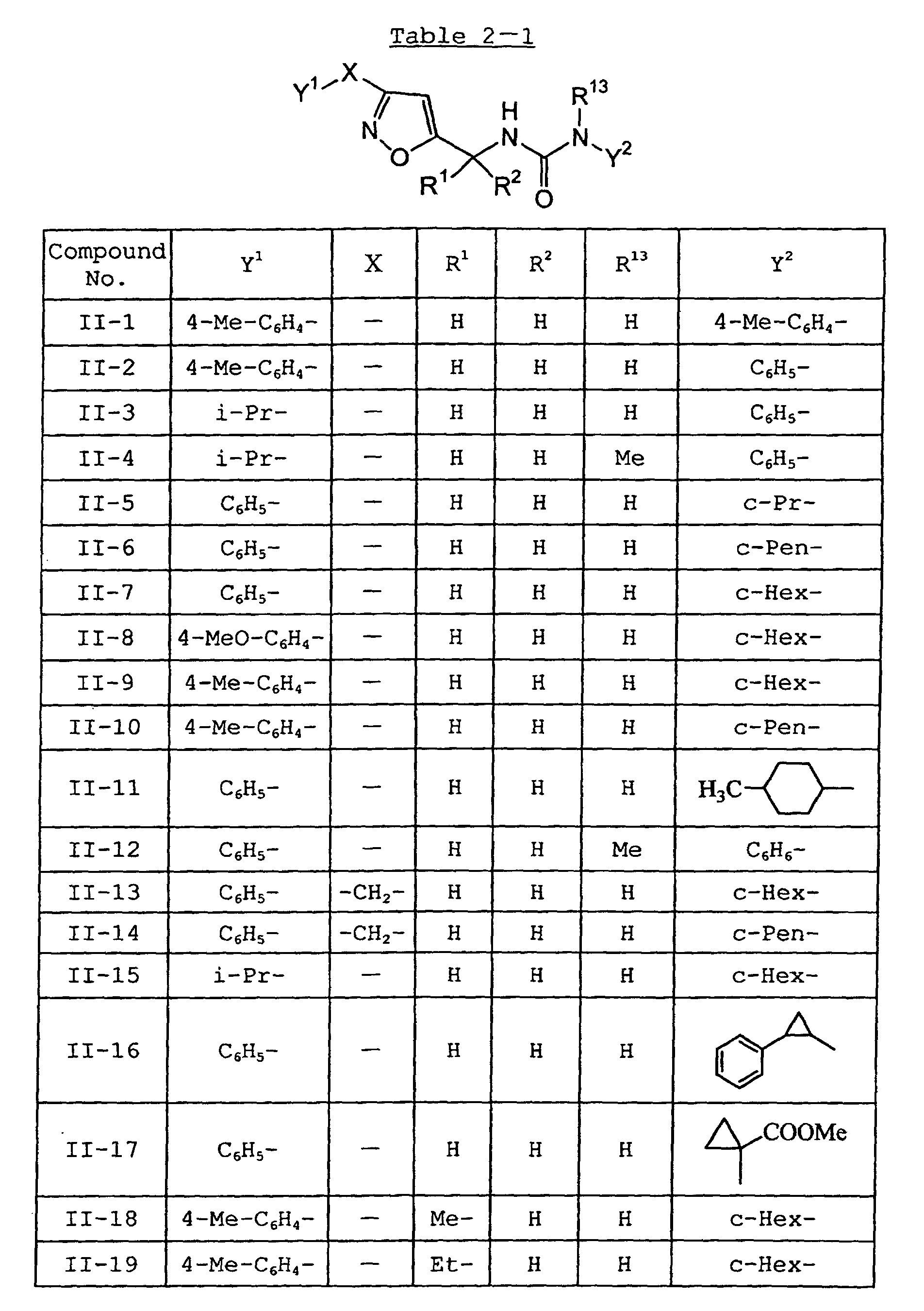

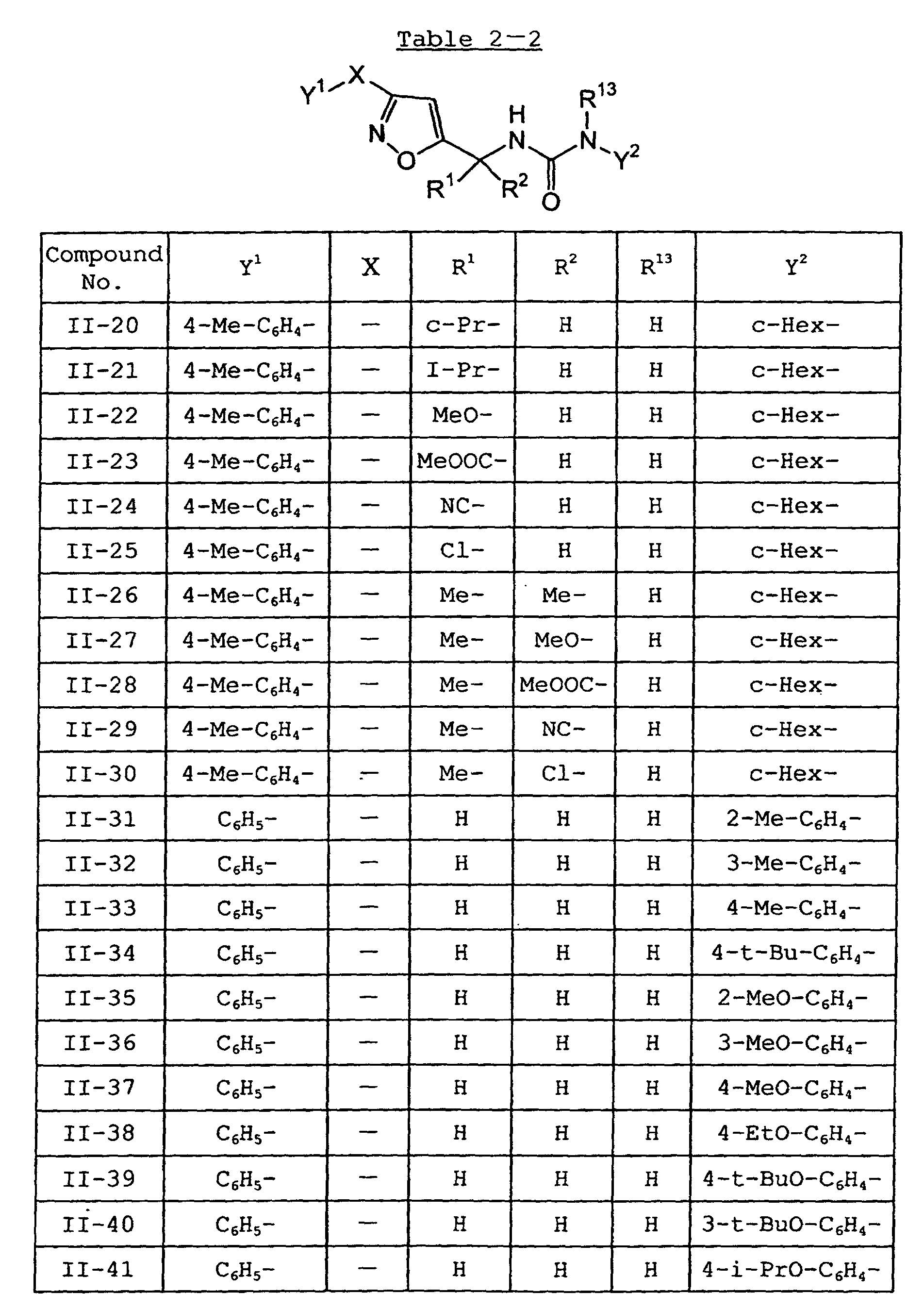

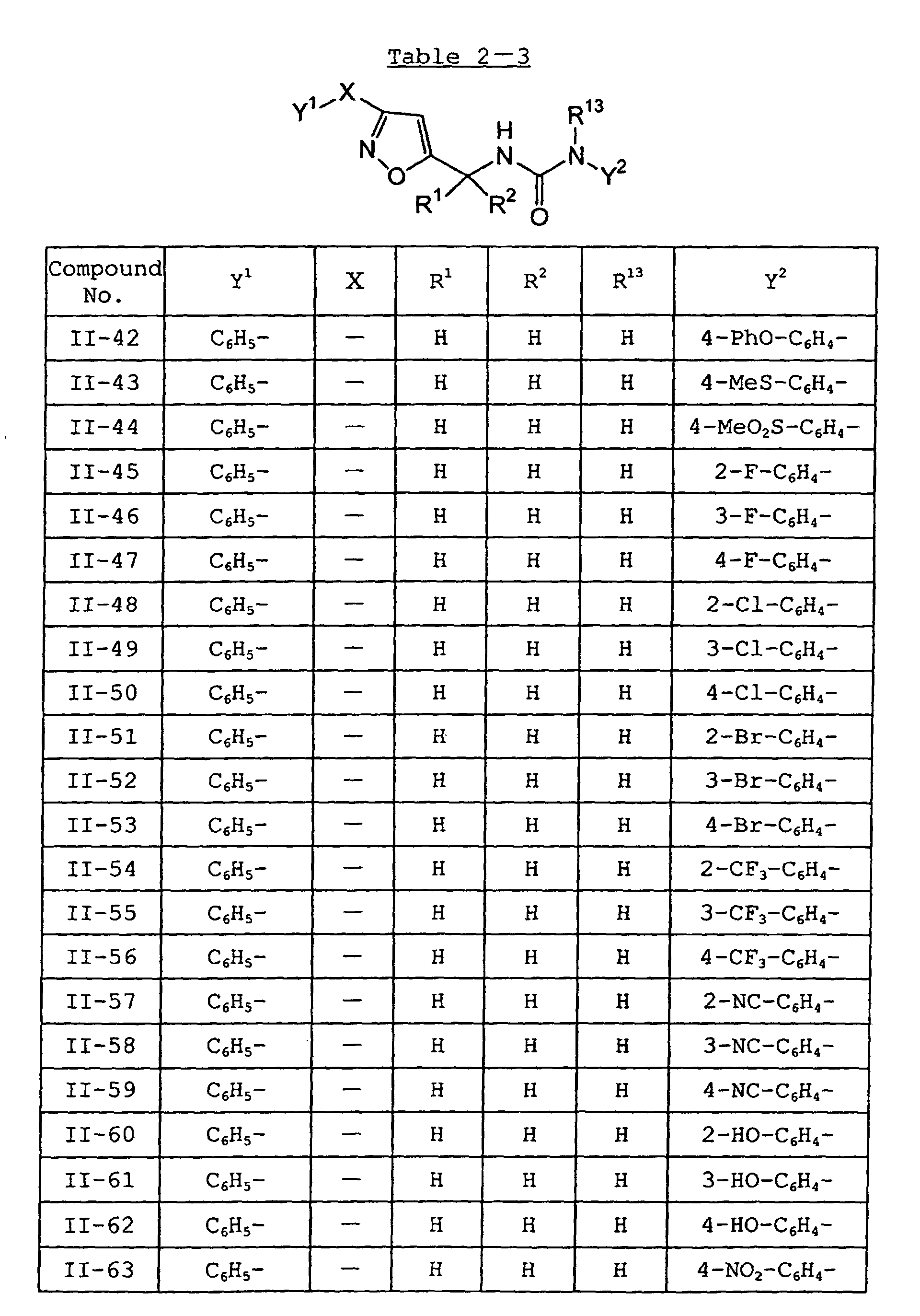

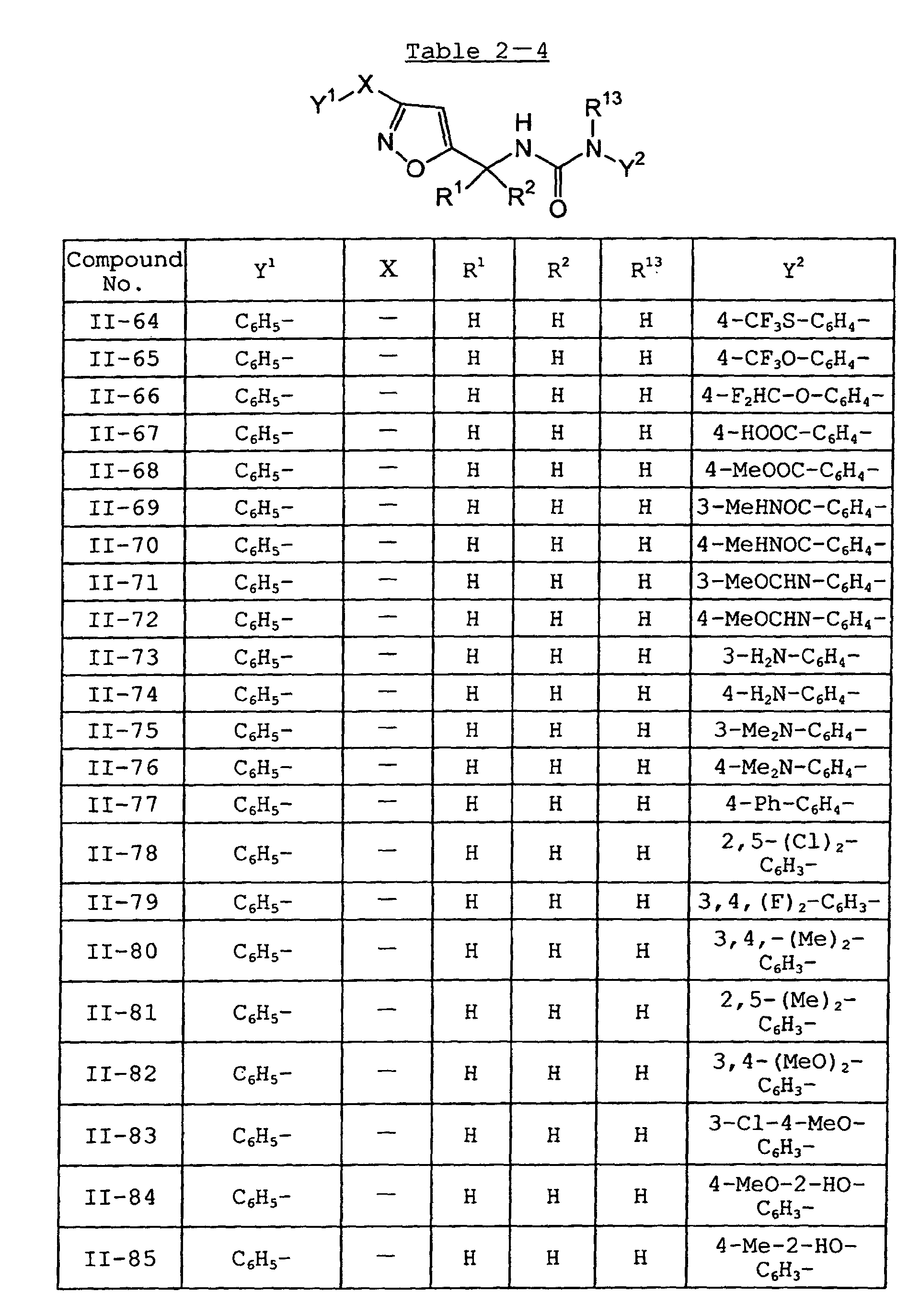

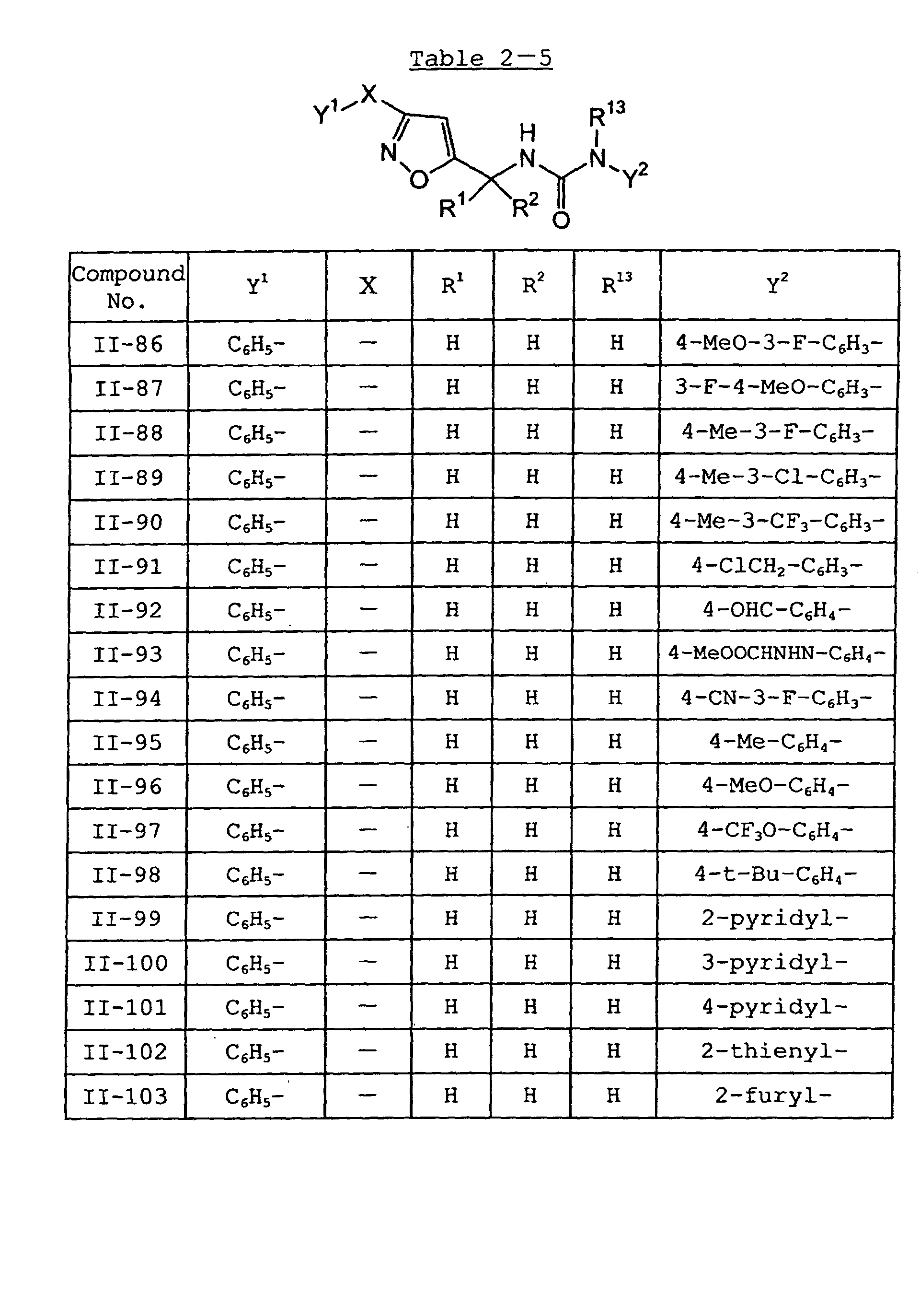

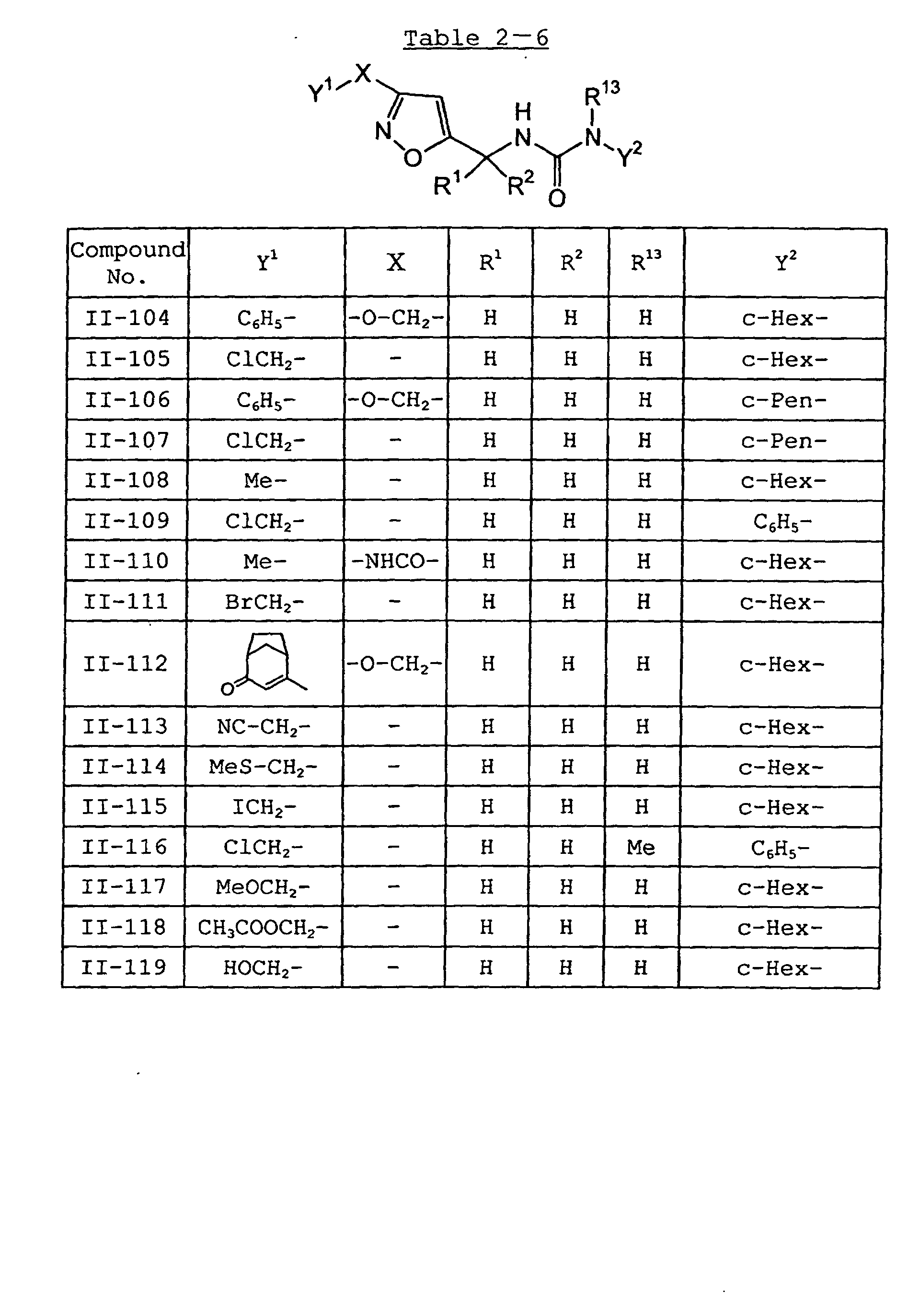

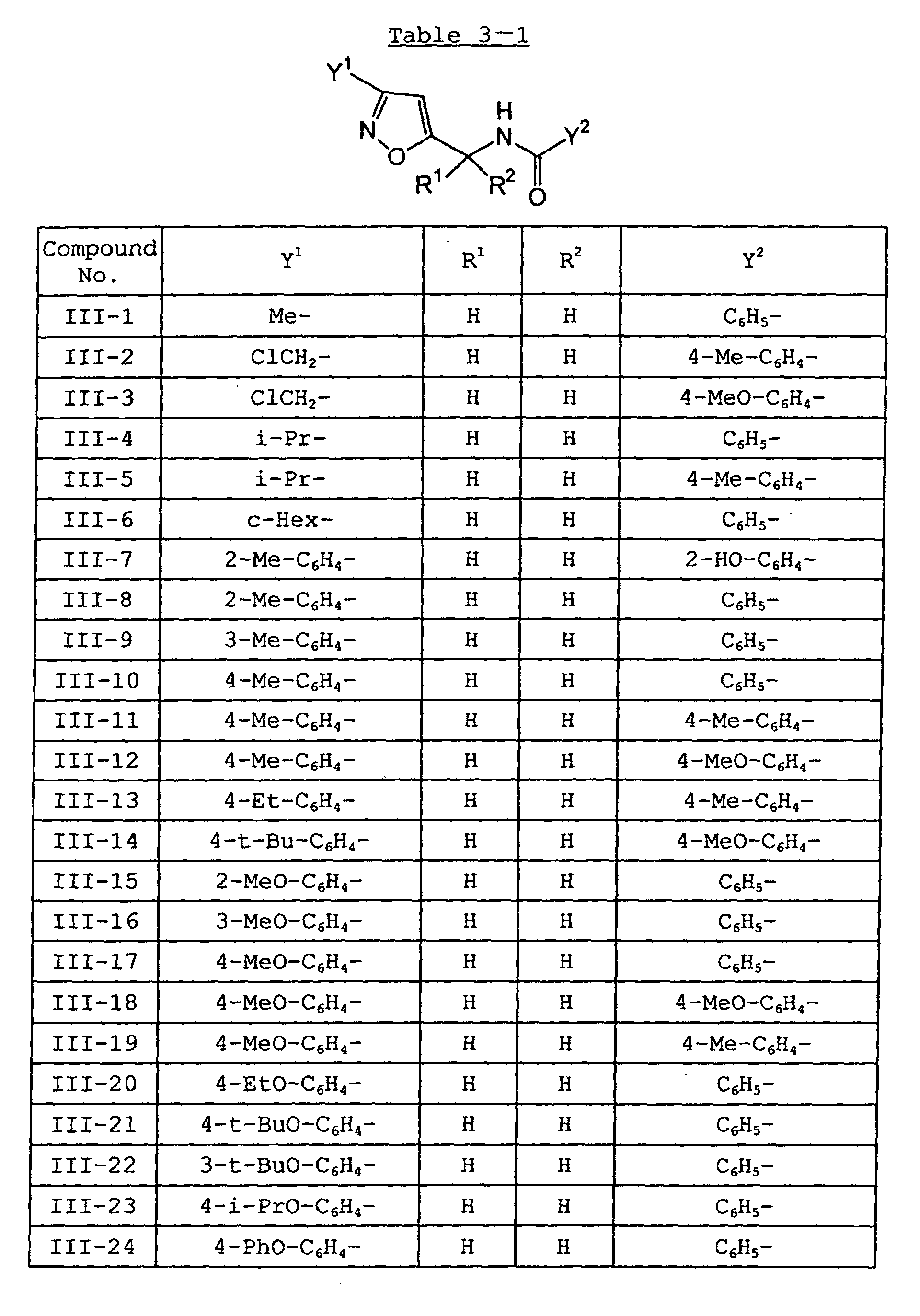

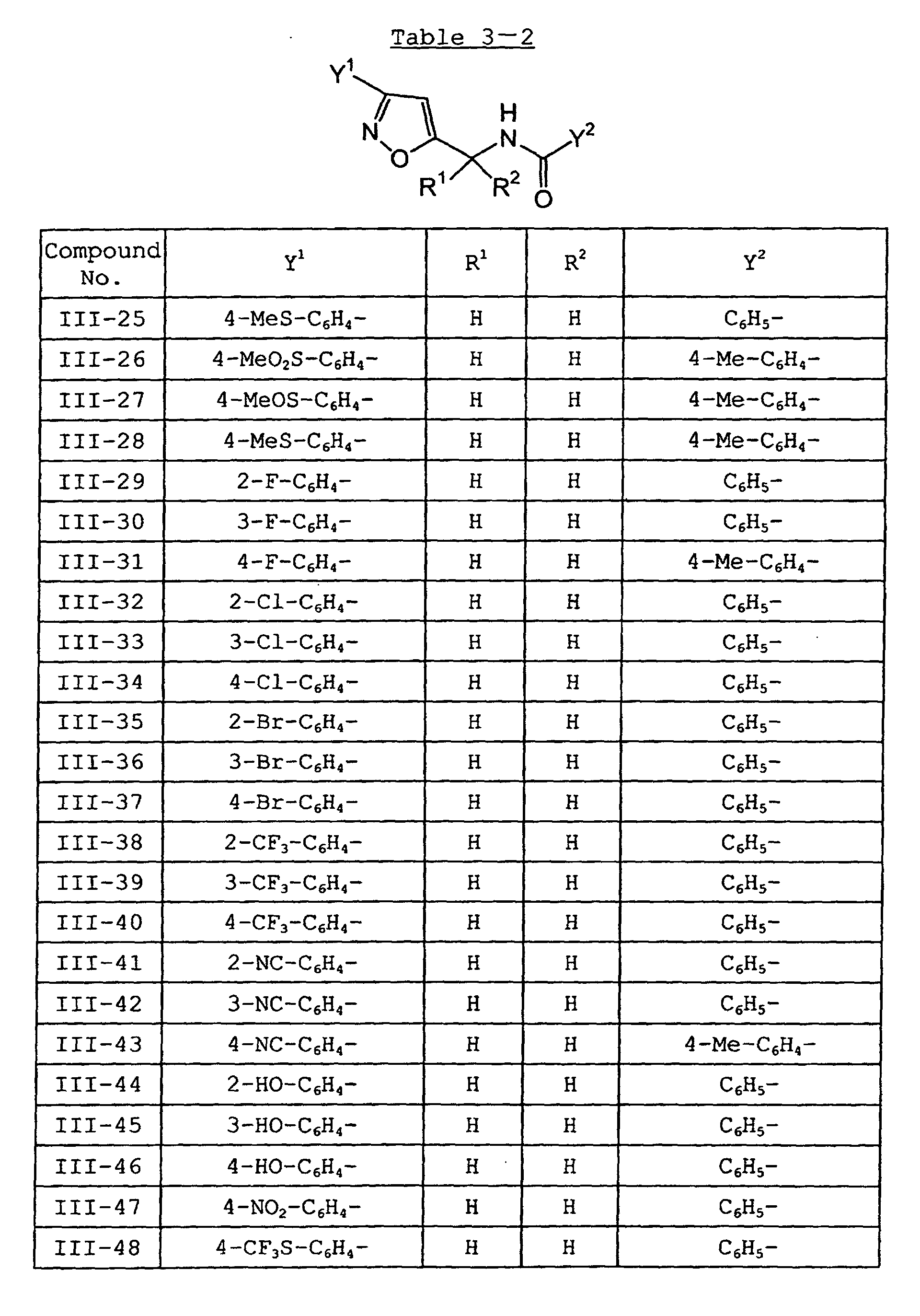

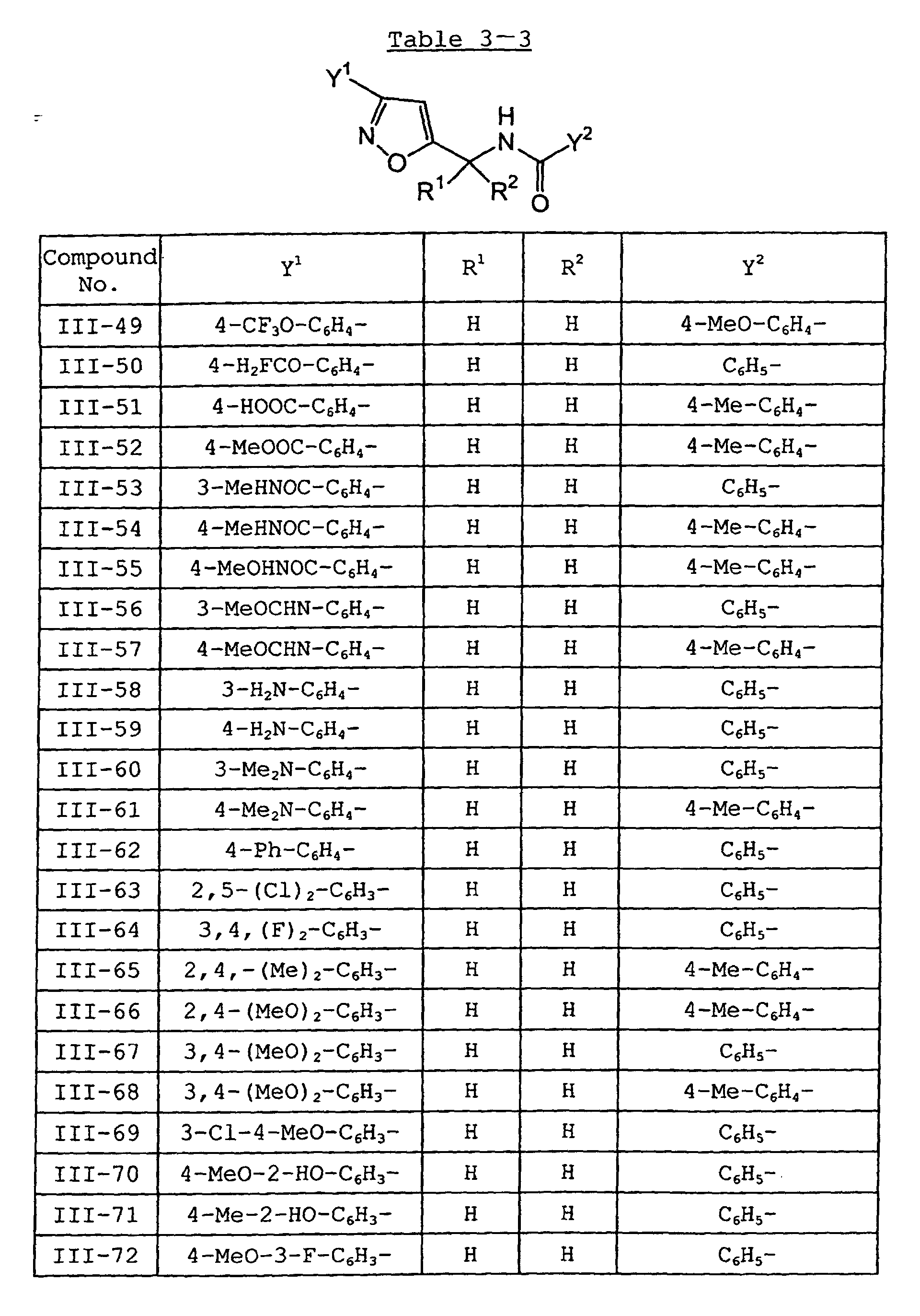

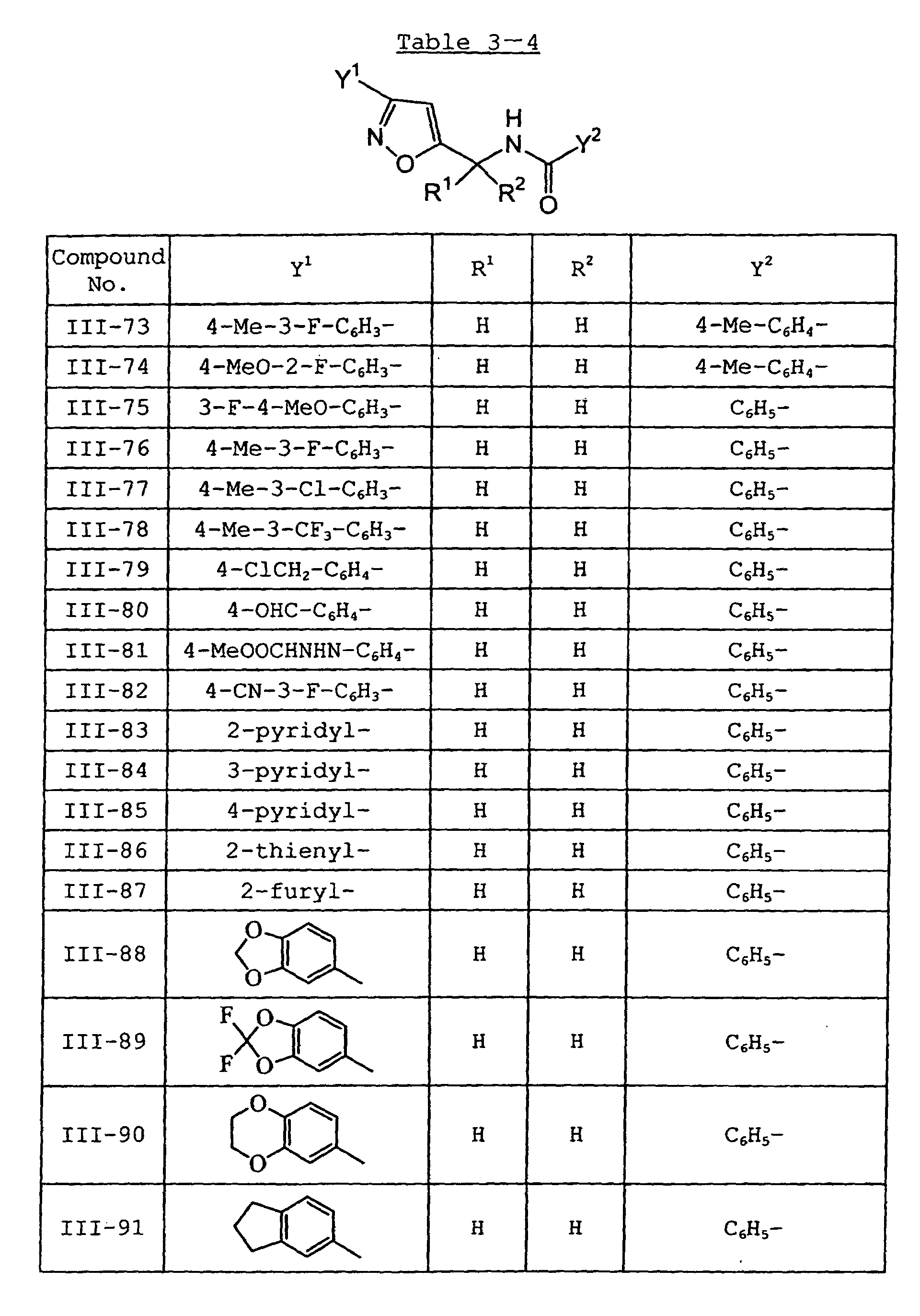

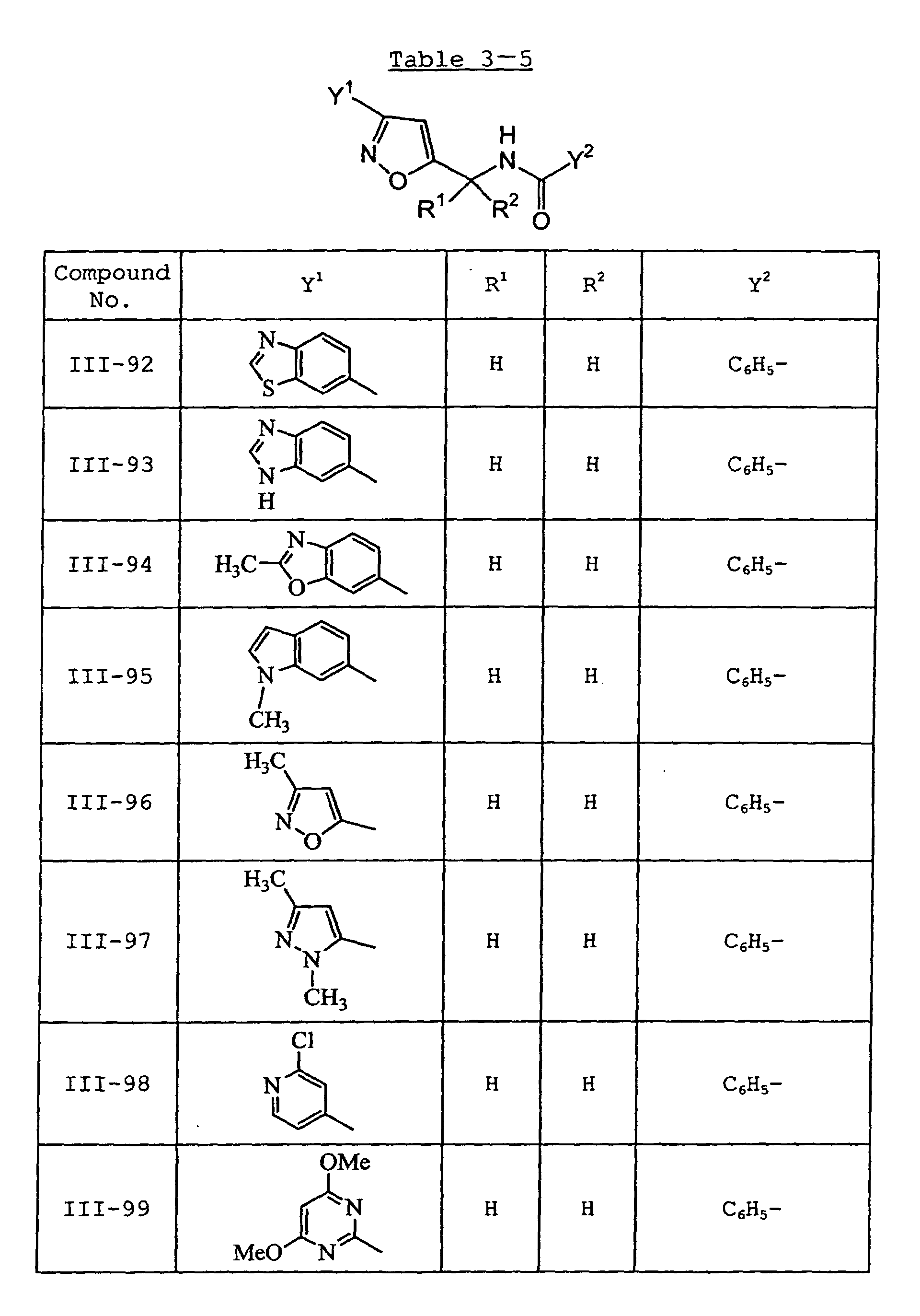

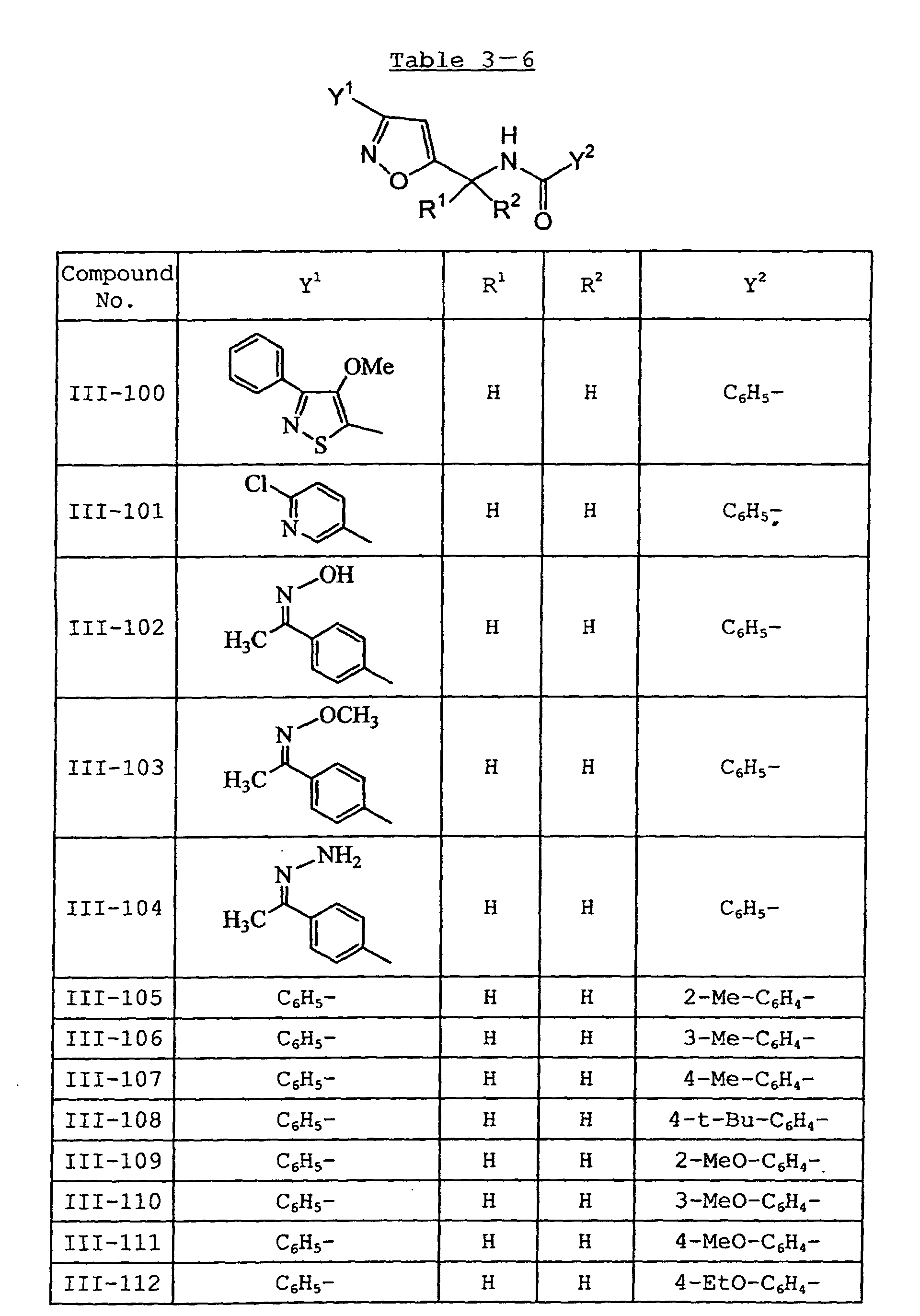

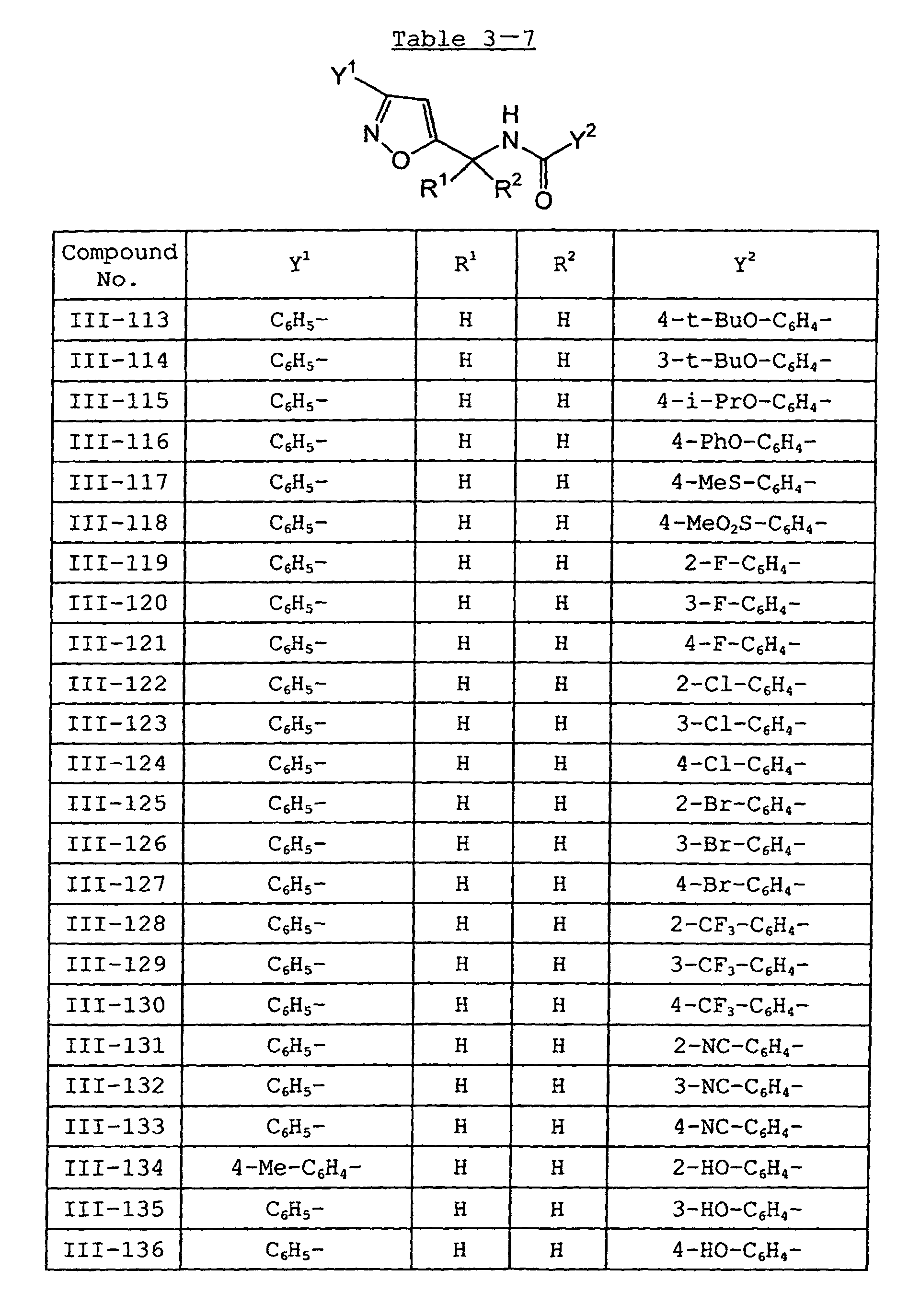

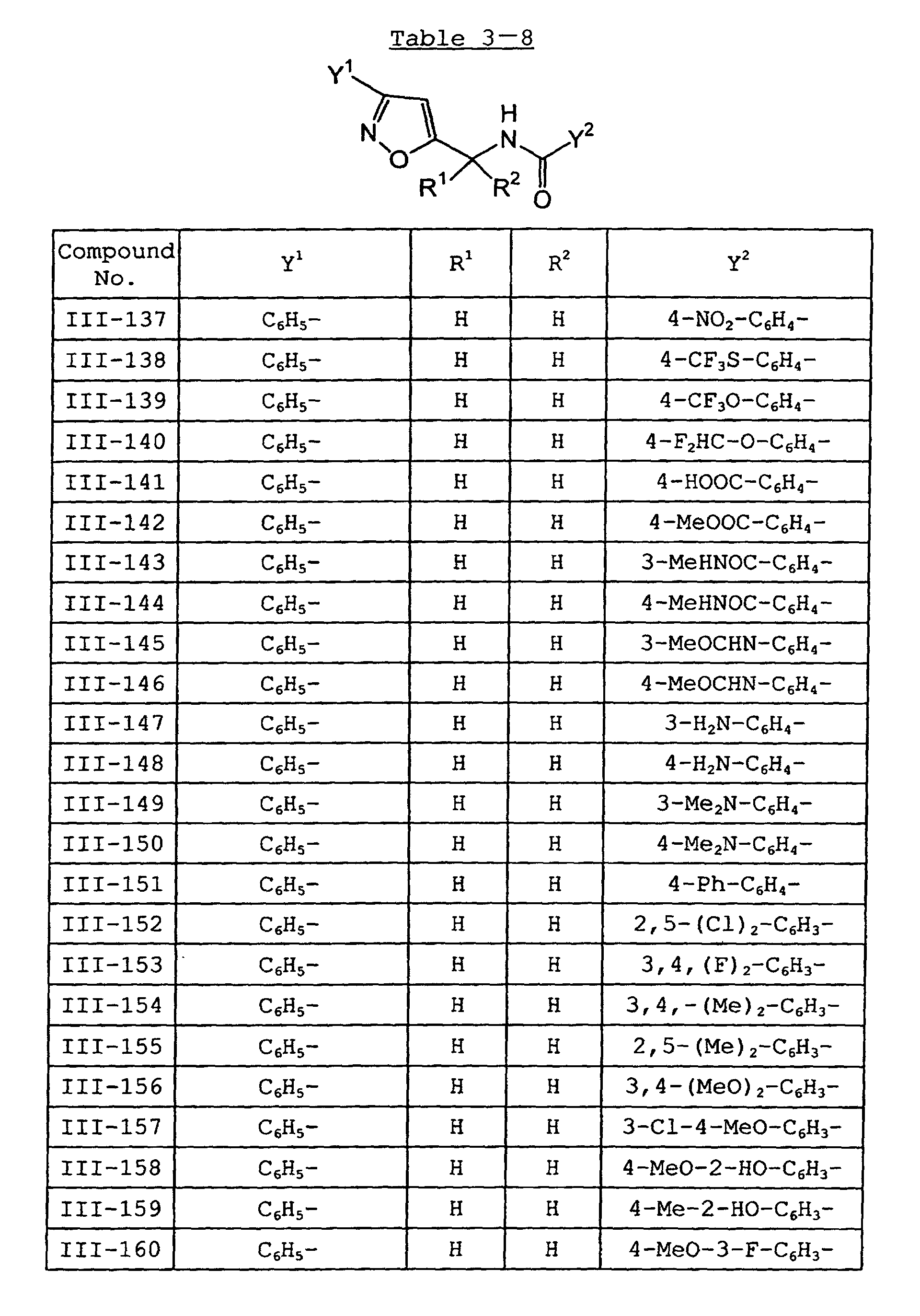

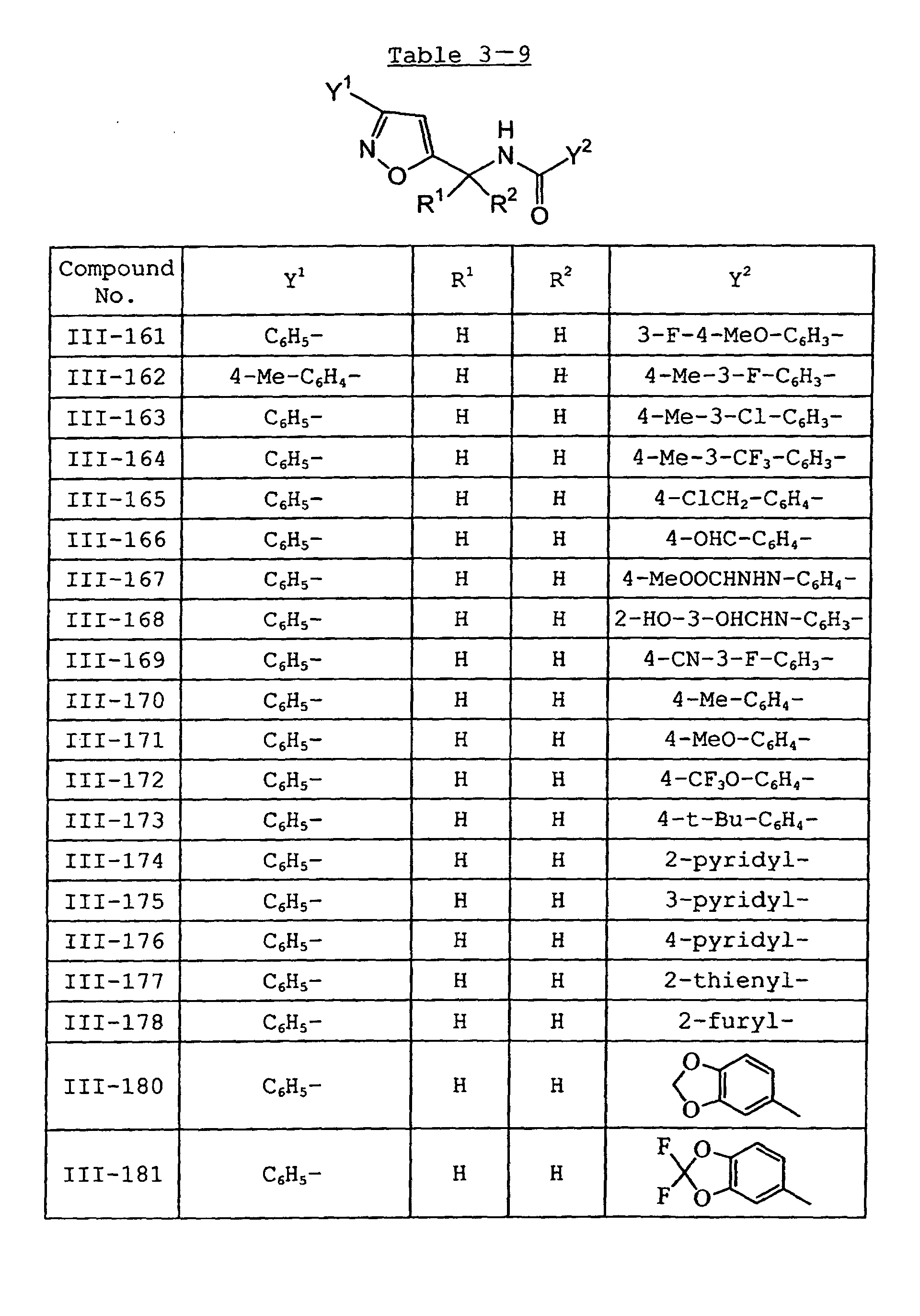

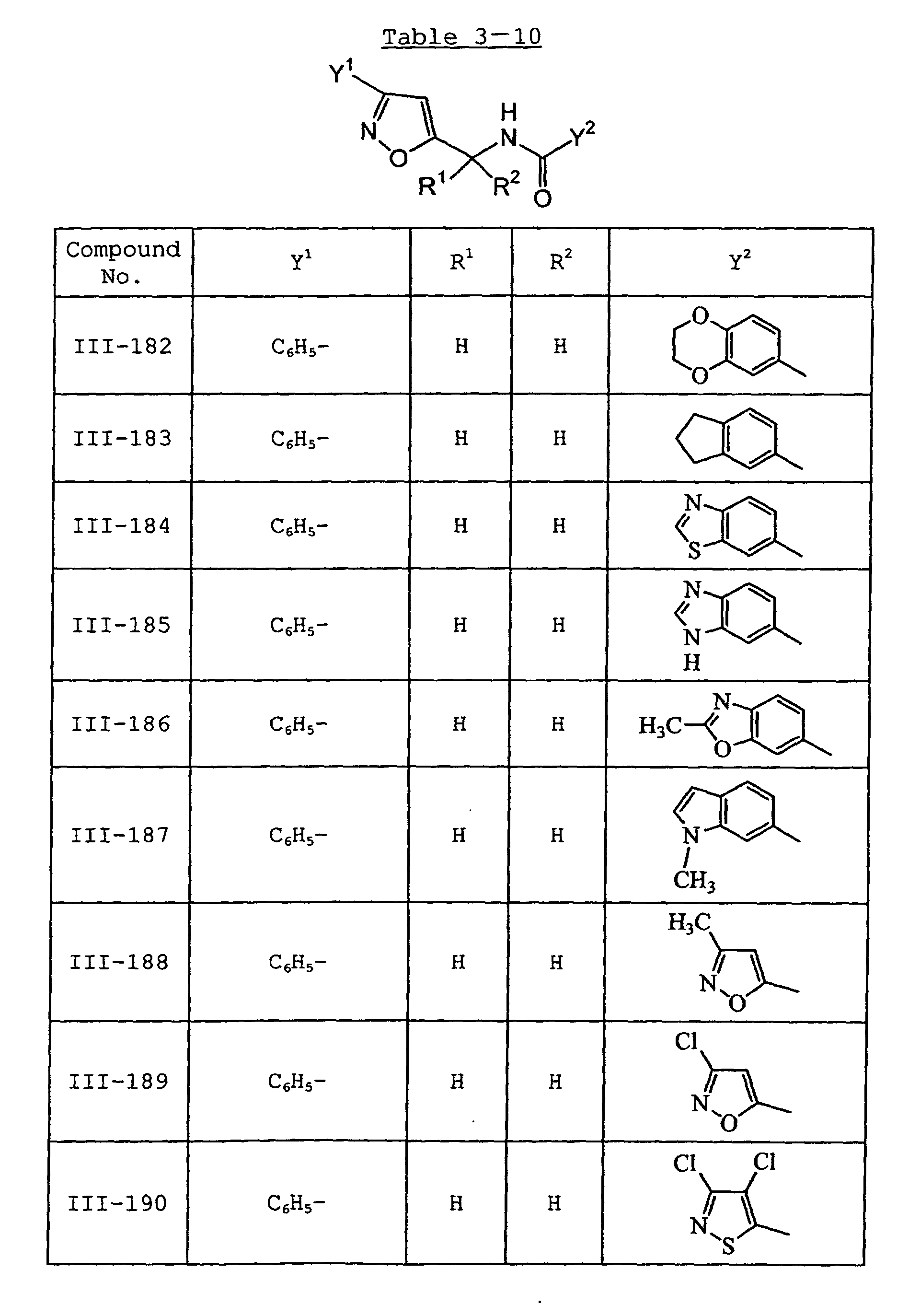

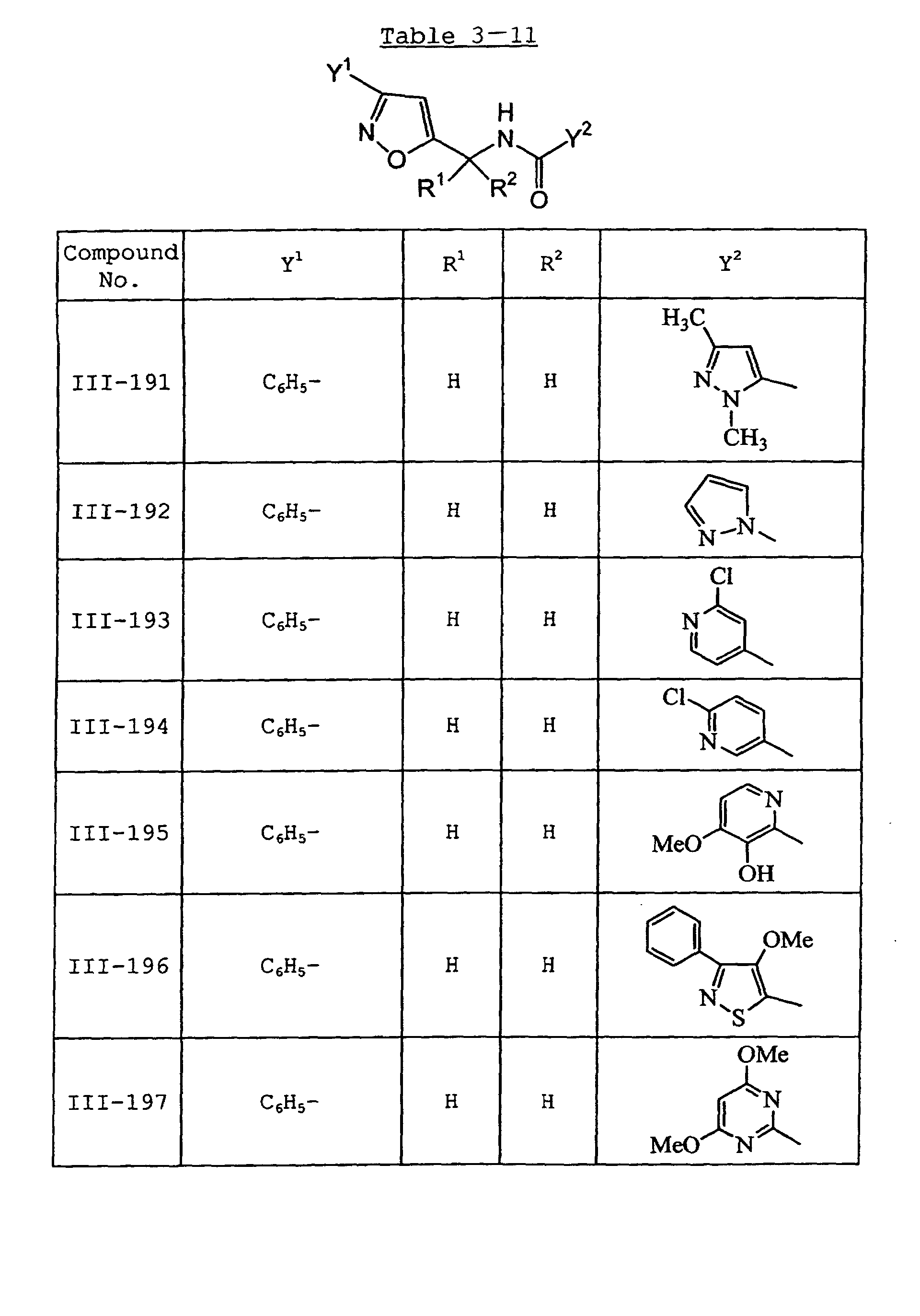

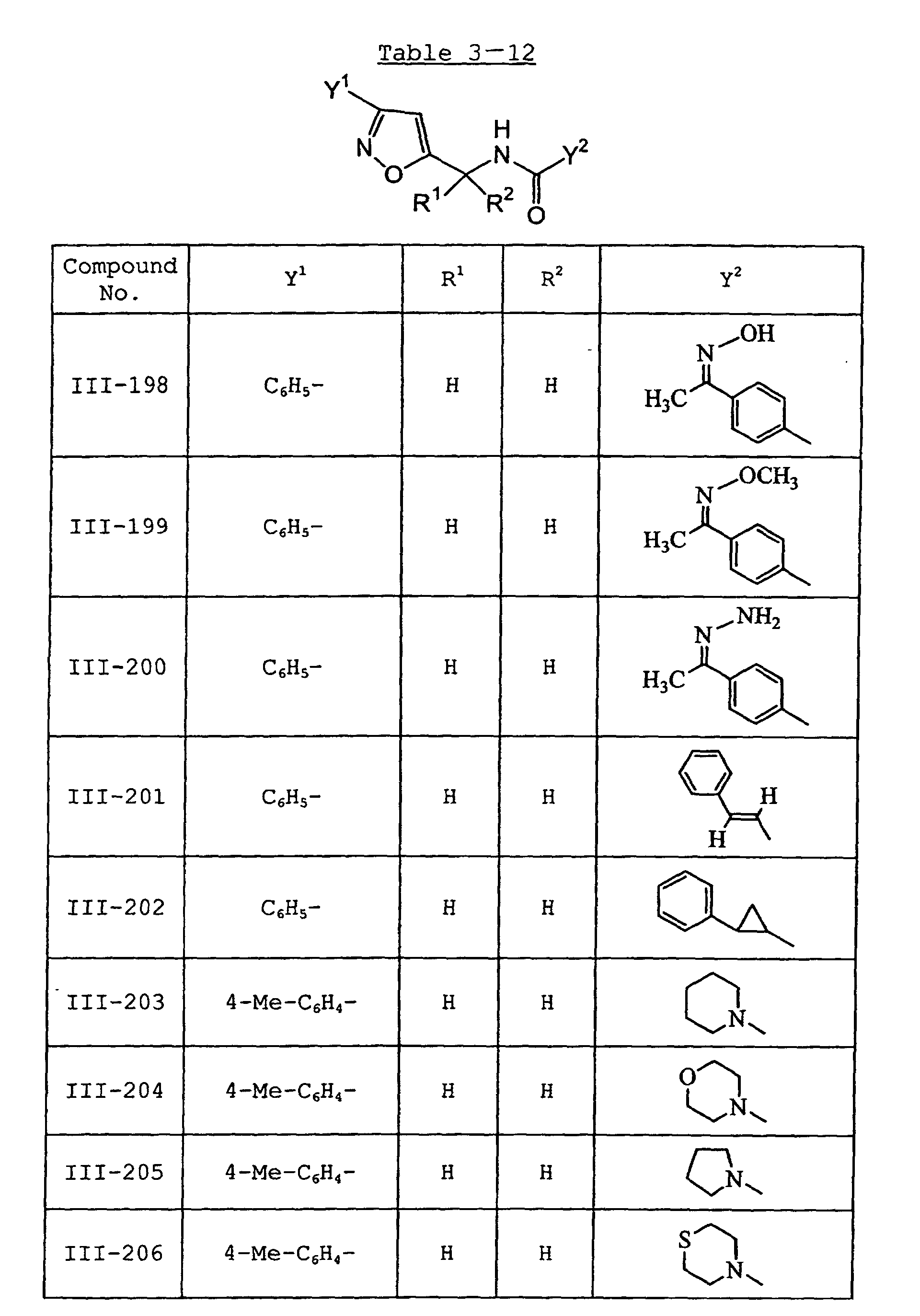

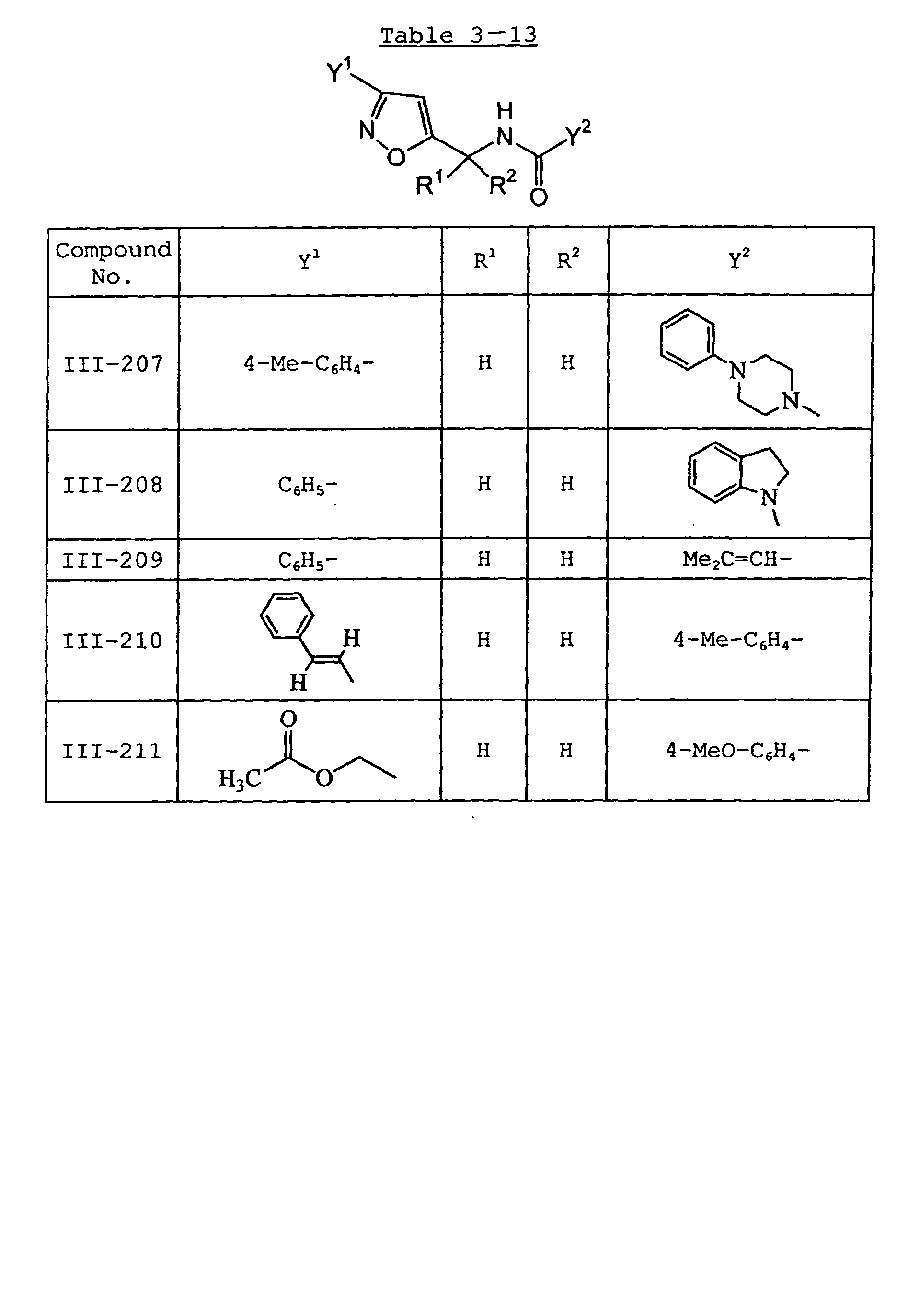

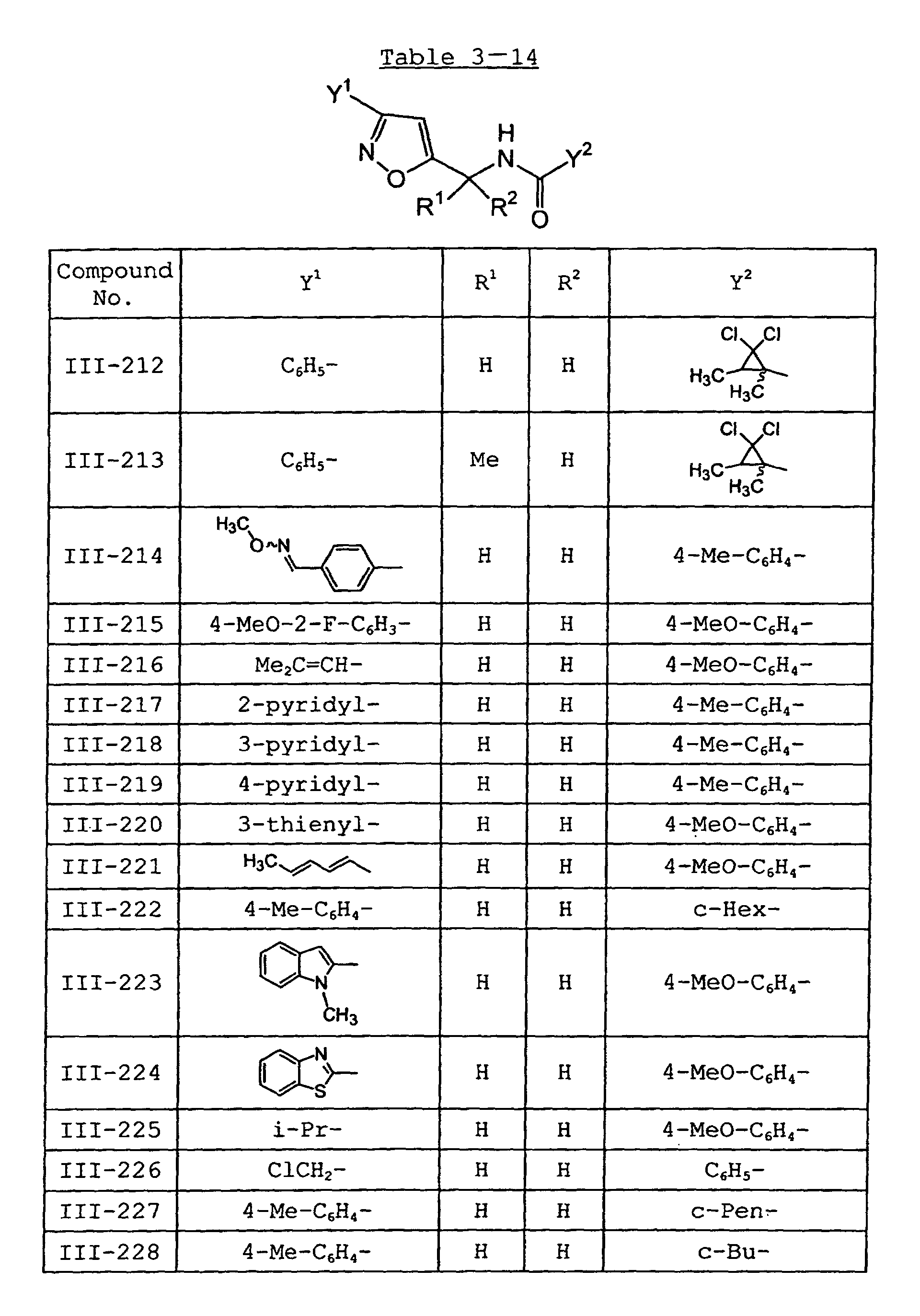

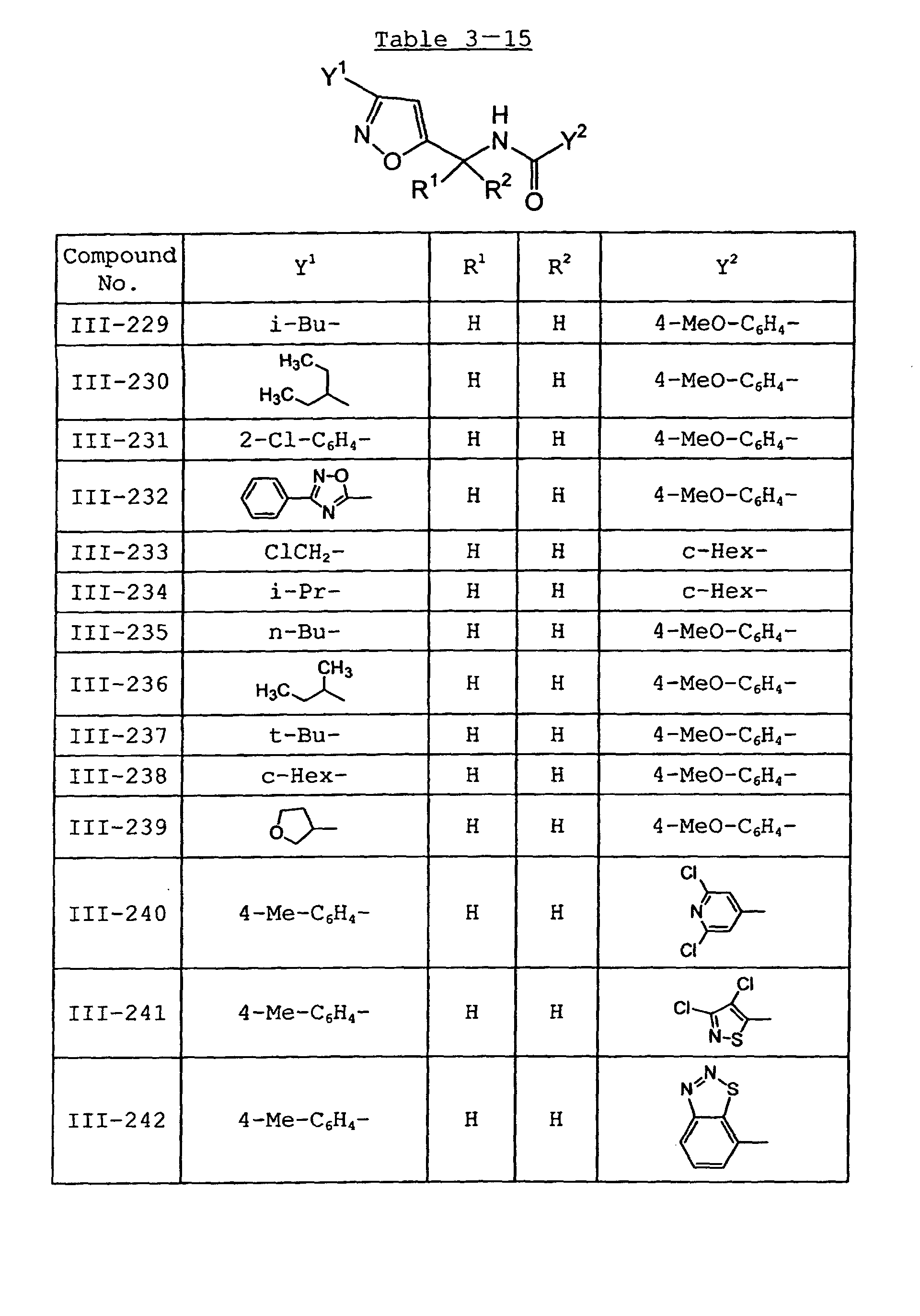

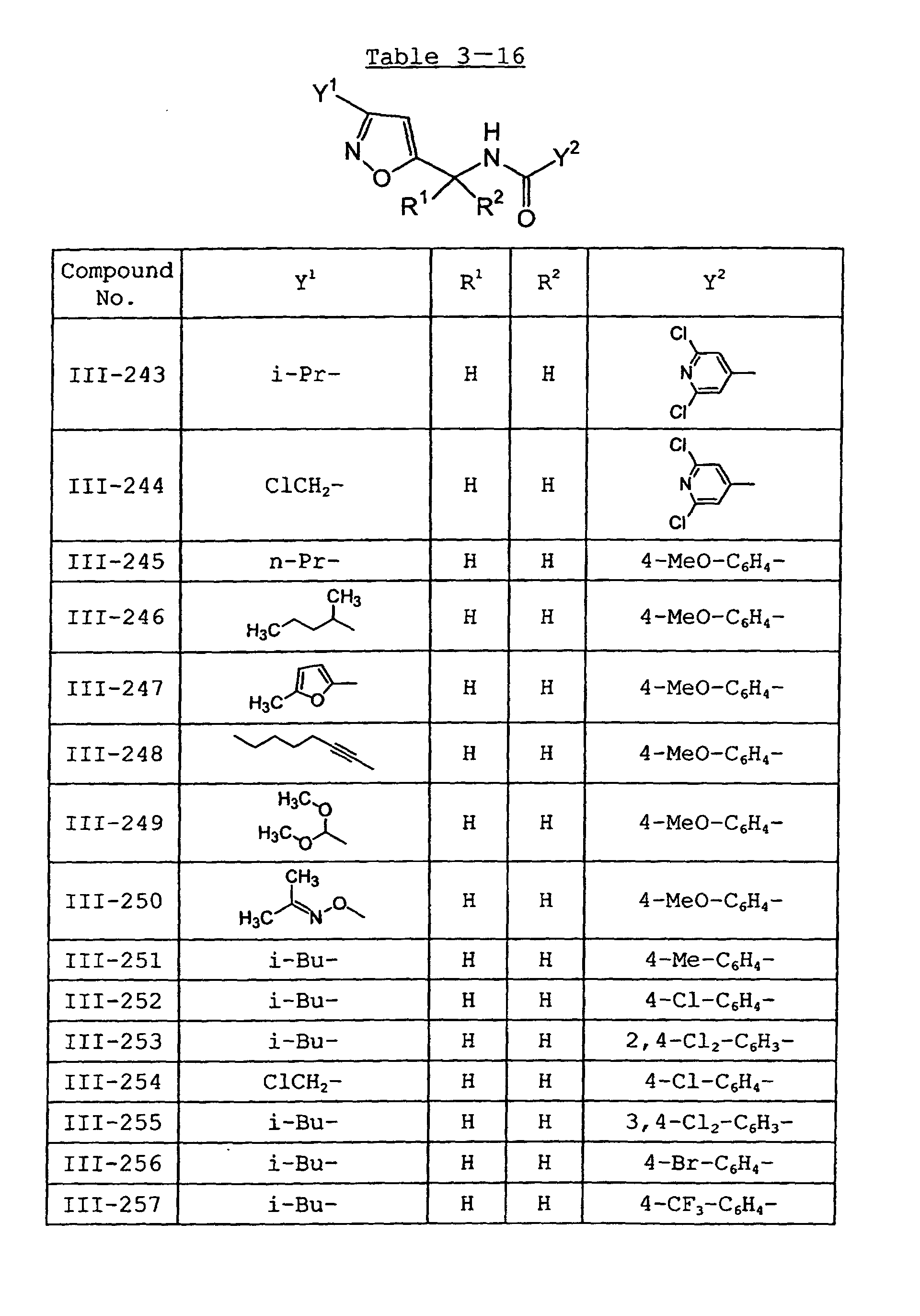

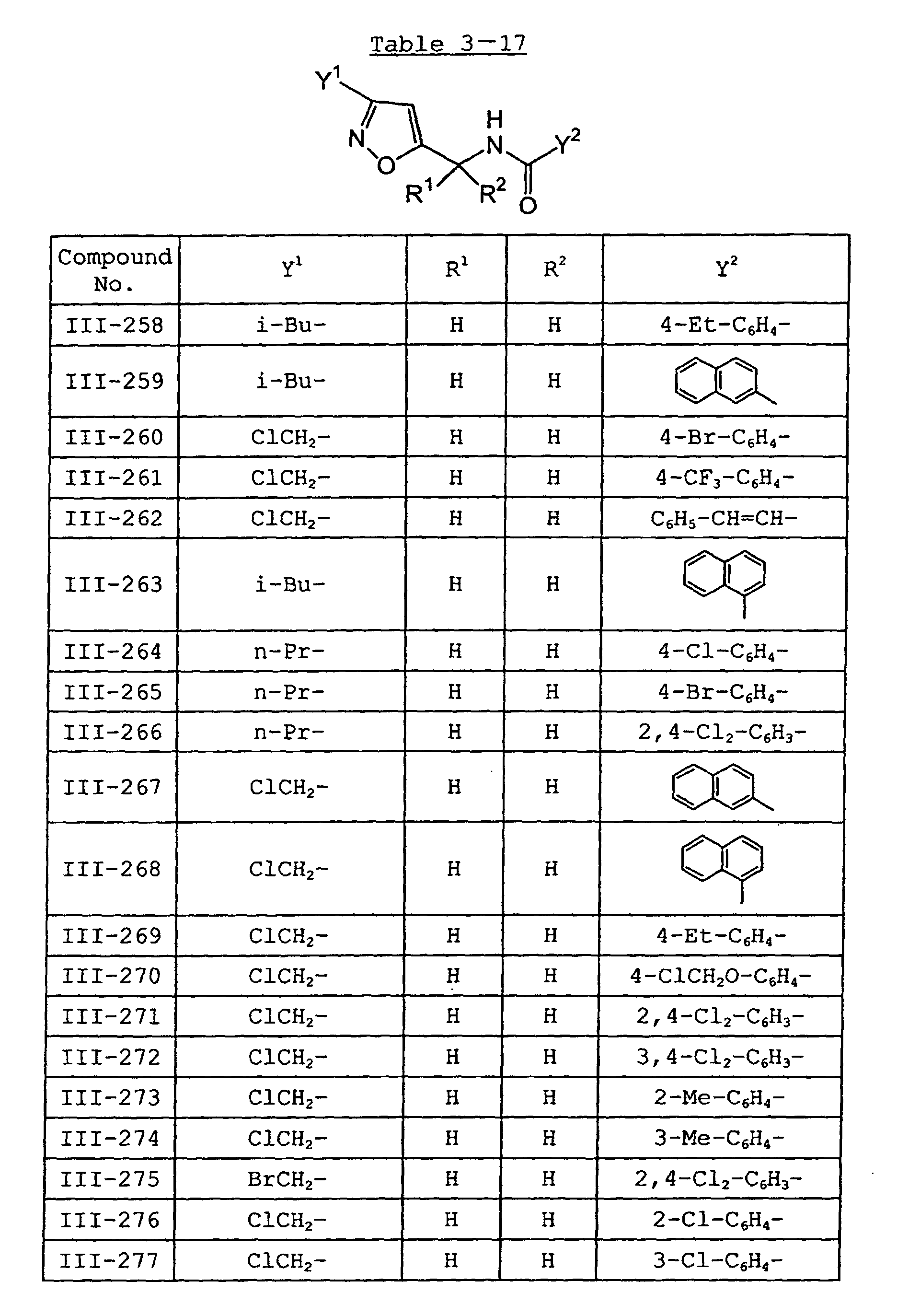

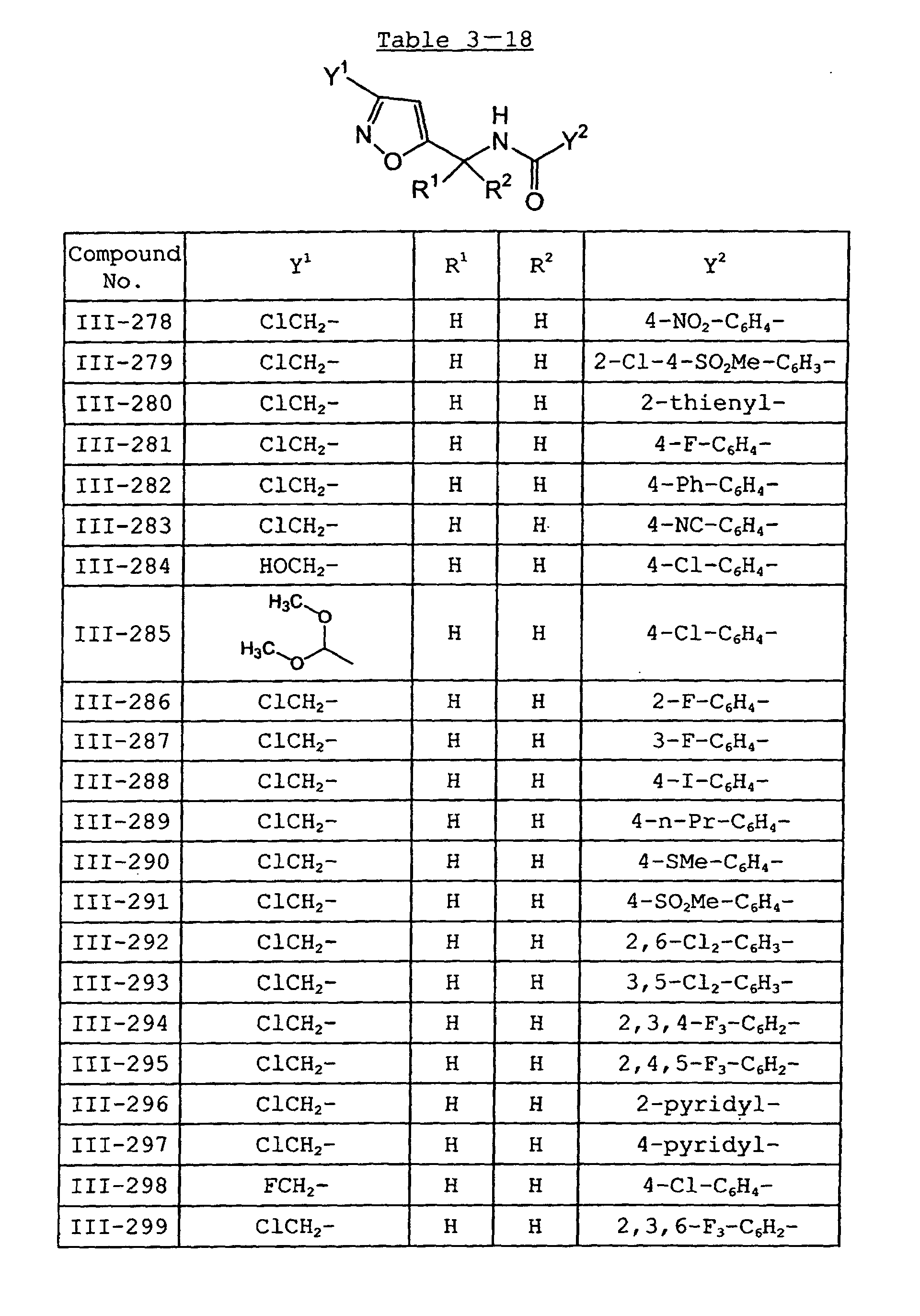

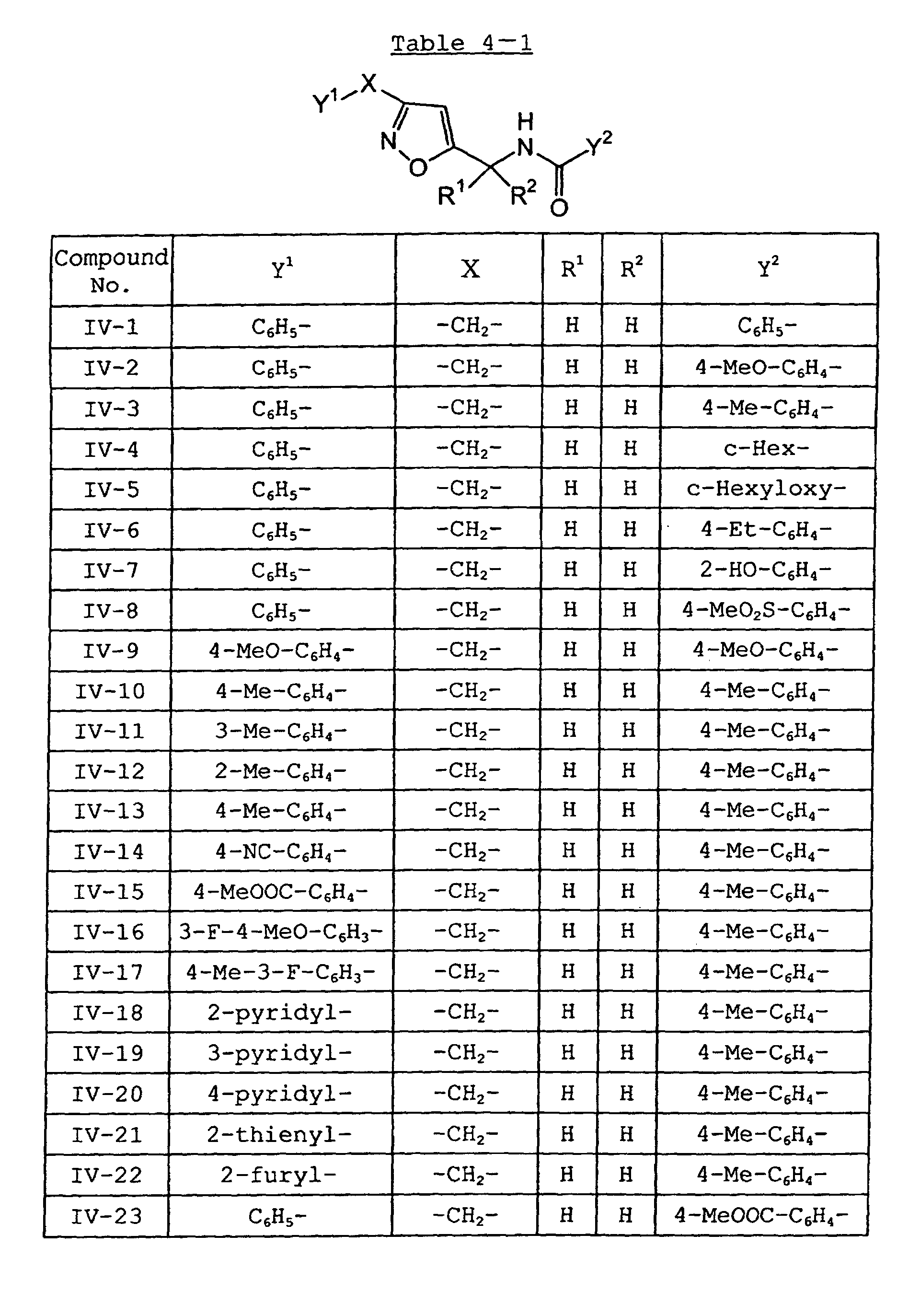

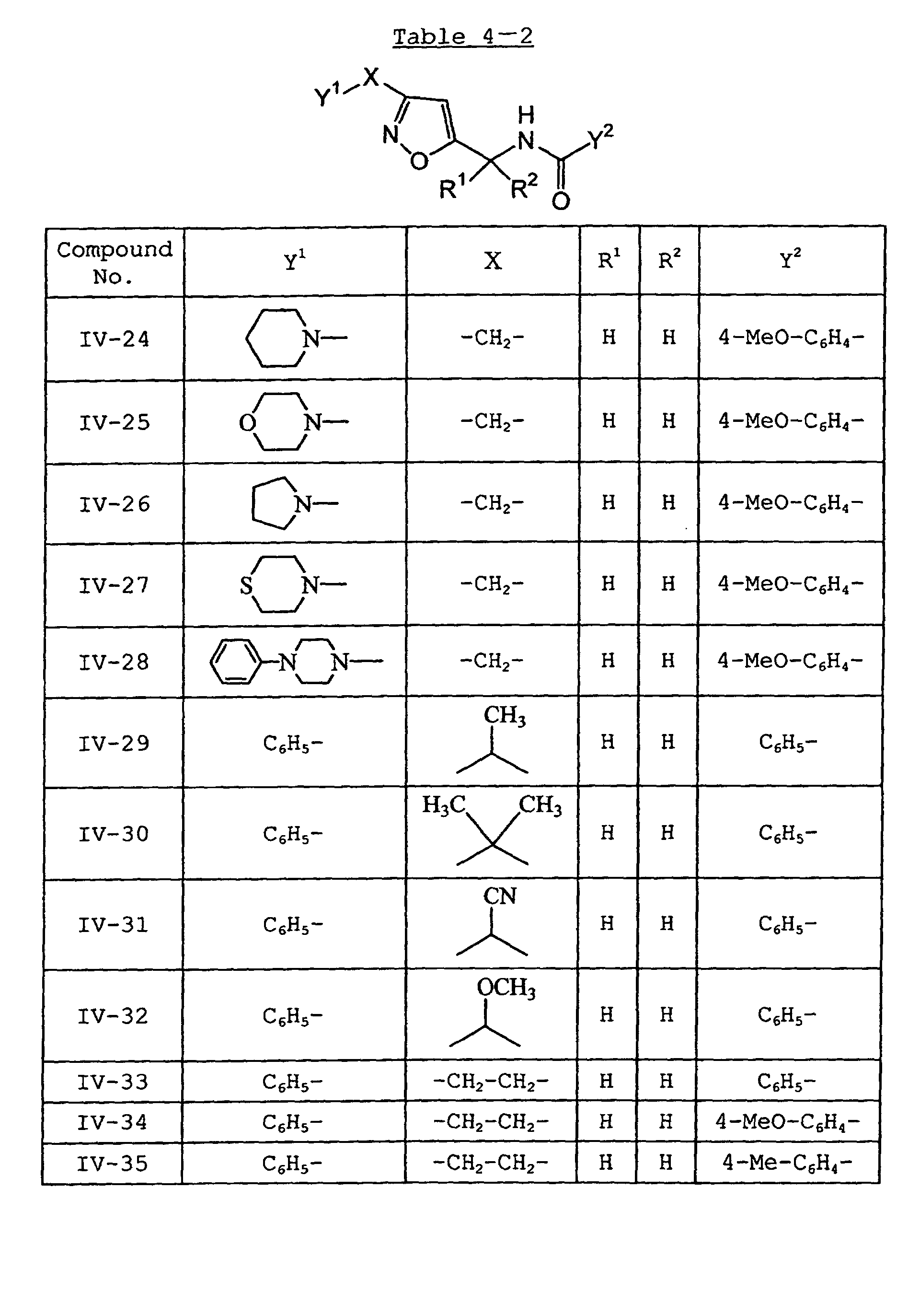

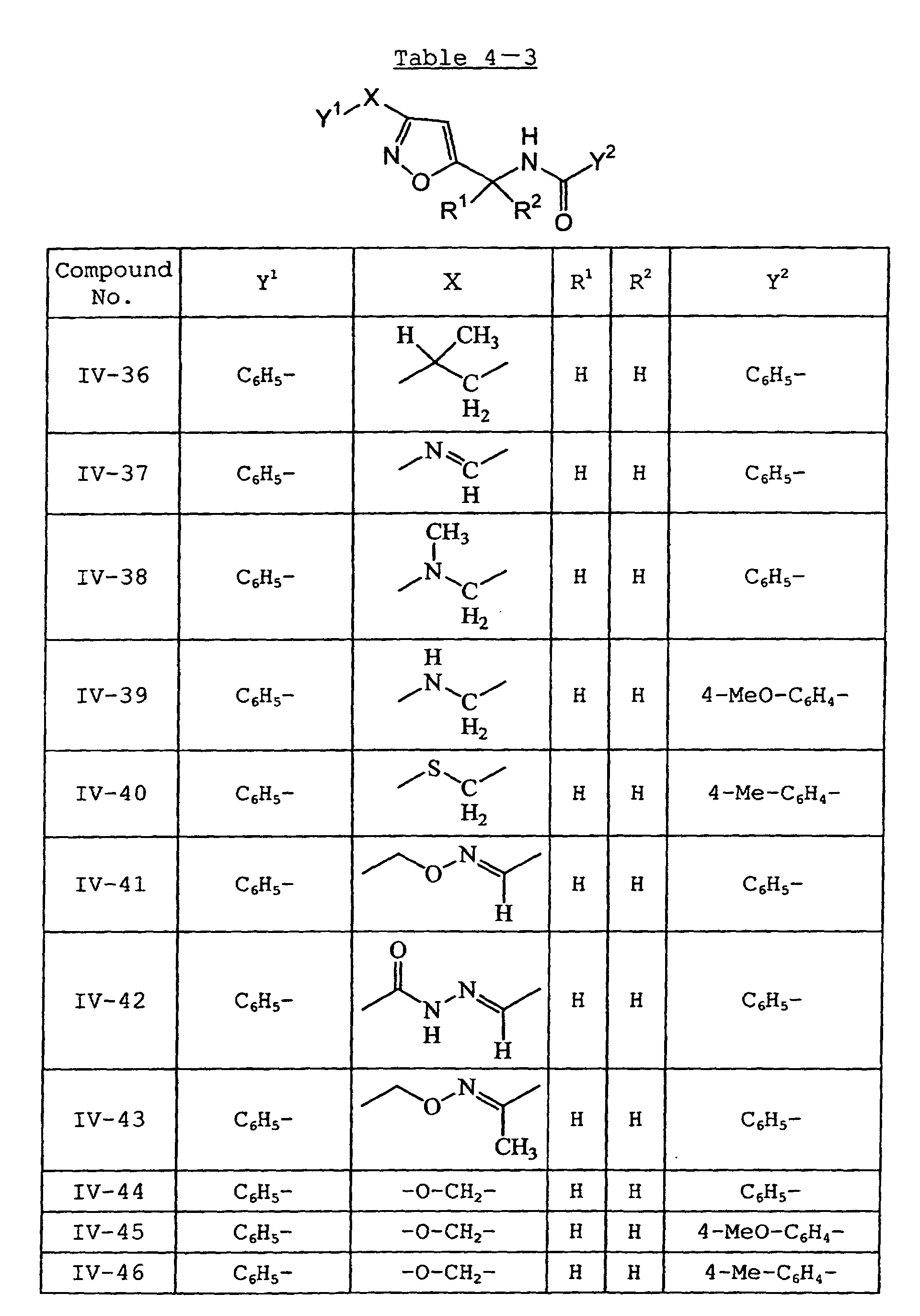

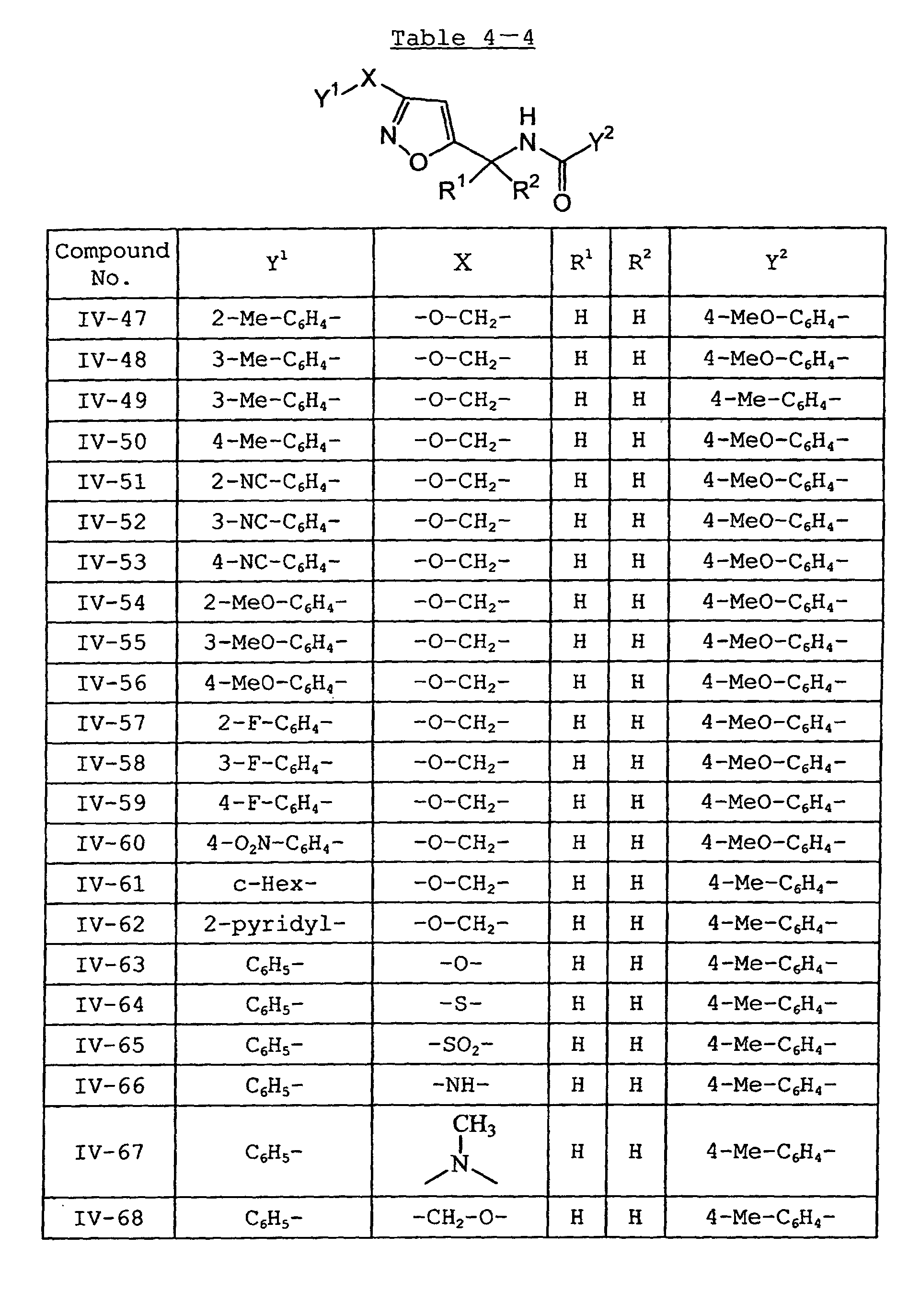

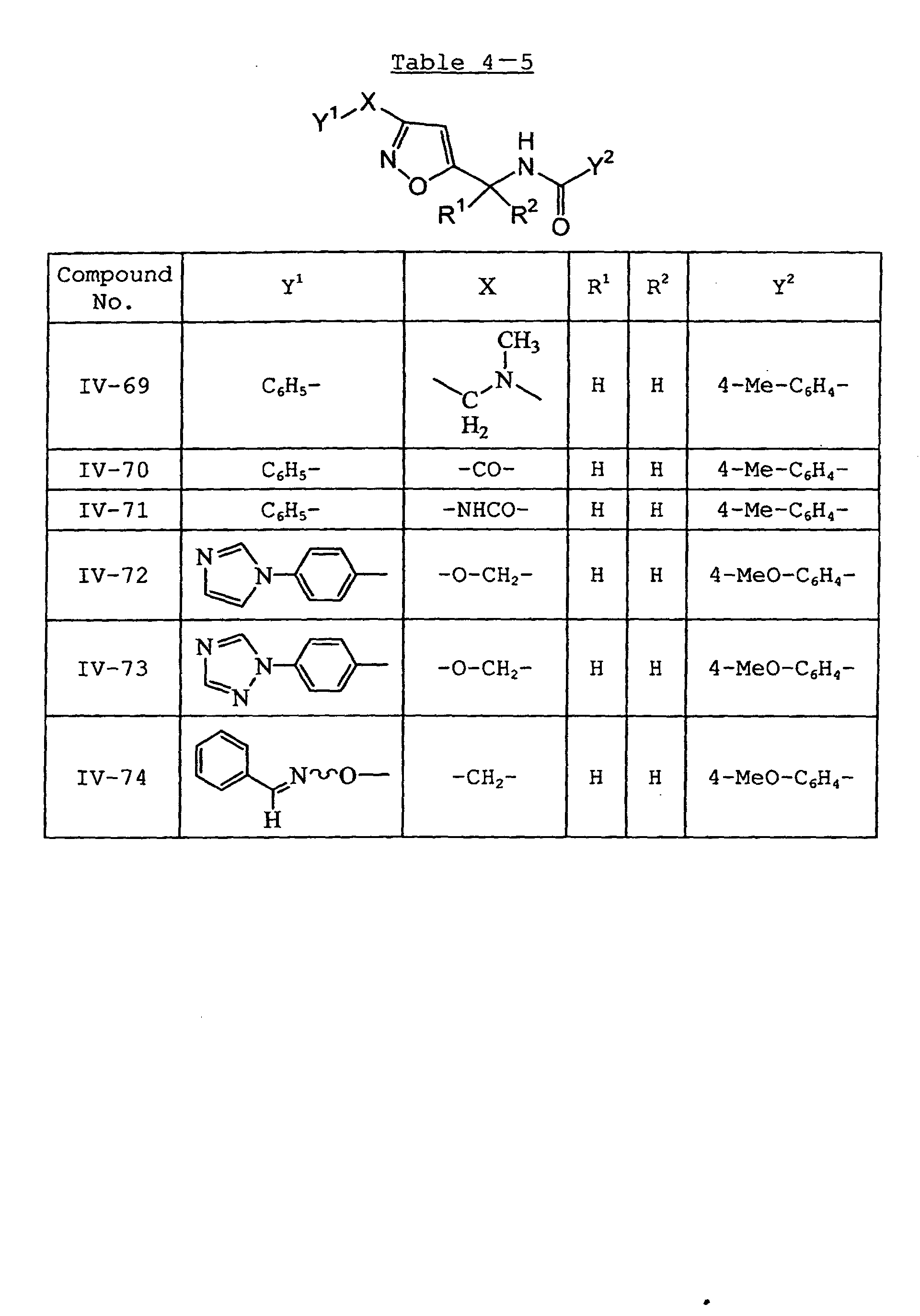

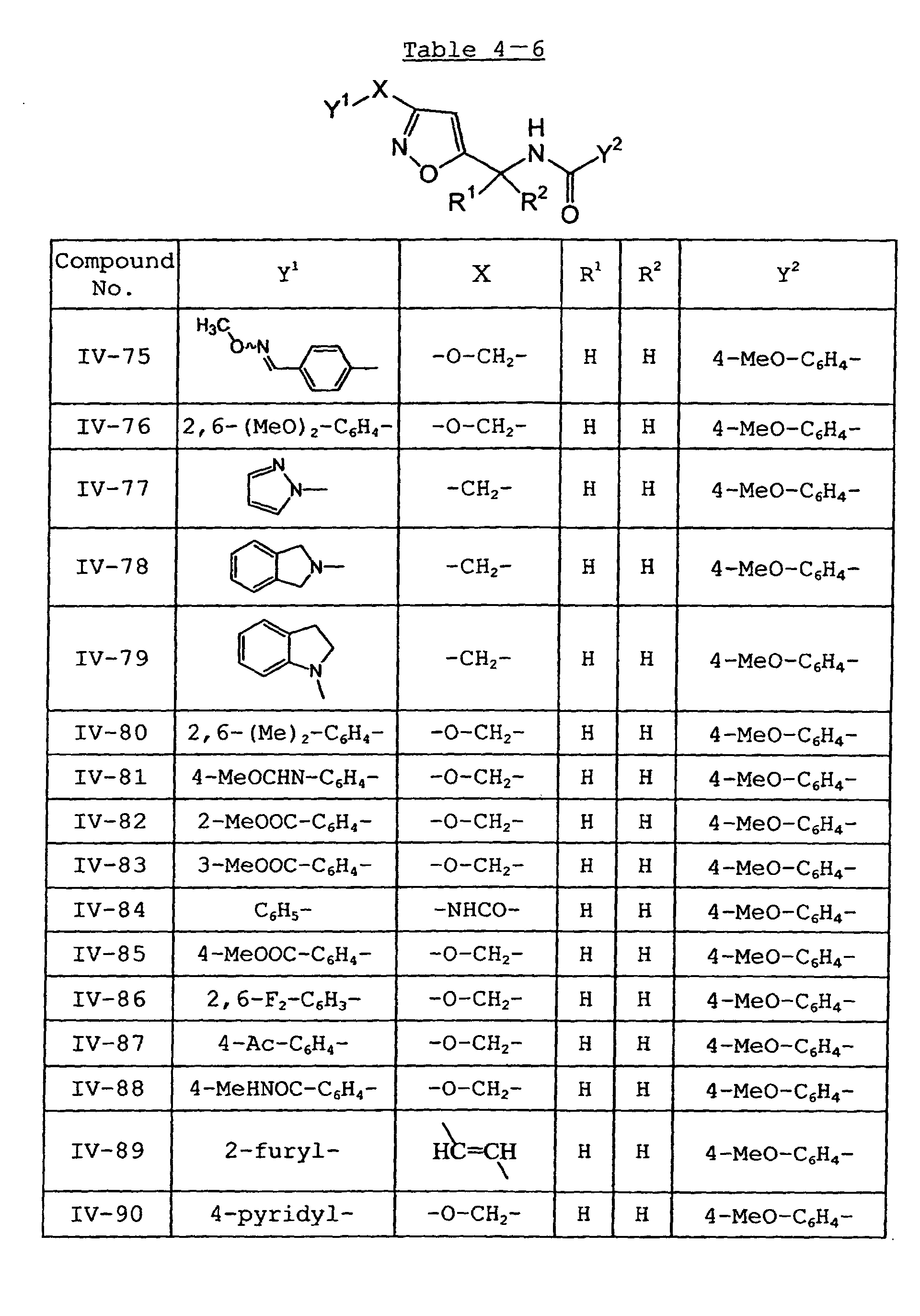

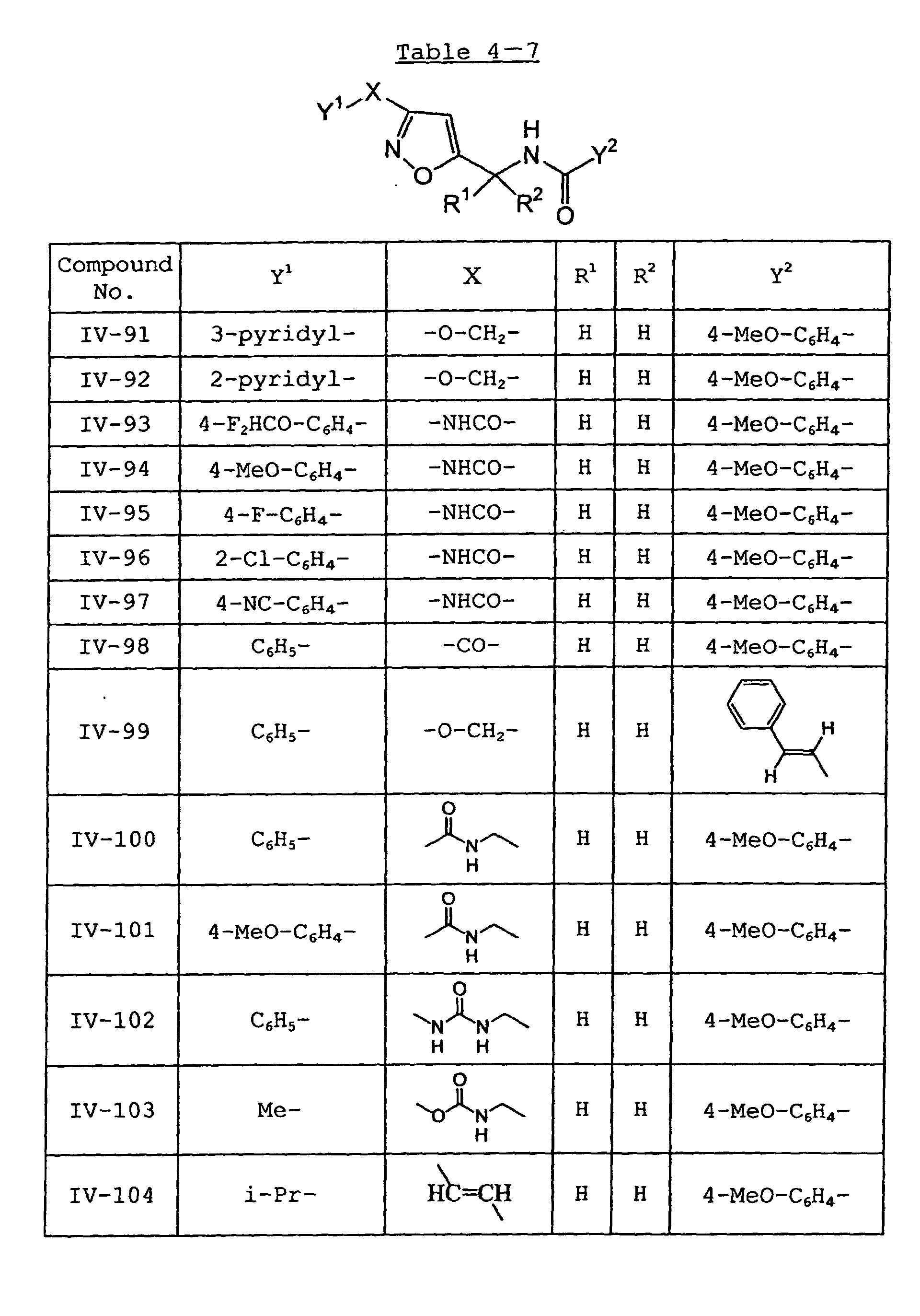

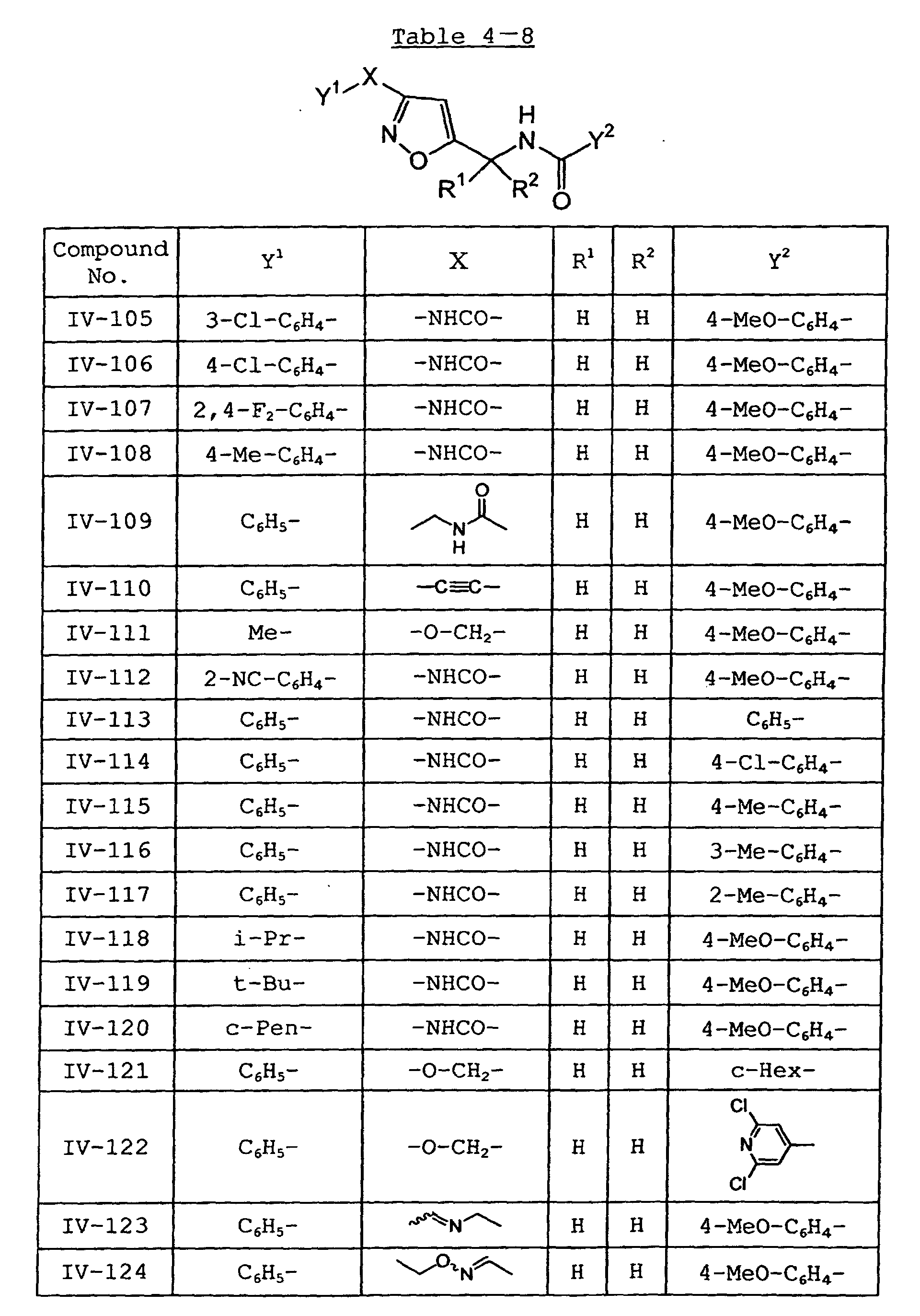

### [Method of manufacturing the compound of the invention]

[1] A substituted 5-alkylamino isoxazole derivative (V) which is an intermediate of the compound of the invention can be prepared by a method shown in the following reaction scheme (1).

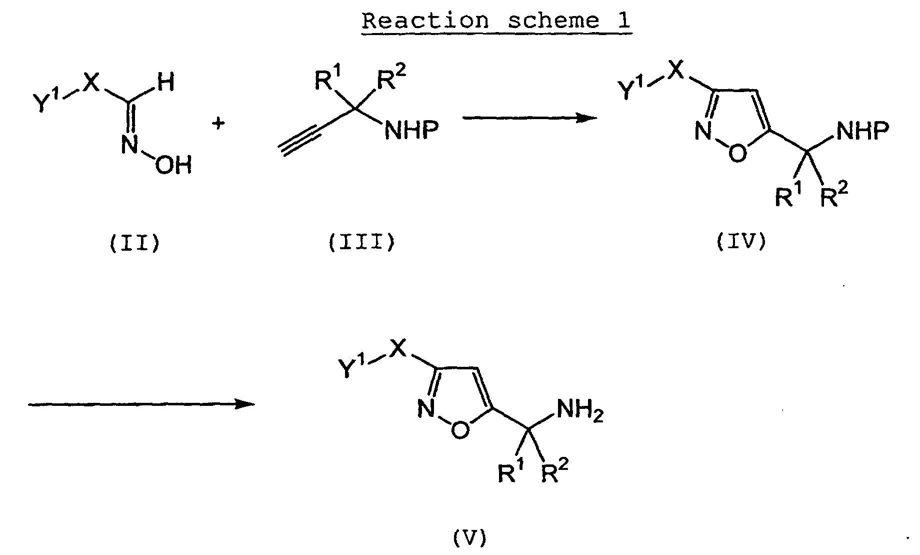

(In the reaction scheme, P represents a protecting group of an amino group, X, Y¹, R¹, and R² represent the same meanings as those defined above.)

That is, a protection body of a substituted 5-alkylamino isoxazole derivative represented by the formula (IV) can be prepared by reacting an oxime derivative represented by the formula (II) with a protected propargyl amine derivative represented by the formula (III) in an appropriate solvent in the presence of Hypolites.

Here, a t-butoxycarbonyl group, a benzyloxycarbonyl group, etc. are used as a protective group of an amino group.

Here, examples of Hypolites include: alkali metals such as sodium hypochlorite, potassium hypochlorite, lithium hypochlorite, sodium hypobromite, and potassium hypobromite; calcium hypochlorite; magnesium hypochlorite; strontium hypochlorite; calcium hypobromite; and magnesium hypobromite. Preferable examples thereof include alkali metal solutions of sodium hypochlorite and potassium hypochlorite.

Preferable examples of the solvent used for the reaction include: hydrocarbons such as hexane, pentane, and cyclohexane; aromatic hydrocarbons such as benzene, toluene, and xylene; primary alcohols such as methanol, ethanol, and n-propanol; ethers such as tetrahydrofuran and dioxane; halogenated hydrocarbons such as chloroform, dichloromethane, carbon tetrachloride, 1,2-dichloroethane, and 1,1,2-trichloroethane; esters such as methyl acetate, ethyl acetate, and propyl acetate; water; acetonitrile; dimethyl formamide; dimethyl sulfoxide; N-methyl-2-pyrrolidone; and arbitrary mixed solvents thereof. More preferable examples thereof include: halogenated hydrocarbons such as chloroform and dichloromethane; and arbitrary mixed solvents selected therefrom.

The reaction temperature is generally between -80°C and 150°C, and preferably between -15°C and 50°C.

Although the reaction time depends on the reaction temperature and the reaction substrate, the reaction is generally completed in 30 minutes to 3 days.

The compound of the formula (IV) which is a reaction intermediate is collected from a reaction mixture by a usual post-treatment. For example, the compound is obtained by: adding water to the reaction mixture; washing with water; and subsequently distilling off the solvent. The resulting objective mixture can be also purified by operations such as column chromatography and recrystallization if necessary.

Subsequently, a substituted 5-aminoalkyl isoxazole derivative represented by the formula (V) can be prepared by reacting the compound of the formula (IV) in an appropriate solvent under an appropriate deprotection condition.

The deprotection condition used here depends on the protecting group. For example, when a t-butoxycarbonyl group is used as the protecting group, proton acid solutions such as an aqueous solution of hydrochloric acid are preferable, and when a benzyloxycarbonyl group is used as the protecting group, it is preferable to perform deprotection under a hydrogen flow in the presence of a catalyst such as palladium carbon or Raney Nickel.

Preferable examples of the solvent used for the reaction include: hydrocarbons such as hexane, pentane, and cyclohexane; aromatic hydrocarbons such as benzene, toluene, and xylene; primary alcohols such as methanol, ethanol, and n-propanol; ethers such as tetrahydrofuran and dioxane; halogenated hydrocarbons such as chloroform, dichloromethane, carbon tetrachloride, 1,2-dichloroethane, and 1,1,2-trichloroethane; esters such as methyl acetate, ethyl acetate, and propyl acetate; water; acetonitrile; dimethyl formamide; dimethyl sulfoxide; and N-methyl-2-pyrrolidone; and arbitrary mixed solvents thereof.

The reaction temperature is generally between -80°C and 150°C, and preferably between -15°C and 50°C.

Although the reaction time depends on the reaction temperature, the reaction substrate, and the reaction reagent, the reaction is generally completed in 30 minutes to 1 week.

The aminoalkyl isoxazole derivative of the formula (V) which is the reaction intermediate is collected from the reaction mixture by the usual post-treatment. For example, in the case of the deprotection condition of the t-butoxycarbonyl group, the derivative is obtained by: making the reaction mixture alkali; then extracting with a solvent; and subsequently distilling off the solvent. The resulting objective mixture can be also purified by operations such as column chromatography and recrystallization if necessary. Also in the case of the deprotection condition of the benzyloxycarbonyl group, the derivative is obtained by: eliminating a reaction catalyst by filtration; and subsequently distilling off the solvent. The resulting objective mixture can be also purified by operations such as column chromatography and recrystallization if necessary.

[2] Also, the substituted 5-alkylamino isoxazole derivative of the formula (V) can be prepared by the method shown in the following reaction scheme 2. 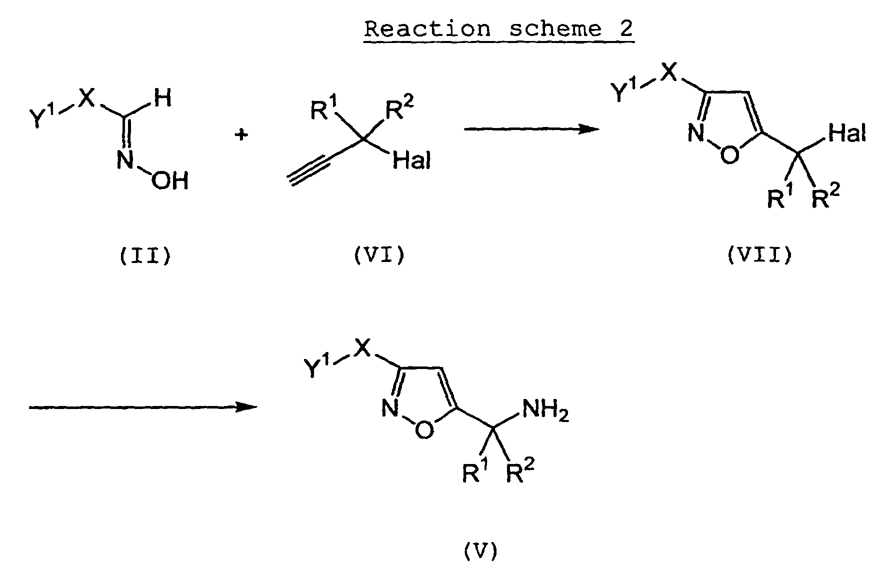 (In the reaction scheme 2, Hal represents a halogen atom, X, Y¹, R¹, and R² represent the same meanings as those defined above.)

That is, a 5-halogenoalkyl isoxazole derivative represented by the formula (VII) can be prepared by reacting an oxime derivative represented by the formula (II) with a propargyl amine derivative represented by the formula (VI) in an appropriate solvent in the presence of Hypolites.

Here, examples of Hypolites include: alkali metals such as sodium hypochlorite, potassium hypochlorite, lithium hypochlorite, sodium hypobromite, and potassium hypobromite; calcium hypochlorite; magnesium hypochlorite; strontium hypochlorite; calcium hypobromite; and magnesium hypobromite. Preferable examples thereof include alkali metal solutions of sodium hypochlorite, and potassium hypochlorite.

Preferable examples of the solvent used for the reaction include: hydrocarbons such as hexane, pentane, and cyclohexane; aromatic hydrocarbons such as benzene, toluene, and xylene; primary alcohols such as methanol, ethanol, and n-propanol; ethers such as tetrahydrofuran and dioxane; halogenated hydrocarbons such as chloroform, dichloromethane, carbon tetrachloride, 1,2-dichloroethane, and 1,1,2-trichloroethane; esters such as methyl acetate, ethyl acetate, and propyl acetate; water; acetonitrile; dimethyl formamide; dimethyl sulfoxide; and N-methyl-2-pyrrolidone; and arbitrary mixed solvents thereof. More preferable examples thereof include: halogenated hydrocarbons such as chloroform and dichloromethane; and arbitrary mixed solvents thereof.

The reaction temperature is generally between -80°C and 150°C, and preferably between -15°C and 50°C.

Although the reaction time depends on the reaction temperature and the reaction substrate, the reaction is generally completed in 30 minutes to 24 hours.

The compound of the formula (VII) which is a reaction intermediate is collected from a reaction mixture by a usual post-treatment. For example, the compound is obtained by: adding water to the reaction mixture; washing with water; and subsequently distilling off the solvent. The resulting objective mixture can be also purified by operations such as column chromatography and recrystallization if necessary.

Subsequently, a substituted 5-aminoalkyl isoxazole represented by the formula (V) can be prepared by reacting the compound of the formula (VII) in an appropriate solvent by using an amine.

Examples of the amines used here include gaseous ammonia, an ammonia-dioxane solution, an ammonia-ethyl alcohol solution, an ammonia-methyl alcohol solution, an ammonia-2-propanol solution, and an aqueous ammonia solution.

Any solvent may be used for the reaction as long as the solvent does not inhibit the reaction: for example, hydrocarbons such as hexane, pentane, and cyclohexane; aromatic hydrocarbons such as benzene, toluene, and xylene; primary alcohols such as methanol, ethanol, and n-propanol; ethers such as tetrahydrofuran and dioxane; halogenated hydrocarbons such as chloroform, dichloromethane, carbon tetrachloride, 1,2-dichloroethane, and 1,1,2-trichloroethane; water; and arbitrary mixed solvents thereof. More preferable examples thereof include halogenated hydrocarbons such as chloroform and dichloromethane.

The reaction temperature is generally between -80°C and 150°C, and preferably between -15°C and 50°C.

Although the reaction time depends on the reaction temperature and the reaction substrate, the reaction is generally completed in 30 minutes to 1 week.

The compound of the formula (V) which is the reaction object is collected from the reaction mixture by the usual post-treatment. For example, the compound is obtained by: making the reaction mixture alkali; then extracting with a solvent; and subsequently distilling off the solvent. The resulting objective mixture can be also purified by operations such as column chromatography and recrystallization if necessary.

The substituted 5-alkylamino isoxazole derivative of the formula (IV) described above can be synthesized by the methods described in JP 52-154525 A, JP 9-509951 A, Tetrahedron Letters, Vol. 34, No. 47, pp7509-7512, 1993, Tetrahedron Letters, Vol. 42 (2001) 1057-1060, Synthetic Communications, 22(13), 1939-1948 (1992), Tetrahedron Letters, Vol. 27, No. 27, pp3181-3182, 1986, Tetrahedron Letters, Vol. 33, No. 22, pp3113-3116, 1992, and the like, and the methods according thereto.

[3] A substituted 5-alkylamino isoxazole derivative represented by the formula (XII) can be prepared by the method shown in the following reaction scheme 3. 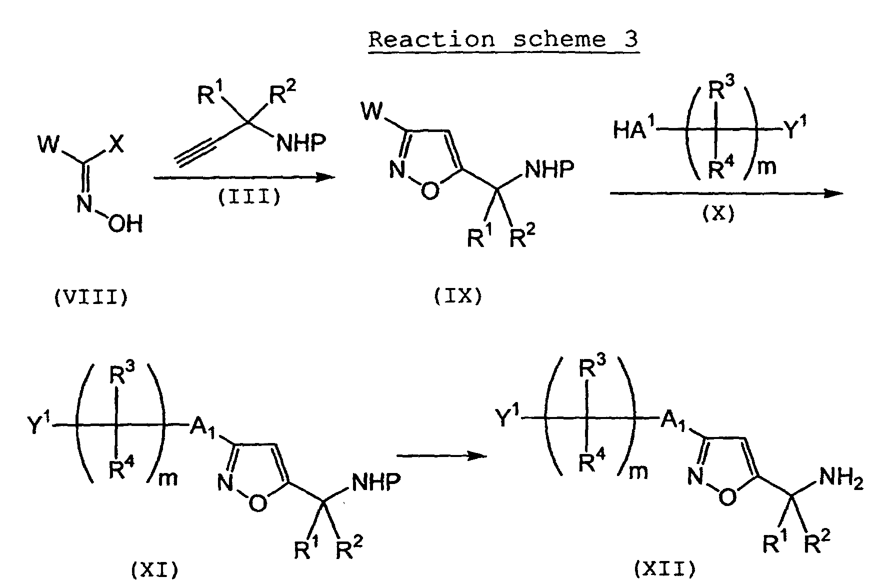 (In the reaction scheme 3, Y¹, R¹, R², R³, R⁴, and m represent the same meanings as those defined above, A¹ represents an oxygen atom, a sulfur atom, or an -NR¹²- group (wherein R¹² represents a hydrogen atom or a lower alkyl group), Hal represents a halogen atom, P represents a protecting group of the amino group, X represents a halogen atom, and W represents a leaving group such as a halogen atom, or a tosyl or mesyl group.)

That is, a protection body of a 3-phenylsulfonyl isoxazole derivative represented by the formula (IX) can be prepared by reacting halo oxime represented by the formula (VIII) with the propargyl amine derivative represented by the formula (III) by virtue of 1,3-dipolar addition in the presence of a base. Subsequently, the protection body of a 5-alkylamino isoxazole derivative represented by the formula (XI) can be prepared by reacting a nucleophilic agent represented by the formula (X). A substituted alkylamino isoxazole derivative represented by the formula (XII) can be prepared by reacting the protection body of the alkylamino isoxazole derivative represented by the formula (XI) under an appropriate deprotection condition.

As the base used for the 1,3-dipolar addition reaction, as the base used in the 1st stage reaction for example, any type of base generally used for this type of reaction may be used without particular limitation. Examples thereof include: alkali metal hydroxides such as sodium hydroxide and potassium hydroxide; alkali earth metal hydroxides such as calcium hydroxide; alkali carbonates such as sodium carbonate and potassium carbonate; and organic bases such as triethylamine, trimethylamine, N,N-dimethylaniline, pyridine, N-methylpiperidine, 1,5-diazabicyclo[4.3.0]non-5-ene (DBN) and 1,8-diazabicyclo[5.4.0]undec-7-ene (DBU).

Any solvent may be used for the 1,3-dipolar addition reaction as long as the solvent does not inhibit the reaction: for example, hydrocarbons such as hexane, pentane, and cyclohexane; aromatic hydrocarbons such as benzene, toluene, and xylene; primary alcohols such as methanol, ethanol, and n-propanol; ethers such as tetrahydrofuran and dioxane; halogenated hydrocarbons such as chloroform, dichloromethane, carbon tetrachloride, 1,2-dichloroethane, and 1,1,2-trichloroethane; water; and arbitrary mixed solvents thereof. Preferable examples thereof include halogenated hydrocarbons such as chloroform and dichloromethane.

The reaction temperature is generally between -80°C and 150°C, and preferably between -15°C and 50°C.

Although the reaction time depends on the reaction temperature and the reaction substrate, the reaction is generally completed in 30 minutes to 1 week.

The compound of the formula (IX) which is a reaction object is collected from the reaction mixture by the usual post-treatment. For example, the compound is obtained by: extracting with a solvent; and subsequently distilling off the solvent. The resulting objective mixture can be also purified by operations such as column chromatography and recrystallization if necessary.

As the base used in the nucleophilic addition reaction of the compound represented by the formula (X) with the compound represented by the formula (IX), any type of base generally used for this type of reaction may be used without particular limitation. Examples thereof include: alkali metal hydroxides such as sodium hydroxide and potassium hydroxide; alkali earth metal hydroxides such as calcium hydroxide; alkali carbonates such as sodium carbonate and potassium carbonate; and organic bases such as triethylamine, trimethylamine, N,N-dimethylaniline, pyridine, N-methylpiperidine, 1,5-diazabicyclo[4.3.0]non-5-ene (DBN), and 1,8-diazabicyclo[5.4.0]undec-7-ene (DBU).

Any solvent may be used for the nucleophilic addition reaction of the compound represented by the formula (X) with the compound represented by the formula (IX) as long as the solvent does not inhibit the reaction including: for example, hydrocarbons such as hexane, pentane, and cyclohexane; aromatic hydrocarbons such as benzene, toluene, and xylene; primary alcohols such as methanol, ethanol, and n-propanol; ethers such as tetrahydrofuran and dioxane; halogenated hydrocarbons such as chloroform, dichloromethane, carbon tetrachloride, 1,2-dichloroethane, and 1,1,2-trichloroethane; water; and arbitrary mixed solvents thereof.

The reaction temperature is generally between -80°C and 150°C, and preferably between -15°C and 50°C.

Although the reaction time depends on the reaction temperature and the reaction substrate, the reaction is generally completed in 30 minutes to 1 week.

The compound of the formula (XI) which is a reaction object is collected from the reaction mixture by the usual post-treatment. For example, the compound is obtained by: extracting with a solvent; and subsequently distilling off the solvent. The resulting objective mixture can be also purified by operations such as column chromatography and recrystallization if necessary.

Subsequently, a substituted 5-aminoalkyl isoxazole derivative represented by the formula (XII) can be prepared by reacting the compound of the formula (XI) in an appropriate solvent under an appropriate deprotection condition.

The deprotection condition used here depends on the protecting group. For example, when a t-butoxycarbonyl group is used as the protecting group, proton acid solutions such as an aqueous solution of hydrochloric acid are preferable, and when a benzyloxycarbonyl group is used as the protecting group, it is preferable to perform deprotection under a hydrogen flow in the presence of a catalyst such as palladium carbon or Raney Nickel.

Preferable examples of the solvent used for the reaction include: hydrocarbons such as hexane, pentane, and cyclohexane; aromatic hydrocarbons such as benzene, toluene, and xylene; primary alcohols such as methanol, ethanol, and n-propanol; ethers such as tetrahydrofuran and dioxane; halogenated hydrocarbons such as chloroform, dichloromethane, carbon tetrachloride, 1,2-dichloroethane, and 1,1,2-trichloroethane; esters such as methyl acetate, ethyl acetate, and propyl acetate; water; acetonitrile; dimethyl formamide; dimethyl sulfoxide; N-methyl-2-pyrrolidone; and arbitrary mixed solvents thereof.

The reaction temperature is generally between -80°C and 150°C, and preferably between -15°C and 50°C.

Although the reaction time depends on the reaction temperature, the reaction substrate, and the reaction reagent, the reaction is generally completed in 30 minutes to 1 week.

The substituted aminoalkyl isoxazole derivative of the formula (XII) is collected from the reaction mixture by the usual post-treatment. For example, in the case of the deprotection condition of the t-butoxycarbonyl group, the derivative is obtained by: making the reaction mixture alkali; then extracting with a solvent; and subsequently distilling off the solvent. The resulting objective mixture can be also purified by operations such as column chromatography and recrystallization if necessary. Also in the case of the deprotection condition of the benzyloxycarbonyl group, the derivative is obtained by: eliminating a reaction catalyst by filtration; and subsequently distilling off the solvent. The resulting objective mixture can be also purified by operations such as column chromatography and recrystallization if necessary.

Halo oxime represented by the formula (VIII) is the compound known in literatures, and for example, there are descriptions of the synthetic methods of the halo oxime and the related reactions in Journal of American Chemical Society, 101, (5), 1319-1320 (1979), Journal of Organic Chemistry, 1988, 53, 5369-5371, Journal of Organic Chemistry, 1983, 46, 1796, Journal of Organic Chemistry, 1984, 49, 4595, Journal of Organic Chemistry, 1987, 52, 2973, JP 11-504651 T, Synthetic Communications, 22(13), 1939-1948 (1992), Tetrahedron Letters, Vol. 27, No. 27, pp3181-3182, 1986, Tetrahedron Letters, Vol. 33, No. 22, pp3113-3116, (1992), and the like.

[4] The compound represented by the formula (I) can be prepared by the method shown in the following reaction scheme 4. 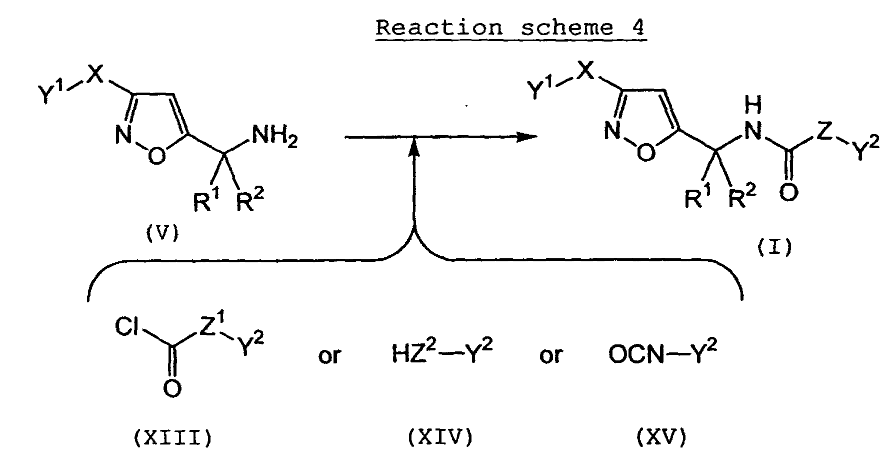 (In the reaction scheme, X, Y¹, Y², R¹, R², and Z represent the same meanings as those defined above, Z¹ represents Z in which B is any one but an NH group, and Z² represents Z which includes B.)

That is, the derivative represented by the formula (I) can be prepared by: reacting a derivative, in which a carbonyl group of an acid chloride represented by the formula (XIII) or of a compound other than the compound of the formula (XIII) is activated, with the substituted 5-alkylamino isoxazole derivative (V) in an appropriate solvent, if necessary in the presence of an appropriate base.

Examples of the derivative in which a carboxyl group of a compound other than the compound of the formula (XIII) is activated used here include: an acid anhydride of the compound of the formula (XIII); a mixed acid anhydride in which the compound of the formula (XIII) is condensed with another acid, o-alkyl carbonate, or the like; and activated esters such as p-nitrophenyl ester, 2-tetrahydropyranyl ester, and 2-pyridyl ester.

The base used here is not particularly limited and may be any type of base generally used for this type of reaction. Examples of the base include: alkali metal hydroxides such as sodium hydroxide and potassium hydroxide; alkali earth metal hydroxides such as calcium hydroxide; alkali carbonates such as sodium carbonate, and potassium carbonate; and organic bases such as triethylamine, trimethylamine, N,N-dimethylaniline, pyridine, N-methylpiperidine, 1,5-diazabicyclo[4.3.0]non-5-ene (DBN), and 1,8-diazabicyclo[5.4.0] undec-7-ene (DBU). Of those, tertiary amines such as triethylamine, pyridine, and N-methylpiperidine are preferable.

Any solvent may be used for the reaction as long as the solvent does not inhibit the reaction: for example, hydrocarbons such as hexane, pentane, and cyclohexane; aromatic hydrocarbons such as benzene, toluene, and xylene; primary alcohols such as methanol, ethanol, and n-propanol; ethers such as tetrahydrofuran and dioxane; halogenated hydrocarbons such as chloroform, dichloromethane, carbon tetrachloride, 1,2-dichloroethane, and 1,1,2-trichloroethane; water; and arbitrary mixed solvents thereof.

The reaction temperature is generally between -80°C and 150°C, and preferably between -15°C and 50°C.

Although the reaction time depends on the reaction temperature and the reaction substrate, the reaction is generally completed in 30 minutes to 1 week.

The compound of the formula (I) which is the reaction object is collected from the reaction mixture by the usual post-treatment. For example, the compound is obtained by: adding water to the reaction mixture; extracting with a solvent; and subsequently distilling off the solvent. The resulting objective mixture can be also purified by operations such as column chromatography and recrystallization if necessary.

In this reaction, the substituted alkylamino isoxazole derivative represented by the formula (XII) may be used in place of the substituted alkylamino isoxazole derivative represented by the formula (V).

Also, the compound of the formula (I) can be prepared by: making the substituted 5-alkylamino isoxazole derivative of the formula (V) act upon an active species obtained by reacting an alcohol derivative, thiol derivative, primary amino derivative, or secondary amino derivative denoted by the formula (XIV) in the presence of an appropriate solvent with an appropriate condensing agent, if necessary in the presence of an appropriate base.

Examples of the condensation agent used include N,N'-carbonyl diimidazole, 4-nitrophenoxy carbonyl chloride, phosgene, and triphosgene.

The base used here is not particularly limited and may be any type of base generally used for this type of reaction. Examples of the base include: alkali metal hydroxides such as sodium hydroxide and potassium hydroxide; alkali earth metal hydroxides such as calcium hydroxide; alkali carbonates such as sodium carbonate and potassium carbonate; and organic bases such as triethylamine, trimethylamine, N,N-dimethylaniline, pyridine, N-methylpiperidine, 1,5-diazabicyclo[4.3.0]non-5-ene (DBN), and 1,8-diazabicyclo[5.4.0]undec-7-ene (DBU). Of those, tertiary amines such as triethylamine, pyridine, and N-methylpiperidine are preferable.

Examples of the solvent used for the reaction include: hydrocarbons such as hexane, pentane, and cyclohexane; aromatic hydrocarbons such as benzene, toluene, and xylene; primary alcohols such as methanol, ethanol, and n-propanol; ethers such as tetrahydrofuran and dioxane; halogenated hydrocarbons such as chloroform, dichloromethane, carbon tetrachloride, 1,2-dichloroethane, and 1,1,2-trichloroethane; water; and arbitrary mixed solvents thereof.

The reaction temperature is generally between -80°C and 150°C, and preferably between -15°C and 50°C.

Although the reaction time depends on the reaction temperature and the reaction substrate, the reaction is generally completed in 30 minutes to 48 hours.

The compound of the formula (I) which is the reaction object is collected from the reaction mixture by the usual post-treatment. For example, the compound is obtained by: adding water to the reaction mixture; extracting with a solvent; and subsequently distilling off the solvent. The resulting objective mixture can be also purified by operations such as column chromatography and recrystallization if necessary.

In this reaction, the substituted alkylamino isoxazole derivative represented by the formula (XII) may be used in place of the substituted alkylamino isoxazole derivative represented by the formula (V).

Also, the compound of the formula (I) can be prepared by reacting an isocyanate derivative of the formula (XV) with the substituted 5-alkylamino isoxazole derivative of the formula (V).

Examples of the solvent used for the reaction include: hydrocarbons such as hexane, pentane, and cyclohexane; aromatic hydrocarbons such as benzene, toluene, and xylene; primary alcohols such as methanol, ethanol, and n-propanol; ethers such as tetrahydrofuran and dioxane; halogenated hydrocarbons such as chloroform, dichloromethane, carbon tetrachloride, 1,2-dichloroethane, and 1,1,2-trichloroethane; water; and arbitrary mixed solvents thereof.

The reaction temperature is generally between -80°C and 150°C, and preferably between -15°C and 50°C.

Although the reaction time depends on the reaction temperature and the reaction substrate, the reaction is generally completed in 30 minutes to 48 hours.

The compound of the formula (I) which is the reaction object is collected from the reaction mixture by the usual post-treatment. For example, the compound is obtained by: adding water to the reaction mixture; extracting with a solvent; and subsequently distilling off the solvent. The resulting objective mixture can be also purified by operations such as column chromatography and recrystallization if necessary.

In this reaction, the substituted alkylamino isoxazole derivative represented by the formula (XII) may be used in place of the substituted alkylamino isoxazole derivative represented by the formula (V).

[5] The derivative represented by the formula (I) can be also manufactured by the method shown in the following reaction scheme 5. 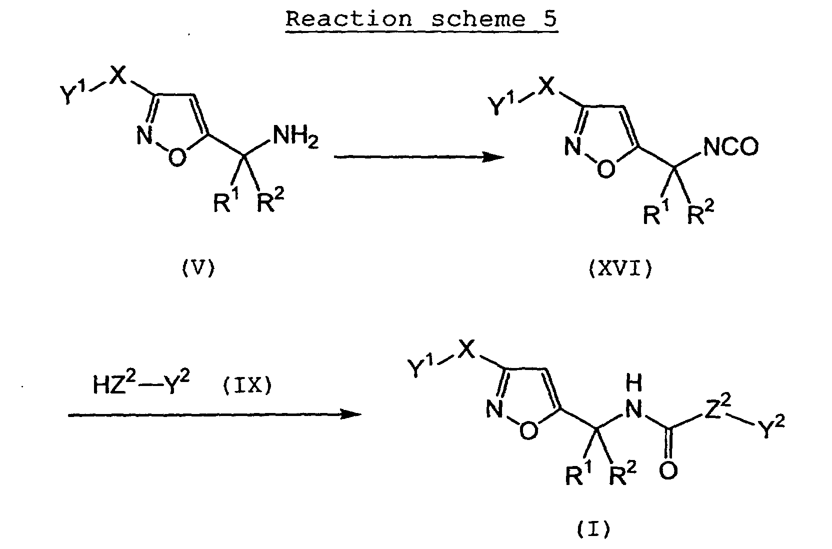 (In the reaction scheme, X, Y¹, Y², R¹, R², and Z² represent the same meanings as those defined above.)

That is, the isocyanate derivative represented by the formula (XVI) can be prepared by using phosgene or triphosgene for the substituted 5-alkylamino isoxazole derivative (V) and reacting in an appropriate solvent, if necessary in the presence of an appropriate base.

The base used here is not particularly limited and may be any type of base generally used for this type of reaction. Examples of the base include: alkali metal hydroxides such as sodium hydroxide and potassium hydroxide; alkali earth metal hydroxides such as calcium hydroxide; alkali carbonates such as sodium carbonate and potassium carbonate; and organic bases such as triethylamine, trimethylamine, N,N-dimethylaniline, pyridine, N-methylpiperidine, 1,5-diazabicyclo[4.3.0]non-5-ene (DBN), and 1,8-diazabicyclo[5.4.0]undec-7-ene (DBU). Of those, tertiary amines such as triethylamine, pyridine, and N-methylpiperidine are preferable.

Examples of the solvent used for the reaction include: hydrocarbons such as hexane, pentane, and cyclohexane; aromatic hydrocarbons such as benzene, toluene, and xylene; primary alcohols such as methanol, ethanol, and n-propanol; ethers such as tetrahydrofuran and dioxane; halogenated hydrocarbons such as chloroform, dichloromethane, carbon tetrachloride, 1,2-dichloroethane, and 1,1,2-trichloroethane; water; and arbitrary mixed solvents thereof.

The reaction temperature is generally between -80°C and 150°C, and preferably between -15°C and 100°C.

Although the reaction time depends on the reaction temperature and the reaction substrate, the reaction is generally completed in 30 minutes to 48 hours.

The compound of the formula (XVI) which is the reaction object is collected from the reaction mixture by the usual post-treatment. For example, the compound is obtained by: adding water to the reaction mixture; extracting with a solvent; and subsequently distilling off the solvent. The resulting objective mixture can be also purified by operations such as column chromatography and recrystallization if necessary.

In this reaction, the substituted alkylamino isoxazole derivative represented by the formula (XII) may be used in place of the substituted alkylamino isoxazole derivative represented by the formula (V).

Subsequently, the compound of the formula (I) can be also manufactured by reacting an alcohol derivative, thiol derivative, primary amino derivative, or secondary amino derivative represented by the formula (IX) with the isocyanate derivative (XVI) in the presence of an appropriate solvent.

Examples of the solvent used for the reaction include: hydrocarbons such as hexane, pentane, and cyclohexane; aromatic hydrocarbons such as benzene, toluene, and xylene; primary alcohols such as methanol, ethanol, and n-propanol; ethers such as tetrahydrofuran and dioxane; halogenated hydrocarbons such as chloroform, dichloromethane, carbon tetrachloride, 1,2-dichloroethane, and 1,1,2-trichloroethane; water; and arbitrary mixed solvents thereof.

The reaction temperature is generally between -80°C and 150°C, and preferably between -15°C and 50°C.

Although the reaction time depends on the reaction temperature and the reaction substrate, the reaction is generally completed in 30 minutes to 48 hours.

The compound of the formula (I) which is the reaction object is collected from the reaction mixture by the usual post-treatment. For example, the compound is obtained by: adding water to the reaction mixture; extracting with a solvent; and subsequently distilling off the solvent. The resulting objective mixture can be also purified by operations such as column chromatography and recrystallization if necessary.

[6] The substituted 5-alkylamino isoxazole derivative represented by the formula (I) can be also manufactured by the method shown in the following reaction scheme 6. 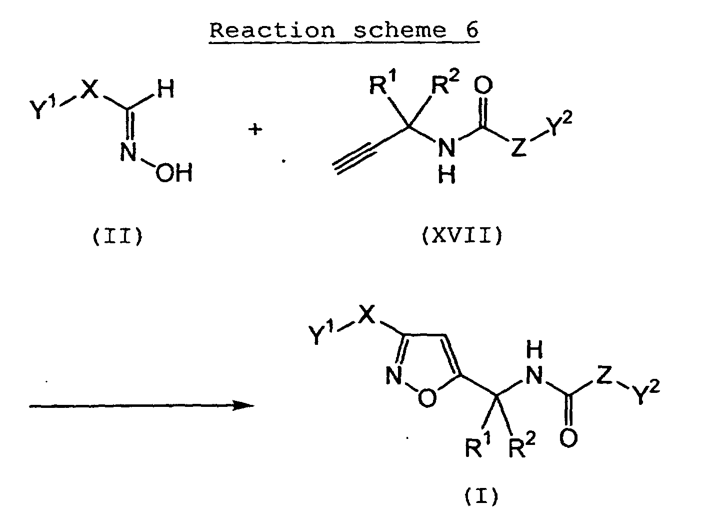 (In the reaction scheme, X, Y¹, Y², R¹, R², and Z represent the same meanings as those defined above.)

That is, the derivative represented by the formula (I) can be prepared by reacting the oxime derivative represented by the formula (II) with a propargyl amine derivative represented by the formula (XVII) in an appropriate solvent in the presence of Hypolites.

Here, examples of Hypolites include: alkali metals such as sodium hypochlorite, potassium hypochlorite, lithium hypochlorite, sodium hypobromite, and potassium hypobromite; calcium hypochlorite; magnesium hypochlorite; strontium hypochlorite; calcium hypobromite; and magnesium hypobromite. Preferable examples thereof include alkali metal solutions of sodium hypochlorite and potassium hypochlorite.

Preferable examples of the solvent used for the reaction include: hydrocarbons such as hexane, pentane, and cyclohexane; aromatic hydrocarbons such as benzene, toluene, and xylene; primary alcohols such as methanol, ethanol, and n-propanol; ethers such as tetrahydrofuran and dioxane; halogenated hydrocarbons such as chloroform, dichloromethane, carbon tetrachloride, 1,2-dichloroethane, and 1,1,2-trichloroethane; esters such as methyl acetate, ethyl acetate, and propyl acetate; water; acetonitrile; dimethyl formamide; dimethyl sulfoxide; N-methyl-2-pyrrolidone; and arbitrary mixed solvents thereof. More preferable examples thereof include halogenated hydrocarbons such as chloroform and dichloromethane.

The reaction temperature is generally between -80°C and 150°C, and preferably between -15°C and 50°C.

Although the reaction time depends on the reaction temperature and the reaction substrate, the reaction is generally completed in 30 minutes to 24 hours.

The compound of the formula (I) is collected from a reaction mixture by a usual post-treatment. For example, the compound is obtained by: adding water to the reaction mixture; washing with water; and subsequently distilling off the solvent. The resulting objective mixture can be also purified by operations such as column chromatography and recrystallization if necessary.

Aldoximes of the compound (II) can be prepared from corresponding aldehydes using a base such as hydroxylamine hydrochloride or sodium acetate in an alcohol solvent such as ethanol at ambient temperature or under the condition of reflux with heating.

Also, the propargyl amine derivative of the formula (XVII) can be prepared from propargyl amine using chloroformates, isocyanates, acid chlorides, or the like.

### [Application as agri-horticultural fungicides]

The compound represented by the formula (I) or (IA) has an action of exterminating noxious organisms and can be used as a agri-horticultural fungicide.

Plant diseases toward which the compound of the present invention have an excellent fungicidal effect include:
*Pyricularia oryzae*;
*Rhizoctonia solani*;
*Gibberella fujikuroi*;
*Erysiphe graminis* f.sp. *tritici*;
*Erysiphe graminis* f.sp. *hordei*;
*Pseudocercosporella herpotrichoides;*
*Puccinia graminis;*
*Colletotrichum graminicola*;
*Septoria tritici;*
*Phynchosporium secalis* f.sp. *hordei;*
*Phytophthora infestans*;
*Alternaria solani*;
*Colletotrichum atramentarium*;
*Thanatephorus cucumeris;*
*Botrytis cinerea*;
*Erysiphe pisi*;
*Cercospora canescens*;
*Sclerotinia sclerotiorum;*
*Colletotrichum phaseolorum*;
*Cercospora canescens*;
*Sclerotinia sclerotiorum;*
*Colletotrichum lindemuthianum;*
*Botrytis cinerea;*
*Colletotrichum truncatum;*
*Cercospora kikuchii;*
*Phakopsora pachyrhizi;*
*Gloeosporium conjac;*
*Septoria perillae;*
*Colletotrichum theae-sinensis;*
*Cercospora beticola;*
*Colletotrichum spinaciae;*
*Peronospora effusa;*
*Alternaria brassicae;*
*Alternaria brassicicola;*
*Sclerotinia sclerotiorum;*
*Botrytis cinerea;*
*Colletotrichum higginsianum;*
*Sclerotinia sclerotiorum;*
*Alternaria brassicae, Alternaria brassicicola;*
*Alternaria cucumerina;*
*Botrytis cinerea;*
*Pseudoperonospora cubensis;*
*Sphaerotheca fuliginea;*
*Phytophthora melonis;*
*Corynespora cassiicola;*
*Sclerotinia sclerotiorum;*
*Alternaria cucumerina*;
*Colletotrichum lagenarium*;
*Fusarium oxysporum* f.sp. *cucumerinum*;
*Pythium cucurbitacearum*, *Rhizoctonia solani*;
*Botrytis cinerea*;
*Phomopsis* sp.;
*Pseudoperonospora cubensis*;
*Sphaerotheca fuliginea*;
*Phytophthora cryptogea*;
*Sclerotinia sclerotiorum;*
*Colletotrichum orbiculare;*
*Pseudoperonospora cubensis*;
*Corynespora cassiicola*;
*Sclerotinia sclerotiorum;*
*Fusarium oxysporum* f.sp. *melonis*;
*Pseudoperonospora cubensis*;
*Fusarium oxysporum*;
*Sclerotinia sclerotiorum*;
*Puccinia cnici-oleracei*;
*Gloeosporium chrysanthemi*, *Gloeosporium carthami*;
*Fusarium oxysporum;*
*Erysiphe heraclei*;
*Sclerotinia intermedia, Sclerotinia sclerotiorum*;
*Alternaria dauci;*
*Alternaria radicina*;
*Fusarium oxysporum*;
*Oidiopsis sicula*;
*Phytophthora capsici*;
*Sclerotinia sclerotiorum*;
*Colletotrichum gloeosporioides*;
*Botrytis cinerea*;
*Fusarium oxysporum* f.sp. *lycopersici*;
*Oidiopsis sicula*;
*Phytophthora infestans*;
*Sclerotinia sclerotiorum;*
*Botrytis cinerea*;
*Fulvia fulva*;
*Phytophthora infestans*;
*Thanatephorus cucumeris;*
*Phytoph thora capsici*;
*Sclerotinia sclerotiorum;*
*Alternaria solani*;
*Fusarium oxysporum*;
*Verticillium dahliae*;
*Sphaerotheca humuli;*
*Phytophthora nicotianae* var. *parasitica*;
*Pythium ultimum* var. *ultimum*;
*Sclerotinia sclerotiorum*;
*Alternaria alternata*;
*Mycosphaerella fragariae;*
*Colletotrichum acutatum, Glomerella cingulata;*
*Botrytis cinerea;*
*Cercospora asparagi;*
*Phomopsis asparagi;*
*Puccinia asparagi-lucidi;*
*Colletotrichum gloeosporioides;*
*Botrytis cinerea;*
*Phytophthora nicotianae;*
*Cladosporium allii-cepae;*
*Fusarium oxysporum* f.sp. *cepae;*
*Sclerotinia sclerotiorum;*
*Septoria alliacea;*
*Alternaria porri;*
*Puccinia allii;*
*Puccinia allii;*
*Botrytis squamosa;*
*Phytophthora porri;*
*Colletotrichum circinans;*
*Alternaria* sp.;
*Botrytis cinerea;*
*Botrytis allii;*
*Pleospora herbarum;*
*Peronospora destructor;*
*Puccinia allii;*
*Botrytis byssoidea;*
*Phytophthora nicotianae;*
*Fusarium oxysporum;*
*Mycosphaerella allicina;*
*Septria alliacea;*
*Alternaria porri;*
*Sclerotinia allii;*
*Puccinia allii;*
*Botrytis squamosa;*
*Phytophthora porri;*
*Colletotrichum circinans;*
*Peronospora destructor;*
*Colletotrichum gloeosporioides;*
*Phyllactinia kakicola;*
*Colletotrichum* ssp., *Glomerella* sp.;
*Cercospora kakivora;*
*Cercospora kaki;*
*Macrophoma kaki;*
*Fusicladium levieri;*
*Phomopsis kakivora;*
*Pseudocercospora fuliginosa;*
*Physalospora kaki;*
*Aureobasidium pullulans, Capnophaeum fuliginodes, Cladosporium herbarum, Microxyphium* sp., *Scorias communis, Tripospermum juglandis;*
*Zygophiala jamaicensis;*
*Gloeosporium kaki;*
*Botrytis cinerea;*
*Pestalotia diospyri;*
*Glomerella cingulata;*
*Mycosphaerella nawae;*
*Podosphaera tridactyla, Sphaerotheca pannosa;*
*Botryosphaeria dothidea;*
*Sclerotinia sclerotiorum;*
*Cladosporium carpophilum;*
*Leucotelium pruni-persicae;*
*Rosellinia necatrix;*
*Fusarium lateritium;*
*Pseudocercospora circumscissa;*
*Sphaceloma pruni-domesticae;*
*Glomerella cingulata;*
*Botrytis cinerea;*
*Monilinia fructicola, Monilinia laxa;*
*Glomerella mume;*
*Sclerotinia sclerotiorum;*
*Podosphaera tridactyla;*
*Rhizopus nigricans;*
*Glomerella cingulata;*
*Botrytis cinerea;*
*Monilinia fructicola;*
*Phyllactinia mali;*
*Phytophthora cactorum, Phytophthora syringae;*
*Venturia pirina;*
*Colletotrichum gloeosporioides;*
*Gymnosporangium asiaticum;*
*Phyllactinia pyri;*
*Phytophthora cactorum, Phytophthora syringae;*
*Venturia nashicola;*
*Alternaria kikuchiana;*
*Leptothyrium pomi;*
*Glomerella cingulata;*
*Botrytis cinerea;*
*Monilinia fructigena;*
*Colletotrichum gloeosporioides;*
*Physalospora piricola;*
*Fusarium oxysporum, Gibberella zeae;*
*Podosphaera tridactyla, Sphaerotheca pannosa;*
*Sclerotinia sclerotiorum;*
*Rhizopus nigricans;*
*Cladosporium carpophilum:*
*Leucotelium pruni-persicae;*
*Stenella* sp.;
*Pseudocercospora circumscissa, Phyllosticta persicae;*
*Gloeosporium laeticolor;*
*Botrytis cinerea;*
*Monilinia fructicola*;
*Phomopsis* sp.;
*Gymnosporangium yamadae*;
*Podosphaera leucotricha*;
*Phytophthora cactorum*, *Phytophthora cambivora*, *Phytophthora syringae*;
*Diplocarpon mali;*
*Cristulariella moricola*;
*Venturia inaequalis*;
*Zygophiala jamaicensis*;
*Glomerella cingulata*;
*Botrytis cinerea;*
*Alternaria alternata;*
*Uncinula necator*;
*Sclerotinia sclerotiorum;*
*Glomerella cingulata*;
*Pseudocercospora vitis;*
*Briosia ampelophaga*;
*Elsinoe ampelina*;
*Phyllosticta ampelicida*;
*Phomopsis viticola*;
*Plasmopara viticola*;
*Glomerella cingulata;*
*Capnodium salicinum*;
*Morenoella quercina*;
*Microsphaera alphitoides;*
*Monochaetia monochaeta;*
*Botrytis cinerea;*
*Phytophthora citrophthora;*
*Diaporthe citri;*
*Mycosphaerella citri, Mycosphaerella horii;*
*Elsinoe fawcettii;*
*Mycosphaerella pinodes;*
*Colletotrichum gloeosporioides;* and
*Colletotrichum gloeosporioides.*

Application of the compound by the invention can be performed by treating plants with an active ingredient by spraying, scattering, applying, or the like, or by treating seeds of the plants, soil surrounding the plants or soil in which the seeds are sowed, and rice paddies and water of hydroponics with the active ingredient. The application can be performed before or after the plant is infected with pathogenic fungi.

The compound of the invention can be used by combining and formulating carriers, surfactants, adjuncts, and the like commonly used in agrichemical formulations.

The aspects of the formulations include dosage forms such as suspension agents, oil agents, emulsions, liquid agents, water soluble agents, water-dispersible powder, flowable agents, paste agents, granules, powder, tablets, granular water-dispersible powder, fumigants, aerosol agents, foaming agents, microcapsule agents, jumbo agents, coating agents for seeds; and ULV (cold mists or warm mists) agents. Such aspects can be prepared by the usual method of mixing at least one compound of the invention with carriers such as appropriate solid carrier, liquid carrier, and liquid gaseous carrier, and if desired, appropriate adjuncts (e.g., surfactants, spreaders, dispersants, stabilizers) for improving dispersibility and other natures of the active ingredient.

Examples of the solid carrier include: vegetable materials (such as crystalline cellulose, starch, wood meal, cork, and coffee shells); fibrous materials; artificial plastic powders; clays (such as kaolin, bentonite, white clay, diatomite, synthetic hydroxylated silicon, fubasami clay, and acid clay); talc and inorganic products (such as vermiculite, montmorillonite, pumica, sulfur powder, apatite, mica, sericite, ground quarts, active carbon, and calcium carbonate); high-molecular compounds (such as polyvinylchloride and petroleum resin); and chemical fertilizers (such as ammonium sulfate, ammonium phosphate, ammonium nitrate, ammonium chloride, calcium chloride, and urea).

Examples of the liquid carrier and diluent include: water; alcohols (such as methanol, ethanol, isopropyl alcohol, cyclohexanol, and aluminum alcohol); ketones (such as acetone, methyl ethyl ketone, and cyclohexanone); ethers (such as ethylcellosolve, butylcellosolve, and dioxane); aromatic hydrocarbons (such as benzene, toluene, xylene, ethylbenzene, and methylnaphthalene); aliphatic hydrocarbons (such as kerosine, isoparaffin, and normal paraffin); esters (such as isopropyl acetate and benzyl acetate); nitriles; amides (such as N,N-dimethylformamide and dimethylsulfoxide); and halogenated hydrocarbons (such as chlorobenzene and trichloroethylene).

Examples of the gaseous carrier, in other words the propellant, include carbon dioxide gas, butane gas, and fluorocarbon.

Examples of the surfactant include: alkyl sulfate; alkyl sulfonate; alkyl arylsulfonate; alkyl arylates and polyoxyethylene compounds thereof; polyethylene glycolether; polyalcohol esters; and sugar alcohol derivatives.

Examples of other adjuncts for the preparation include: binders and dispersants such as casein, gelatin, polysaccharides (such as starch, gum arabic, cellulose derivatives, and alginic acid), lignin derivatives, bentonite, synthetic water-soluble high molecules (such as polyvinyl alcohol, polyvinyl pyrrolidone, and polyacrylic acid); and stabilizers such as PAP (acidic phosphoric isopropyl), BHT (2,6-di-tert-butyl-4-methylphenol), BHA (2-tert-butyl-4-methoxyphenol), vegetable oil, molasses, agar, mineral oil, fatty acid, and fatty ester.

Generally, each of the above preparations contains at least one compound (I) of the invention at 0.1 to 95% by weight, preferably at 1 to 80% by weight. Those preparations can be treated alone or in dilution for the plants by spraying, scattering, applying, or the like, or for seeds of the plants, soil surrounding the plants or soil in which the seeds are sowed, and rice paddies and water of hydroponics. When the compound of the invention is sprayed, scattered, or applied onto the plants, the compound is used at a concentration of about 1 to 50,000 ppm, preferably about 50 to 1,000 ppm. When treated for the soil surrounding the plants or water of rice paddies, the compound is used at an amount of about 1 g to 5 kg/hectare, preferably about 2 g to 100 g/hectare. When treated for the soil in which the seeds are sowed, the compound is typically used at an amount of about 0.1 g to 1 kg/m², preferably 0.5 g to 500 g/m².

The compound of the invention can be formulated by combining with one or more of the other agrichemical ingredients (fungicide, bactericide, insecticide, acaridide, herbicide, plant growth regulator, etc.) for expanding effects. Examples of such agrichemicals are shown below, but the examples of agrichemicals to be combined are not limited thereto.

Examples of the combinable fungicide include acibenzolar-s-methyl, azoxystrobin, isoprothiolane, ipconazole, iprodione, iprovalicarb, iminoctadine acetate, iminoctadine albesilate, epoxyconazole, basic copper chloride, oxadixyl, kasugamycin, carpropamid, quinoxyfen, captan, kresoxim-methyl, chlorothalonil, diazofamid, diethofencarb, diclocymet, diclomedin, dithianon, zineb, simeconazole, dimethirimol, cyproconazole, cymoxanil, silthiofam, streptomycin, spiroxamine, sulfenic acid, thiabenzazole, thiophanete methyl, thiuram, thifluzamide, tecloftalam, tebuconazole, triadimefon, triazine, tricyclazole, triflumizole, trifloxystrobin, triforine, triclofos methyl, copper nonylphenol sulfonate, paclobutrazol, validamycin, picoxystrobin, hydroxyisoxazole, pyraclostrobin, pyroquilon, famoxadone, fenarimol, fenoxanil, ferimzone, fenhexamid, fthalide, blasticidin S, furametpyr, fluazinam, fluquinconazole, fludioxonil, flutolanil, prochloraz, procymidone, propiconazole, probenazole, pefurazoate, benomyl, pencycuron, fosetyl, polyoxine, manzeb, metalaxyl, metominostrobin, mepanipyrim, mepronil, organic copper, organic nickel, edifenphos, and iprobenfos.

Examples of the combinable bactericide include tecloftalam and oxolinic acid.

Examples of the combinable insecticide include acetamiprid, acephate, isoxathion, imidacloprid, ethiofencarb, ethylthiometon, ethofenprox, oxamyl, cartap, carbosulfan, clothianidin, chromafenozide, chlorpyrifos methyl, cycloprothrin, dinotefuran, dimethylvinphos, dimethoate, cyfluthrin, diprofezine, silafluofen, spinosad, sulprofos, diazinon, thiacloprid, thiomethoxam, thiodicarb, thiocyclam, thiometon, tebufenozide, nitenpyram, vamidothion, pymetrozine, pyraclofos, pyridaphenthion, fipronil, fopronil, buprofezin, furathiocarb, flucythrinate, fluvalinate, propaphos, permethrin, bensultap, benthiazole, benfuracarb, malathion, methasulfocarb, monocrotophos, malathion, fenobucarb, tetrachlorvinphos, trichlorophon, EPN, iprobenfos, fenitrothion, isoprocarb, carbaryl, phenthoate, propoxur, and XMC.

Examples of the combinable acaricides include etoxazole, kelthane, fenbutatin oxide, tebufenpyrad, halfenprox, bifenazate, pyrimidifen, pirimiphos methyl, fenpropathrin, fluvalinate, phosalone, milbemectin, propargite, and dichlorvos.

Examples of the combinable herbicide include ioxynil, azimsulfuron, asulam, atrazine, anilofos, alachlor, imazamox ammonium, imazosulfuron, indanofan, esprocarb, ethoxysulfuron, etobenzanid, chlorate, oxadiclomefon, cafenstrole, quizalofop ethyl, cumyluron, glyphosate ammonium, glyphosate isopropylamine, glyphosate trimesium, glyphosate sodium, glyphosinate, clethodim, clomeprop, cyanazine, cyclosulfamuron, diquat, cyhalofop butyl, cyhalofop methyl, diflufenican, dimethametryn, dimethenamid, simetryn, dimepiperate, cinmethylin, lime nitrogen, sethoxydim, dymron, thifensulfuron methyl, thenylchlor, tepraloxydim, trifluralin, naproanilide, paraquat, halosulfuron methyl, bialaphos, bispyribac sodium, bifenox, piperophos, pyrazoxyfen, pyrazosulfuron ethyl, pyrazolate, pyraflufenethyl, pyridate, pyributicarb, pyriminobac methyl, fenoxapropethyl, fentrazamide, butachlor, butamifos, fluazifop, pretilachlor, prometryn, bromobutide, pelargonic acid, bensulfuron methyl, benzobicyclon, benzofenap, bentazone, benthiocarb, pendimethalin, pentoxazone, benfuresate, methyl isocyanate, metolachlor, metribuzin, mephenacet, molinate, linuron, lenacil, quinoclamine, simazine, dichlobenil, diuron, propanil, chlorpropham, MCPA ethyl, MCPA thioethyl, MCPA sodium, MCPB, bensulide, and 2,4-PA.

Examples of the combinable plant growth regulator include inabenfide, indoleacetic acid, uniconazole, calcium peroxide, nicotinamide, paclobutrazol, prohexadione calcium, and benzylaminopurin.

### BEST MODE FOR CARRYING OUT THE INVENTION

Next, the present invention is illustrated by citing preparation examples and evaluation examples of the compound of the invention, but the invention is not limited thereto.

In the examples, data on ¹H-NMR were represented as δ values measured by an R-1900 type spectrometer (90 MHz) supplied from Hitachi Ltd., using tetramethylsilane as an internal standard and CDCl₃ as a solvent. However, for the compound which is difficult to dissolve in CDCl₃, the measurement was performed by adding an appropriate amount of DMSO-d₆.

### Example 1: Preparation of phenyl {[3-(2-chlorophenyl)-5-isoxazolyl]methyl}carbamate (Compound Number I-28)

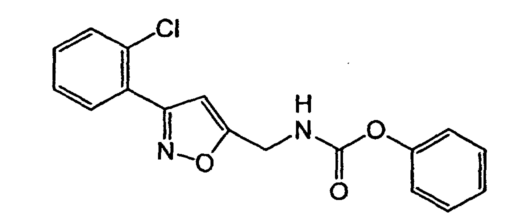

To a solution of 0.51 g of 5-aminomethyl-3-(2-chlorophenyl)isoxazole and 0.38 g of diisopropylethylamine in 30 ml of dichloromethane, 0.46 g of phenyl chloroformate was added at 0°C, the temperature was raised to room temperature, and subsequently the solution was stirred for 5 hours. After the completion of the reaction, the solution was concentrated under reduced pressure, and subsequently purified on silica gel column chromatography to yield 0.75 g as a colorless oil having the following physical property.
NMR : δ 4.52(2H,d), 5.49-5.88(1H,brs), 6.48(1H,s), 6.99-7.80(9H,m)

### Example 2: Preparation of 3-[(3-phenyl-5-isoxazolyl)methyl]-1-(4-phenoxyphenyl)urea (Compound Number II-42)

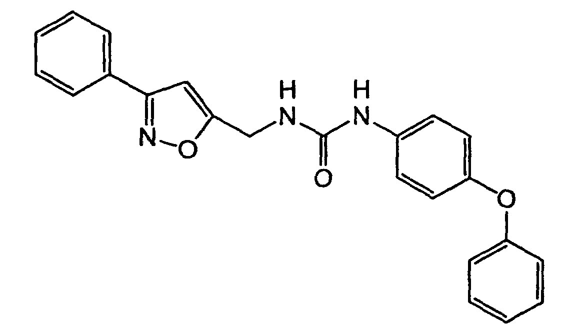

To a solution of 0.82 g of 5-aminomethyl-3-phenylisoxazole in 40 ml of ethanol, 1.00 g of p-phenoxyphenylisocyanate was added at room temperature, and then the whole was stirred overnight. The reaction solution was concentrated under reduced pressure, and subsequently a precipitated crystal was filtrated and washed with isopropylether to yield 1.60 g as a colorless crystal having the following physical property.
Melting point: 179 to 181°C.

### Example 3: Preparation of 3-[(3-phenyl-5-isoxazolyl]methyl]-1-methyl-1-phenyl urea (Compound Number II-12)

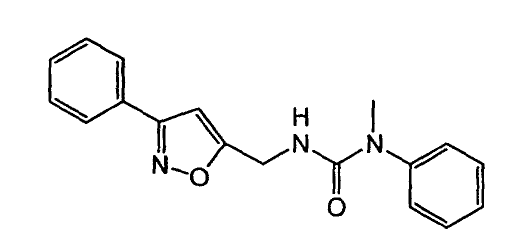

To a solution of 1.00 g of 5-aminomethyl-3-phenylisoxazole and 0.89 g of diisopropylethylamine in 20 ml of dichloromethane, 0.97 g of N-methyl N-phenylcarbamoylchloride was added at 0°C, the temperature was raised to room temperature, and subsequently the solution was stirred overnight. After the completion of the reaction, the solution was concentrated under reduced pressure, and subsequently purified on silica gel column chromatography to yield 1.48 g as a colorless crystal having the following physical property.
Melting point: 114 to 117°C.

### Example 4: Preparation of N-[(3-phenyl-5-isoxazolyl)methyl]-4- methoxy benzamide (Compound Number III-111)

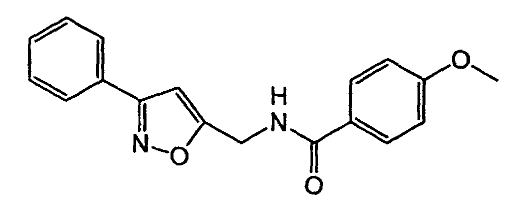

To a solution of 0.60 g of 5-aminomethyl-3-phenylisoxazole and 0.53 g of diisopropylethylamine in 30 ml of dichloromethane, 0.59 g of p-methoxybenzoylchloride was added at 0°C, the temperature was raised to room temperature, and subsequently the solution was stirred for overnight. After the completion of the reaction, the solution was concentrated under reduced pressure, and subsequently purified on silica gel column chromatography to yield 0.90 g as a colorless crystal having the following physical property.
Melting point: 182 to 184°C.

### Example 5: Preparation of phenyl {[3-(4-methoxyphenyl)-5-isoxazolyl]methyl}carbamate (Compound Number I-26)

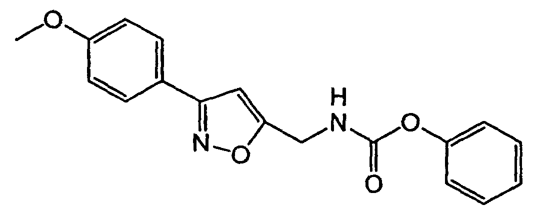

To a solution of 2.00 g of 4-metoxy benzaldoxime and 2.78 g of phenyl 2-propynyl carbamate in 50 ml of dichloromethane, 11.8 g of 10% sodium hypochlorite aqueous solution was dripped over 30 minutesat 0°C. The temperature was raised to room temperature, and then the solution was stirred for 5 hours. Water was added, the solution was extracted twice with ethyl acetate, and an extract was washed with a saturated aqueous solution of sodium sulfite. The extract was dried on anhydrous magnesium sulfate, and subsequently purified on column chromatography to yield 2.25 g as a colorless powder having the following physical property.
Melting point: 102 to 105°C.

### Example 6: Preparation of 3-{[3-(4-tolyl)-5-isoxazolyl]methyl}-1-phenyl urea (Compound Number II-2)

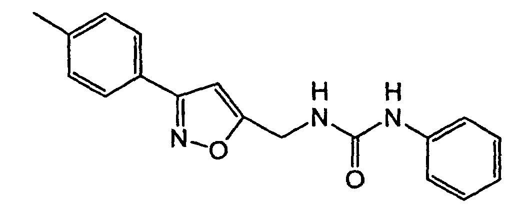

To a solution of 4.66 g of 4-methyl benzaldoxime and 1.50 g of 3-(2-propynyl)-1-phenyl urea in 50 ml of dichloromethane, 36.6 g of 7.0% sodium hypochlorite aqueous solution was dripped over 1 hour at 0°C. The temperature was raised to room temperature, and then the solution was stirred for 7 hours. Water was added, the solution was extracted twice with ethyl acetate, and an extract was washed with a saturated aqueous solution of sodium sulfite. The extract was dried on anhydrous magnesium sulfate, and subsequently purified on column chromatography to yield 1.87 g as a colorless powder having the following physical property.
Melting point: 176 to 178°C.

### Example 7: Preparation of N-{[3-(4-methoxyphenyl)-5-isoxazolyl]methyl}-4-methoxy benzamide (Compound Number III-18)

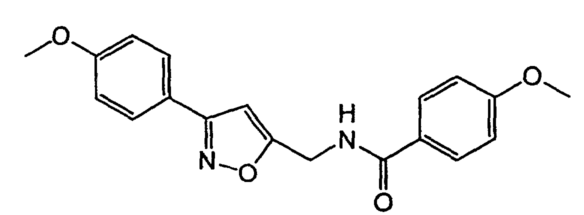

To a solution of 4.90 g of 4-metoxy benzaldoxime and 1.50 g of N-(2-propynyl)-1-p-methoxy benzamide in 50 ml of dichloromethane, 37.7 g of 6.4% sodium hypochlorite aqueous solution was dripped over 1 hour at 0°C. The temperature was raised to room temperature, and then the solution was stirred overnight. Water was added, the solution was extracted twice with ethyl acetate, and an extract was washed with a saturated aqueous solution of sodium sulfite. The extract was dried on anhydrous magnesium sulfate, and subsequently purified on column chromatography to yield 2.36 g as a colorless powder having the following physical property.
Melting point: 144 to 155°C.

### Example 8: Preparation of N-{[3-(2-methylbenzyl)-5-isoxazolyl]methyl}-4-methyl benzamide (Compound Number IV-12)

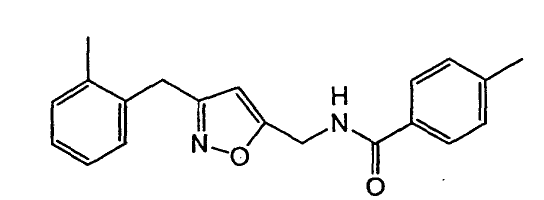

To a solution of 7.75 g of 2-methyl benzylaldoxime and 1.50 g of N-(2-propynyl)-1-p-methyl benzamide in 70 ml of dichloromethane, 87.9 g of 4.4% sodium hypochlorite aqueous solution was dripped over 30 minutesat 0°C. The temperature was raised to room temperature, and then the solution was stirred for 2 hours. Water was added, the solution was extracted twice with ethyl acetate, and an extract was washed with a saturated aqueous solution of sodium sulfite. The extract was dried on anhydrous magnesium sulfate, and subsequently purified on column chromatography to yield 2.12 g as a colorless oil having the following physical property.
NMR : δ 2.27(3H,s), 2.36(3H,s), 3.93(2H,s), 4.62(2H,d), 5.83(1H,s), 6.48-6.78(1H,brs), 7.13(4H,s), 7.20(2H,d), 7.67(2H,d)

### Example 9: Preparation of N-[(3-isopropyl-5-isoxazolyl)methyl]-4-methyl benzamide (Compound Number III-5)

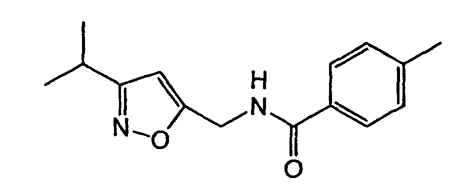

To a solution of 7.54 g of isopropylaldoxime and 1.50 g of N-(2-propynyl)-1-p-methyl benzamide in 100 ml of dichloromethane, 161.2 g of 4.0% sodium hypochlorite aqueous solution was dripped over 30 minutesat 0°C. The temperature was raised to room temperature, and then the solution was stirred overnight. Water was added, the solution was extracted twice with ethyl acetate, and an extract was washed with a saturated aqueous solution of sodium sulfite. The extract was dried on anhydrous magnesium sulfate, and subsequently purified on column chromatography to yield 0.56 g of a colorless oil having the following physical property.
Melting point: 107 to 116°C.
NMR : δ 1.25(6H,d), 2.38(3H,s), 2.78-3.24(1H,m), 4.69(2H,d), 6.08(1H,s), 6.85(1H,br), 7.21(2H,d), 7.71(2H,d)

### Example 10: Preparation of N-[(3-phenyloxymethyl-5-isoxazolyl)methyl]-4-methoxy benzamide (Compound Number IV-45)

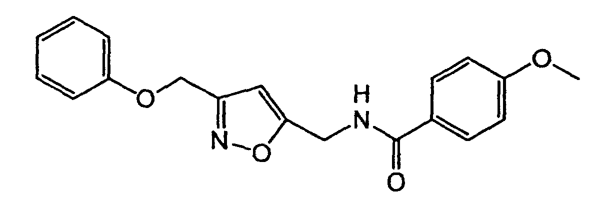

To a solution of 5.99 g of phenyloxymethylaldoxime and 1.50 g of N-(2-propynyl)-1-p-methoxy benzamide, 65.6 g of 4.5% sodium hypochlorite aqueous solution was dripped over 30 minutesat 0°C. The temperature was raised to room temperature, and then the solution was stirred overnight. Water was added, the solution was extracted twice with ethyl acetate, and an extract was washed with a saturated aqueous solution of sodium sulfite. The extract was dried on anhydrous magnesium sulfate, and subsequently purified on column chromatography to yield 1.00 g as a colorless paste having the following physical property.
NMR : δ 3.82(3H,s), 4.72(2H,d), 5.12(2H,s), 6.34(1H,s), 6.41-6.65(1H,brs), 6.75-7.88(9H,m)

### Example 11: Preparation of 3-[(3-phenyl-5-isoxazolyl)methyl]-1-cyclohexyl urea (Compound Number II-7)

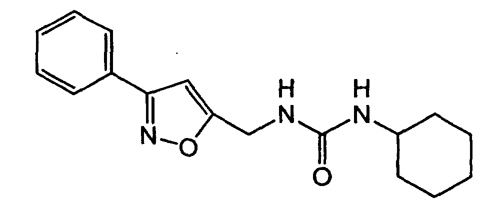

To a solution of 1.00 g of 5-aminomethyl-3-phenylisoxazole in 30 ml of ethanol, 0.79 g of cyclohexylisocyanate was added at room temperature, and then the whole was stirred overnight. The reaction solution was concentrated under reduced pressure, and subsequently a precipitated crystal was filtrated and washed with isopropylether to yield 1.65 g as a colorless crystal having the following physical property.
Melting point: 155 to 158°C.

### Example 12: Preparation of phenyl {[3-(2-pyrizyl)-5-isoxazolyl]methyl}carbamate (Compound Number I-31)

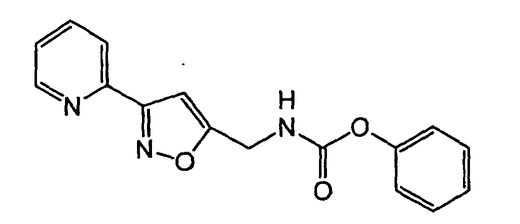

To a solution of 1.00 g of 5-aminomethyl-3-(2-pyrizyl)isoxazole and 0.89 g of diisopropylethylamine in 30 ml of dichloromethane, 0.89 g of phenyl chloroformate was added at 0°C, the temperature was raised to room temperature, and subsequently the solution was stirred for 1 hour. After the completion of the reaction, the solution was concentrated under reduced pressure, and subsequently purified on silica gel column chromatography to yield 1.62 g as a colorless crystal having the following physical property.
Melting point: 104 to 107°C.

### Example 13: Preparation of N-[(3-phenyl-5-isoxazalyl)methyl]-1-pyrazolylamide (Compound Number III-192)

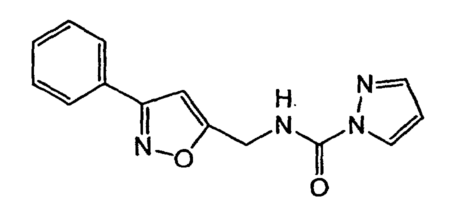

To a solution of 0.78 g of pyrazole in 30 ml of tetrahydrofuran, 0.93 of N,N'-carbonyl diimidazole was added at room temperature, and the whole was stirred for one hour. Subsequently, 1.00 g of 5-aminomethyl-3-phenylisoxazole was added to the reaction solution, and the whole was stirred overnight. After the completion of the reaction, the reaction solution was concentrated under reduced pressure, and purified on silica gel column chromatography to yield 1.51 g as a colorless crystal having the following physical property.
Melting point: 137 to 138°C.

### Example 14: Preparation of 3-[(3-phenyl-5-isoxazolyl)methyl]-1-(4-pyridyl) urea (Compound Number II-101)

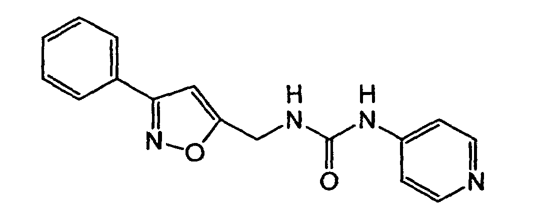

To a solution of 0.65 g of 4-aminopyridine in 30 ml of tetrahydrofuran, 0.93 of N,N'-carbonyl diimidazole was added at room temperature, and the whole was stirred for one hour. Subsequently, 1.00 g of 5-aminomethyl-3-phenylisoxazole was added to the reaction solution, and the whole was stirred overnight. After the completion of the reaction, the reaction solution was concentrated under reduced pressure, and purified on silica gel column chromatography to yield 1.62 g as a colorless crystal having the following physical property.
Melting point: 156 to 162°C.

### Example 15: Preparation of N-[(3-chloromethyl-5-isoxazolyl)methyl]-4-methoxy benzamide (Compound Number III-3)

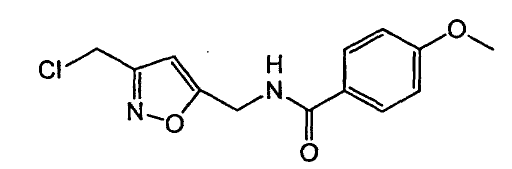

To a solution of 47.7 g of chloromethylaldoxime and 6.43 g of N-(2-propynyl)-1-p-methoxy benzamide in 150 ml of dichloromethane, 380 g of 10% sodium hypochlorite aqueous solution was dripped over 1 hour at 0°C. The temperature was raised to room temperature, and then the solution was stirred overnight. Water was added, the solution was extracted twice with ethyl acetate, and an extract was washed with a saturated aqueous solution of sodium sulfite. The extract was dried on anhydrous magnesium sulfate, and subsequently purified on column chromatography to yield 5.83 g as a colorless solid having the following physical property.
Melting point: 93 to 110°C.
NMR : δ 3.86(3H,s), 4.53(2H,s), 4.72(2H,d), 6.31(1H,s), 6.69(1H,brs), 6.92(2H,d), 7.75(2H,d)

### Example 16: Preparation of N-{[3-(2-methylphenyl)oxymethyl-5-isoxazolyl]methyl}-4-methoxy benzamide (Compound Number IV-47)

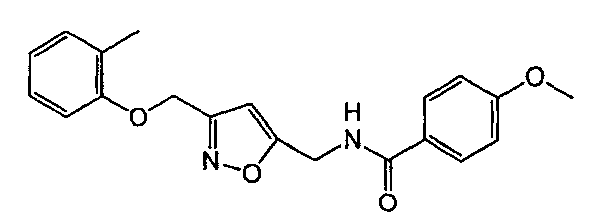

To a solution of 1.20 g of N-[(3-chloromethyl-5-isoxazolyl)methyl]-4-methoxy benzamide and 1.85 g of 2-methylphenol in 30 ml of dimethylformamide, 0.89 g of potassium carbonate was added at room temperature. The temperature was raised to 100°C, and the solution was stirred for 5 hours. Water was added, the solution was extracted twice with ethyl acetate, and a collected organic layer was washed with water and saturated sodium chloride solution. The organic layer was dried on anhydrous magnesium sulfate, and then purified on column chromatography to yield 0.65 g as a colorless solid having the following physical property.
Melting point: 115 to 117°C
NMR : δ 2.20(3H,s), 3.80(3H,s), 4.68(2H,d), 5.08(2H,s), 6.30(1H,s), 6.61-7.82(9H,m)

### Example 17

According to the methods described in Examples 1 to 16, the compounds in the following Examples 17(1) to 17(115) were obtained.

### Example 17(1): Production of [(3-isopropyl-5-isoxazolyl)methyl]phenylcarbamate (Compound number I-4):

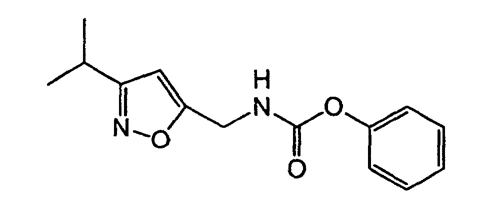

Property: oily;
NMR : δ 1.28(6H, d), 2.75-3.29(1H, m), 4.52(2H, d), 5.34-5.72(1H, brs), 6.12(1H, s), 7.00-7.48(5H, m)

### Example 17(2): Preparation of phenyl [(3-cyclohexyl-5-isoxazolyl)methyl] carbamate (Compound number I-6):

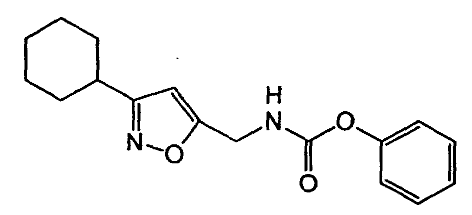

Property: paste;
NMR : δ 1.08-2.10(10H, m), 2.48-2.91(1H, brs), 4.42(2H, d), 5.80-6.00(1H, brs), 6.02(1H, s), 6.98-7.48(5H, m)

### Example 17(3): Preparation of phenyl {[3-(4-tolyl)-5-isoxazolyl)methyl] carbamate (Compound number I-25):

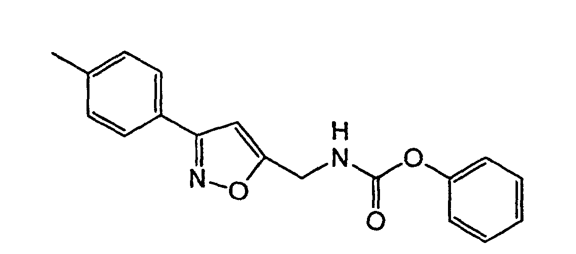

Melting point: 119 to 123°C.

### Example 17(4): Preparation of phenyl {[3-(4-fluorophenyl)-5-isoxazolyl)methyl] carbamate (Compound number I-27):

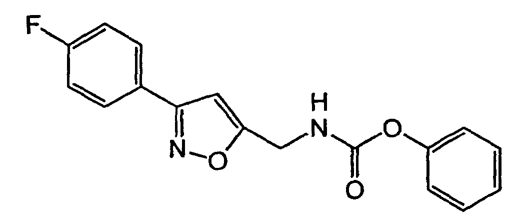

Melting point: 114 to 116°C.

### Example 17(5): Preparation of phenyl {[3-(3-chlorophenyl)-5-isoxazolyl)methyl] carbamate (Compound number I-29):

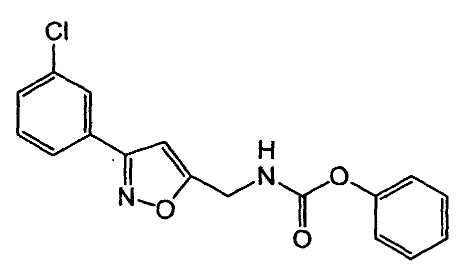

Melting point: 103 to 105°C.

### Example 17(6): Preparation of phenyl {[3-(4-chlorophenyl)-5-isoxazolyl)methyl] carbamate (Compound number I-30):

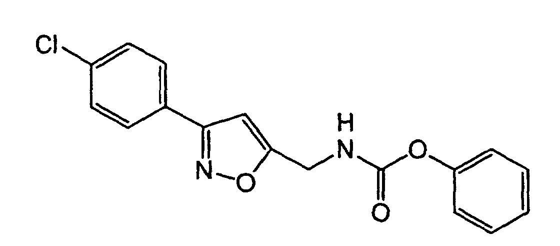

Melting point: 135 to 137°C.

### Example 17(7): Preparation of phenyl {[3-(2-thienyl)-5-isoxazolyl)methyl] carbamate (Compound number I-32):

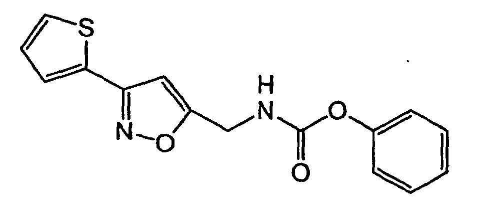

Melting point: 104 to 107°C.

### Example 17(8): Preparation of phenyl {[3-(2-furyl)-5-isoxazolyl)methyl] carbamate (Compound number I-33):

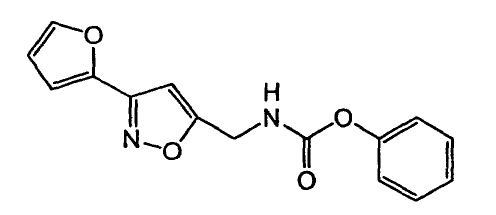

Melting point: 89 to 92°C.

### Example 17(9): Preparation of phenyl [(3-benzyl-5-isoxazolyl)methyl] carbamate (Compound number I-38):

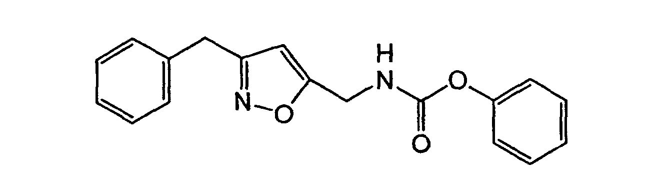

Melting point: 135 to 137°C.

### Example 17(10): Preparation of benzyl [(3-phenyl-5-isoxazolyl)methyl] carbamate (Compound number I-41):

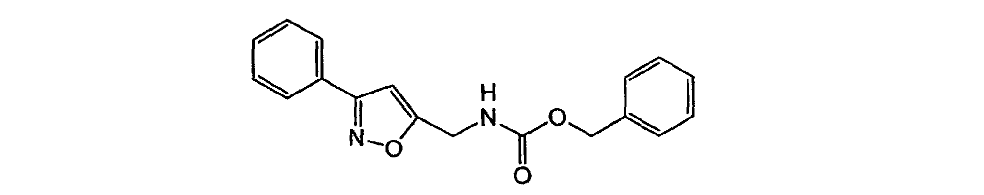

Melting point: 104 to 105°C.

### Example 17(11): Preparation of [(3-phenyl-5-isoxazolyl)methyl]2-propynyl carbamate (Compound number I-42) :

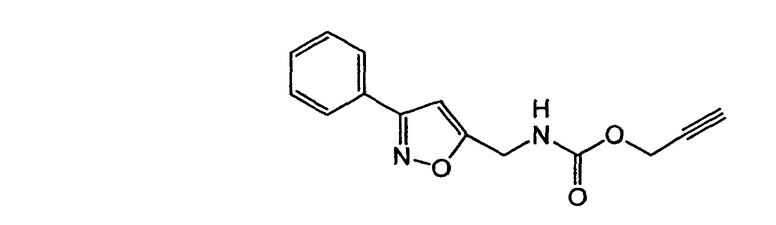

Melting point: 102 to 104°C.

### Example 17(12): Preparation of 3-[(3-phenyl-5-isoxazolyl)methyl]-1-(methyl-1-phenylethyl)urea (Compound number I-45):

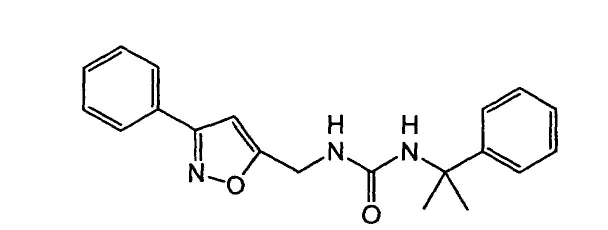

Melting point: 160 to 164°C.

### Example 17(13): Preparation of N-[(3-phenyl-5-isoxazolyl)methyl]-2-phenylpropanamide (Compound number I-46) :

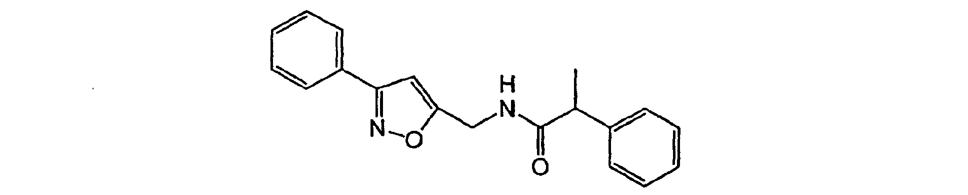

Melting point: 110 to 112°C.

### Example 17(14): Preparation of N-[(3-isopropyl-5-isoxazolyl)methyl]-2-phenylpropanamide (Compound number I-47) :

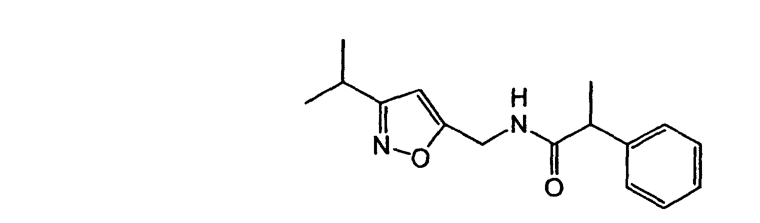

Property: oily;
NMR : δ 0.80-1.66(9H, m), 2.67-3.14(1H, m), 3.27-3.81(1H, m), 4.40(2H, d), 5.80(1H,s), 6.22-6.51(1H, br), 7.26(5H, s)

### Example 17(15): Preparation of N-[(3-phenyl-5-isoxazolyl)methyl]-2-phenylethanamide (Compound number I-48):

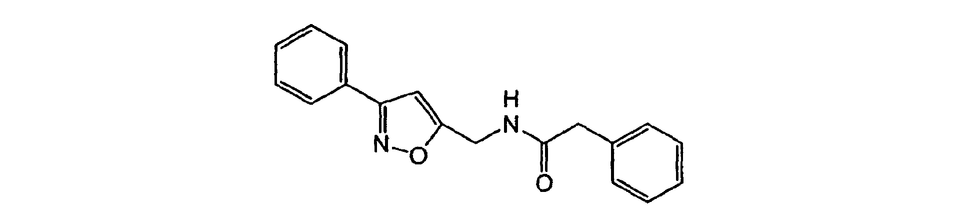

Melting point: 153 to 154°C.

### Example 17(16): Preparation of N-[(3-phenyl-5-isoxazolyl)methyl]-2-chloro-2-phenylethanamide (Compound number I-49):

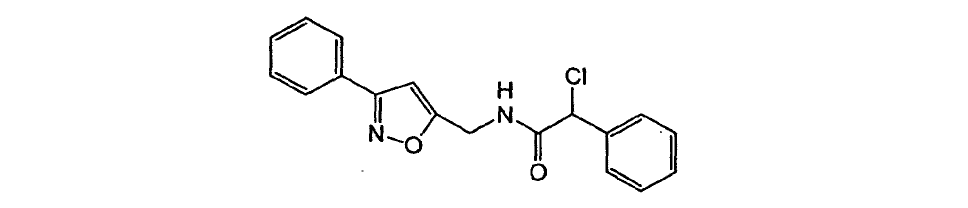

Melting point: 121 to 125°C.

### Example 17(17): Preparation of 1-[(3-isopropyl-5-isoxazolyl)methyl]-3-phenylurea (Compound number II-3):

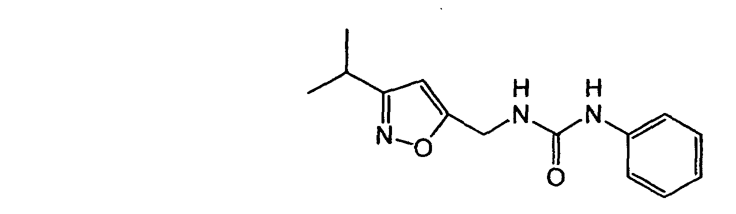

Property: paste;
NMR : δ 1.08(6H, d), 2.69-3.14(1H, m), 4.33(2H, d), 5.98(1H, s), 6.22-6.44(1H, brs), 6.86-7.31(5H, m), 7.92(1H, d)

### Example 17(18): Preparation of 1-[(3-isopropyl-5-isoxazolyl)methyl]-3-phenyl-3-methylurea (Compound number II-4):

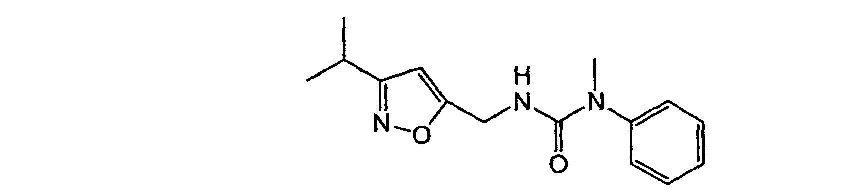

Property: oily;
NMR : δ 1.24(6H, d), 2.72-3.21(1H, m), 4.42(2H, d), 4.77-5.04(1H, brs), 5.99(1H, s), 7.10-7.52(5H, m)

### Example 17(19): Preparation of 1-{[3-(4-tolyl)-5-isoxazolyl]methyl}-3-cyclohexylurea (Compound number II-9):

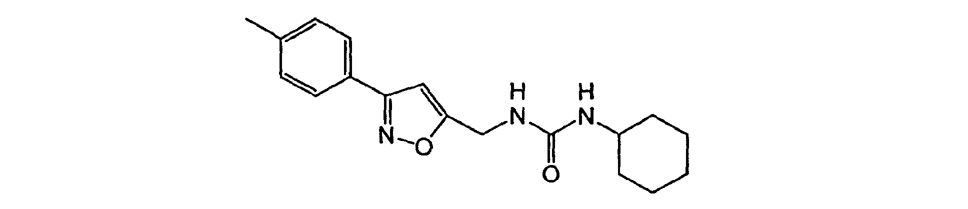

Melting point: 163 to 170°C.

### Example 17(20): Preparation of 1-[(3-benzyl-5-isoxazolyl)methyl]-3-cyclohexylurea (Compound number II-13):

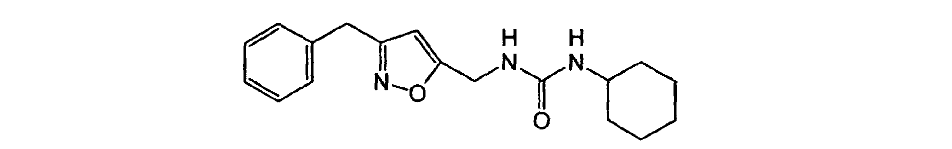

Property: oily;
NMR : δ 0.80-2.05(10H, m), 3.22-3.68(1H, brs), 3.92(2H, s), 4.35(2H, d), 5.23(1H, brt), 7.20(5H, brs)

### Example 17(21): Preparation of 1-[(3-benzyl-5-isoxazolyl)methyl]-3-cyclopentylurea (Compound number II-14) :

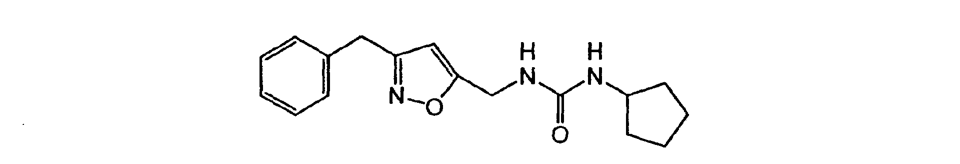

Melting point: 125 to 129°C.

### Example 17(22): Preparation of 1-[(3-isopropyl-5-isoxazolyl)methyl]-3-cyclohexylurea (Compound number II-15):

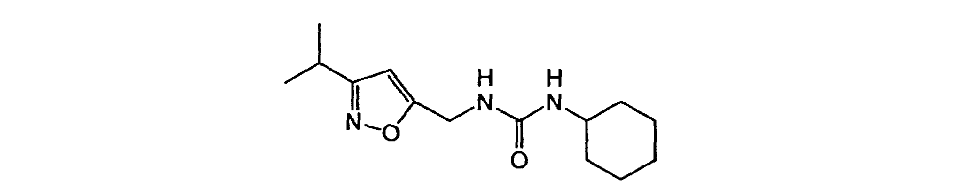

Property: oily;
NMR : δ 0.48-2.08(16H, m), 3.28-3.63(2H, m), 4.35-4.60(1H, brs), 4.43(2H, d), 4.90(1H, Brt), 6.12(1H, s)

### Example 17(23): Preparation of 1-[(3-phenyl-5-isoxazolyl)methyl]-3-(2-chlorophenyl)urea (Compound number II-48) :

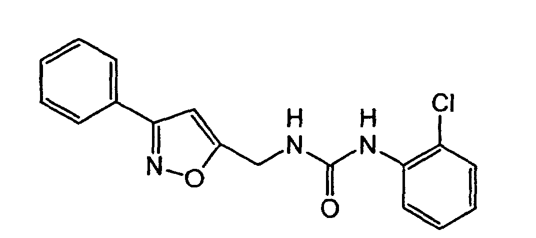

Melting point: 166 to 169°C.

### Example 17(24): Preparation of 1-[(3-phenyl-5-isoxazolyl)methyl]-3-(3-chlorophenyl)urea (Compound number II-49):

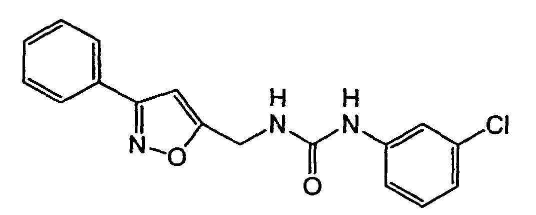

NMR : δ 4.58(2H, d) 6.51(1H, s) 6.82-7.86(10H, m) 8.08(1H, brs).

### Example 17(25): Preparation of 1-[(3-phenyl-5-isoxazolyl)methyl]-3-(4-chlorophenyl)urea (Compound number II-50):

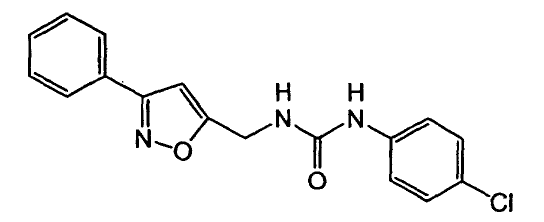

Melting point: 212 to 219°C.

### Example 17(26): Preparation of 1-[(3-phenyl-5-isoxazolyl)methyl]-3-(2-pyridyl)urea (Compound number II-99) :

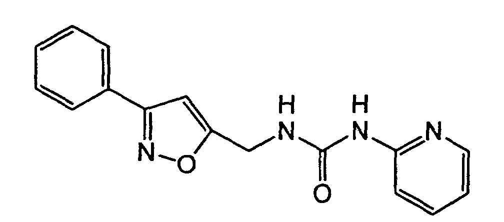

M.P.: 211 to 212°C.

### Example 17(27): Preparation of 1-[(3-phenyl-5-isoxazolyl)methyl]-3-(3-pyridyl)urea (Compound number II-100) :

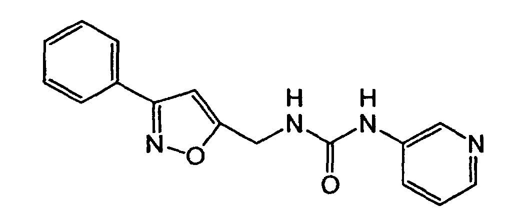

Melting point: 186 to 188°C.

### Example 17(28): Preparation of N-[(3-methyl-5-isoxazolyl)methyl]benzamide (Compound number III-1):

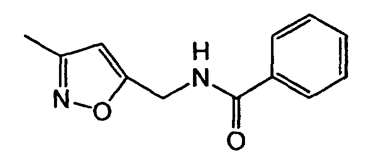

Melting point: 106 to 107°C.

### Example 17(29): Preparation of N-[(3-chloromethyl-5-isoxazolyl)methyl]-4-methylbenzamide (Compound number III-2) :

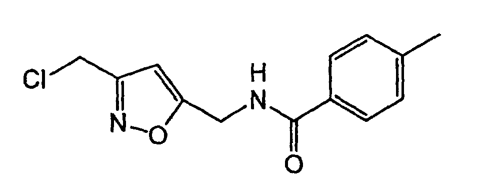

Property: paste;
NMR : δ 2.39(3H, s), 4.53(2H, s), 4.72(2H, d), 6.32(1H, s), 6.63-6.92(1H, brs), 7.21(2H, d), 7.68(2H, d)

### Example 17(30): Preparation of N-[(3-isopropyl-5-isoxazolyl)methyl]benzamide (Compound number III-4):

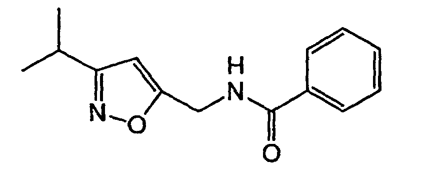

Property: oily;
NMR : δ 1.25(6H, d), 2.80-3.27(1H, m), 4.71(2H, d), 6.09(1H, s), 6.66(1H, brs), 7,28-7.88(5H, m)

### Example 17(31): Preparation of N-[(3-cyclohexyl-5-isoxazolyl)methyl]benzamide (Compound number III-6):

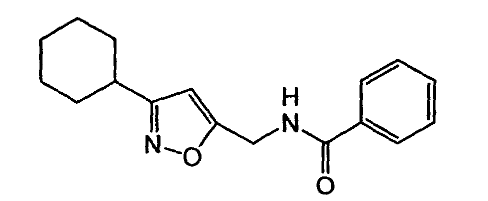

Melting point: 89 to 93°C.

### Example 17(32): Preparation of N-{[3-(4-tolyl)-5-isoxazolyl]methyl}benzamide (Compound number III-10):

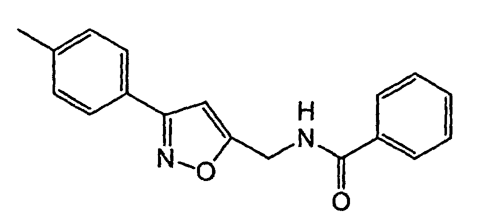

Melting point: 154 to 156°C.

### Example 17(33): Preparation of N-{[3-(4-tolyl)-5-isoxazolyl]methyl}-4-methylbenzamide (Compound number III-11) :

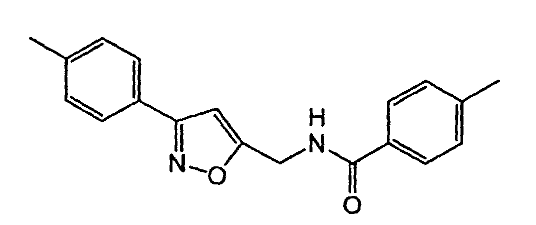

Melting point: 164 to 166°C.

### Example 17(34): Preparation of N-{[3-(4-tolyl)-5-isoxazolyl]methyl}-4-methoxybenzamide (Compound number III-12) :

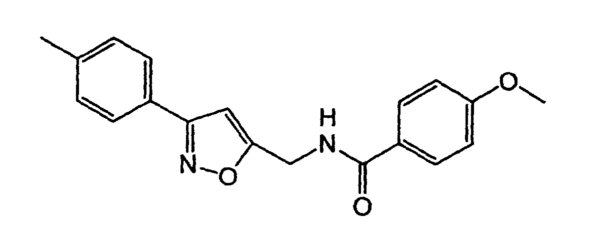

Melting point: 174 to 175°C.

### Example 17(35): Preparation of N-{[3-(4-ethylphenyl)-5-isoxazolyl]methyl}-4-methoxybenzamide (Compound number III-13) :

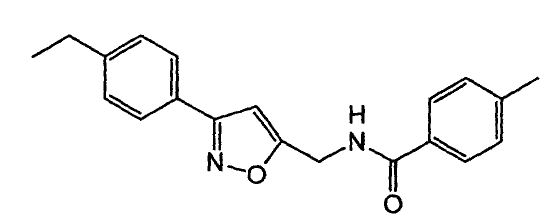

Melting point: 150 to 152°C.

### Example 17(36): Preparation of N-{[3-(4-t-butylphenyl)-5-isoxazolyl]methyl}-4-methoxybenzamide (Compound number III-14):

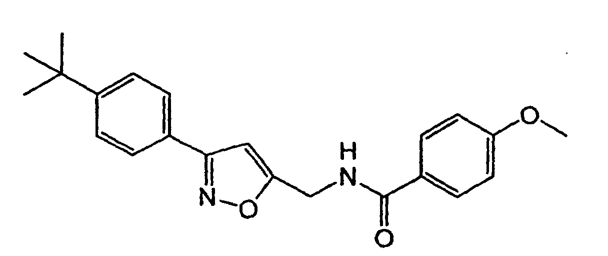

Melting point: 179 to 180°C.

### Example 17(37): Preparation of N-{[3-(4-methoxyphenyl)-5-isoxazolyl]methyl}benzamide (Compound number III-17):

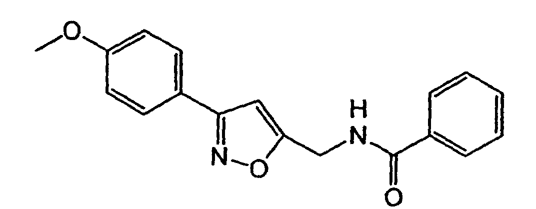

Melting point: 125 to 127°C.

### Example 17(38): Preparation of N-{[3-(4-methoxyphenyl)-5-isoxazolyl]methyl}-4-methylbenzamide (Compound number III-19) :

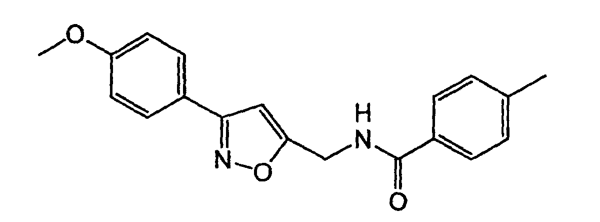

Melting point: 139 to 142°C;
NMR : δ 2.37(3H, s), 3.81(3H, s), 4.75(2H, d), 6.43(1H, s), 6.69(1H, brs), 6.82-7.83(8H, m)

### Example 17(39): Preparation of N-{[3-(4-mesylphenyl)-5-isoxazolyl]methyl}-4-methylbenzamide (Compound number III-26) :

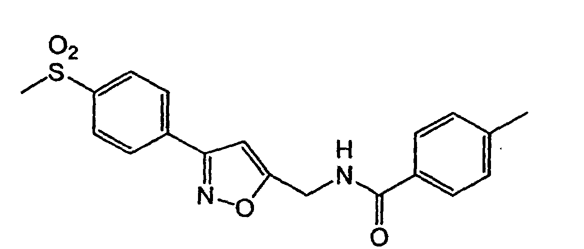

NMR : δ 2.37(3H, s), 3.08(3H, s), 4.78 (2H, d), 6.61(1H, s), 7.09-8.08(9H, m)

### Example 17(40): Preparation of N-{[3-(4-methanesulfinylphenyl)-5-isoxazolyl]methyl}-4-methylbenzamide (Compound number III-27):

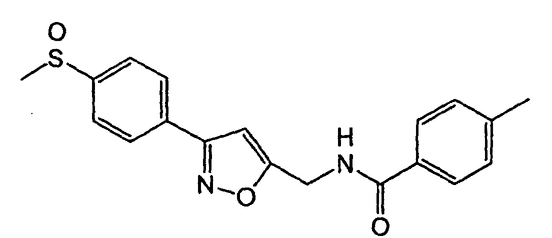

NMR : δ 2.37(3H, s), 2.72(3H, s), 4.78(2H, d), 5.99(1H, s), 7.09-8.08(9H, m)

### Example 17(41): Preparation of N-{[3-(4-methylthiophenyl)-5-isoxazolyl]methyl}-4-methylbenzamide (Compound number III-28) :

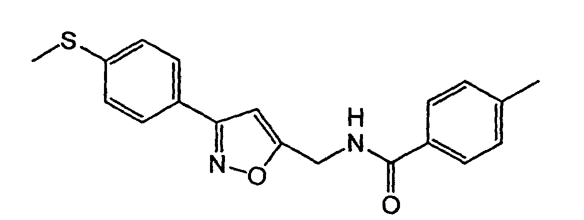

Melting point: 135 to 155°C.

### Example 17(42): Preparation of N-{[3-(4-fluorophenyl)-5-isoxazolyl]methyl}-4-methylbenzamide (Compound number III-31) :

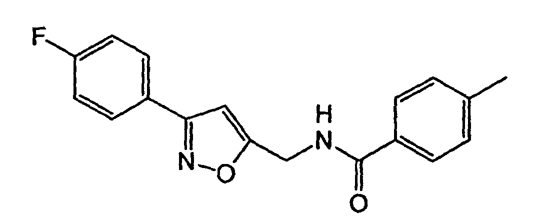

Melting point: 167 to 168°C;
NMR : δ 2.67(3H, s), 4.78(2H, d), 6.50(1H, s), 6.60-7.93(9H, m)ₒ

### Example 17(43): Preparation of N-{[3-(4-chlorophenyl)-5-isoxazolyl]methyl}benzamide (Compound number III-34):

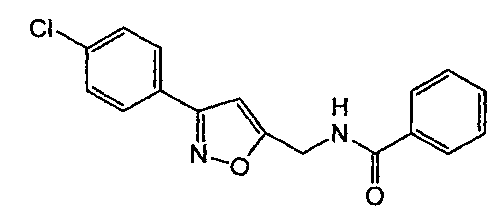

Melting point: 172 to 173°C.

### Example 17(44): Preparation of N-{[3-(4-bromophenyl)-5-isoxazolyl]methyl}benzamide (Compound number III-37):

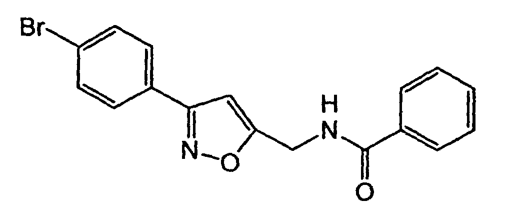

Melting point: 184 to 187°C.

### Example 17(45): Preparation of N-{[3-(4-trifluoromethylphenyl)-5-isoxazolyl]methyl}benzamide (Compound number III-40):

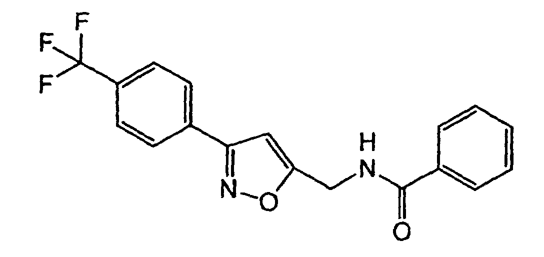

Melting point: 179 to 180°C.

### Example 17(46): Preparation of N-{[3-(4-cyanophenyl)-5-isoxazolyl]methyl}-4-methylbenzamide (Compound number III-43):

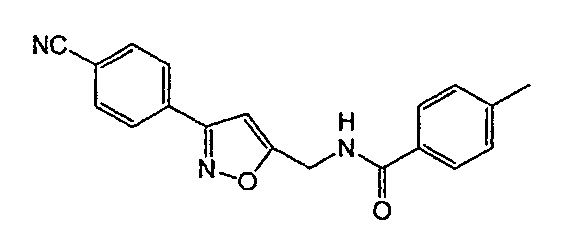

NMR : δ 2.33(3H, s), 4.79(2H, d), 6.44-6.85(1H, br), 6.59(1H, s), 7.10-8.87(8H, m)

### Example 17(47): Preparation of N-{[3-(4-trifluoromethoxyphenyl)-5-isoxazolyl]methyl}-4-methoxybenzamide (Compound number III-49):

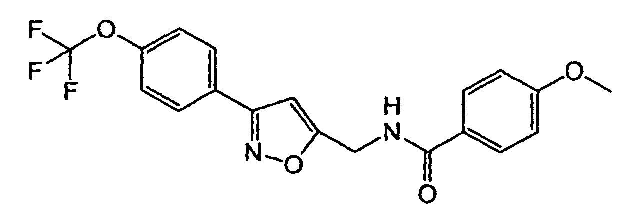

Melting point: 188 to 191°C.

### Example 17(48): Preparation of N-{[3-(4-carboxyphenyl)-5-isoxazolyl]methyl}-4-methylbenzamide (Compound number III-51):

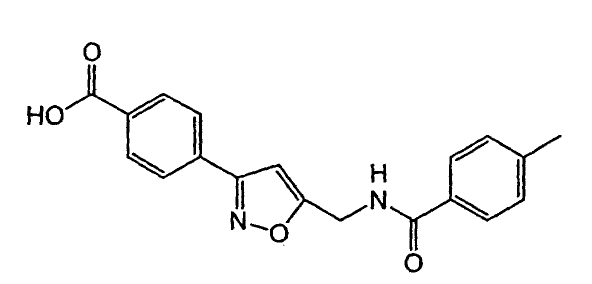

Melting point: 248°C or more (degradation).

### Example 17(49): Preparation of N-{[3-(4-methoxycarbonylphenyl)-5-isoxazolyl]methyl}-4-methylbenzamide (Compound number III-52):

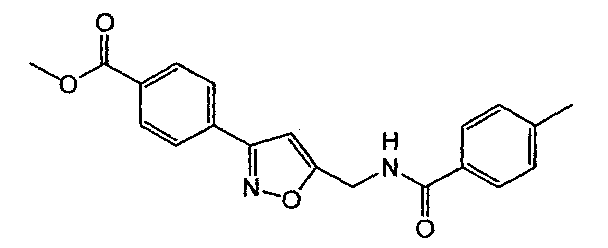

Melting point: 173 to 175°C;
NMR : δ 2.34(3H, s), 3.92(3H, d), 4.76(2H, d), 6.53(1H, s), 6.92-8.15(9H, m).

### Example 17(50): Preparation of N-{[3-(4-methylaminocarbonylphenyl)-5-isoxazolyl]methyl}-4-methylbenzamide (Compound number III-54):

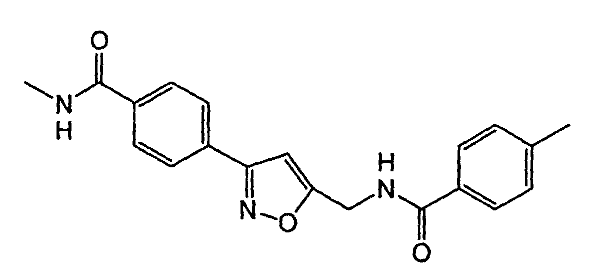

Melting point: 261 to 262°C.

### Example 17(51): Preparation of N-{[3-(4-acetamidophenyl)-5-isoxazolyl]methyl}-4-methylbenzamide (Compound number III-57) :

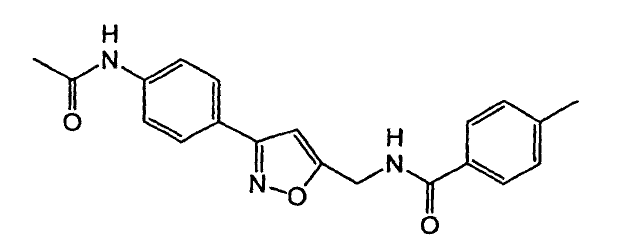

Melting point: > 200°C.

### Example 17(52): Preparation of N-{[3-(4-dimethylaminophenyl)-5-isoxazolyl]methyl}-4-methylbenzamide (Compound number III-61):

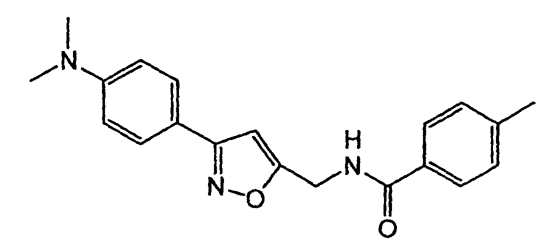

NMR : δ 2.33(3H, s), 2.99(6H, s), 4.74(2H, d), 6.46(1H, s), 6.53-8.07(9H, m)

### Example 17(53): Preparation of N-{[3-(2,4-dimethylphenyl)-5-isoxazolyl]methyl}-4-methylbenzamide (Compound number III-65) :

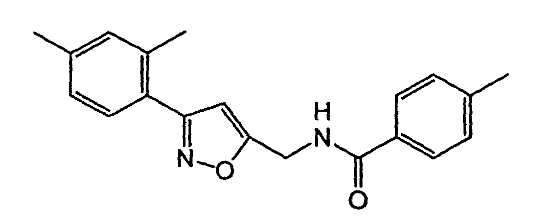

Melting point: 138 to 140°C.

### Example 17(54): Preparation of N-{[3-(2,4-dimethoxyphenyl)-5-isoxazolyl]methyl}-4-methylbenzamide (Compound number III-66) :

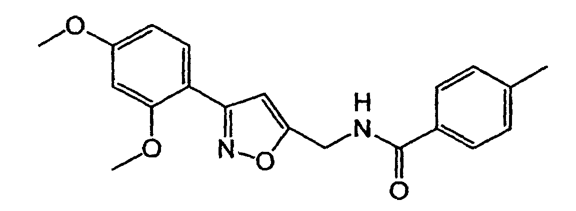

Melting point: 157 to 158°C.

### Example 17(55): Preparation of N-{[3-(3,4-dimethoxyphenyl)-5-isoxazolyl]methyl}benzamide (Compound number III-67):

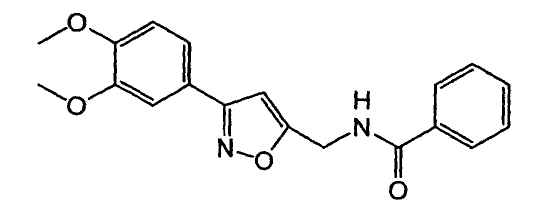

Melting point: 122 to 130°C.

### Example 17(56): Preparation of N-{[3-(3,4-dimethoxyphenyl)-5-isoxazolyl]methyl}-4-methylbenzamide (Compound number III-68) :

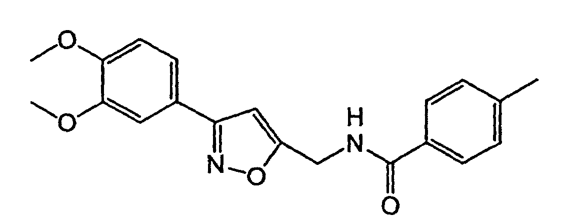

Melting point: 160 to 162°C;
NMR : δ 2.32 (3H, s), 3.82(6H, s), 4.72(2H, d), 6.43(1H, s), 6.72-7.83(8H, m)

### Example 17(57): Preparation of N-{[3-(3-fluoro-4-methylphenyl)-5-isoxazolyl]methyl}-4-methylbenzamide (Compound number III-73):

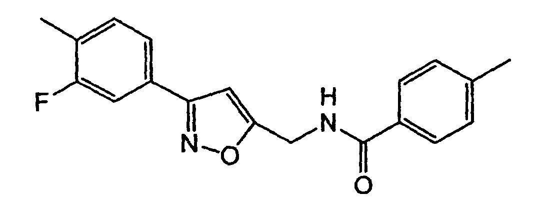

Melting point: 165 to 166°C.

### Example 17(58): Preparation of N-{[3-(2-fluoro-4-methoxyphenyl)-5-isoxazolyl]methyl}-4-methylbenzamide (Compound number III-74):

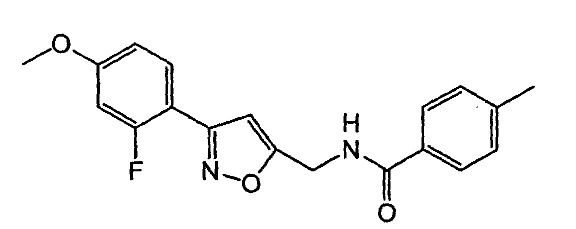

Melting point: 154 to 155°C.

### Example 17(59): Preparation of N-[(3-phenyl-5-isoxazolyl)methyl]-4-methylbenzamide (Compound number III-107):

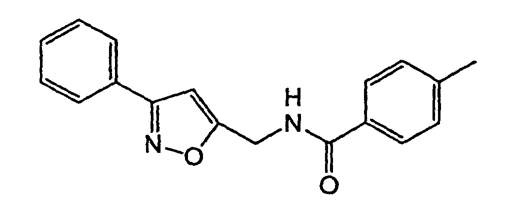

Melting point: 154 to 156°C.

### Example 17 (60) : Preparation of N-[(3-phenyl-5-isoxazolyl)methyl]-4-t-butylbenzamide (Compound number III-108) :

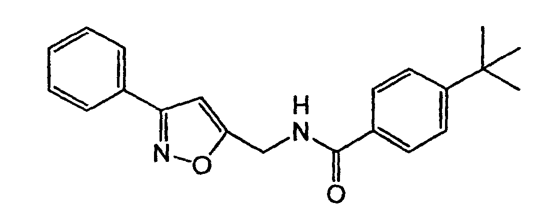

Melting point: 135 to 140°C.

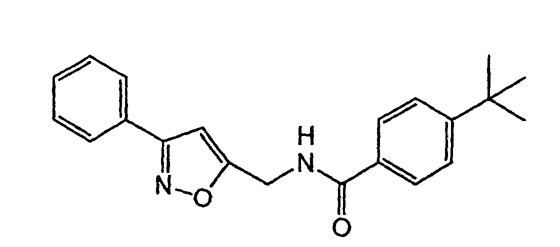

### Example 17(61) : Preparation of N-[(3-phenyl-5-isoxazolyl)methyl]-2-methoxybenzamide (Compound number III-109) :

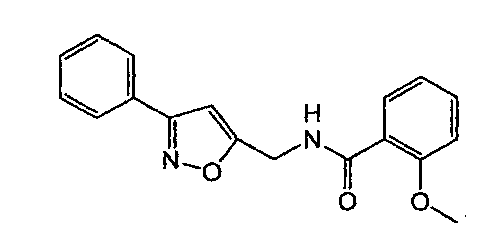

Property: oily;
NMR : δ 4.00(3H, s) 4.81(2H, d) 6.53(1H, s) 6.87-8.55(10H, m)

### Example 17(62): Preparation of N-[(3-phenyl-5-isoxazolyl)methyl]-4-fluorobenzamide (Compound number III-121) :

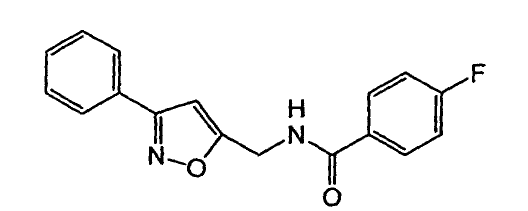

Melting point: 126 to 131°C.

### Example 17(63): Preparation of N-[(3-phenyl-5-isoxazolyl)methyl]-2-chlorobenzamide (Compound number III-122) :

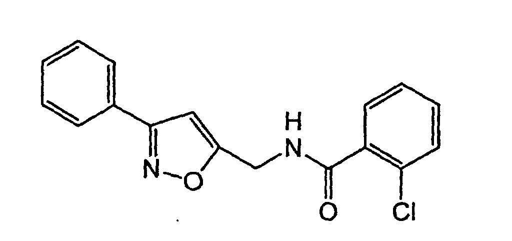

Melting point: 170 to 175°C.

### Example 17(64): Preparation of N-[(3-phenyl-5-isoxazolyl)methyl]-3-chlorobenzamide (Compound number III-123) :

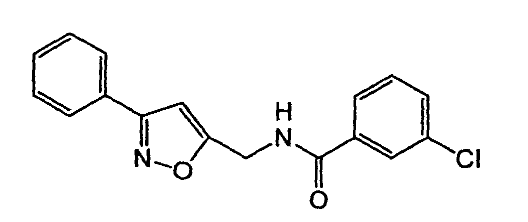

Melting point: 113 to 115°C.

### Example 17(65): Preparation of N-[(3-phenyl-5-isoxazolyl)methyl]-4-chlorobenzamide (Compound number III-124) :

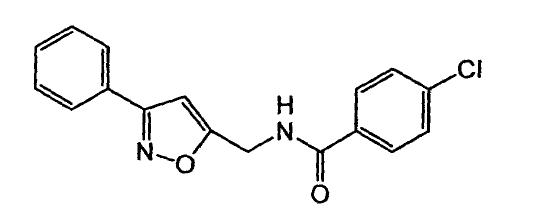

Melting point: 148 to 151°C.

### Example 17(66): Preparation of N-[(3-phenyl-5-isoxazolyl)methyl]-4-trifluoromethylbenzamide (Compound number III-130):

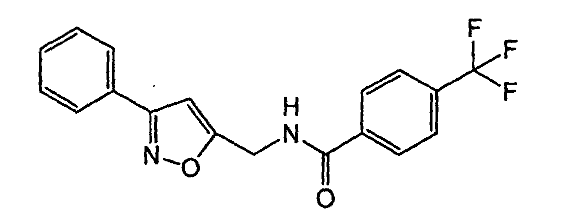

Melting point: 157 to 168°C.

### Example 17(67): Preparation of N-[(3-phenyl-5-isoxazolyl)methyl]-4-cyanobenzamide (Compound number III-133):

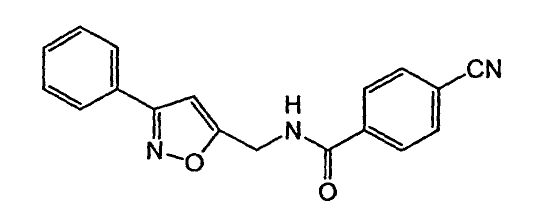

Melting point: 144 to 145°C.

### Example 17(68): Preparation of N-[(3-(4-tolyl)-5-isoxazolyl)methyl]-2-hydroxybenzamide (Compound number III-134) :

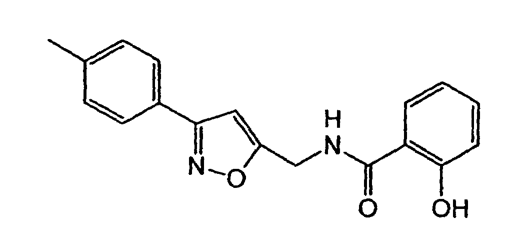

Melting point: 120 to 132°C.

### Example 17(69): Preparation of N-[(3-phenyl-5-isoxazolyl)methyl]-4-nitrobenzamide (Compound number III-137) :

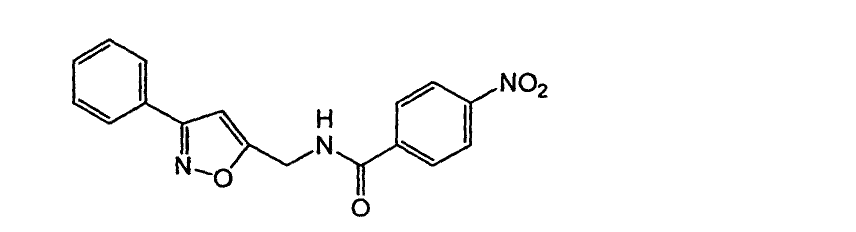

Melting point: 166 to 174°C.

### Example 17(70): Preparation of N-[(3-phenyl-5-isoxazolyl)methyl]-4-trifluoromethoxybenzamide (Compound number III-139):

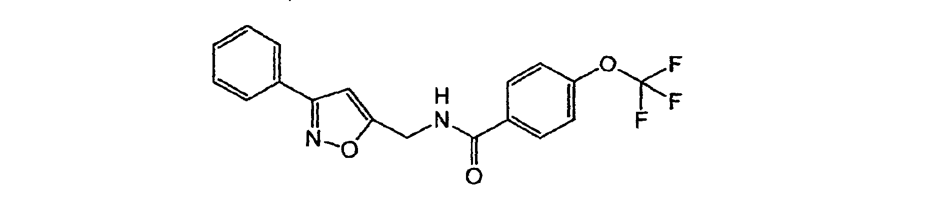

Melting point: 140 to 141°C.

### Example 17(71): Preparation of N-[(3-phenyl-5-isoxazolyl)methyl]-3,4-dimethoxybenzamide (Compound number III-156):

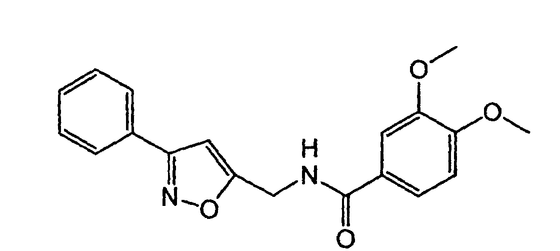

Melting point: 157 to 169°C.

### Example 17(72): Preparation of N-[(3-(4-tolyl)-5-isoxazolyl)methyl]-3-fluoro-4-methylbenzamide (Compound number III-162):

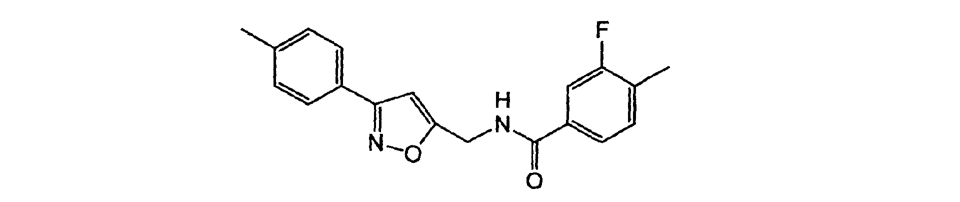

Melting point: 148 to 154°C.

### Example 17(73): Preparation of (3-phenyl-5-isoxazolyl)methylaminocarbonyl-2-pyridine (Compound number III-174):

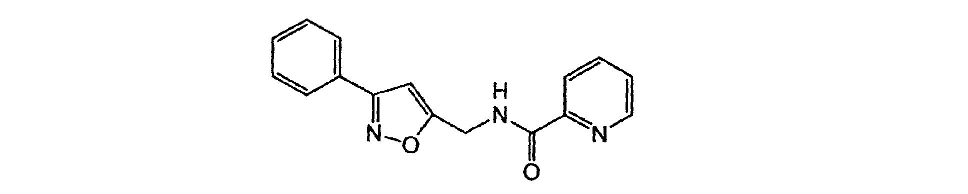

Melting point: 125 to 127°C.

### Example 17(74): Preparation of (3-phenyl-5-isoxazolyl)methylaminocarbonyl-3-pyridine (Compound number III-175):

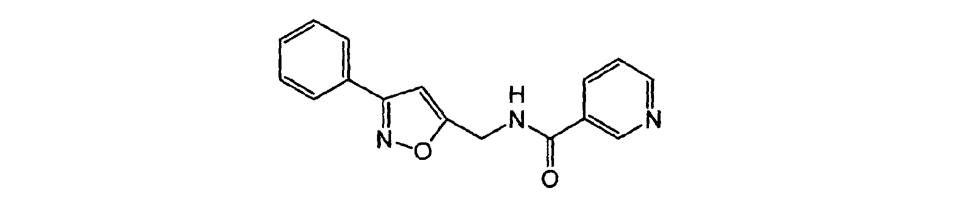

Melting point: 130 to 132°C.

### Example 17(75): Preparation of (3-phenyl-5-isoxazolyl)methylaminocarbonyl-4-pyridine (Compound number III-176):

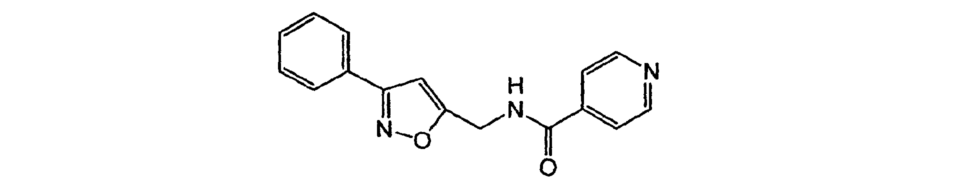

Melting point: 125 to 135°C.

### Example 17(76): Preparation of (3-phenyl-5-isoxazolyl)methylaminocarbonyl-2-thiophene (Compound number III-177):

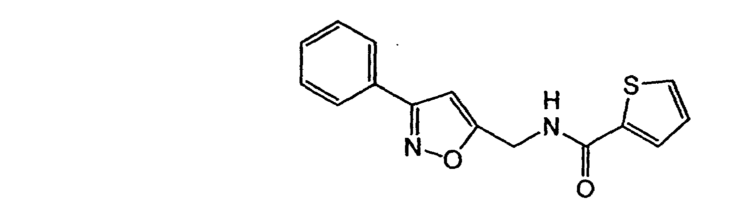

Melting point: 141 to 155°C.

### Example 17(77): Preparation of 5-(3-phenyl-5-isoxazolyl)methylaminocarbonyl-1,3-benzodioxol (Compound number III-180):

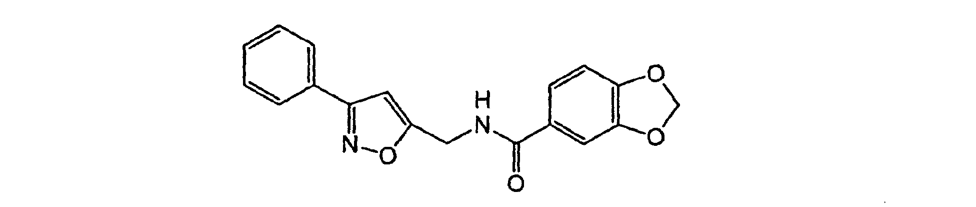

Melting point: 175 to 183°C.

### Example 17(78): Preparation of (E)-N-[(3-phenyl-5-isoxazolyl)methyl]-3-phenylpropenamide (Compound number III-201) :

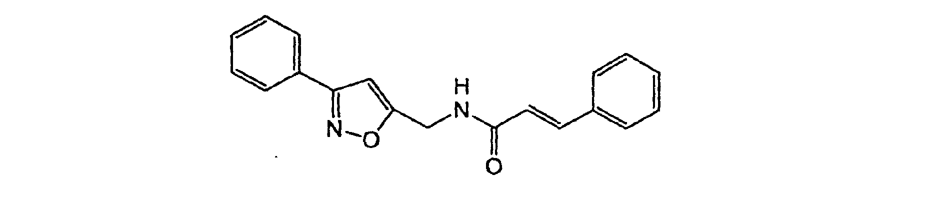

Melting point: 155 to 157°C.

### Example 17(79): Preparation of 1-[(3-phenyl-5-isoxazolyl)methylaminocarbonyl]-2-phenylcyclopropane (Compound number III-202):

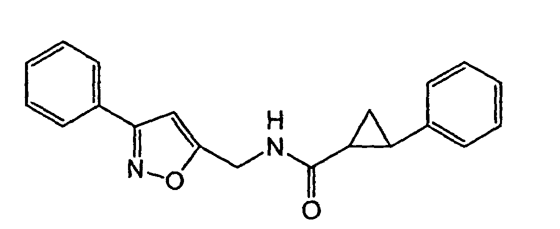

Melting point: 152 to 154°C.

### Example 17(80): Preparation of N-[(3-phenyl-5-isoxazolyl)methylaminocarbonyl]indoline (Compound number III-208):

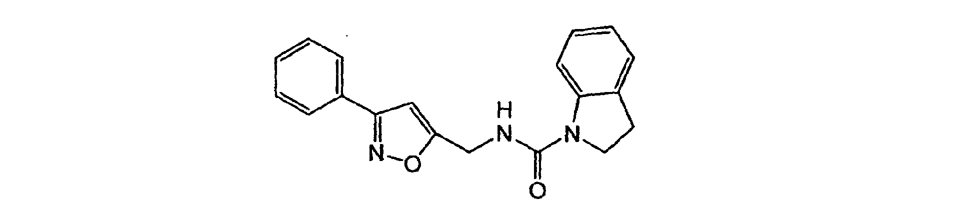

Melting point: 137 to 139°C.

### Example 17(81): Preparation of N-[(3-phenyl-5-isoxazolyl)methyl]-3-methyl-2-butenamide (Compound number III-209):

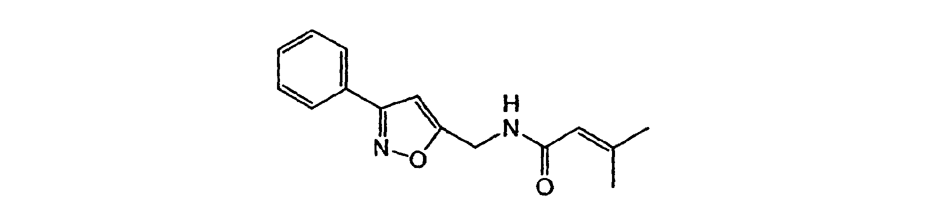

Melting point: 97 to 98°C.

### Example 17(82): Preparation of N-{[3-(2-phenylethenyl)-5-isoxazolyl)methyl]-4-methylbenzamide (Compound number III-210) :

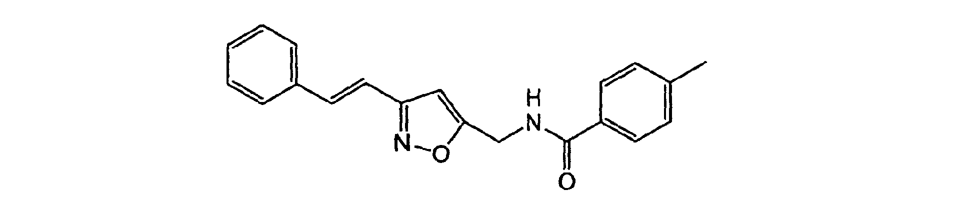

Melting point: 151 to 156°C.

### Example 17(83): Preparation of N-[(3-acetyloxymethyl-5-isoxazolyl)methyl]-4-methoxylbenzamide (Compound number III-211):

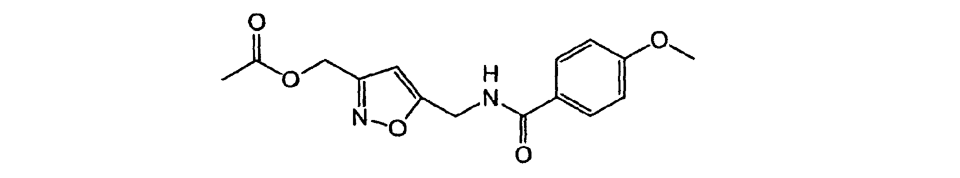

Property: paste;
NMR : δ 2.09(3H, s), 3.80 (3H, s), 4.68(2H, d), 5.09(2H, d), 6.21(1H, s), 6.87(2H, d), 7.19-7.40(1H, brt), 7.78(2H, d)

### Example 17(84): Preparation of N-[(3-benzyl-5-isoxazolyl)methyl]benzamide (Compound number IV-1):

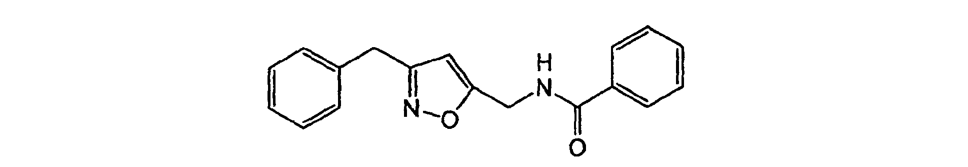

Property: oily;
NMR : δ 3.92(2H, s), 4.61(2H, d), 5.94(1H, s), 6.83-7.88(11H, m)

### Example 17(85): Preparation of N-[(3-benzyl-5-isoxazolyl)methyl]-4-methoxybenzamide (Compound number IV-2) :

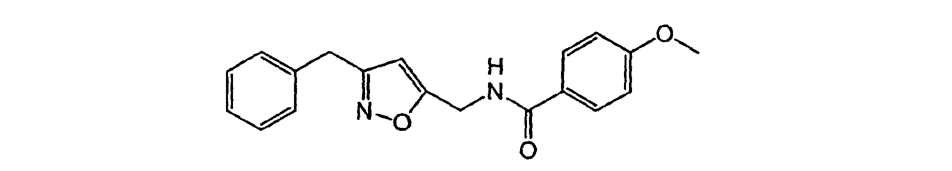

Property: oily;
NMR : δ 3.80(3H, s), 3.93(2H, s), 4.61(2H, d), 5.98(1H, s), 6.86(2H, d), 7.21(5H, s), 7.73(2H, d)

### Example 17(86): Preparation of N-[(3-benzyl-5-isoxazolyl)methyl]-4-methylbenzamide (Compound number IV-3):

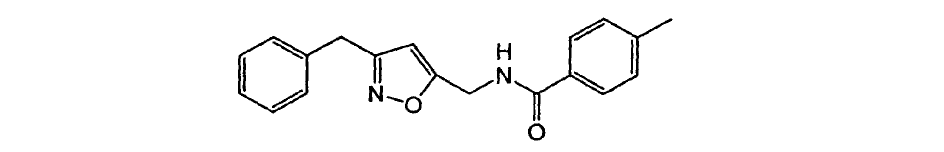

Melting point: 119 to 124°C.

### Example 17(87): Preparation of (3-benzyl-5-isoxazolyl)methylaminocarbonylcyclohexane (Compound number IV-4):

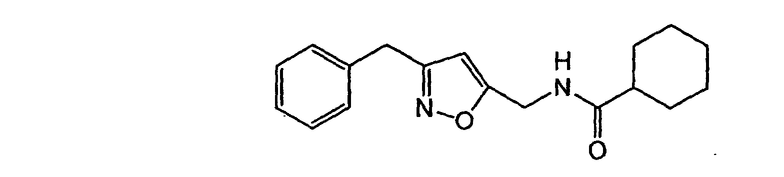

Property: oily;
NMR : δ 1.06-2.30(11H, m), 3.99(2H, s), 4.50(2H, d), 5.71-6.04(1H, br), 5.89(1H, s), 7.21(5H, s)

### Example 17(88): Preparation of [(3-benzyl-5-isoxazolyl)methyl]cyclohexylcarbamate (Compound number IV-5) :

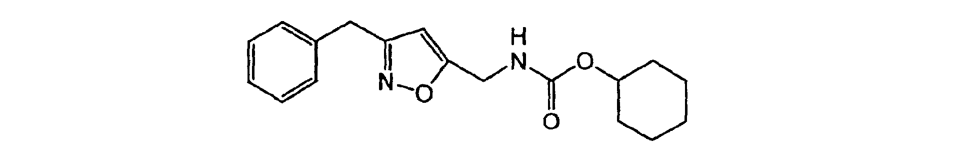

Melting point: 98 to 104°C.

### Example 17(89): Preparation of N-[(3-benzyl-5-isoxazolyl)methyl]-4-ethylbenzamide (Compound number IV-6):

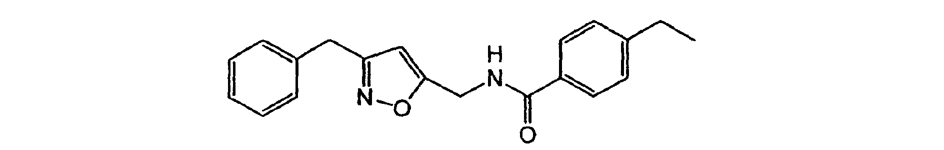

Property: oily;
NMR : δ 1.22(3H, t), 2.70(2H, q), 3.93(2H, s), 4.67(2H, d), 5.99(1H, s), 6.40-6.72(1H, brs), 7.08-7.47(7H, m), 7.69(2H, d)

### Example 17(90): Preparation of N-[(3-benzyl-5-isoxazolyl)methyl]-2-hydroxybenzamide (Compound number IV-7) :

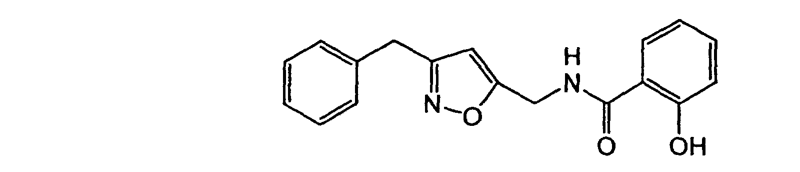

Property: paste;
NMR : δ 3.92(2H, s), 4.60(2H, d), 5.99(1H, s), 6.47-7.56(10H, m), 11.4(1H, s)

### Example 17(91): Preparation of N-[(3-benzyl-5-isoxazolyl)methyl]-4-methanesulfonylbenzamide (Compound number IV-8):

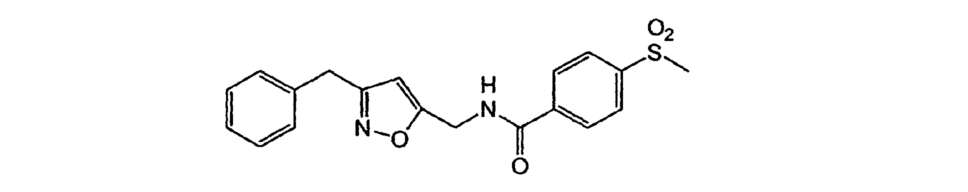

Melting point: 148 to 157°C;
NMR : δ 3.04 (3H, s), 3.97(2H, s), 4.69(2H, d), 6.01(1H, s), 6.95-7.55(6H, m), 7.93(4H, s)

### Example 17(92): Preparation of N-{[3-(4-methoxybenzyl)-5-isoxazolyl]methyl}-4-methoxybenzamide (Compound number IV-9) :

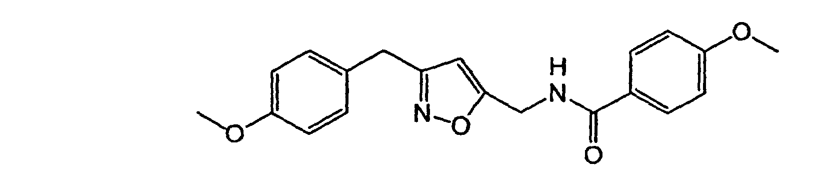

Melting point: 114 to 117°C.

### Example 17(93): Preparation of N-{[3-(4-methylbenzyl)-5-isoxazolyl]methyl}-4-methylbenzamide (Compound number IV-10):

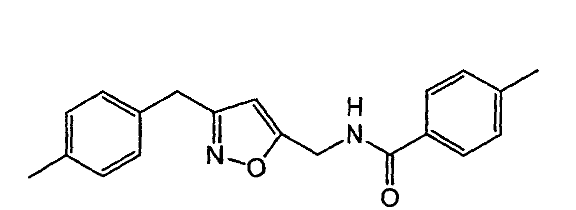

Melting point: 86 to 97°C.

### Example 17(94): Preparation of N-{[3-(3-methylbenzyl)-5-isoxazolyl]methyl}-4-methylbenzamide (Compound number IV-11) :

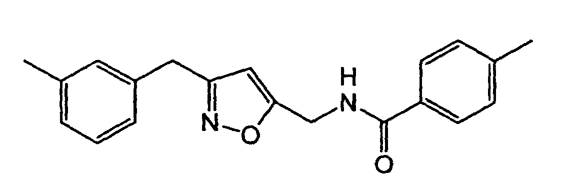

Property: oily;
NMR : δ 2.31(3H, s), 2.40(3H, s), 3.92(2H, s), 4.65(2H, d), 5.99(1H, s), 6.53-6.87(1H, brs), 6.87-7.86(8H, m)

### Example 17(95): Preparation of N-[(3-benzyl-5-isoxazolyl)methyl]-4-methoxycarbonylbenzamide (Compound number IV-23):

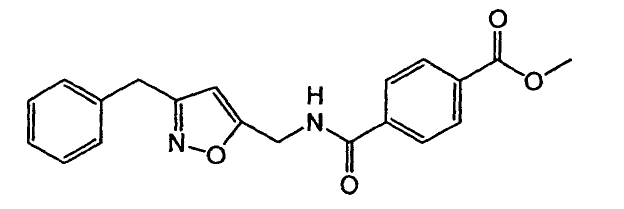

Melting point: 134 to 140°C.

### Example 17(96): Preparation of N-{[3-(1-phenylethyl)-5-isoxazolyl]methyl}benzamide (Compound number IV-29):

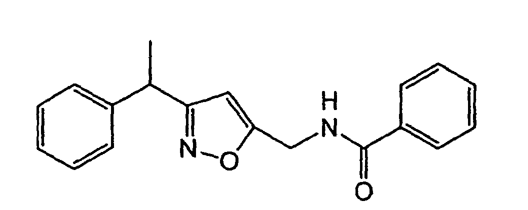

Melting point: 100 to 107°C.

### Example 17(97): Preparation of N-{[3-(2-phenylethyl)-5-isoxazolyl]methyl}benzamide (Compound number IV-33):

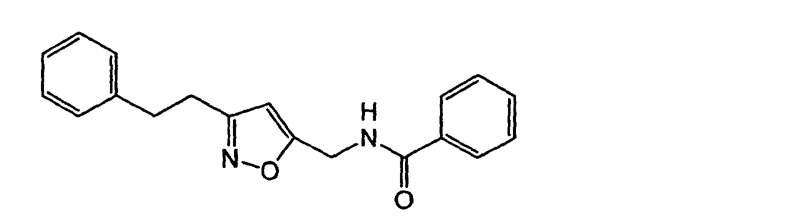

Melting point: 53 to 83°C.

### Example 17(98): Preparation of N-{[3-(2-phenylethyl)-5-isoxazolyl]methyl}-4-methoxybenzamide (Compound number IV-34):

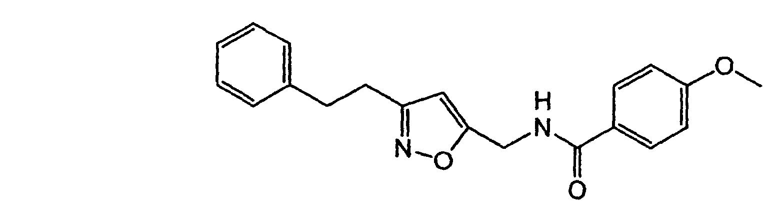

Melting point: 119 to 131°C.

### Example 17(99): Preparation of N-{[3-(2-phenylethyl)-5-isoxazolyl]methyl}-4-methylbenzamide (Compound number IV-35) :

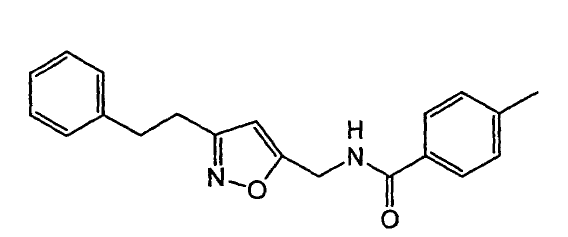

Melting point: 131 to 137°C.

### Example 17(100): Preparation of N-[(3-phenylthiomethyl-5-isoxazolyl)methyl]-4-methylbenzamide (Compound number IV-40) :

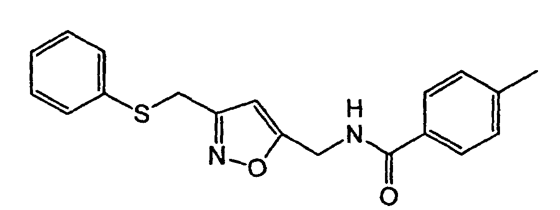

Melting point: 79 to 85°C;
NMR : δ 2.40(3H, s), 4.04(2H, s), 4.62 (2H, d), 6.13(1H, s), 6.76(1H, s), 7.03-7.74(10H, m)

### Example 17(101): Preparation of N-[(3-phenoxymethyl-5-isoxazolyl)methyl]-4-methylbenzamide (Compound number IV-46) :

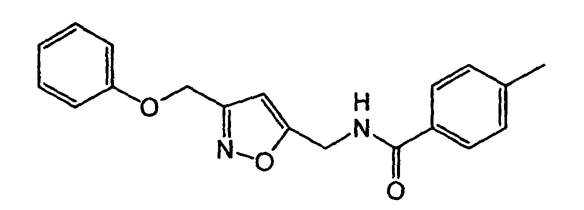

Property: paste;
NMR : δ 2.36 (3H, s), 4.67 (2H, d), 5.07(2H, s), 6.30(1H, s), 6.52-7.78(10H, m)

### Example 17(102): Preparation of N-{[3-(3-methylphenyl)oxymethyl-5-isoxazolyl]methyl}-4-methoxybenzamide (Compound number IV-48):

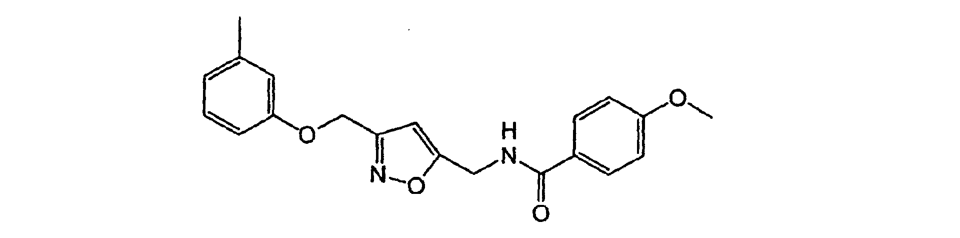

Melting point: 86 to 90°C;
NMR : δ 2.25(3H, s), 3.75(3H, s), 4.62(2H, s), 5.00(2H, s), 6.27(1H, s), 6.60-7.83(9H, m)

### Example 17(103): Preparation of N-{[3-(3-methylphenyl)oxymethyl-5-isoxazolyl]methyl}-4-methylbenzamide (Compound number IV-49):

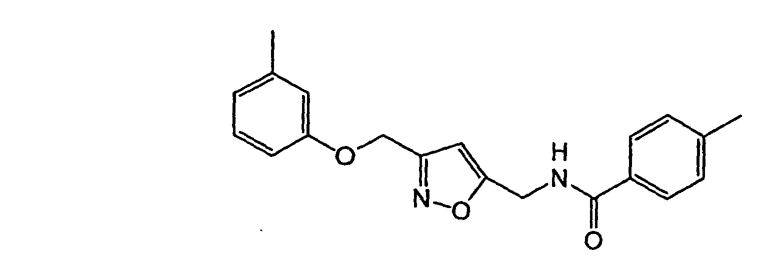

Melting point: 94 to 96°C;
NMR : δ 2.31(3H, s), 2.35(3H, s), 4.72(2H, d), 5.05(2H, s), 6.33(1H, s), 6.61-7.76(9H, m)

### Example 17(104): Preparation of N-{[3-(4-methylphenyl)oxymethyl-5-isoxazolyl]methyl}-4-methoxybenzamide (Compound number IV-50):

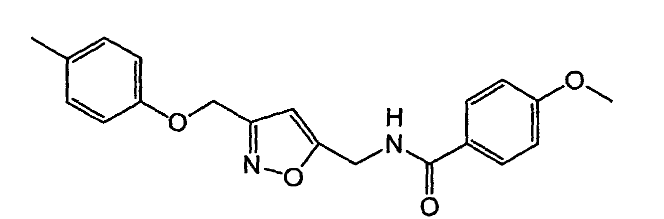

Melting point: 124 to 127°C;
NMR : δ 2.27 (3H, s), 3.80(3H, s), 4.68(2H, s), 5.03(2H, s), 6.33(1H, s), 6.64-8.00(9H, m)

### Example 17(105): Preparation of N-{[3-(2-cyanophenyl)oxymethyl-5-isoxazolyl]methyl}-4-methoxybenzamide (Compound number IV-51):

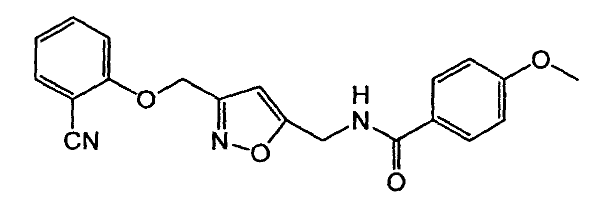

Melting point: 111 to 114°C.

### Example 17(106): Preparation of N-{[3-(3-cyanophenyl)oxymethyl-5-isoxazolyl]methyl}-4-methoxybenzamide (Compound number IV-52):

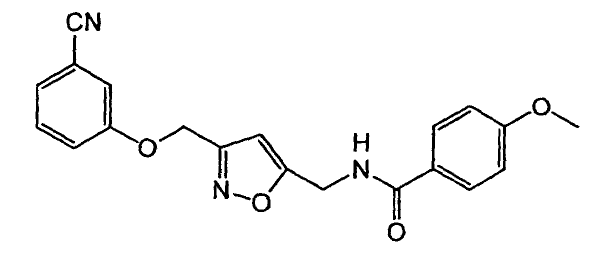

Melting point: 128 to 130°C.

### Example 17(107): Preparation of N-{[3-(4-Cyanophenyl)oxymethyl-5-isoxazolyl]methyl}-4-methoxybenzamide (Compound number IV-53):

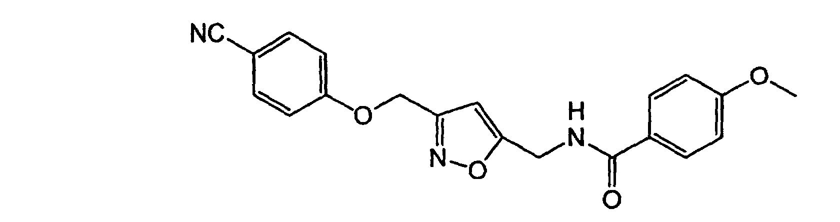

Melting point: 119 to 121°C;
NMR : δ 3.81 (3H, s), 4.72(2H, d), 5.14(2H, s), 6.32(1H, s), 6.73-8.02(9H, m)

### Example 17(108): Preparation of N-{[3-(2-methoxyphenyl)oxymethyl-5-isoxazolyl)methyl}-4-methoxybenzamide (Compound number IV-54):

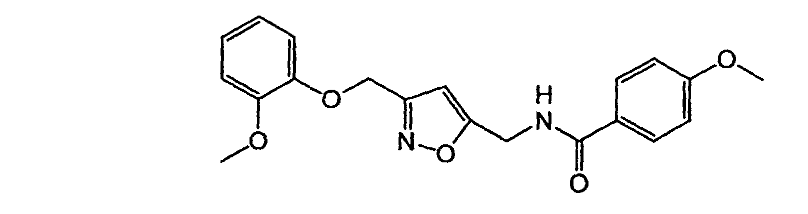

Property: oily;
NMR : δ 3.75 (6H, s), 4.60 (2H, d), 5.07(2H, s), 6.28(1H, s), 6.69-7.90(9H, m)

### Example 17(109): Preparation of N-{[3-(3-methoxyphenyl)oxymethyl-5-isoxazolyl]methyl}-4-methoxybenzamide (Compound number IV-55):

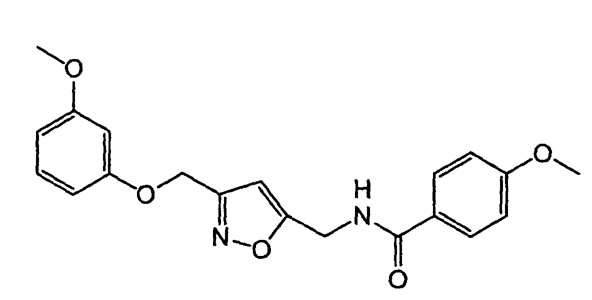

Melting point: 95 to 104°C.

### Example 17(110): Preparation of N-{[3-(4-methoxyphenyl)oxymethyl-5-isoxazolyl]methyl}-4-methoxybenzamide (Compound number IV-56):

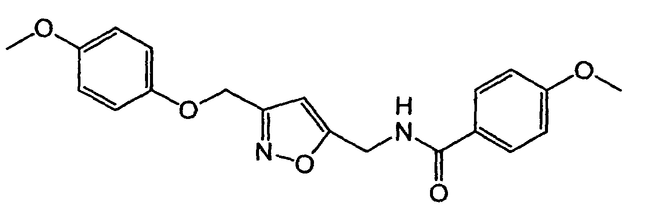

Melting point: 115 to 121°C.

### Example 17(111): Preparation of N-{[3-(2-fluorophenyl)oxymethyl-5-isoxazolyl]methyl}-4-methoxybenzamide (Compound number IV-57):

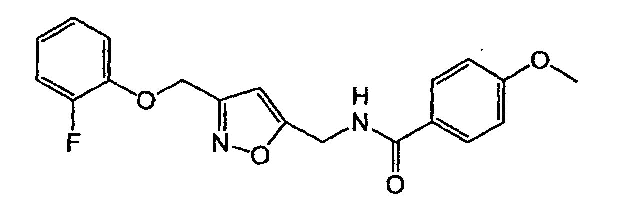

Melting point: 97 to 102°C.

### Example 17(112): Preparation of N-{[3-(3-fluorophenyl)oxymethyl-5-isoxazolyl]methyl}-4-methoxybenzamide (Compound number IV-58):

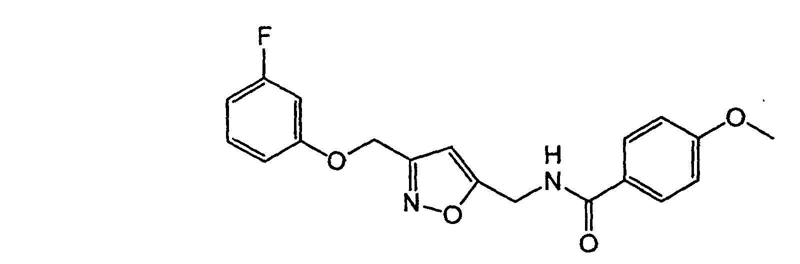

Melting point: 95 to 106°C.

### Example 17(113): Preparation of N-{[3-(4-fluorophenyl)oxymethyl-5-isoxazolyl]methyl}-4-methoxybenzamide (Compound number IV-59):

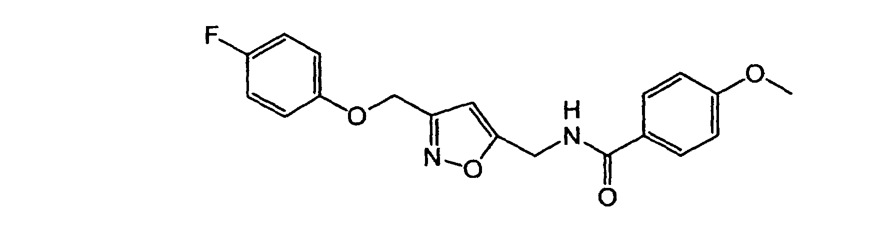

Melting point: 95 to 106°C.

### Example 17(114): Preparation of N-{[3-(4-nitrophenyl)oxymethyl-5-isoxazolyl]methyl}-4-methoxybenzamide (Compound number IV-60):

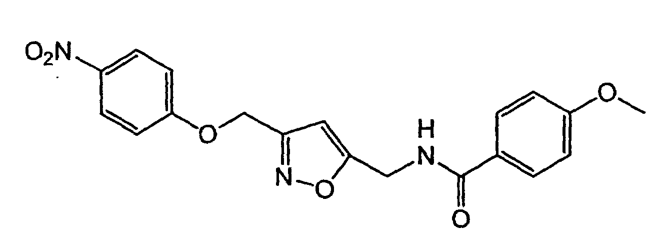

Melting point: 151 to 154°C.

### Example 17(115): Preparation of N-{[3-(imidazol-2-ylphenyl)oxymethyl-5-isoxazolyl]methyl}-4-methoxybenzamide (Compound number IV-72):

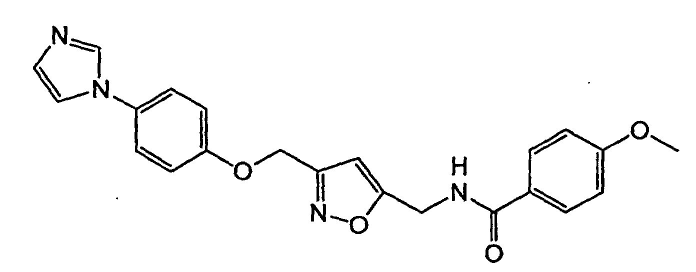

Melting point: 138 to 142°C;
NMR : δ 3.80(3H, s), 4.70(2H, d), 5.08(2H, s), 6.32(1H, s), 6.78-7.93(12H, m)

### Example 17(116): Preparation of {3-[(4-methoxyphenyl)carbonylaminomethyl]-5-isoxazolyl]methoxyiminobenzyl (Compound number IV-74):

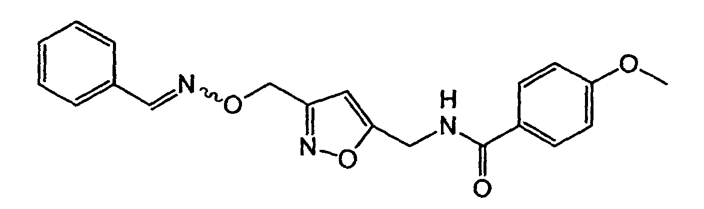

Property: amorphous;
NMR : δ 3.76(3H, s), 4.65(2H, d), 5.18(2H, s), 5.32(1H, s), 6.25(1H, s), 6.73-8.09(11H, m)

### Example 18

According to the methods described in Examples 1 to 16, the compounds in the following Examples 18(1) to 18(173) were obtained.

### Example 18(1): Preparation of N-[(3-isopropyl-5-isoxazolyl)methyl]phenoxyacetamide (Compound number I-62):

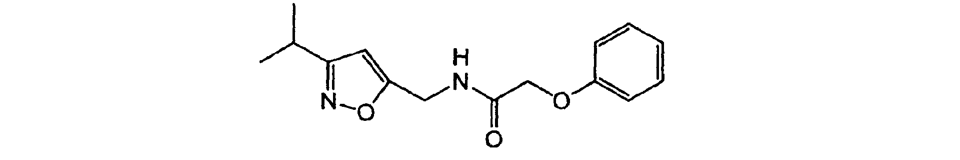

Melting point: 80 to 86°C.

### Example 18(2): Preparation of N-[(3-isopropyl-5-isoxazolyl)methyl]phenylacetamide (Compound number I-63):

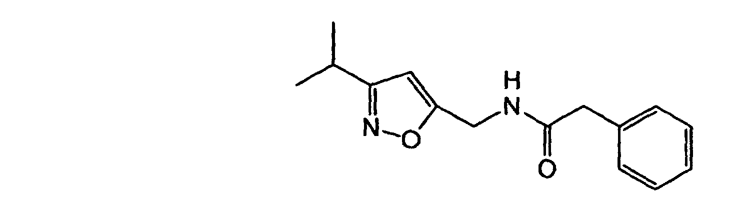

Melting point: 54 to 66°C.

### Example 18(3): Preparation of phenyl [(3-isobutyl-5-isoxazolyl)methyl] carbamate (Compound number I-8):

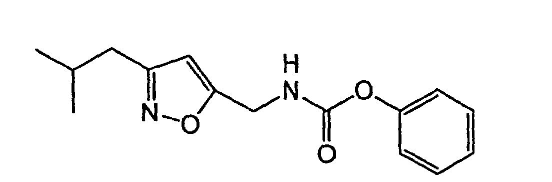

Property: oily;
NMR : δ 0.91(3H, s), 0.99(3H, s), 1.72-2.22(1H, m), 2.52(2H, d), 4.52(2H, d), 5.47(1H, br), 6.06(1H, s), 7.02-7.48(5H, m)

### Example 18(4): Preparation of N-[(3-isobutyl-5-isoxazolyl)methyl]phenylacetamide (Compound number I-64):

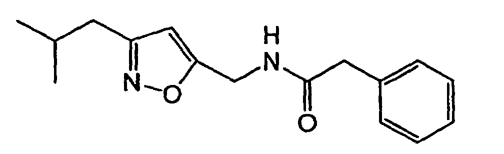

Property: oily;
NMR : δ 0.88(3H, s), 0.93(3H, s), 1.72-2.14(1H, m), 2.48(2H, d), 3.59(2H, s), 4.48(2H, d), 5.88(1H, s), 7.12-7.44(5H, m)

### Example 18(5): Preparation of N-[(3-chloromethyl-5-isoxazolyl)methyl]phenoxyacetamide (Compound number I-65):

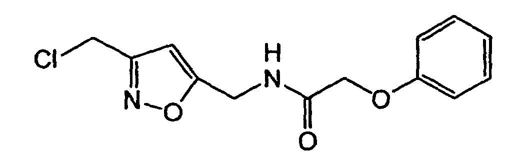

Melting point: 89 to 99°C.

### Example 18(6): Preparation of phenyl [(3-propyl-5-isoxazolyl)methyl] carbamate (Compound number I-3):

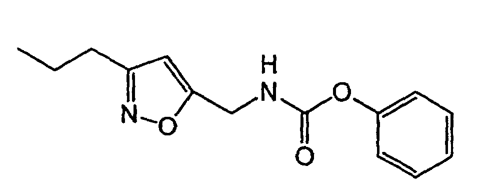

Property: oily;
NMR : δ 0.92 (3H, t), 1.43-1.88(2H, m), 2.60(2H, t), 4.48(2H, d), 5.89(1H, br), 6.07(1H, s), 7.00-7.50(5H, m)

### Example 18(7): Preparation of phenyl [(3-chloromethyl-5-isoxazolyl)methyl] carbamate (Compound number I-66):

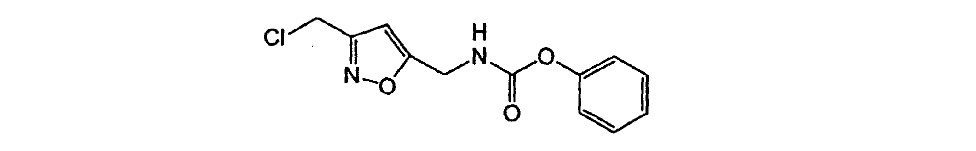

Melting point: 79 to 81°C.

### Example 18(8): Preparation of N-[(3-chloromethyl-5-isoxazolyl)methyl]phenylacetamide (Compound number I-67):

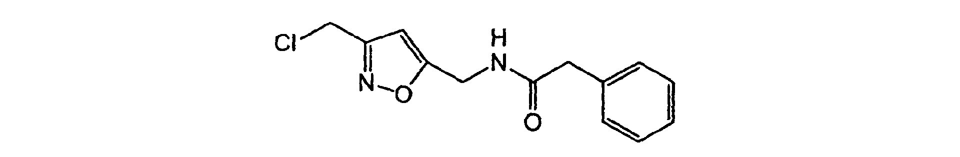

Melting point: 102 to 103°C.

### Example 18(9): Preparation of 3-[3-(4-tolyl)-5-isoxazolyl]methyl-1-cyclopentylurea (Compound number II-10):

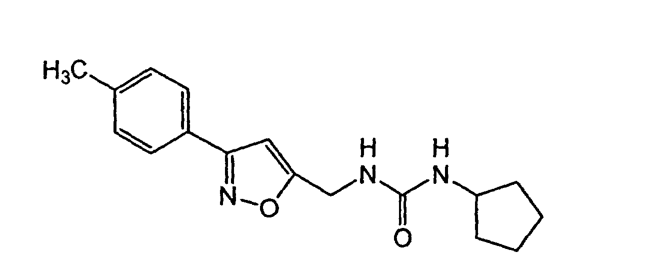

Melting point: 163 to 164°C.

### Example 18(10): Preparation of 3-(3-phenoxymethyl-5-isoxazolyl)methyl-1-cyclohexylurea (Compound number II-104):

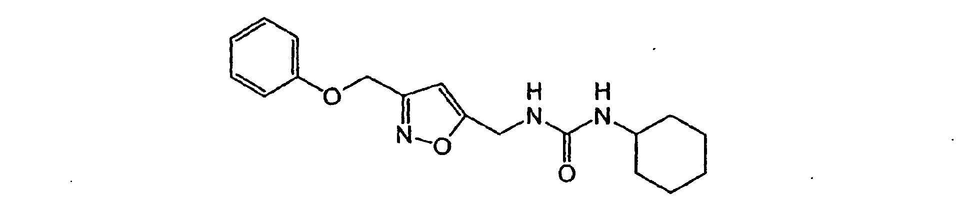

Melting point: 137 to 138°C.

### Example 18(11): Preparation of 3-(3-chloromethyl-5-isoxazolyl)methyl-1-cyclohexylurea (Compound number II-105):

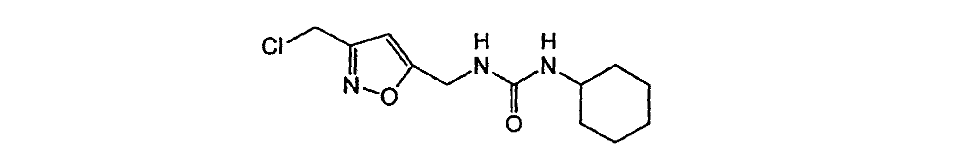

Melting point: 96 to 107°C.

### Example 18(12): Preparation of 3-(3-phenoxymethyl-5-isoxazolyl)methyl-1-cyclopentylurea (Compound number II-106) :

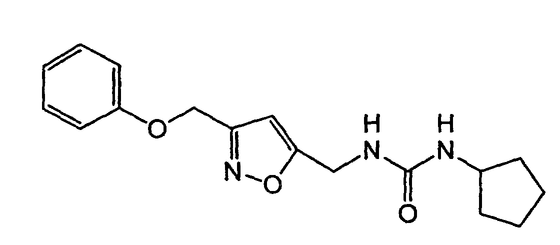

NMR : δ 1.10-2.20(8H, m), 3.74-4.12(1H, m), 4.42(2H, d), 5.08(2H, s), 5.13(1H, br), 5.60(1H, br), 6.28(1H, s), 6.82-7.40(5H, m)

### Example 18(13): Preparation of 3-(3-chloromethyl-5-isoxazolyl)methyl-1-cyclopentylurea (Compound number II-107):

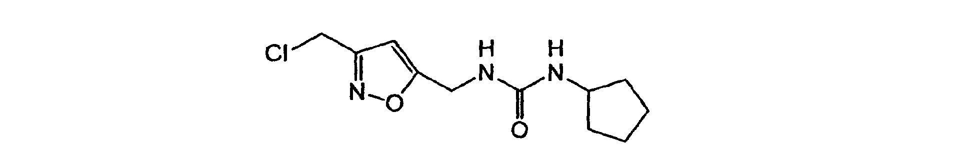

Property: oily;
NMR : δ 0.72-2.20(8H, m), 3.80-4.20(1H, m), 4.50(2H, d), 4.53(2H, s), 4.40-5.35(2H,m), 6.28(1H, s)

### Example 18(14): Preparation of 3-(3-methyl-5-isoxazolyl)methyl-1-cyclohexylurea (Compound number II-108):

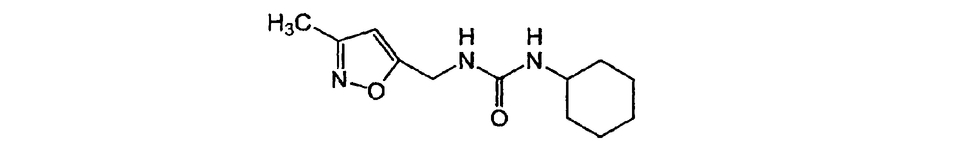

Melting point: 119 to 122°C.

### Example 18(15): Preparation of 3-(3-chloromethyl-5-isoxazolyl)methyl-1-phenylurea (Compound number II-109):

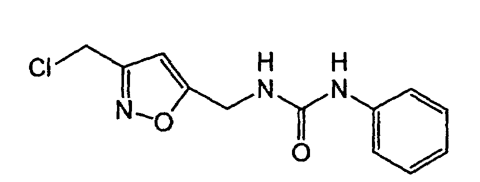

Melting point: 144 to 146°C.

### Example 18(16): Preparation of 3-(3-methylaminocarbonyl-5-isoxazolyl)methyl-1-cyclohexylurea (Compound number II-110):

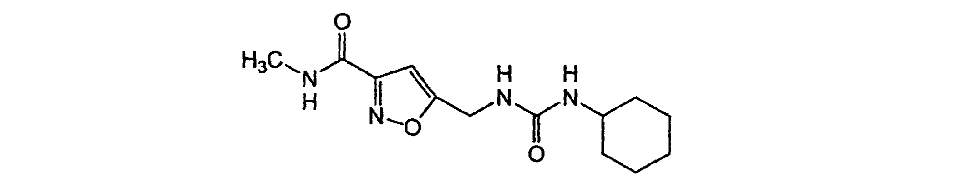

Melting point: 197 to 198°C.

### Example 18(17): Preparation of 3-(3-bromomethyl-5-isoxazolyl)methyl-1-cyclohexylurea (Compound number II-111):

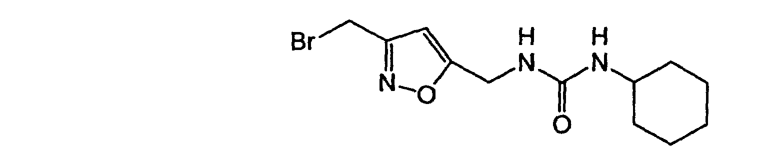

Melting point: 137 to 139°C.

### Example 18(18): Preparation of 3-[3-(bicyclo[3.2.1]oct-2-en-4-on-2-yl)oxymethyl-5-isoxazolyl]methyl-1-cyclohexylurea (Compound number II-112):

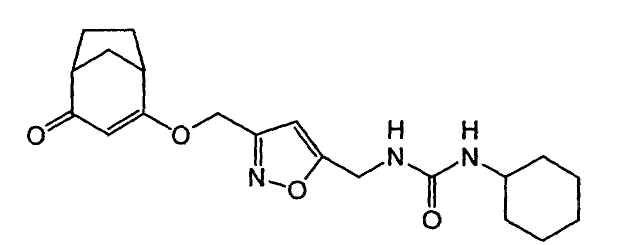

Property: gummy;
NMR : δ 0.60-2.40(16H, m), 2.90(2H, m), 3.50(1H, m), 4.50(2H, d), 4.85(2H, s), 5.00(1H, br), 5.10(1H, s), 5.50(1H, b), 6.25(1H, s)

### Example 18(19): Preparation of 3-(3-cyanomethyl-5-isoxazolyl)methyl-1-cyclohexylurea (Compound number II-113):

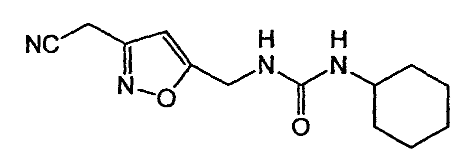

Melting point: 140 to 150°C.

### Example 18(20): Preparation of 3-(3-methylthiomethyl-5-isoxazolyl)methyl-1-cyclohexylurea (Compound number II-114):

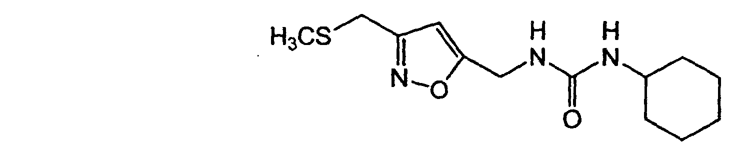

Melting point: 123 to 130°C.

### Example 18(21): Preparation of 3-(3-iodomethyl-5-isoxazolyl)methyl-1-cyclohexylurea (Compound number II-115):

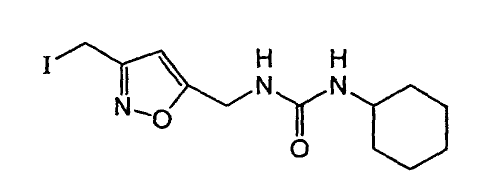

Melting point: 136 to 139°C.

### Example 18(22): Preparation of 3-[(3-chloromethyl-5-isoxazolyl)methyl]-1-methyl-1-phenylurea (Compound number II-116):

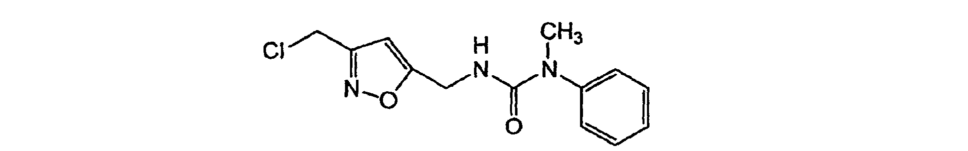

Property: oily;
NMR : δ 3.25(3H,s), 4.50(2H,d), 4.55(2H,s), 4.70-4.90(1H,brs), 6.25(1H,s), 7.10-7.60(5H,m)

### Example 18(23): Preparation of 3-(3-methoxymethyl-5-isoxazolyl)methyl-1-cyclohexylurea (Compound number II-117):

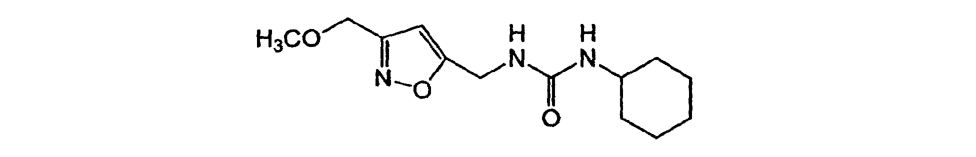

Melting point: 98 to 99°C.

### Example 18(24): Preparation of 3-(3-acetyloxymethyl-5-isoxazolyl)methyl-1-cyclohexylurea (Compound number II-118):

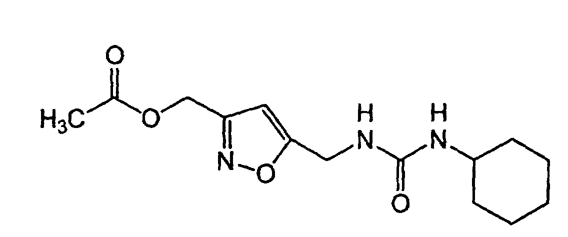

Melting point: 89 to 100°C.

### Example 18(25): Preparation of 3-(3-hydroxymethyl-5-isoxazolyl)methyl-1-cyclohexylurea (Compound number II-119):

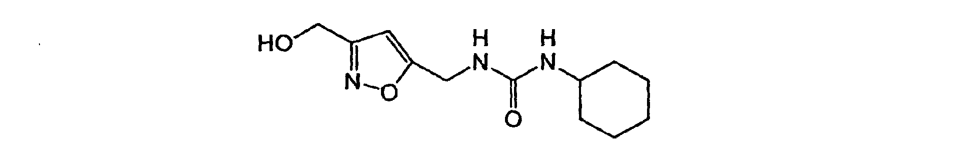

Melting point: 114 to 116°C.

### Example 18(26): Preparation of N-[(3-phenyl-5-isoxazolyl)methyl]-2,2-dichloro-1,3-dimethylcyclopropanecarboxamide (Compound number III-212):

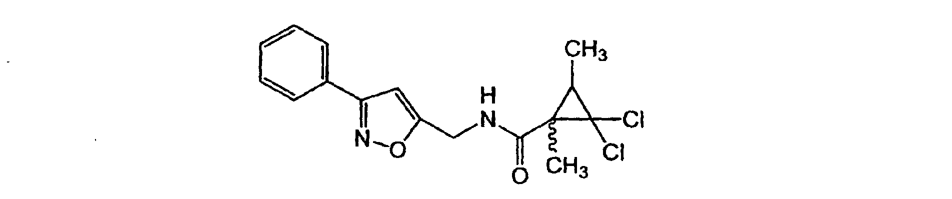

Property: oily;
NMR : δ 1.07-2.50(7H, m), 4.65(2H, d), 4.61(1H, br), 6.52(1H, s), 7.32-7.87(5H, m)

### Example 18(27): Preparation of N-[1-(3-phenyl-5-isoxazolyl)ethyl]-2,2-dichloro-1,3-dimethylcyclopropanecarboxamide (Compound number III-213):

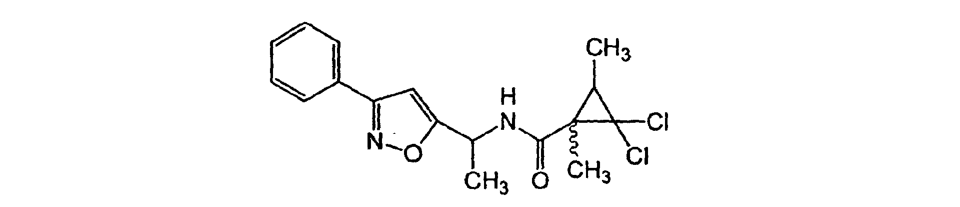

Property: gummy;
NMR : δ 1.07-2.47(10H, m), 5.21-5.65(1H, m), 6.02-6.35(1H, br), 6.41(0.5H, s), 6.48(0.5H, s), 7.32-7.89(5H, m)

### Example 18(28): Preparation of N-{[3-(methoxyiminomethyl)phenyl-5-isoxazolyl]methyl}-4-methylbenzamide (Compound number III-214):

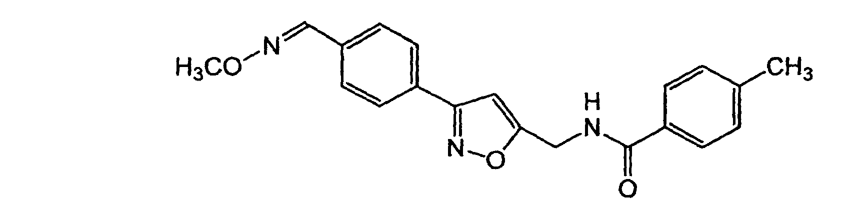

Melting point: 154 to 164°C.

### Example 18(29): Preparation of N-{[3-(2-fluoro-4-methoxyphenyl)-5-isoxazolyl]methyl}-4-methoxylbenzamide (Compound number III-215):

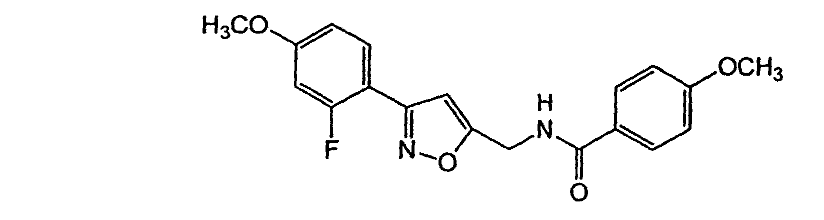

Melting point: 129 to 131°C.

### Example 18(30): Preparation of N-{[3-(2-methyl-1-propenyl)-5-isoxazolyl]methyl}-4-methoxylbenzamide (Compound number III-216):

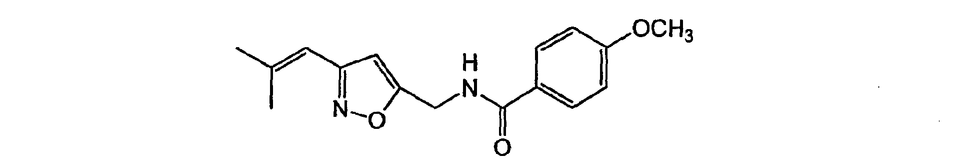

Melting point: 74 to 85°C.

### Example 18(31): Preparation of N-{[3-(2-pyridyl)-5-isoxazolyl]methyl}-4-methylbenzamide (Compound number III-217):

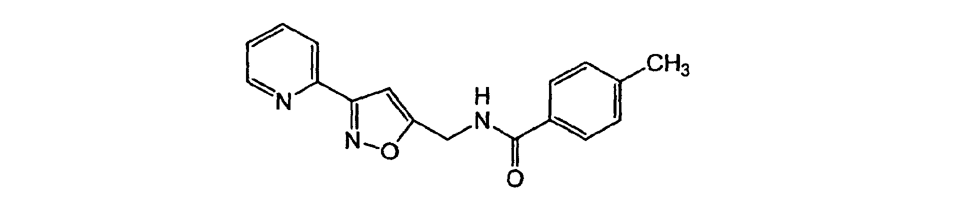

Melting point: 141 to 142°C.

### Example 18(32): Preparation of N-{[3-(3-pyridyl)-5-isoxazolyl]methyl}-4-methylbenzamide (Compound number III-218) :

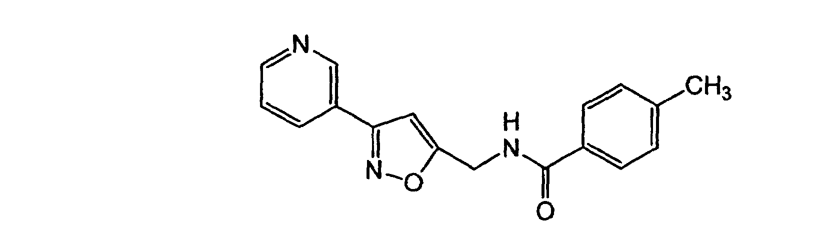

Melting point: 127 to 129°C.

### Example 18(33): Preparation of N-{[3-(4-pyridyl)-5-isoxazolyl]methyl}-4-methylbenzamide (Compound number III-219) :

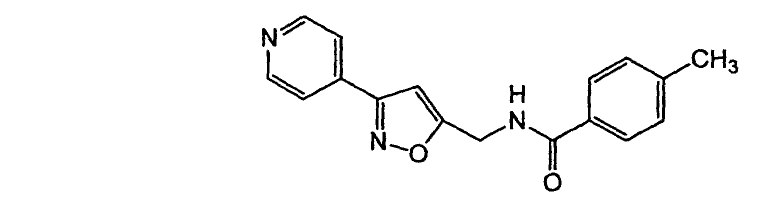

NMR : δ 2.40 (3H, s), 4.82 (2H, d), 6.62(1H, s), 6.98(1H, br), 7.13-8.88(8H, m)

### Example 18(34): Preparation of N-{[3-(3-thienyl)-5-isoxazolyl]methyl}-4-methoxylbenzamide (Compound number III-220) :

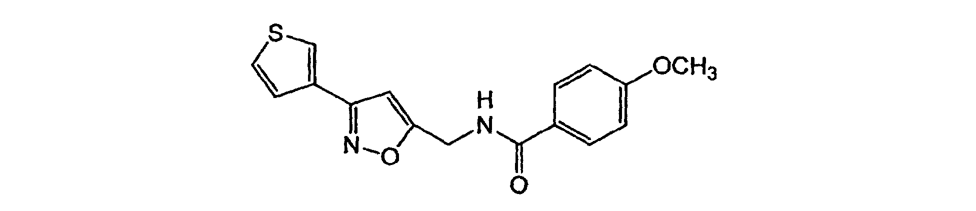

Melting point: 142 to 155°C.

### Example 18(35): Preparation of N-{[3-(1,3-pentadienyl)-5-isoxazolyl]methyl}-4-methoxylbenzamide (Compound number III-221):

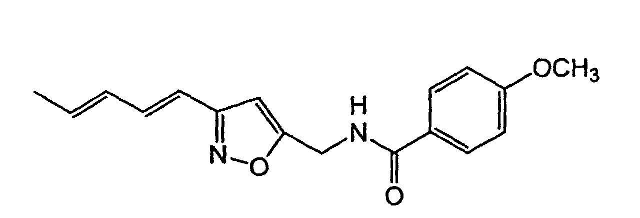

Melting point: 151 to 154°C.

### Example 18(36): Preparation of N-{[3-(4-tolyl)-5-isoxazolyl]methyl}cyclohexanecarboxamide (Compound number III-222):

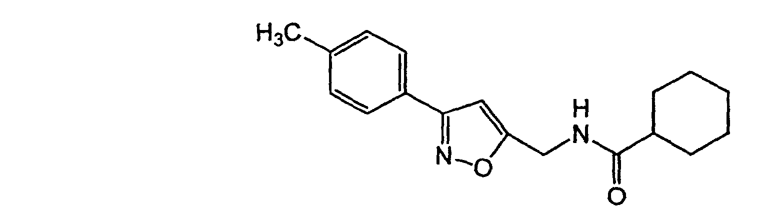

Melting point: 162 to 164°C.

### Example 18(37): Preparation of N-{[3-(4-tolyl)-5-isoxazolyl]methyl}-4-morpholinecarboxamide (Compound number III-204):

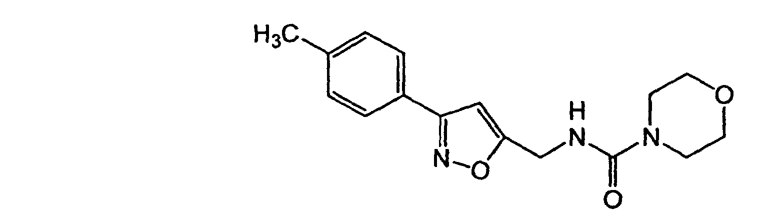

Melting point: 118 to 123°C.

### Example 18(38): Preparation of N-{[3-(1-methylindol-2-yl)-5-isoxazolyl]methyl}-4-methoxybenzamide (Compound number III-223) :

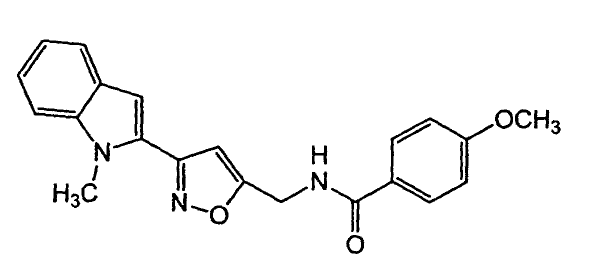

Melting point: 110 to 114°C.

### Example 18(39): Preparation of N-{[3-(4-tolyl)-5-isoxazolyl]methyl}-4-phenylpiperazine-1-carboxamide (Compound number III-207):

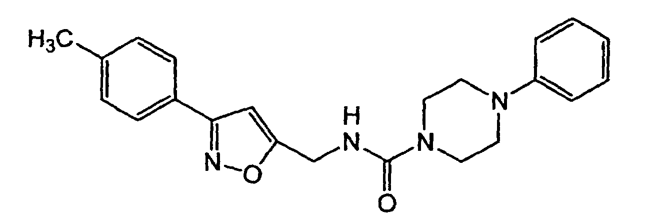

Melting point: 177 to 179°C.

### Example 18(40): Preparation of N-{[3-(4-tolyl)-5-isoxazolyl]methyl)-1-pyridinecarboxamide (Compound number III-203):

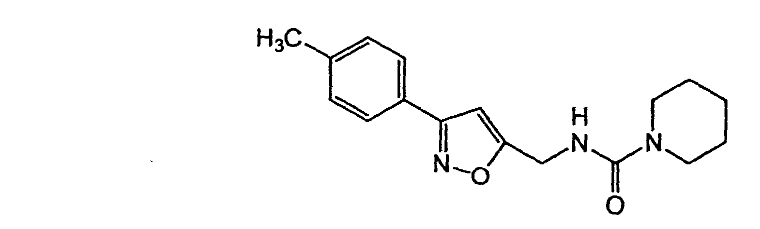

Melting point: 130 to 133°C.

### Example 18(41): Preparation of N-{[3-(4-tolyl)-5-isoxazolyl]methyl}-1-pyrrolidinecarboxamide (Compound number III-205) :

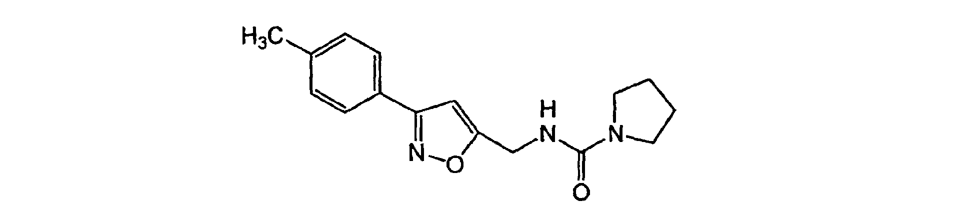

Melting point: 131 to 142°C.

### Example 18(42): Preparation of N-{[3-(benzo[d]thiazol-2-yl)-5-isoxazolyl]methyl}-4-methoxybenzamide (Compound number III-224):

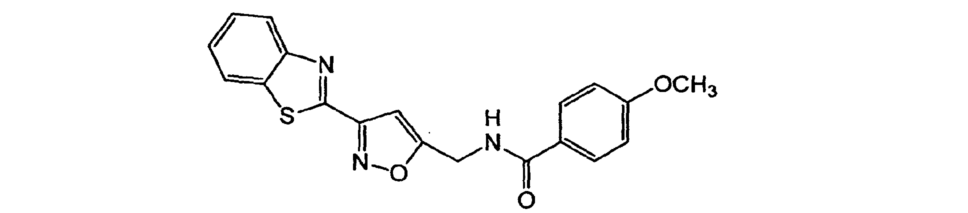

NMR : δ 3.85 (3H, s), 4.74(2H, d), 6.72(1H, s), 6.69-8.34(4H, m), 9.42(1H, br)

### Example 18(43): Preparation of N-[(3-isopropyl-5-isoxazolyl)methyl]-4-methoxybenzamide (Compound number III-225) :

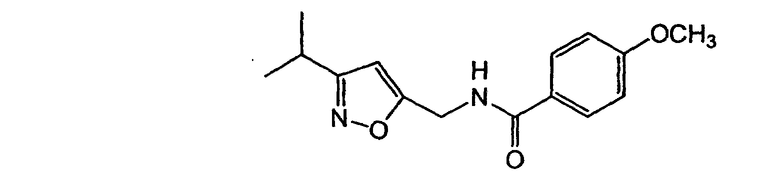

Melting point: 114 to 119°C.

### Example 18(44): Preparation of N-[(3-chloromethyl-5-isoxazolyl)methyl]benzamide (Compound number III-226):

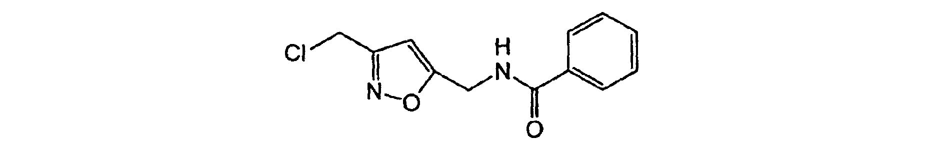

Melting point: 109 to 117°C.

### Example 18(45): Preparation of N-{[3-(4-tolyl)-5-isoxazolyl]methyl}cyclopentanecarboxamide (Compound number III-227):

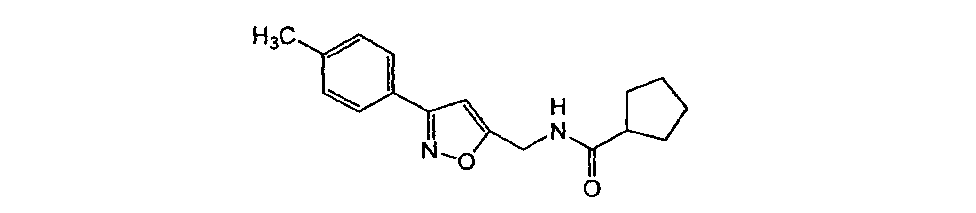

Melting point: 162 to 164°C.

### Example 18(46): Preparation of N-{[3-(4-tolyl)-5-isoxazolyl]methyl}cyclobutanecarboxamide (Compound number III-228):

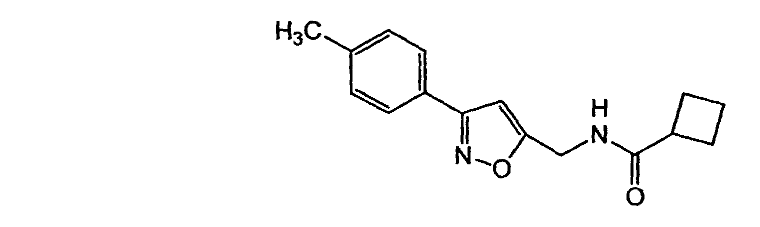

Melting point: 168 to 170°C.

### Example 18(47): Preparation of N-[(3-isobutyl-5-isoxazolyl)methyl]-4-methoxybenzamide (Compound number III-229) :

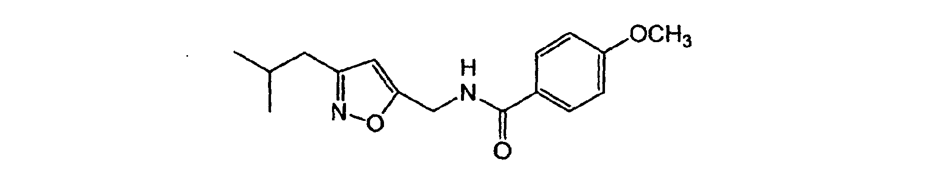

Melting point: 94 to 113°C.

### Example 18(48): Preparation of N-{[3-(1-ethylpropyl)-5-isoxazolyl]methyl}-4-methoxybenzamide (Compound number III-230) :

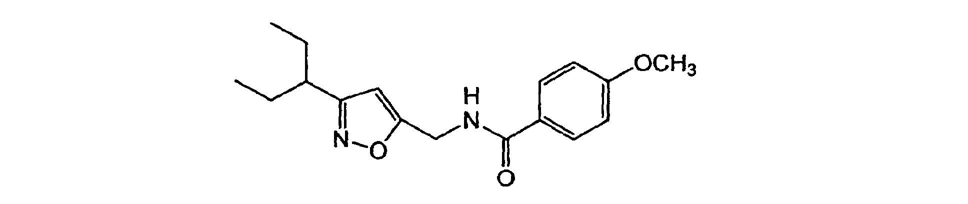

Melting point: 95 to 114°C.

### Example 18(49): Preparation of N-{[3-(2-chlorophenyl)-5-isoxazolyl]methyl}-4-methoxybenzamide (Compound number III-231):

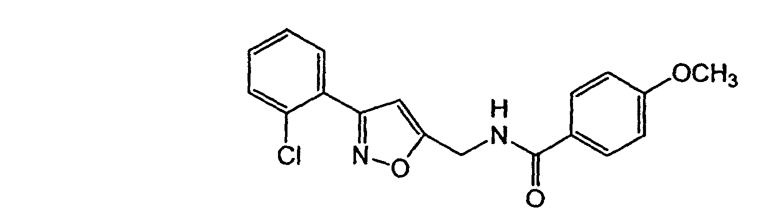

Melting point: 87 to 101°C.

### Example 18(50): Preparation of N-{[3-(3-phenyl-1,2,4-oxadiazol-5-yl)-5-isoxazolyl]methyl}-4-methoxybenzamide (Compound number III-232):

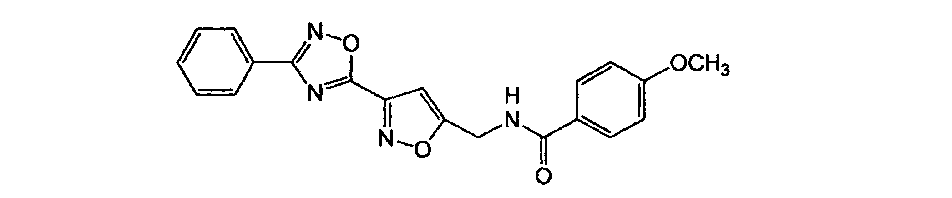

Melting point: 150 to 162°C.

### Example 18(51): Preparation of N-[(3-chloromethyl-5-isoxazolyl)methyl]cyclohexanecarboxamide (Compound number III-233):

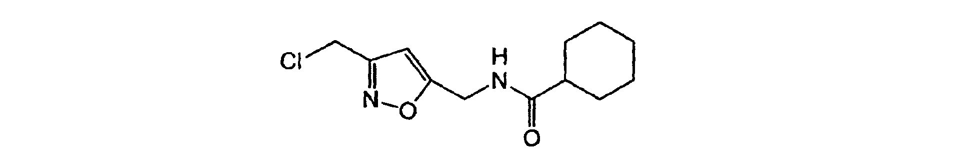

Property: paste;
NMR : δ 1.03-2.33(11H, m), 4.52(2H, s), 4.52(2H, d), 6.03(1H, br), 6.26(1H, s)

### Example 18(52): Preparation of N-[(3-isopropyl-5-isoxazolyl)methyl]cyclohexanecarboxamide (Compound number III-234):

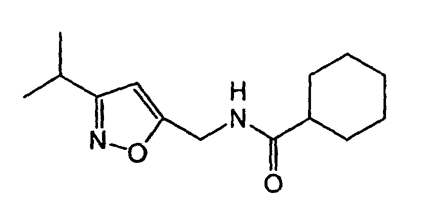

Property: oily;
NMR : δ 0.87-2.27(10H, m), 1.20(3H, s), 1.30(3H, s), 2.82-3.23(1H, m), 4.50(2H, d), 5.92(1H, br), 6.00(1H, s)

### Example 18(53): Preparation of N-[(3-butyl-5-isoxazolyl)methyl]-4-methoxybenzamide (Compound number III-235) :

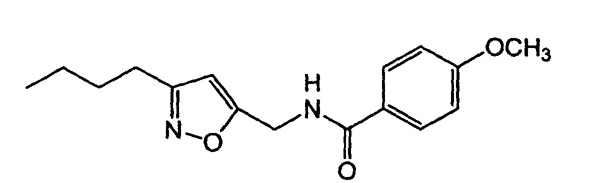

Melting point: 101 to 104°C.

### Example 18(54): Preparation of N-[(3-sec-butyl-5-isoxazolyl)methyl]-4-methoxybenzamide (Compound number III-236) :

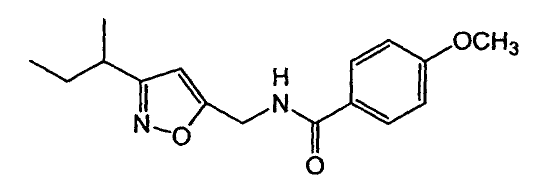

Melting point: 70 to 80°C.

### Example 18(55): Preparation of N-[(3-tert-butyl-5-isoxazolyl)methyl]-4-methoxybenzamide (Compound number III-237):

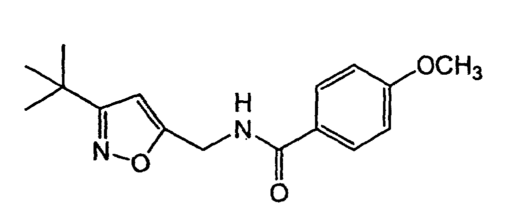

Melting point: 125 to 132°C.

### Example 18(56): Preparation of N-[(3-cyclohexyl-5-isoxazolyl)methyl]-4-methoxybenzamide (Compound number III-238) :

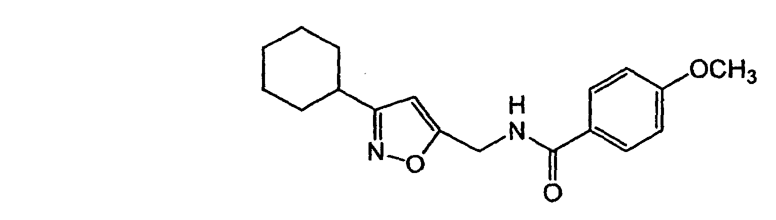

Melting point: 93 to 107°C.

### Example 18(57): Preparation of N-{[3-(oxolan-3-yl)-5-isoxazolyl]methyl}-4-methoxybenzamide (Compound number III-239) :

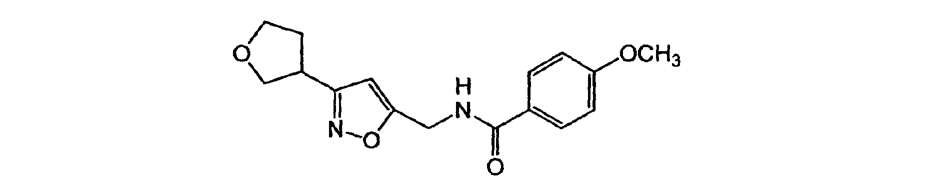

Melting point: 113 to 117°C.

### Example 18(58): Preparation of N-{[3-(4-tolyl)-5-isoxazolyl]methyl}-1,4-thiazinane-4-carboxamide (Compound number III-206):

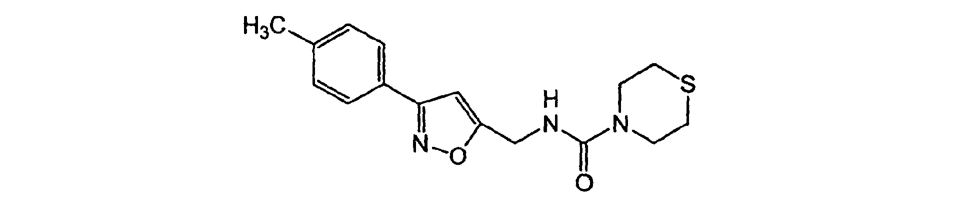

Melting point: 141 to 145°C.

### Example 18(59): Preparation of N-{[3-(4-tolyl)-5-isoxazolyl]methyl}-2,6-dichloropyridine-4-carboxamide (Compound number III-240):

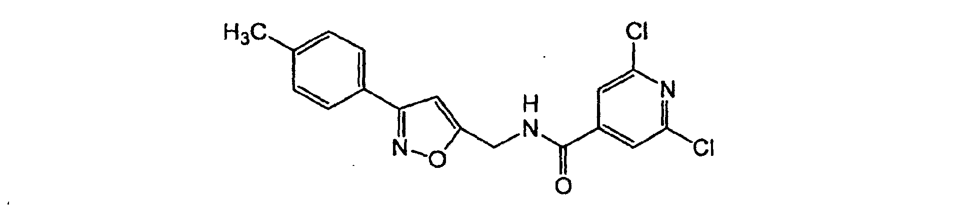

Melting point: 149 to 151°C.

### Example 18(60): Preparation of N-{[3-(4-tolyl)-5-isoxazolyl]methyl}-3,4-dichloroisothiazole-5-carboxamide (Compound number III-241):

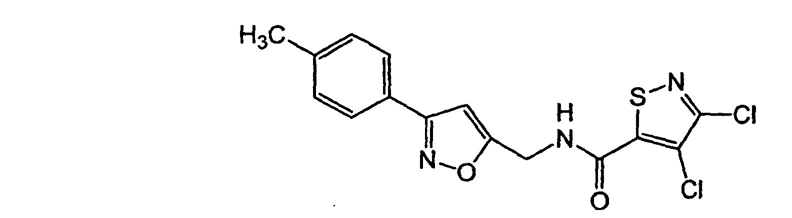

Melting point: 155 to 157°C.

### Example 18(61): Preparation of N-{[3-(4-tolyl)-5-isoxazolyl]methyl}-benzo[d][1,2,3]thiazole-7-carboxamide (Compound number III-242):

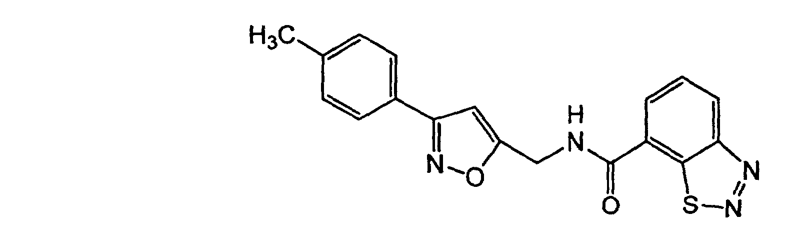

Melting point: 178 to 192°C.

### Example 18(62): Preparation of N-[(3-isopropyl-5-isoxazolyl)methyl]-2,6-dichloropyridine-4-carboxamide (Compound number III-243):

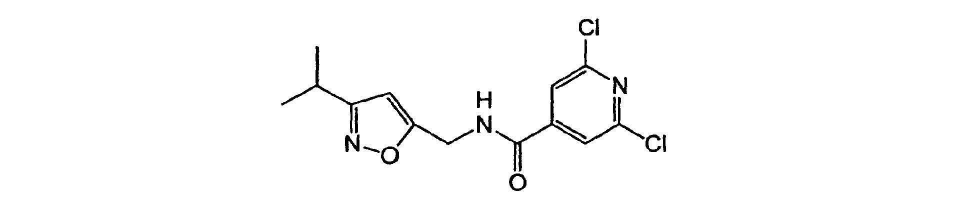

Property: oily;
NMR : δ 1.19 (3H, s), 1.28(3H, s), 2.73-3.22(1H, m), 4.70(2H, d), 6.13(1H, s), 7.68(2H, s), 8.00(1H, br)

### Example 18(63): Preparation of N-[(3-chloromethyl-5-isoxazolyl)methyl]-2,6-dichloropyridine-4-carboxamide (Compound number III-244) :

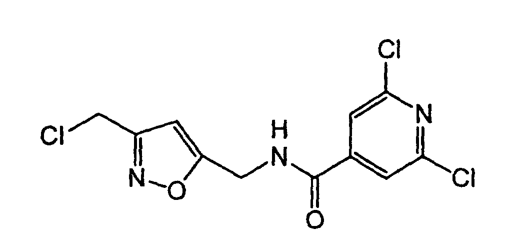

NMR :δ 4.54(2H, s), 4.73(2H, d), 6.34(1H, s), 6.81(1H, br), 7.59(2H, s)

### Example 18(64): Preparation of N-[(3-propyl-5-isoxazolyl)methyl]-4-methoxybenzamide (Compound number III-245) :

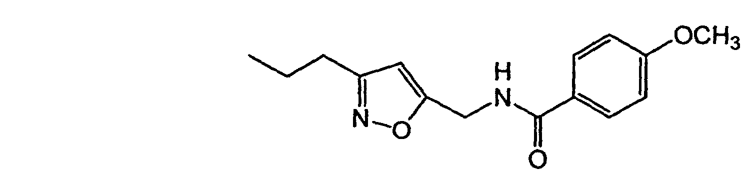

Melting point: 97 to 102°C.

### Example 18(65): Preparation of N-[(3-sec-pentyl-5-isoxazolyl)methyl]-4-methoxybenzamide (Compound number III-246) :

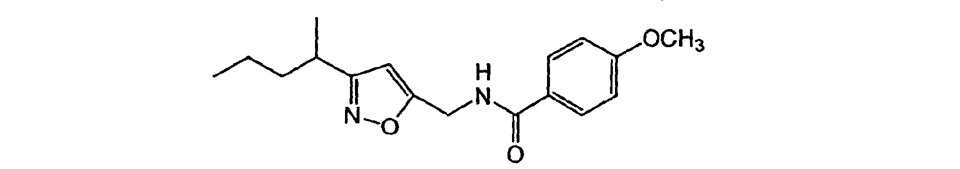

Melting point: 72 to 76°C.

### Example 18 (66) : Preparation of N-{[3-(2-methylfuran-5-yl)-5-isoxazolyl]methyl}-4-methoxybenzamide (Compound number III-247) :

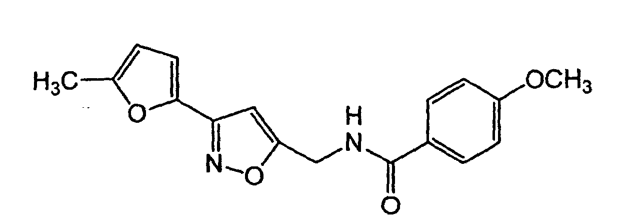

Melting point: 107 to 109°C.

### Example 18(67): Preparation of N-{[3-(1-heptynyl)-5-isoxazolyl]methyl}-4-methoxybenzamide (Compound number III-248):

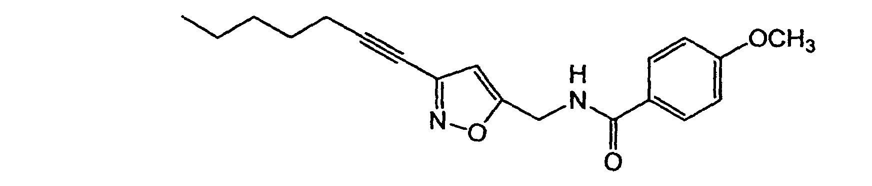

Melting point: 96 to 102°C.

### Example 18(68): Preparation of N-[(3-dimethoxymethyl-5-isoxazolyl)methyl]-4-methoxybenzamide (Compound number III-249) :

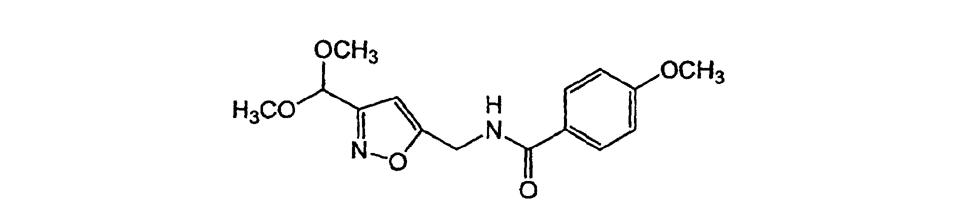

Property: paste;
NMR : δ 3.36(6H, s), 3.83(3H, s), 4.73(2H, d), 5.40(1H, s), 6.28(1H, s), 6.73(1H, br), 6.88(2H, d), 7.74(2H, d) Example 18(69): Preparation of N-[(3-isopropyliminoxy-5-isoxazolyl)methyl]-4-methoxybenzamide (Compound number III-250) : 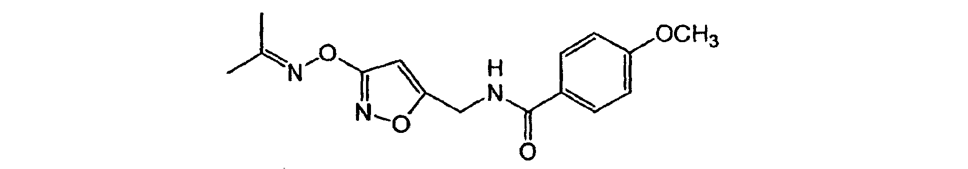 Property: paste;
NMR : δ 1.81(6H, s), 3.83(3H, s), 4.68(2H, d), 5.02(2H, s), 6.22(1H, s), 6.86(2H, d), 7.74(2H, d)

### Example 18(70): Preparation of N-[(3-isobutyl-5-isoxazolyl)methyl]-4-methylbenzamide (Compound number III-251) :

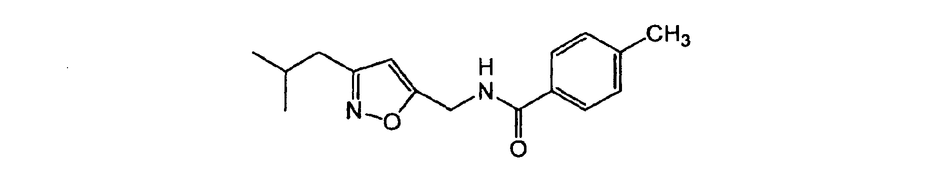

Melting point: 131 to 134°C.

### Example 18(71): Preparation of N-[(3-isobutyl-5-isoxazolyl)methyl]-4-chlorobenzamide (Compound number III-252) :

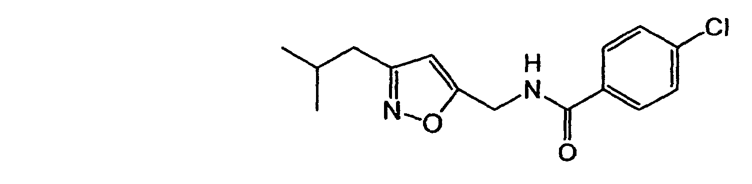

Melting point: 122 to 124°C.

### Example 18(72): Preparation of N-[(3-isobutyl-5-isoxazolyl)methyl]-2,4-dichlorobenzamide (Compound number III-253):

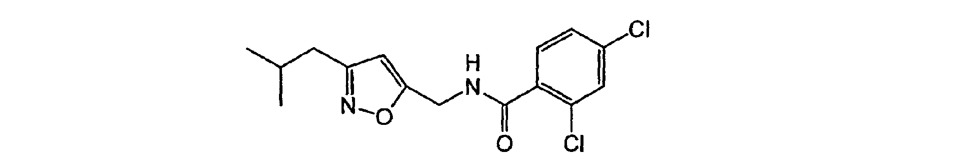

Property: oily;
NMR : δ 0.88(3H, s), 0.96 (3H, s), 1.76-2 .13 (1H, m), 2.51(2H, d), 4.71(2H, d), 6.09(1H, s), 6.84(1H, br), 7.16-7.70(3H, m) Example 18(73): Preparation of N-[(3-chloromethyl-5-isoxazolyl)methyl]-4-chlorobenzamide (Compound number III-254): 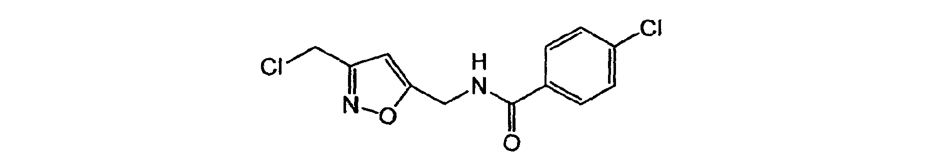 Melting point: 145 to 147°C.

### Example 18(74): Preparation of N-[(3-isobutyl-5-isoxazolyl)methyl]-3,4-dichlorobenzamide (Compound number III-255):

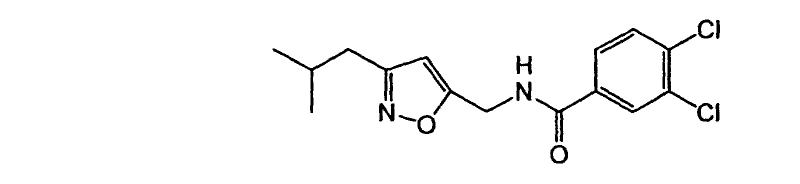

Melting point: 68 to 74°C.

### Example 18(75): Preparation of N-[(3-isobutyl-5-isoxazolyl)methyl]-4-bromobenzamide (Compound number III-256) :

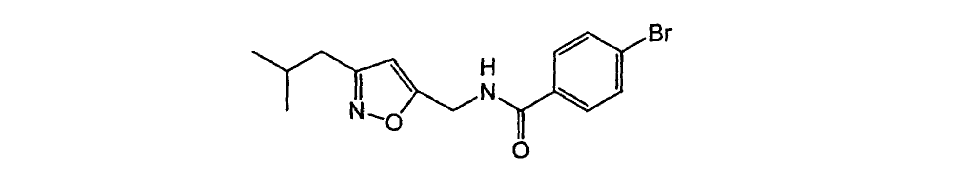

Melting point: 124 to 125°C.

### Example 18(76): Preparation of N-[(3-isobutyl-5-isoxazolyl)methyl]-4-trifluoromethylbenzamide (Compound number III-257):

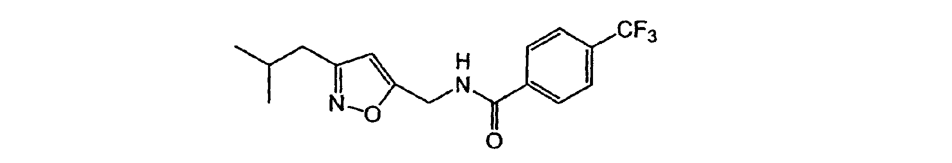

Melting point: 111 to 112°C.

### Example 18(77): Preparation of N-[(3-isobutyl-5-isoxazolyl)methyl]-4-ethylbenzamide (Compound number III-258) :

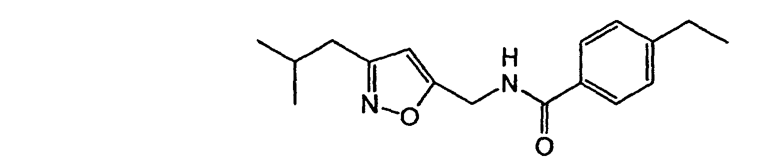

Melting point: 127 to 128°C.

### Example 18(78): Preparation of N-[(3-isobutyl-5-isoxazolyl)methyl]-2-naphthalenecarboxamide (Compound number III-259):

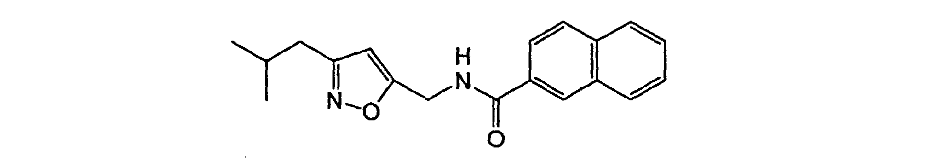

Melting point: 109 to 118°C.

### Example 18(79): Preparation of N-[(3-chloromethyl-5-isoxazolyl)methyl]-4-bromobenzamide (Compound number III-260) :

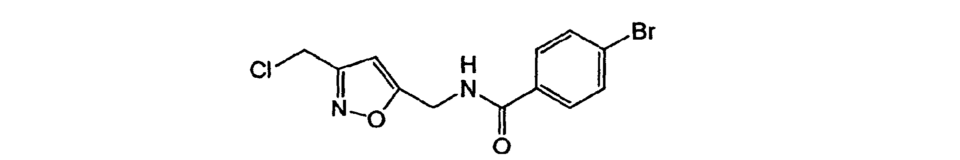

NMR : δ 4.55(2H, s), 4.70(2H, d), 6.35(1H, s), 7.64(4H, d), 7.97(1H, br)

### Example 18(80): Preparation of N-[(3-chloromethyl-5-isoxazolyl)methyl]-4-trifluoromethylbenzamide (Compound number III-261):

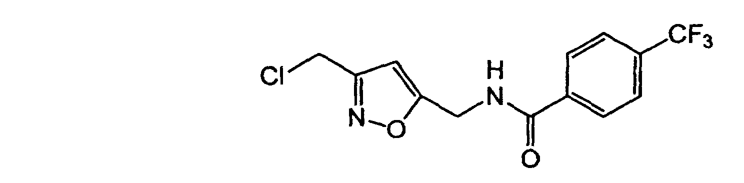

Melting point: 145 to 147°C.

### Example 18(81): Preparation of N-[(3-isobutyl-5-isoxazolyl)methyl]-1-naphthalenecarboxamide (Compound number III-262):

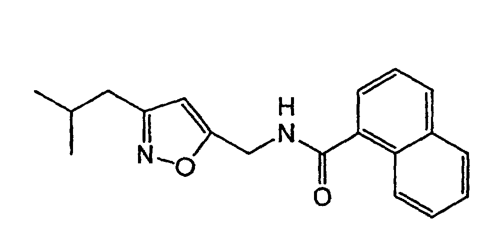

Melting point: 89 to 92°C.

### Example 18(82): Preparation of N-[(3-propyl-5-isoxazolyl)methyl]-4-chlorobenzamide (Compound number III-263) :

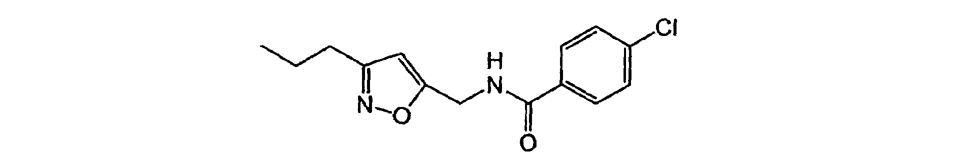

Melting point: 133 to 134°C.

### Example 18(83): Preparation of N-[(3-propyl-5-isoxazolyl)methyl]-4-bromobenzamide (Compound number III-264):

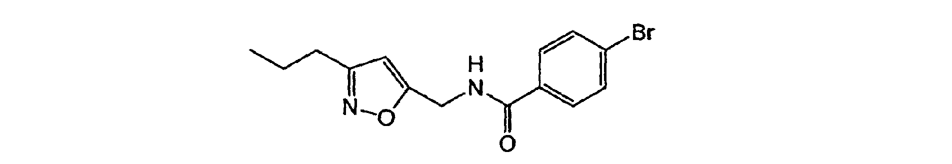

Melting point: 132 to 133°C.

### Example 18(84): Preparation of N-[(3-propyl-5-isoxazolyl)methyl]-2,4-dichlorobenzamide (Compound number III-265):

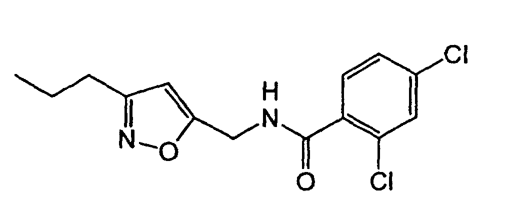

Melting point: 67 to 71°C.

### Example 18(85): Preparation of N-[(3-chloromethyl-5-isoxazolyl)methyl]-β-phenylacrylamide (Compound number III-266) :

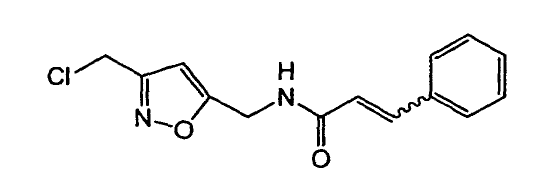

NMR : δ 4.52 (2H, s), 4.67(2H, d), 6.30(1H, s), 6.35(0.5H, s), 6.52(0.5H, s), 6.80(1H, br), 7.13-7.75(6H, m)

### Example 18(86): Preparation of N-[(3-chloromethyl-5-isoxazolyl)methyl]-2-naphthalenecarboxamide (Compound number III-267):

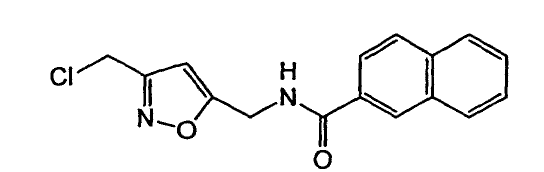

Melting point: 106 to 119°C.

### Example 18(87): Preparation of N-[(3-chloromethyl-5-isoxazolyl)methyl]-1-naphthalenecarboxamide (Compound number III-268):

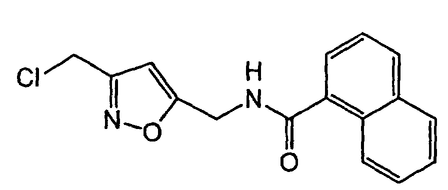

Melting point: 120 to 123°C.

### Example 18(88): Preparation of N-[(3-chloromethyl-5-isoxazolyl)methyl]-4-ethylbenzamide (Compound number III-269):

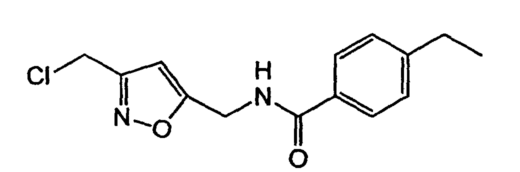

NMR : δ 1.22(3H, t), 2.65(2H, q), 4.51(2H, s), 4.72(2H, d), 6.31(1H, s), 6.72(1H, br), 7.20(2H, d), 7.71(2H, d)

### Example 18(89): Preparation of N-[(3-chloromethyl-5-isoxazolyl)methyl]-4-chloromethyloxybenzamide (Compound number III-270):

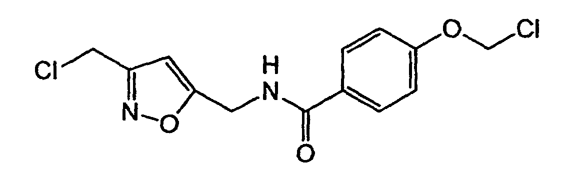

Melting point: 85 to 88°C.

### Example 18(90): Preparation of N-[(3-chloromethyl-5-isoxazolyl)methyl]-2,4-dichlorobenzamide (Compound number III-271):

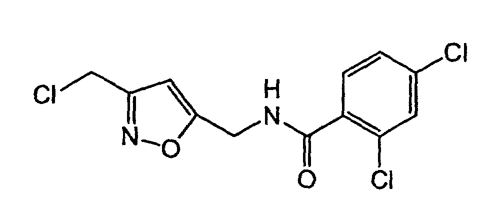

Melting point: 90 to 91°C.

### Example 18(91): Preparation of N-[(3-chloromethyl-5-isoxazolyl)methyl]-3,4-dichlorobenzamide (Compound number III-272):

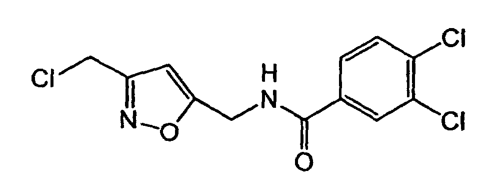

Melting point: 119 to 121°C.

### Example 18(92): Preparation of N-[(3-chloromethyl-5-isoxazolyl)methyl]-2-methylbenzamide (Compound number III-273) :

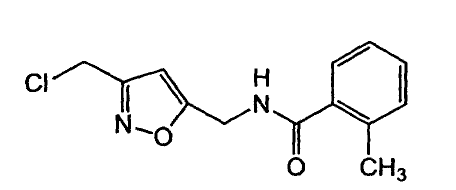

Melting point: 76 to 83°C.

### Example 18(93): Preparation of N-[(3-chloromethyl-5-isoxazolyl)methyl]-3-methylbenzamide (Compound number III-274) :

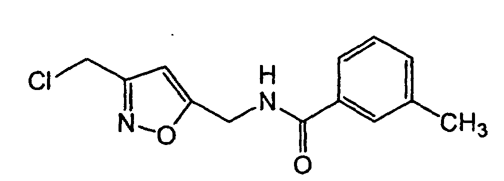

Melting point: 87 to 93°C.

### Example 18(94): Preparation of N-[(3-bromomethyl-5-isoxazolyl)methyl]-2,4-dichlorobenzamide (Compound number III-275) :

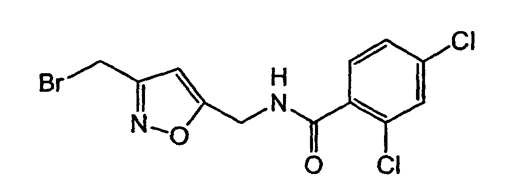

Melting point: 108 to 109°C.

### Example 18(95): Preparation of N-[(3-chloromethyl-5-isoxazolyl)methyl]-2-chlorobenzamide (Compound number III-276) :

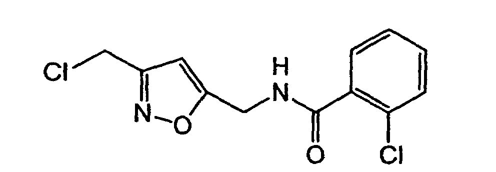

Melting point: 108 to 109°C.

### Example 18(96): Preparation of N-[(3-chloromethyl-5-isoxazolyl)methyl]-3-chlorobenzamide (Compound number III-277) :

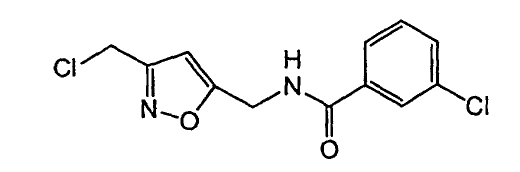

Melting point: 102 to 104°C.

### Example 18(97): Preparation of N-[(3-chloromethyl-5-isoxazolyl)methyl]-4-nitrobenzamide (Compound number III-278) :

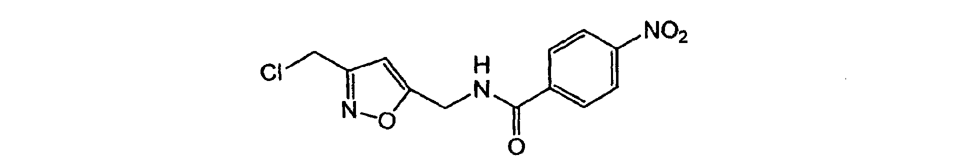

Melting point: 125 to 128°C.

### Example 18(98): Preparation of N-[(3-chloromethyl-5-isoxazolyl)methyl]-2-chloro-4-methanesulfonylbenzamide (Compound number III-279):

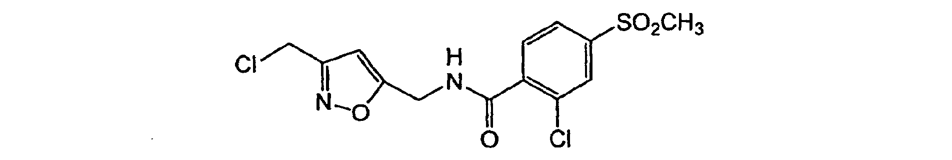

Melting point: 107 to 110°C.

### Example 18(99): Preparation of N-[(3-chloromethyl-5-isoxazolyl)methyl]-2-thiophenecarboxamide (Compound number III-280):

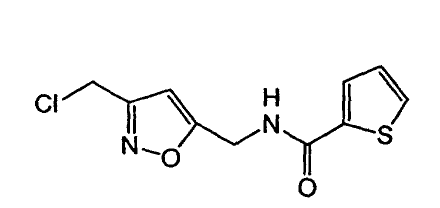

Melting point: 117 to 125°C.

### Example 18(100): Preparation of N-[(3-chloromethyl-5-isoxazolyl)methyl]-4-fluorobenzamide (Compound number III-281) :

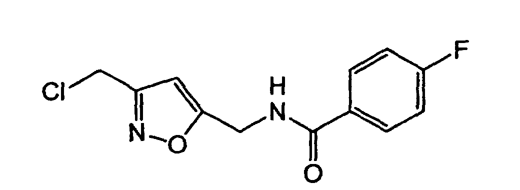

Melting point: 116 to 120°C.

### Example 18(101): Preparation of N-[(3-chloromethyl-5-isoxazolyl)methyl]-4-phenylbenzamide (Compound number III-282):

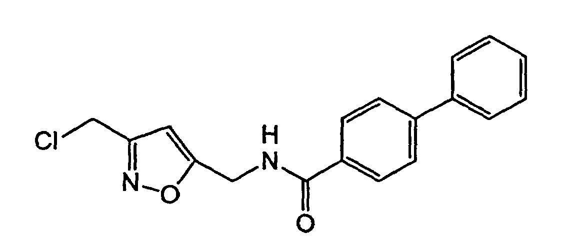

Melting point: 129 to 139°C.

### Example 18(102): Preparation of N-[(3-chloromethyl-5-isoxazolyl)methyl)-4-cyanobenzamide (Compound number III-283):

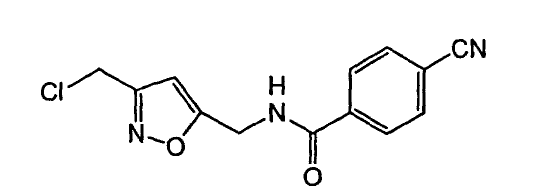

Melting point: 124 to 126°C.

### Example 18(103): Preparation of N-[(3-hydroxymethyl-5-isoxazolyl)methyl]-4-chlorobenzamide (Compound number III-284) :

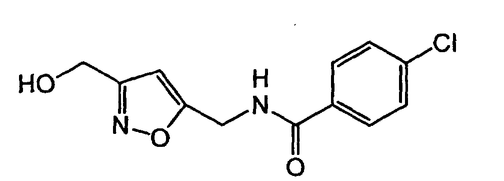

Melting point: 135 to 139°C.

### Example 18(104): Preparation of N-[(3-dimethoxymethyl-5-isoxazolyl)methyl]-4-chlorobenzamide (Compound number III-285) :

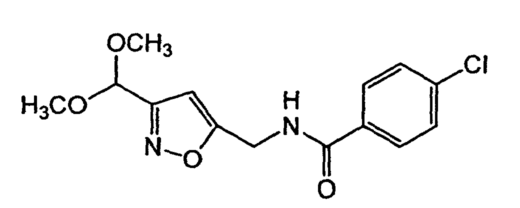

Melting point: 84 to 86°C.

### Example 18(105): Preparation of N-[(3-chloromethyl-5-isoxazolyl)methyl]-2-fluorobenzamide (Compound number III-286) :

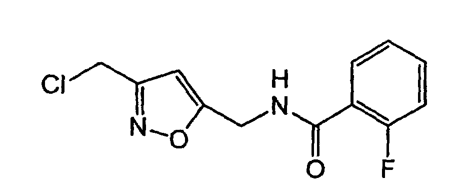

Melting point: 95 to 97°C.

### Example 18(106): Preparation of N-[(3-chloromethyl-5-isoxazolyl)methyl]-3-fluorobenzamide (Compound number III-287):

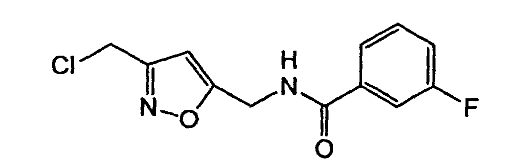

Melting point: 117 to 118°C.

### Example 18(107): Preparation of N-[(3-chloromethyl-5-isoxazolyl)methyl]-4-iodobenzamide (Compound number III-288) :

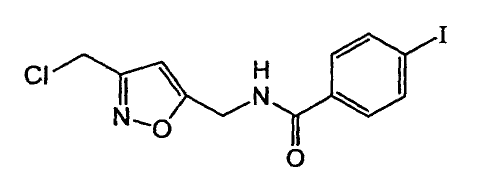

Melting point: 169 to 172°C.

### Example 18(108): Preparation of N-[(3-chloromethyl-5-isoxazolyl)methyl]-4-propylbenzamide (Compound number III-289) :

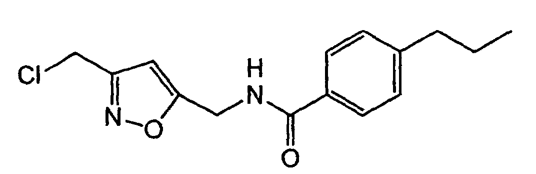

Melting point: 110 to 113°C.

### Example 18(109): Preparation of N-[(3-chloromethyl-5-isoxazolyl)methyl]-4-methylthiobenzamide (Compound number III-290):

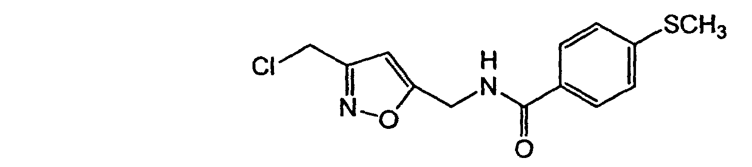

Melting point: 147 to 149°C.

### Example 18(110): Preparation of N-[(3-chloromethyl-5-isoxazolyl)methyl]-4-methanesulfonylbenzamide (Compound number III-291):

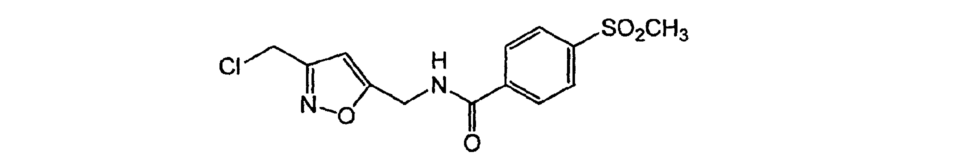

Melting point: 134 to 135°C.

### Example 18(111): Preparation of N-[(3-chloromethyl-5-isoxazolyl)methyl]-2,6-dichlorobenzamide (Compound number III-292):

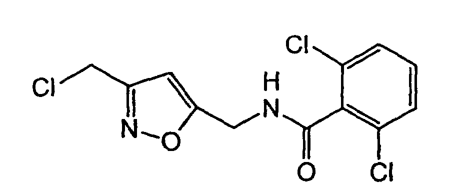

Melting point: 94 to 95°C.

### Example 18(112): Preparation of N-[(3-chloromethyl-5-isoxazolyl)methyl]-3,5-dichlorobenzamide (Compound number III-293):

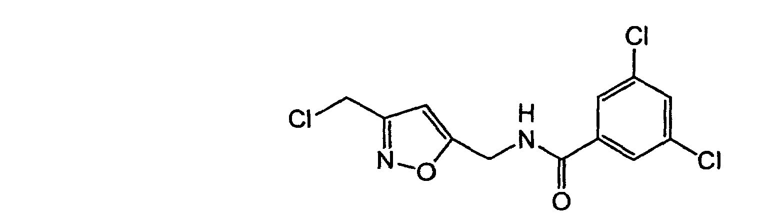

Melting point: 136 to 137°C.

### Example 18(113): Preparation of N-[(3-chloromethyl-5-isoxazolyl)methyl]-2,3,4-trifluorobenzamide (Compound number III-294):

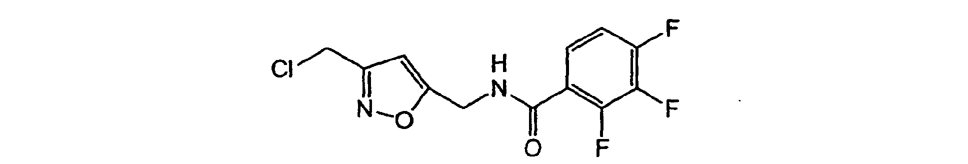

Melting point: 105 to 106°C.

### Example 18(114): Preparation of N-[(3-chloromethyl-5-isoxazolyl)methyl]-2,4,5-trifluorobenzamide (Compound number III-295):

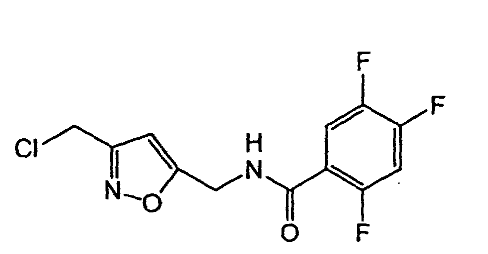

Melting point: 116 to 118°C.

### Example 18(115): Preparation of N-[(3-chloromethyl-5-isoxazolyl)methyl]-2-pyridinecarboxamide (Compound number III-296) :

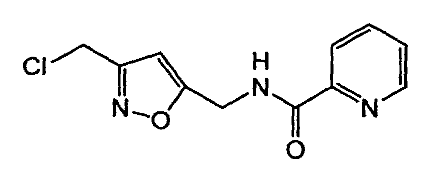

Melting point: 132 to 134°C.

### Example 18(116): Preparation of N-[(3-chloromethyl-5-isoxazolyl)methyl]-4-pyridinecarboxamide (Compound number III-297):

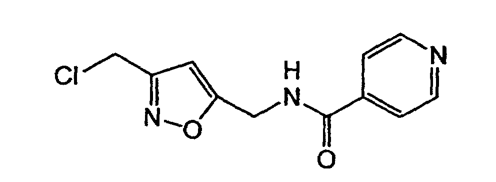

Melting point: 104 to 105°C.

### Example 18(117): Preparation of N-[(3-fluoromethyl-5-isoxazolyl)methyl]-4-chlorobenzamide (Compound number III-298) :

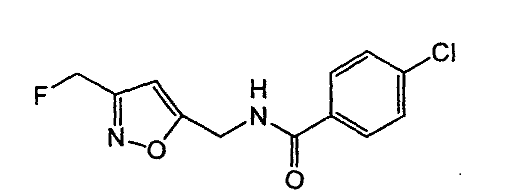

Melting point: 147 to 149°C.

### Example 18(118): Preparation of N-[(3-chloromethyl-5-isoxazolyl)methyl]-2,3,6-trifluorobenzamide (Compound number III-299):

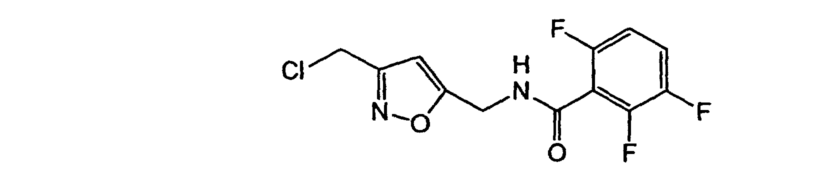

Property: oily;
NMR : δ 4.50(2H,s), 4.60(2H,d), 6.25(1H,s), 6.60-7.00(1H,m), 7.00-7.20(1H,m), 7.45-7.70(1H,brs)

### Example 18(119): Preparation of N-{[3-(1,2,4-triazol-1-yl)phenyloxymethyl-5-isoxazolyl]methyl}-4-methoxybenzamide (Compound number IV-73):

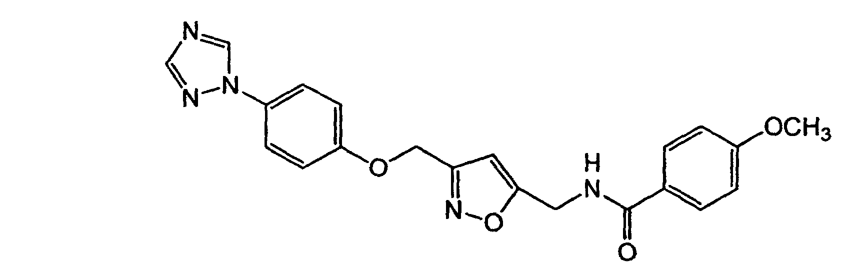

NMR : δ 3.87(3H, s), 4.72(2H, d), 5.17(2H, s), 6.35(1H, s), 6.80-8.50(10H, m), 9.08(1H, s)

### Example 18(120): Preparation of N-{[3-(4-methoxyiminomethylphenyl)oxymethyl-5-isoxazolyl]methyl}-4-methoxybenzamide (Compound number IV-75):

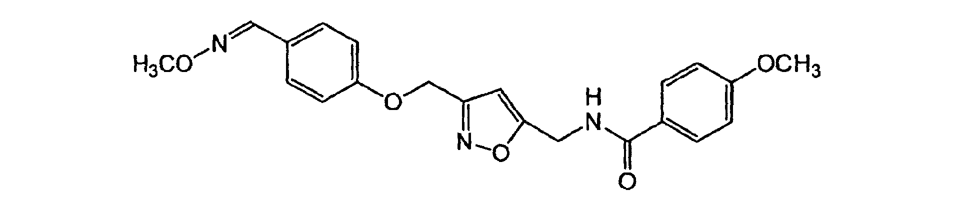

Melting point: 143 to 151°C.

### Example 18(121): Preparation of N-{[3-(2,6-dimethoxyphenyl)oxymethyl-5-isoxazolyl]methyl}-4-methoxybenzamide (Compound number IV-76):

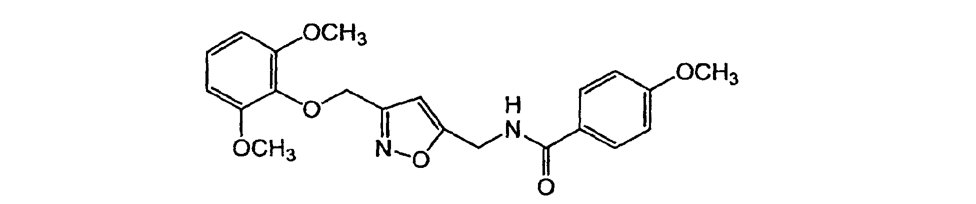

Property: paste;
NMR : δ 3.73 (3H, s), 3.80 (3H, s), 3.83 (3H, s), 4.67(2H, d), 5.04(2H, s), 6.45(1H, s), 6.49-7.85(8H, m)

### Example 18(122): Preparation of N-{[3-(1,2-diazol-1-yl)methyl-5-isoxazolyl]methyl}-4-methoxybenzamide (Compound number IV-77):

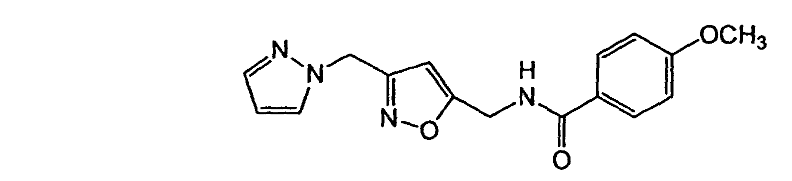

Property: paste;
NMR : δ 3.81(3H, s), 4.68(2H, s), 4.68(2H, d), 6.24(1H, s), 6.28(1H, br), 6.70(1H, br), 6.77-7.90(6H, m)

### Example 18(123): Preparation of N-{[3-(1-piperidyl)methyl-5-isoxazolyl]methyl}-4-methoxybenzamide (Compound number IV-24) :

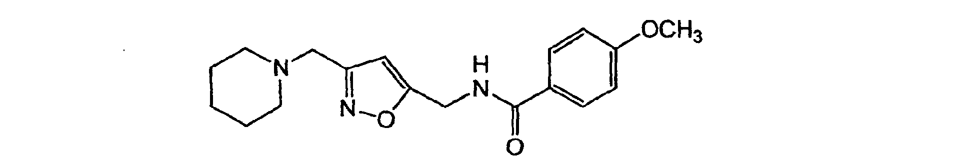

Property: oily;
NMR : δ 3.51(2H, s), 3.84(3H, s), 4.73(2H, d), 6.27(1H, s), 6.73(1H, br), 6.91(2H, d), 7.77(2H, d)

### Example 18(124): Preparation of N-[(3-morpholinomethyl-5-isoxazolyl)methyl]-4-methoxybenzamide (Compound number IV-25):

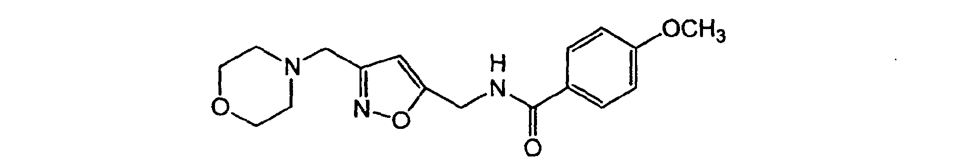

Property: paste;
NMR : δ 2.32-2.52(4H, m), 3.52(2H, s), 3.56-3.73(4H, m), 3.83(3H, s), 4.68(2H, d), 6.21(1H, s), 6.87(2H, d), 7.30(1H, br), 7.80(1H, d)

### Example 18(125): Preparation of N-{[3-(1,3-dihydroindol-2-yl)methyl-5-isoxazolyl]methyl}-4-methoxybenzamide (Compound number IV-78):

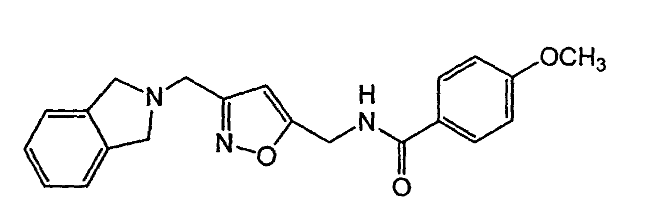

Property: paste;
NMR : δ 3.84(3H, s), 3.92(2H, s), 3.98(4H, s), 4.72(2H, d), 6.28(1H, s), 6.56(1H, br), 6.90(2H, d), 7.14(4H, s), 7.73(2H, d)

### Example 18(126): Preparation of N-{[3-(2,3-dihydroindol-1-yl)methyl-5-isoxazolyl]methyl}-4-methoxybenzamide (Compound number IV-79):

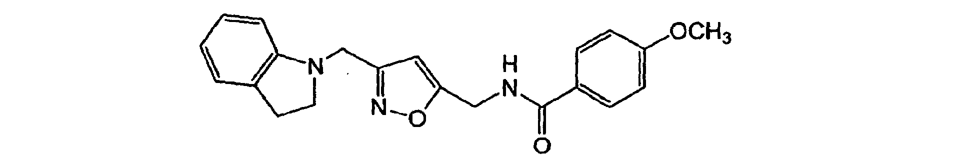

Property: paste;
NMR : δ 2.80-3.28(2H, m), 3.80(3H, s), 3.72-4.28(2H, m), 4.68(2H, d), 5.28(2H, s), 6.30(1H, s), 6.72-7.87(9H, m)

### Example 18(127): Preparation of N-{[3-(2,6-dimethylphenyl)oxymethyl-5-isoxazolyl]methyl}-4-methoxybenzamide (Compound number IV-80):

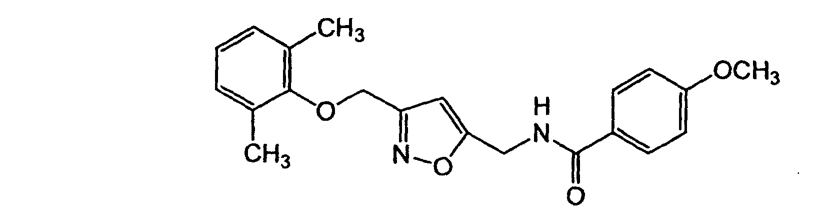

Melting point: 139 to 146°C.

### Example 18(128): Preparation of N-{[3-(4-acetylaminophenyl)oxymethyl-5-isoxazolyl]methyl}-4-methoxybenzamide (Compound number IV-81):

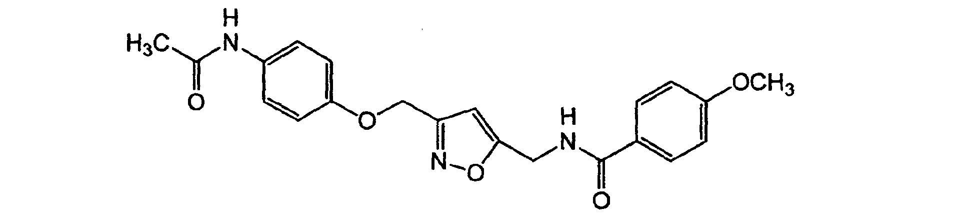

Melting point: 183 to 188°C.

### Example 18(129): Preparation of N-{[3-(2-methoxycarbonylphenyl)oxymethyl-5-isoxazolyl]methyly-4-methoxybenzamide (Compound number IV-82):

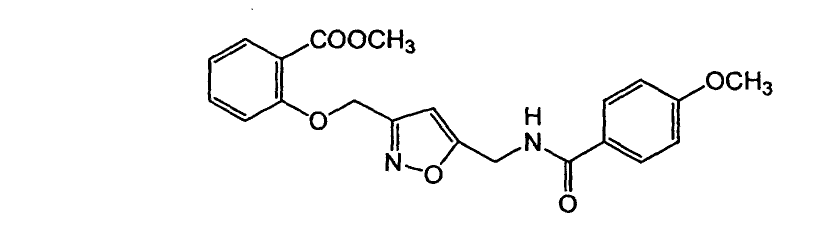

Melting point: 85 to 88°C.

### Example 18(130): Preparation of N-{[3-(3-methoxycarbonylphenyl)oxymethyl-5-isoxazolyl]methyl}-4-methoxybenzamide (Compound number IV-83):

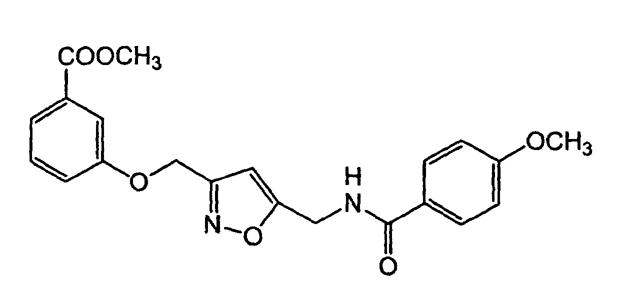

Melting point: 106 to 112°C.

### Example 18(131): Preparation of N-[(3-phenylaminocarbonyl-5-isoxazolyl)methyl]-4-methoxybenzamide (Compound number IV-84) :

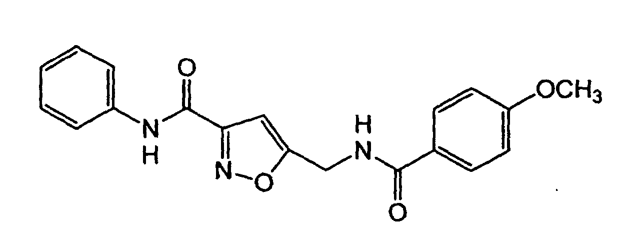

Melting point: 186°C.

### Example 18(132): Preparation of N-{[3-(4-methoxycarbonylphenyl)oxymethyl-5-isoxazolyl]methyl}-4-methoxybenzamide (Compound number IV-85):

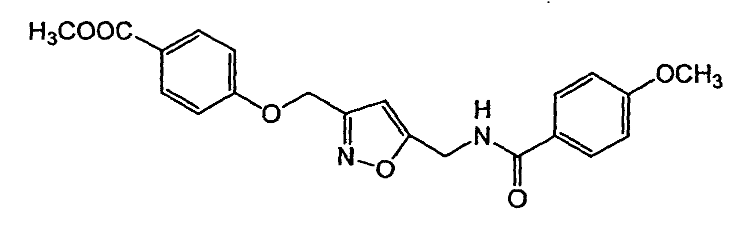

Melting point: 155 to 162°C.

### Example 18(133): Preparation of N-{[3-(2,6-difluorophenyl)oxymethyl-5-isoxazolyl]methyl}-4-methoxybenzamide (Compound number IV-86):

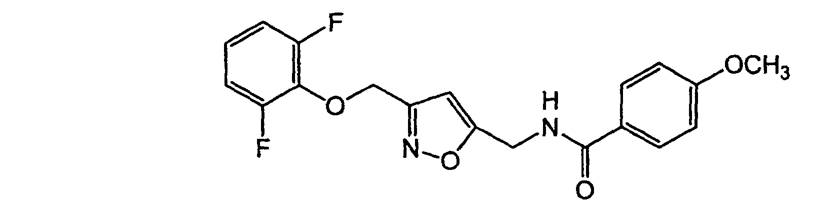

Melting point: 111 to 116°C.

### Example 18(134): Preparation of N-{[3-(4-phenylpiperazinyl)methyl-5-isoxazolyl]methyl}-4-methoxybenzamide (Compound number IV-28):

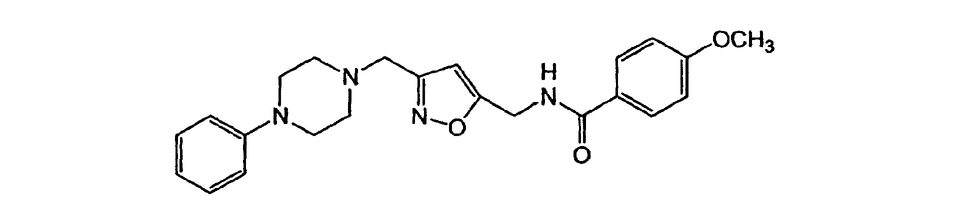

Melting point: 148 to 158°C.

### Example 18(135): Preparation of N-{[3-(4-acetylphenyl)oxymethyl-5-isoxazolyl]methyl}-4-methoxybenzamide (Compound number IV-87):

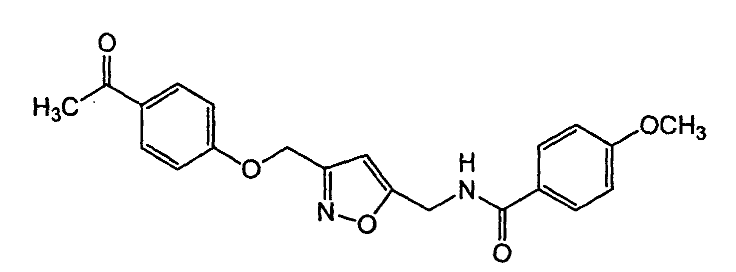

Melting point: 148 to 156°C.

### Example 18(136): Preparation of N-{[3-(4-methylaminocarbonylphenyl)oxymethyl-5-isoxazolyl]methyl}-4-methoxybenzamide (Compound number IV-88):

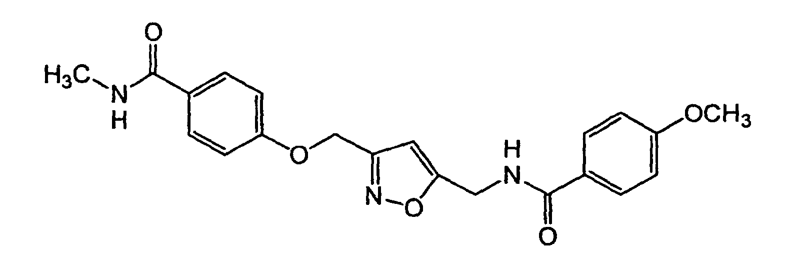

Melting point: 211 to 214°C.

### Example 18(137): Preparation of N-{[3-(2-(2-furyl)ethenyl)-5-isoxazolyl]methyl}-4-methoxybenzamide (Compound number IV-89) :

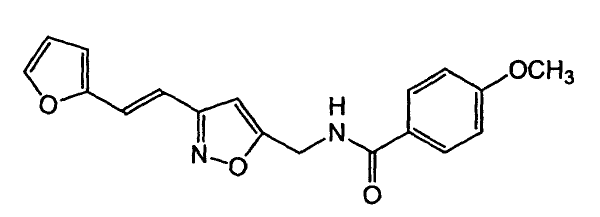

Melting point: 159 to 164°C.

### Example 18(138): Preparation of N-{[3-(4-pyridyl)oxymethyl-5-isoxazolyl]methyl}-4-methoxybenzamide (Compound number IV-90):

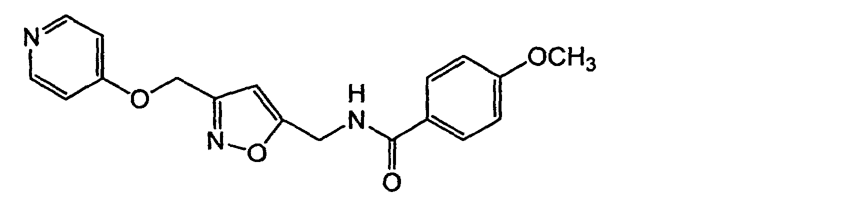

Melting point: 154 to 159°C.

### Example 18(139): Preparation of N-{[3-(3-pyridyl)oxymethyl-5-isoxazolyl]methyl)-4-methoxybenzamide (Compound number IV-91) :

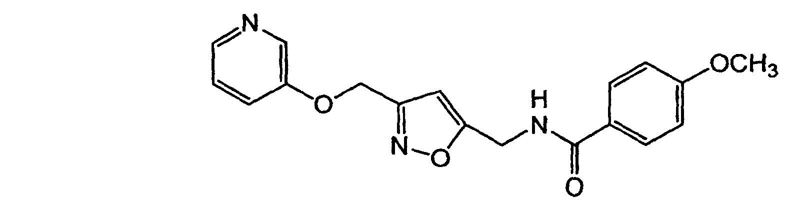

Property: oily;
NMR : δ 3.78(3H, s), 4.66(2H, d), 5.08(2H, s), 6.32(1H, s), 6.70-8.35(9H, m)

### Example 18(140): Preparation of N-{[3-(2-pyridyl)oxymethyl-5-isoxazolyl]methyl}-4-methoxybenzamide (Compound number IV-92):

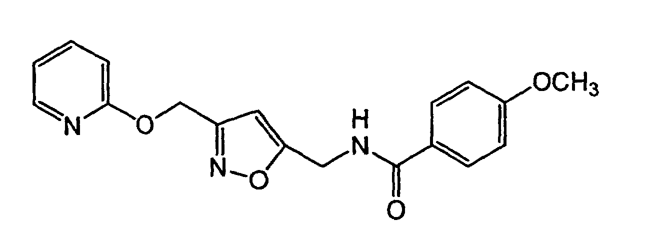

Melting point: 169 to 172°C.

### Example 18(141): Preparation of N-{[3-(4-difluoromethyloxyphenyl)aminocarbonyl-5-isoxazolyl]methyl}-4-methoxybenzamide (Compound number IV-93):

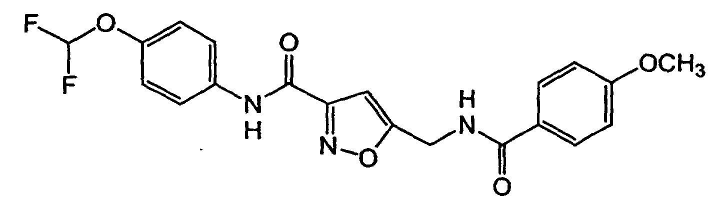

NMR : δ 3.85(3H, s), 4.76(2H, d), 6.60-8.00(9H, m), 8.30(1H, d), 9.30(1H, br)

### Example 18(142): Preparation of N-{[3-(4-methoxyphenyl)aminocarbonyl-5-isoxazolyl]methyl}-4-methoxybenzamide (Compound number IV-94):

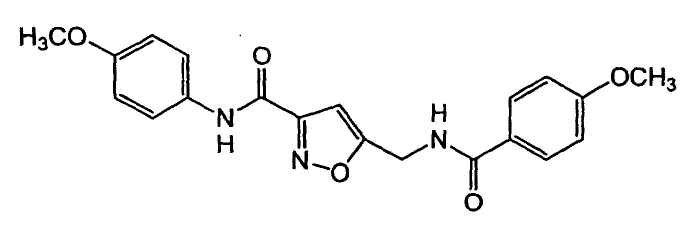

Melting point: 186 to 188°C.

### Example 18 (143) : Preparation of N-{[3-(4-fluorophenyl)aminocarbonyl-5-isoxazolyl]methyl}-4-methoxybenzamide (Compound number IV-95):

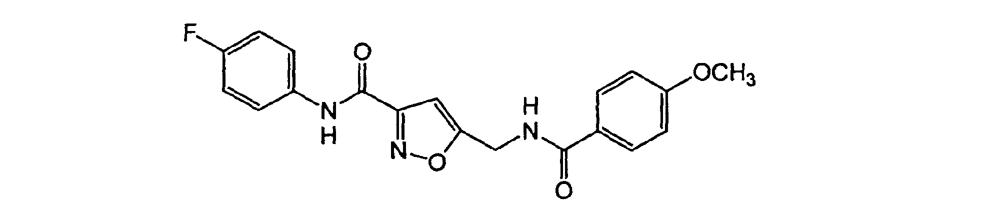

Melting point: 191 to 193°C.

### Example 18(144): Preparation of N-{[3-(2-chlorophenyl)aminocarbonyl-5-isoxazolyl]methyl}-4-methoxybenzamide (Compound number IV-96):

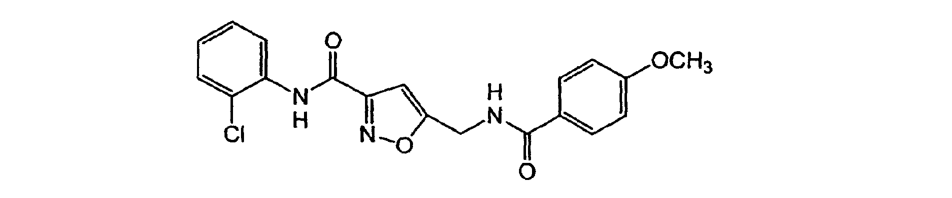

NMR : δ 3.83(3H, s), 4.76(2H,d), 6.60-8.00(9H, m), 8.20(1H, d), 9.10(1H, br)

### Example 18(145): Preparation of N-{[3-(4-cyanophenyl)aminocarbonyl-5-isoxazolyl]methyl}-4-methoxybenzamide (Compound number IV-97):

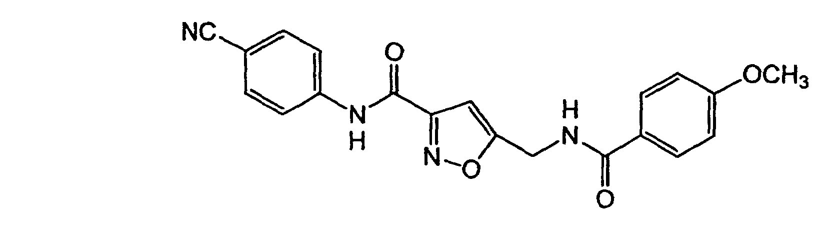

Melting point: 138 to 140°C.

### Example 18(146): Preparation of N-[(3-benzoyl-5-isoxazolyl)methyl]-4-methoxybenzamide (Compound number IV-98):

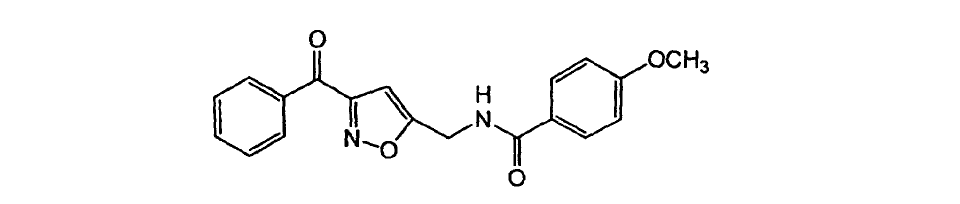

Melting point: 118 to 127°C.

### Example 18(147): Preparation of N-[(3-phenoxymethyl-5-isoxazolyl)methyl]-β-phenylacrylamide (Compound number IV-99):

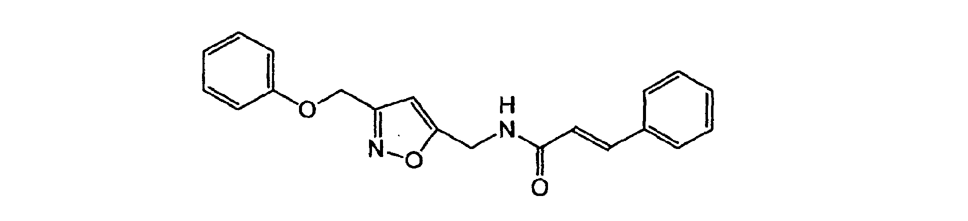

Melting point: 110 to 116°C.

### Example 18(148): Preparation of N-[(3-benzoylaminomethyl-5-isoxazolyl)methyl]-4-methoxybenzamide (Compound number IV-100) :

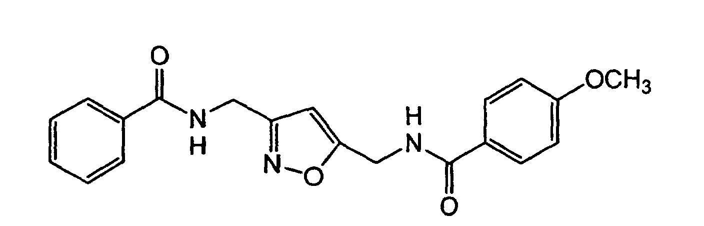

Melting point: 118 to 124°C.

### Example 18(149): Preparation of N-{[3-(4-methoxybenzoyl)aminomethyl-5-isoxazolyl]methyl}-4-methoxybenzamide (Compound number IV-101):

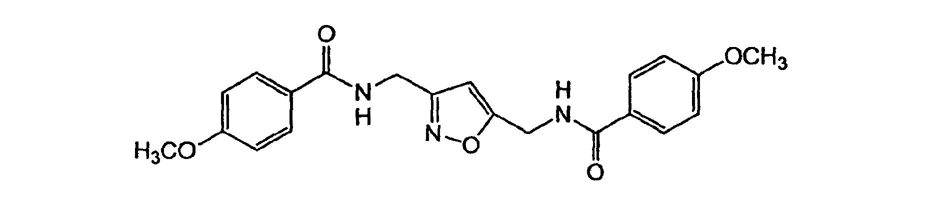

Melting point: 176 to 179°C.

### Example 18(150): Preparation of N-[(3-phenylaminocarbonylaminomethyl-5-isoxazolyl)methyl]-4-methoxybenzamide (Compound number IV-102):

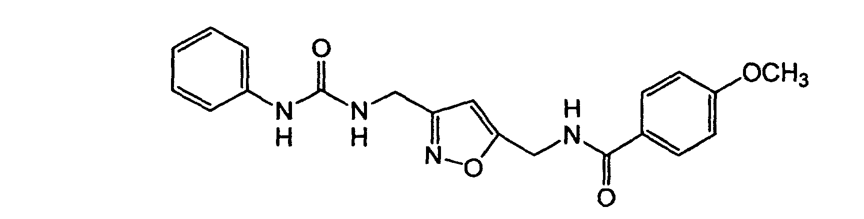

Melting point: 169 to 178°C.

### Example 18(151): Preparation of N-[(3-methoxycarbonylaminomethyl-5-isoxazolyl)methyl]-4-methoxybenzamide (Compound number IV-103):

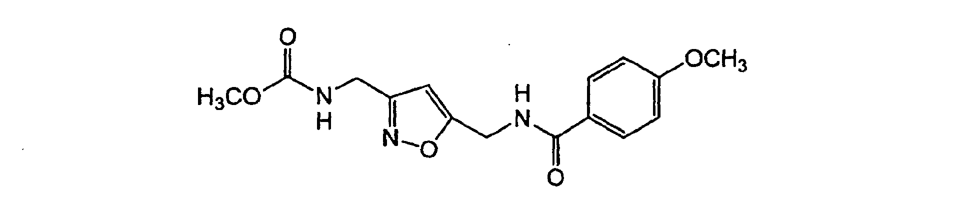

Melting point: 143 to 145°C.

### Example 18(152): Preparation of N-{[3-(4-methyl-1-butenyl)-5-isoxazolyl]methyl}-4-methoxybenzamide (Compound number IV-104) :

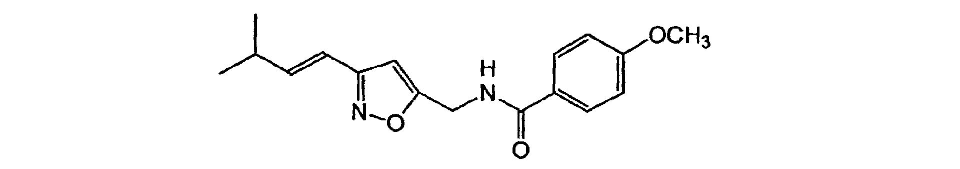

Melting point: 104 to 113°C.

### Example 18(153): Preparation of N-{[3-(3-chlorophenyl)aminocarbonyl-5-isoxazolyl]methyl}-4-methoxybenzamide (Compound number IV-105):

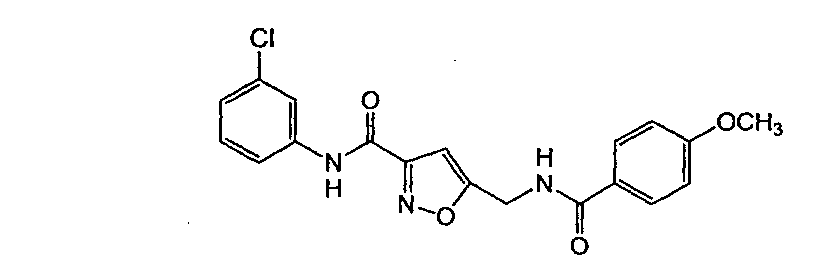

Melting point: 164 to 166°C.

### Example 18(154): Preparation of N-{[3-(4-chlorophenyl)aminocarbonyl-5-isoxazolyl)methyl}-4-methoxybenzamide (Compound number IV-106):

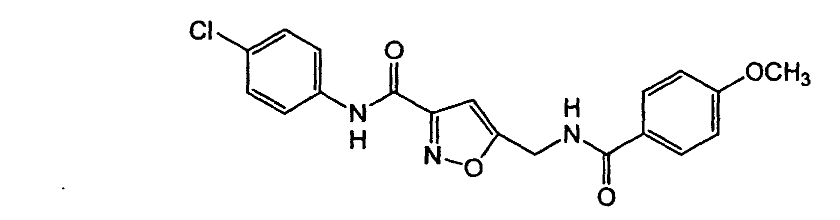

Melting point: 185 to 186°C.

### Example 18(155): Preparation of N-{[3-(2,4-difluorophenyl)aminocarbonyl-5-isoxazolyl]methyl}-4-methoxybenzamide (Compound number IV-107):

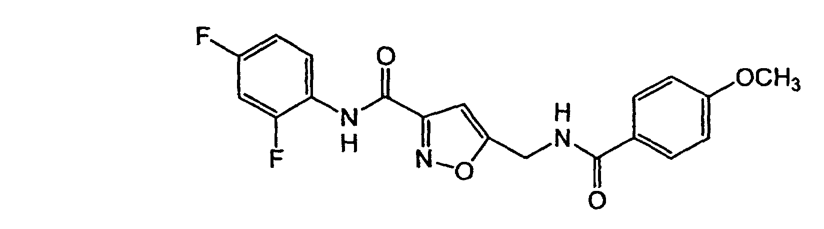

Melting point: 165 to 167°C.

### Example 18(156): Preparation of N-{[3-(4-tolyl)aminocarbonyl-5-isoxazolyl]methyl}-4-methoxybenzamide (Compound number IV-108):

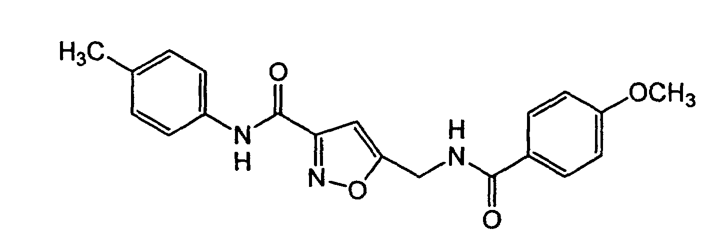

Melting point: 174 to 176°C.

### Example 18(157): Preparation of N-[(3-benzylaminocarbonyl-5-isoxazolyl)methyl]-4-methoxybenzamide (Compound number IV-109) :

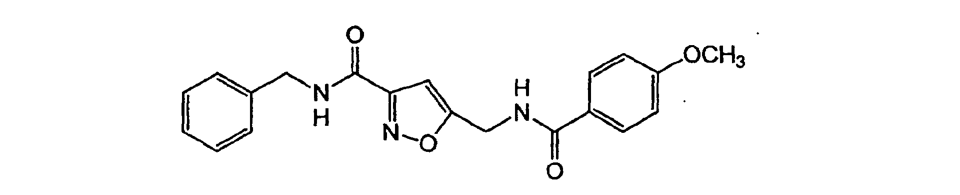

Melting point: 171 to 172°C.

### Example 18(158): Preparation of N-[(3-phenoxymethyl-5-isoxazolyl)methyl]benzamide (Compound number IV-44):

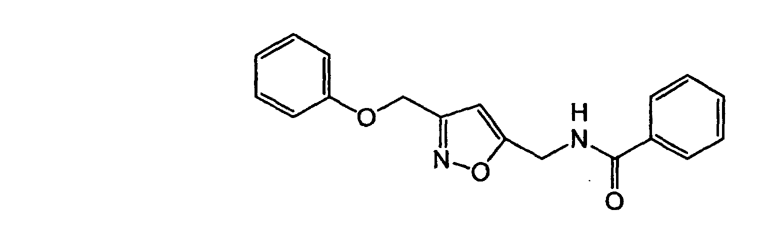

Melting point: 85 to 87°C.

### Example 18(159): Preparation of N-[(3-phenylethynyl-5-isoxazolyl)methyl]-4-methoxybenzamide (Compound number IV-110):

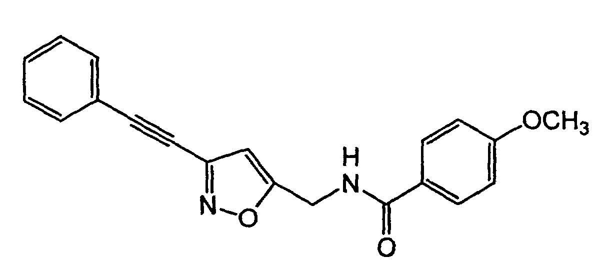

Melting point: 88 to 101°C.

### Example 18(160): Preparation of N-[(3-methoxymethyl-5-isoxazolyl)methyl]-4-methoxybenzamide (Compound number IV-111) :

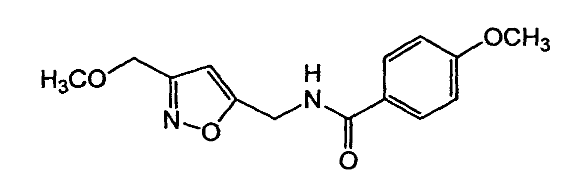

Melting point: 71 to 85°C.

### Example 18(161): Preparation of N-{[3-(2-cyanophenyl)aminocarbonyl-5-isoxazolyl]methyl}-4-methoxybenzamide (Compound number IV-112):

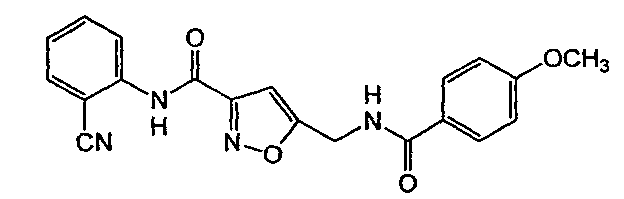

Property: gum-like;
NMR : δ 3.86(3H, s), 4.75(2H, d), 6.40-8.00(10H, m), 8.20(1H, br), 9.40(1H, br)

### Example 18(162): Preparation of N-[(3-phenylaminocarbonyl-5-isoxazolyl)methyl]benzamide (Compound number IV-113):

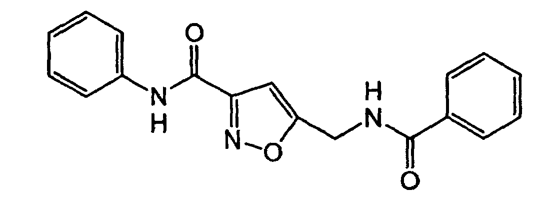

Melting point: 177 to 178°C.

### Example 18(163): Preparation of N-[(3-phenylaminocarbonyl-5-isoxazolyl)methyl]-4-chlorobenzamide (Compound number IV-114):

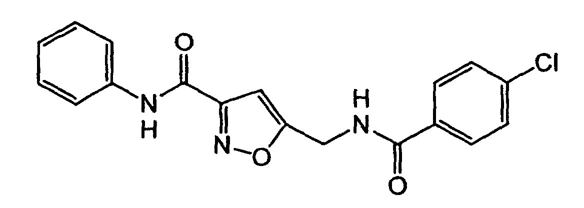

Melting point: 200°C or more.

### Example 18(164): Preparation of N-[(3-phenylaminocarbonyl-5-isoxazolyl)methyl]-4-methylbenzamide (Compound number IV-115) :

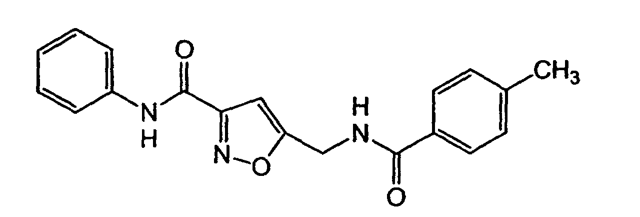

Melting point: 193 to 195°C.

### Example 18(165): Preparation of N-[(3-phenylaminocarbonyl-5-isoxazolyl)methyl]-3-methylbenzamide (Compound number IV-116) :

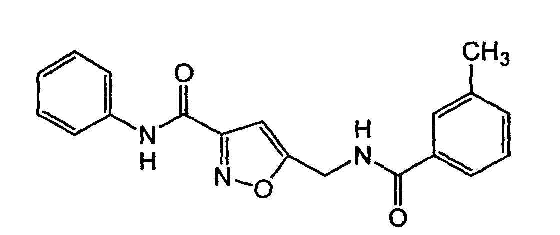

Melting point: 151 to 152°C.

### Example 18(166): Preparation of N-[(3-phenylaminocarbonyl-5-isoxazolyl)methyl]-2-methylbenzamide (Compound number IV-117):

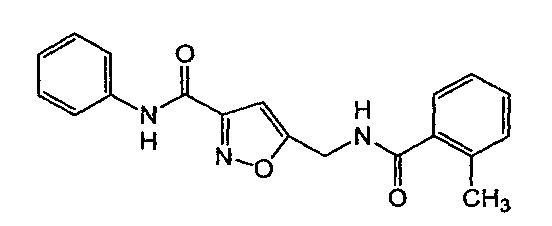

Melting point: 162 to 163°C.

### Example 18(167): Preparation of N-[(3-isopropylaminocarbonyl-5-isoxazolyl)methyl]-4-methoxybenzamide (Compound number IV-118):

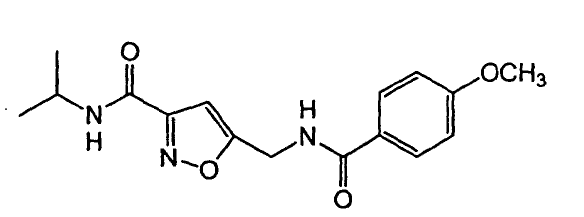

Melting point: 171 to 172°C.

### Example 18(168): Preparation of N-[(3-tert-butylaminocarbonyl-5-isoxazolyl)methyl]-4-methoxybenzamide (Compound number IV-119):

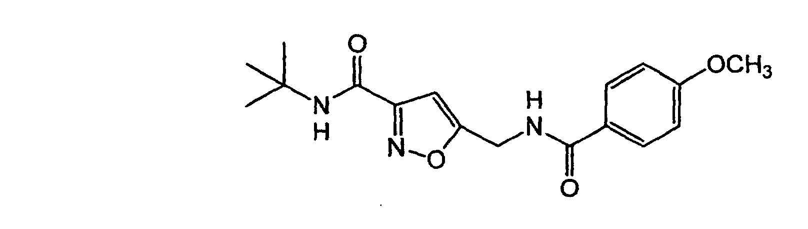

Melting point: 128 to 129°C.

### Example 18(169): Preparation of N-[(3-cyclopentylaminocarbonyl-5-isoxazolyl)methyl]-4-methoxybenzamide (Compound number IV-120):

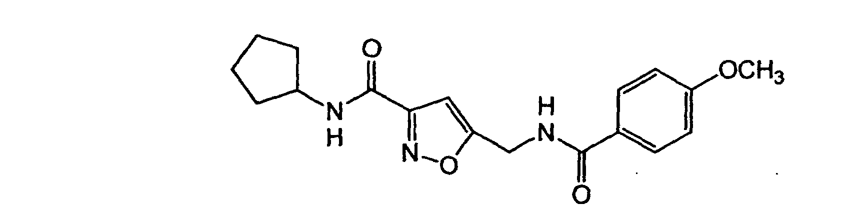

Melting point: 138 to 140°C.

### Example 18(170): Preparation of N-[(3-phenoxymethyl-5-isoxazolyl)methyl]cyclohexanecarboxamide (Compound number IV-121) :

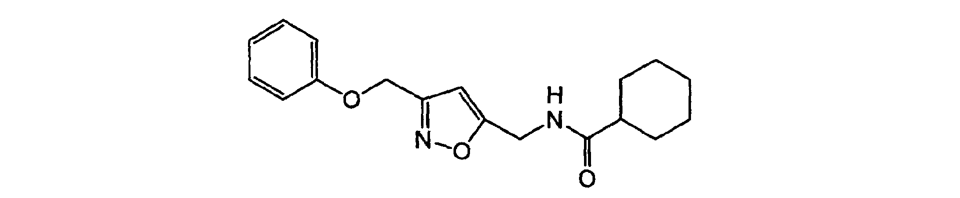

Melting point: 86 to 93°C.

### Example 18(171): Preparation of N-[(3-phenoxymethyl-5-isoxazolyl)methyl]-2,6-dichloropyridine-4-carboxamide (Compound number IV-122):

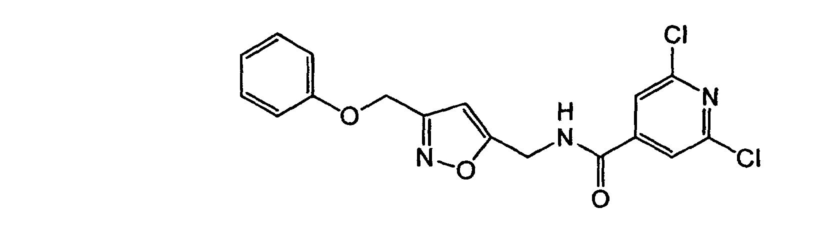

Melting point: 93 to 99°C.

### Example 18(172): Preparation of N-[(3-benzyliminomethyl-5-isoxazolyl)methyl]-4-methoxybenzamide (Compound number IV-123) :

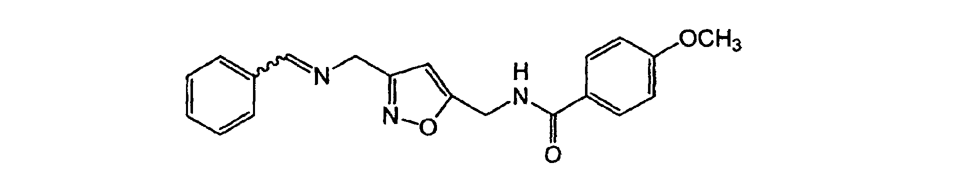

Property: amorphous;
NMR : δ 3.74(3H, s), 4.66(2H, d), 4.74 (2H, s), 6.23(1H, s), 6.84(2H, d), 7.13-7.89(7H, m), 8.35(1H, s)

### Example 18(173): Preparation of N-[(3-benzyloxyiminomethyl-5-isoxazolyl)methyl]-4-methoxybenzamide (Compound number IV-124) :

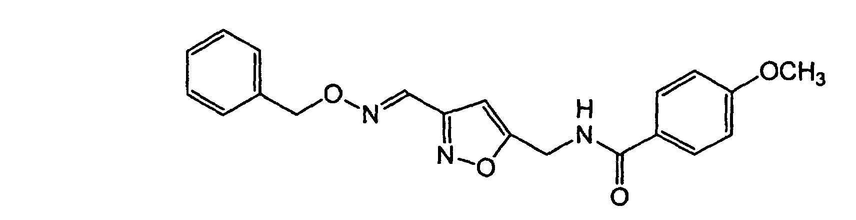

NMR : δ 3.82 (3H, s), 4.71 (2H, d), 5.19 (2H, s), 6.51(1H, s), 6.74(1H, br), 6.89(2H, d), 7.18-7.45(5H, m), 7.75(2H, d), 8.15(1H, s)

### [Formulation Example]

### Formulation Example 1

The compound of I-25 (16 parts by mass), 28 parts by mass of bentonite, 52 parts by mass of talc, 2 parts by mass of sodium dodecylbenzene sulfonate, and 2 parts by mass of sodium lignin sulfonate were mixed and milled. About 20 parts by mass of water was added, and the mixture was kneaded with a kneader, subsequently granulated through a granulator, then dried and sorted to afford granules.

### Formulation Example 2

The compound of II-3 (20 parts by mass), 30 parts by mass of diatomite, 36 parts by mass of clay, 5 parts by mass of white carbon, 4 parts by mass of sodium lignin sulfonate, and 5 parts by mass of sodium alkyl naphthalene sulfonate were mixed and milled to afford water-dispersible powder.

### Formulation Example 3

The compound of III-12 (20 parts by mass), 65 parts by mass of kaolin clay, and 15 parts by mass of talc were thoroughly mixed and milled to afford 20% powder.

### Formulation Example 4

The compound of IV-8 (12.5 parts by mass), 4 parts by mass of ethylene glycol, 5 parts by mass of SORPOL 3940 (trade name, supplied from TOHO Chemical Industry Co., Ltd.), 1 part by mass of RUNOX 1000C (trade name, supplied from TOHO Chemical Industry Co., Ltd.), and 77 parts by mass of water were thoroughly mixed, and milled in a wet manner until an average particle size became 5 µm or less. Subsequently, 0.5 part by mass of xanthane gum was mixed to afford a flowable agent.

### Formulation Example 5

The compound of I-28 (20 parts by mass ), 14 parts by mass of polyoxyethylene styrylphenyl ether, 10 parts by mass of calcium dodecylbenzene sulfonate, and 56 parts by mass of xylene were mixed to afford an emulsion.

### Formulation Example 6

The compound of II-100 (17 parts by mass), 9.5 parts by mass of potassium chloride, 2 parts by mass of NEWCALGEN FS-1 (trade name, supplied from Takemoto Yushi Co., Ltd.), 1 part by mass of dextrin, and 0.5 part by mass of xanthane gum were mixed, and 20 parts by mass of water was added. The mixture was kneaded with a kneader, granulated through a granulator, and then dried/sorted to afford granules. Those granules were filled in a pouched container made from water-soluble film, and a filling opening was sealed with heat to afford a jumbo agent.

### [Evaluation Examples]

Next, Evaluation Examples of the compounds of the invention as the agri-horticultural fungicides are described.

The following compounds A and B described in JP 52-154525 A were used as reference drugs.

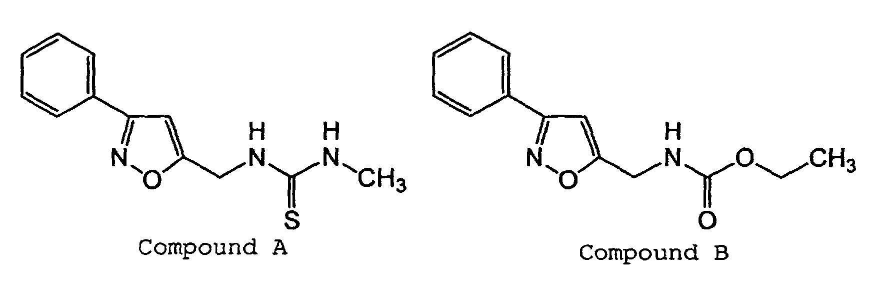

### Evaluation Example 1. Effect test for rice blast

The formulation of the compound of the invention prepared according to Formulation Example 2 was diluted with water to have a concentration of 200 ppm, and subsequently a sufficient amount of the solution was sprayed with a spraygun on rice (cultivar: Koshihikari, planting 15 seedlings) in a 3 leaf stage grown in a plastic pot with a diameter of 6 cm. Next day, a sporal suspension of *Pyricularia oryzae* which was a causative fungus of the rice blast was sprayed to inoculate. The pot was retained at 22°C under a wet condition for 24 hours, subsequently left in a green house for 7 days. Then, lesion numbers on the third leaves were grossly surveyed, and evaluated according to the criteria shown in Table 5. The results are shown in Table 6.

**Table 5**

| Evaluation | Lesion area rate (%) |
|---|---|
| 5 | 0 |
| 4 | 1∼5 |
| 3 | 6∼10 |
| 2 | 11∼20 |
| 1 | 21∼41 |
| 0 | 41 or more |

**Table 6-1 :**

| Effect for rice blast | | | | | |
|---|---|---|---|---|---|
| Compound No. | Example No. | Evaluation | Compound No. | Example No | Evaluation |
| I-4 | 17(1) | 5 | II-106 | 18(12) | 3 |
| I-6 | 17(2) | 5 | II-108 | 18(14) | 1 |
| I-8 | 18(3) | 3 | II-110 | 18(16) | 1 |
| I-25 | 17(3) | 5 | II-112 | 18(18) | 3 |
| I-26 | 5 | 5 | II-113 | 18(19) | 2 |
| I-27 | 17(4) | 5 | II-114 | 18(20) | 3 |
| I-28 | 1 | 4 | II-115 | 18 (21) | 3 |
| I-29 | 17(5) | 5 | III-2 | 17(29) | 4 |
| I-30 | 17(6) | 5 | III-4 | 17(30) | 3 |
| I-31 | 12 | 4 | III-5 | 9 | 4 |
| I-32 | 17(7) | 5 | III-6 | 17(31) | 5 |
| I-33 | 17(8) | 5 | III-10 | 17(32) | 5 |
| I-38 | 17(9) | 5 | III-11 | 17(33) | 5 |
| I-41 | 17(10) | 3 | III-12 | 17(34) | 5 |
| I-45 | 17(12) | 4 | III-13 | 17(35) | 5 |
| I-47 | 17(14) | 4 | III-14 | 17(36) | 5 |
| I-48 | 17(15) | 4 | III-17 | 17(37) | 5 |
| I-49 | 17(16) | 3 | III-18 | 7 | 5 |
| I-63 | 18(2) | 2 | III-19 | 17(38) | 5 |
| I-64 | 18(4) | 2 | III-26 | 17(39) | 5 |
| II-2 | 6 | 5 | III-27 | 17(40) | 5 |
| II-3 | 17(17) | 3 | III-28 | 17(41) | 5 |
| II-7 | 11 | 5 | III-31 | 17(42) | 5 |
| II-9 | 17(19) | 5 | III-34 | 17(43) | 5 |
| II-10 | 18(9) | 4 | III-37 | 17(44) | 5 |
| II-12 | 3 | 4 | III-40 | 17(45) | 5 |
| II-13 | 17(20) | 5 | III-43 | 17(46) | 5 |
| II-14 | 17(21) | 4 | III-49 | 17(47) | 5 |
| II-15 | 17(22) | 3 | III-51 | 17(48) | 5 |
| II-48 | 17(23) | 3 | III-52 | 17(49) | 5 |
| II-49 | 17 (24) | 4 | III-57 | 17(51) | 2 |
| II-50 | 17(25) | 5 | III-61 | 17(52) | 5 |
| II-99 | 17(26) | 3 | III-65 | 17(53) | 5 |
| II-100 | 17(27) | 4 | III-66 | 17(54) | 5 |
| II-104 | 18(10) | 5 | III-67 | 17(55) | 5 |
| II-105 | 18 (11) | 2 | III-68 | 17(56) | 5 |

**Table 6-2 :**

| Effect for rice blast | | | | | |
|---|---|---|---|---|---|
| Compound No. | Example No. | Evaluation | Compound No. | Example No. | Evaluation |
| III-73 | 17(57) | 5 | III-223 | 18(38) | 5 |
| III-74 | 17(58) | 5 | III-224 | 18(42) | 2 |
| III-107 | 17(59) | 5 | III-225 | 18(43) | 4 |
| III-108 | 17(60) | 2 | III-227 | 18(45) | 5 |
| III-109 | 17(61) | 4 | III-228 | 18(46) | 4 |
| III-111 | 4 | 5 | III-229 | 18(47) | 5 |
| III-121 | 17(62) | 5 | III-230 | 18(48) | 5 |
| III-122 | 17(63) | 4 | III-231 | 18(49) | 5 |
| III-123 | 17(64) | 5 | III-232 | 18(50) | 3 |
| III-124 | 17(65) | 5 | III-235 | 18(53) | 4 |
| III-130 | 17(66) | 4 | III-236 | 18(54) | 4 |
| III-133 | 17(67) | 4 | III-237 | 18(55) | 3 |
| III-134 | 17(68) | 3 | III-238 | 18(56) | 4 |
| III-137 | 17(69) | 4 | III-240 | 18(59) | 2 |
| III-139 | 17(70) | 5 | III-243 | 18(62) | 2 |
| III-156 | 17(71) | 4 | III-245 | 18(64) | 5 |
| III-162 | 17(72) | 5 | III-246 | 18(65) | 3 |
| III-174 | 17(73) | 4 | III-247 | 18(66) | 5 |
| III-177 | 17(76) | 4 | III-248 | 18(67) | 3 |
| III-180 | 17(77) | 5 | III-249 | 18(68) | 2 |
| III-201 | 17(78) | 5 | III-250 | 18(69) | 2 |
| III-203 | 18(40) | 2 | III-251 | 18(70) | 5 |
| III-204 | 18(37) | 2 | III-252 | 18(71) | 5 |
| III-208 | 17(80) | 4 | III-253 | 18(72) | 5 |
| III-210 | 17(82) | 5 | III-255 | 18(74) | 4 |
| III-212 | 18(26) | 2 | III-256 | 18(75) | 5 |
| III-213 | 18(27) | 3 | III-257 | 18(76) | 3 |
| III-214 | 18(28) | 4 | III-258 | 18(77) | 5 |
| III-215 | 18(29) | 5 | III-259 | 18(78) | 5 |
| III-216 | 18(30) | 4 | III-262 | 18(81) | 5 |
| III-217 | 18(31) | 4 | III-263 | 18(82) | 5 |
| III-218 | 18(32) | 3 | III-264 | 18(83) | 5 |
| III-219 | 18(33) | 3 | III-265 | 18(84) | 5 |
| III-220 | 18(34) | 3 | III-275 | 18(94) | 2 |
| III-221 | 18(35) | 2 | IV-1 | 17(84) | 5 |
| III-222 | 18(36) | 3 | IV-2 | 17(85) | 5 |

**Table 6-3 :**

| Effect for rice blast | | | | | |
|---|---|---|---|---|---|
| Compound No. | Example No. | Evaluation | Compound No. | Example No. | Evaluation |
| IV-3 | 17(86) | 5 | IV-84 | 18(131) | 5 |
| IV-4 | 17(87) | 4 | IV-86 | 18(133) | 5 |
| IV-5 | 17(88) | 4 | IV-87 | 18(135) | 5 |
| IV-6 | 17(89) | 5 | IV-88 | 18(136) | 3 |
| IV-8 | 17(91) | 3 | IV-89 | 18(137) | 5 |
| IV-9 | 17(92) | 5 | IV-90 | 18(138) | 3 |
| IV-10 | 17(93) | 5 | IV-91 | 18(139) | 3 |
| IV-11 | 17(94) | 5 | IV-92 | 18(140) | 4 |
| IV-12 | 8 | 5 | IV-93 | 18(141) | 4 |
| IV-29 | 17(96) | 3 | IV-94 | 18(142) | 2 |
| IV-33 | 17(97) | 5 | IV-95 | 18(143) | 4 |
| IV-34 | 17(98) | 5 | IV-96 | 18(144) | 4 |
| IV-35 | 17(99) | 5 | IV-97 | 18(145) | 5 |
| IV-40 | 17(100) | 3 | IV-98 | 18(146) | 2 |
| IV-44 | 18(158) | 5 | IV-99 | 18(147) | 4 |
| IV-45 | 10 | 5 | IV-104 | 18(152) | 4 |
| IV-46 | 17(101) | 5 | IV-105 | 18(153) | 3 |
| IV-47 | 16 | 5 | IV-106 | 18(154) | 2 |
| IV-48 | 17(102) | 3 | IV-107 | 18(155) | 5 |
| IV-49 | 17(103) | 2 | IV-108 | 18(156) | 2 |
| IV-50 | 17(104) | 5 | IV-110 | 18(159) | 5 |
| IV-51 | 17(105) | 2 | IV-111 | 18(160) | 3 |
| IV-52 | 17(106) | 3 | IV-112 | 18(161) | 2 |
| IV-53 | 17(107) | 5 | IV-113 | 18(162) | 4 |
| IV-54 | 17(108) | 3 | IV-114 | 18(163) | 5 |
| IV-55 | 17(109) | 4 | IV-115 | 18(164) | 5 |
| IV-56 | 17(110) | 3 | IV-116 | 18(165) | 5 |
| IV-57 | 17(111) | 5 | IV-117 | 18(166) | 4 |
| IV-58 | 17(112) | 5 | IV-119 | 18(168) | 2 |
| IV-59 | 17(113) | 5 | IV-120 | 18(169) | 3 |
| IV-60 | 17(114) | 5 | IV-121 | 18(170) | 4 |
| IV-72 | 17(115) | 2 | IV-122 | 18(171) | 3 |
| IV-73 | 18(119) | 2 | IV-123 | 18(172) | 2 |
| IV-74 | 17(116) | 4 | IV-124 | 18(173) | 5 |
| IV-75 | 18(120) | 2 | Compound A | | 0 |
| IV-80 | 18(127) | 4 | Compound B | | 0 |

### Evaluation Example 2. Effect test for cucumber anthracnose

The formulation of the compound of the invention prepared according to Formulation Example 2 was diluted with water to have a concentration of 200 ppm, and subsequently a sufficient amount of the solution was sprayed with a spraygun on the first and second leaves of cucumber (cultivar: Tokiwa Hikari 3-go P-type) in a 3 leaf stage grown in a plastic pot with a diameter of 6 cm. Next day, a sporal suspension of *Colletotrichum lagenarium* which was a causative fungus of the cucumber anthracnose was sprayed to inoculate. The pot was retained at 22°C under a wet condition for 24 hours, subsequently left in a green house for 7 days. Then, lesion numbers on the first and second leaves were grossly surveyed, and evaluated according to the same criteria as those shown in Table 5. The results are shown in Table 7.

**Table 7-1 :**

| Effect for cucumber anthracnose | | | | | |
|---|---|---|---|---|---|
| Compound No. | Example No. | Evaluation | Compound No. | Example No. | Evaluation |
| I-4 | 17(1) | 5 | III-11 | 17(33) | 3 |
| I-6 | 17(2) | 5 | III-12 | 17(34) | 5 |
| I-8 | 18(3) | 3 | III-13 | 17(35) | 5 |
| I-26 | 5 | 5 | III-14 | 17(36) | 3 |
| I-27 | 17(4) | 5 | III-17 | 17(37) | 5 |
| I-28 | 1 | 5 | III-18 | 7 | 5 |
| I-29 | 17(5) | 5 | III-19 | 17(38) | 5 |
| I-30 | 17(6) | 5 | III-26 | 17(39) | 5 |
| I-33 | 17(8) | 5 | III-27 | 17(40) | 5 |
| I-38 | 17(9) | 5 | III-28 | 17(41) | 4 |
| I-41 | 17(10) | 4 | III-31 | 17(42) | 5 |
| I-45 | 17(12) | 4 | III-34 | 17(43) | 5 |
| I-47 | 17(14) | 4 | III-37 | 17(44) | 3 |
| I-64 | 18(4) | 3 | III-40 | 17(45) | 5 |
| II-7 | 11 | 5 | III-43 | 17(46) | 4 |
| II-9 | 17(19) | 5 | III-49 | 17(47) | 5 |
| II-10 | 18(9) | 5 | III-51 | 17(48) | 4 |
| II-12 | 3 | 4 | III-52 | 17(49) | 5 |
| II-13 | 17(20) | 5 | III-61 | 17(52) | 4 |
| II-14 | 17(21) | 5 | III-65 | 17(53) | 5 |
| II-15 | 17(22) | 4 | III-66 | 17(54) | 5 |
| II-48 | 17(23) | 4 | III-67 | 17(55) | 5 |
| II-100 | 17(27) | 4 | III-68 | 17(56) | 5 |
| II-104 | 18(10) | 5 | III-73 | 17(57) | 4 |
| II-106 | 18(12) | 2 | III-74 | 17(58) | 5 |
| II-108 | 18(14) | 2 | III-107 | 17(59) | 5 |
| II-110 | 18(16) | 1 | III-108 | 17(60) | 3 |
| II-112 | 18(18) | 3 | III-109 | 17(61) | 5 |
| II-113 | 18(19) | 1 | III-111 | 4 | 5 |
| II-114 | 18(20) | 3 | III-121 | 17(62) | 5 |
| II-115 | 18(21) | 2 | III-122 | 17(63) | 4 |
| III-2 | 17(29) | 4 | III-123 | 17(64) | 5 |
| III-4 | 17(30) | 4 | III-124 | 17(65) | 4 |
| III-5 | 9 | 4 | III-130 | 17(66) | 4 |
| III-6 | 17(31) | 4 | III-133 | 17(67) | 4 |
| III-10 | 17(32) | 5 | III-134 | 17(68) | 5 |

**Table 7-2 :**

| Effect for cucumber anthracnose | | | | | |
|---|---|---|---|---|---|
| Compound No. | Example No. | Evaluation | Compound No. | Example No. | Evaluation |
| III-137 | 17(69) | 5 | III-246 | 18(65) | 3 |
| III-139 | 17(70) | 5 | III-247 | 18(66) | 5 |
| III-156 | 17(71) | 4 | III-248 | 18(67) | 2 |
| III-162 | 17(72) | 5 | III-249 | 18(68) | 2 |
| III-174 | 17(73) | 4 | III-250 | 18(69) | 2 |
| III-177 | 17(76) | 4 | III-251 | 18(70) | 5 |
| III-180 | 17(77) | 5 | III-252 | 18(71) | 5 |
| III-201 | 17(78) | 5 | III-253 | 18(72) | 5 |
| III-204 | 18(37) | 2 | III-255 | 18(74) | 4 |
| III-208 | 17(80) | 4 | III-256 | 18(75) | 5 |
| III-210 | 17(82) | 5 | III-257 | 18(76) | 4 |
| III-214 | 18 (28) | 5 | III-258 | 18(77) | 4 |
| III-215 | 18(29) | 5 | III-259 | 18(78) | 4 |
| III-216 | 18(30) | 4 | III-262 | 18(81) | 5 |
| III-217 | 18(31) | 4 | III-263 | 18(82) | 5 |
| III-218 | 18(32) | 4 | III-264 | 18(83) | 5 |
| III-219 | 18(33) | 3 | III-265 | 18(84) | 4 |
| III-220 | 18(34) | 3 | III-275 | 18(94) | 2 |
| III-221 | 18(35) | 2 | IV-1 | 17(84) | 5 |
| III-222 | 18(36) | 2 | IV-2 | 17(85) | 5 |
| III-223 | 18(38) | 5 | IV-3 | 17(86) | 5 |
| III-224 | 18(42) | 2 | IV-4 | 17(87) | 4 |
| III-225 | 18(43) | 4 | IV-9 | 17(92) | 5 |
| III-227 | 18(45) | 5 | IV-10 | 17(93) | 5 |
| III-228 | 18(46) | 3 | IV-11 | 17(94) | 5 |
| III-229 | 18(47) | 5 | IV-12 | 8 | 5 |
| III-230 | 18(48) | 5 | IV-29 | 17(96) | 3 |
| III-231 | 18(49) | 5 | IV-33 | 17(97) | 5 |
| III-232 | 18(50) | 2 | IV-34 | 17(98) | 5 |
| III-235 | 18(53) | 4 | IV-35 | 17(99) | 5 |
| III-236 | 18(54) | 4 | IV-40 | 17(100) | 2 |
| III-237 | 18(55) | 3 | IV-44 | 18(158) | 5 |
| III-238 | 18(56) | 5 | IV-45 | 10 | 5 |
| III-240 | 18(59) | 2 | IV-46 | 17(101) | 5 |
| III-243 | 18(62) | 1 | IV-47 | 16 | 5 |
| III-245 | 18(64) | 5 | IV-48 | 17(102) | 2 |

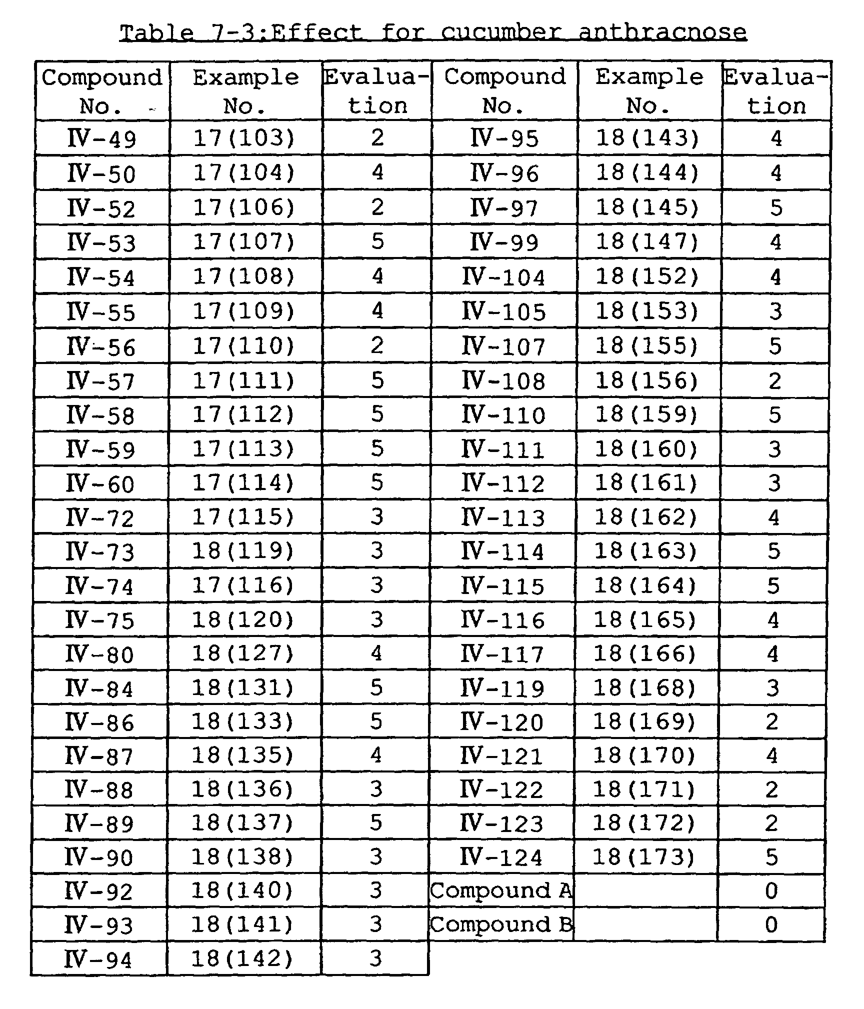

### Evaluation Example 3. Effect test for tomato late blight

The active ingredient compound was diluted with water containing 0.01% Tween 20 to have a concentration of 125 ppm. The amount of this drug solution corresponding to 200 L/10 a was sprayed with a spraygun on tomato in a 5 leaf stage (cultivar: Sugar Lump) grown in a plastic pot with a diameter of 6 cm. Next day, a zoosporangium suspension of *Phytophthora infestans* which was the causative fungus of tomato late blight was sprayed to inoculate. The pot was retained at 22°C under a wet condition for 16 hours, then left in a green house for 3 days. Subsequently, diseased area rates of the third, fourth and fifth true leaves were grossly surveyed, and evaluated according to the same criteria as those in Table 5. The results are shown in Table 8.

**Table 8:**

| Effect for tomato late blight | | |
|---|---|---|
| Compound No. | Example No. | Evaluation |
| 1-65 | 18(5) | 4 |
| 1-67 | 18(8) | 2 |
| II-105 | 18(11) | 5 |
| II-107 | 18(13) | 4 |
| II-111 | 18(17) | 5 |
| II-115 | 18(21) | 3 |
| III-2 | 17(29) | 5 |
| III-3 | 15 | 2 |
| III-226 | 18(44) | 3 |
| III-233 | 18(51) | 2 |
| III-254 | 18(73) | 5 |
| III-260 | 18(79) | 3 |
| III-261 | 18(80) | 2 |
| III-266 | 18(85) | 1 |
| III-267 | 18(86) | 5 |
| III-268 | 18(87) | 0 |
| III-269 | 18(88) | 5 |
| III-270 | 18(89) | 2 |
| III-271 | 18(90) | 1 |
| III-272 | 18(91) | 5 |
| III-273 | 18(92) | 0 |
| III-274 | 18(93) | 4 |
| III-277 | 18(96) | 4 |
| III-278 | 18(97) | 4 |
| III-279 | 18(98) | 5 |
| IV-78 | 18(125) | 1 |
| Compound A | | 0 |
| Compound B | | 0 |

### Evaluation Example 4. Effect test for cucumber downy mildew

The active ingredient compound was diluted with water containing 0.01% Tween 20 to have a concentration of 125 ppm. The amount of this drug solution corresponding to 200 L/10 a was sprayed with a spraygun on cucumber in a 3 leaf stage (cultivar: Hikari 3-go P-type) grown in a plastic pot with a diameter of 6 cm. Next day, a zoosporangium suspension of *Pseudoperonospora cubensis* which was the causative fungus of cucumber downy mildew was sprayed to inoculate. The pot was retained at 22°C under a wet condition for 18 hours, then left in a green house for 3 days. Subsequently, diseased area rates of the first and second true leaves were grossly surveyed, and evaluated according to the same criteria as those in Table 5. The results are shown in Table 9.

**Table 9:**

| Effect for cucumber downy mildew | | |
|---|---|---|
| Compound No. | Example No. | Evaluation |
| I-65 | 18(5) | 2 |
| II-105 | 18(11) | 4 |
| II-107 | 18(13) | 2 |
| II-111 | 18(17) | 3 |
| II-115 | 18(21) | 1 |
| III-2 | 17(29) | 5 |
| III-3 | 15 | 4 |
| III-204 | 18(37) | 4 |
| III-222 | 18(36) | 4 |
| III-226 | 18(44) | 4 |
| III-254 | 18(73) | 3 |
| III-267 | 18(86) | 5 |
| III-269 | 18(88) | 5 |
| III-272 | 18(91) | 3 |
| III-274 | 18(93) | 2 |
| III-277 | 18(96) | 2 |
| III-278 | 18(97) | 2 |
| III-279 | 18(98) | 3 |
| Compound A | | 0 |
| Compound B | | 0 |

### INDUSTRIAL APPLICABILITY

The substituted isoxazole alkylamine derivative represented by the formula (I) or (IA) exhibits the disease control effect on plant disease damages, particularly on rice blast and cucumber anthracnose, in a low drug amount, is safe for crops, and is useful as the agri-horticultural fungicide.

## Claims

1. A substituted isoxazole alkylamine derivative represented by the formula (I): 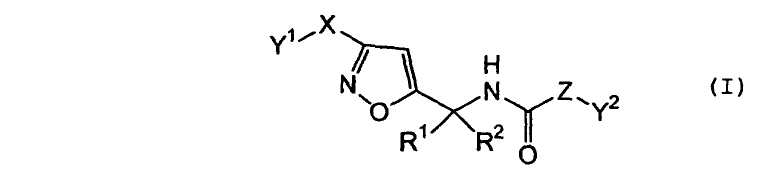 wherein R¹ and R² may be the same or different, and each represent a hydrogen atom, a lower alkyl group which may be substituted, a lower alkenyl group, a lower alkynyl group, a cycloalkyl group which may be substituted, a lower alkoxy group, a lower alkoxycarbonyl group, a lower alkylthio group, a halogen atom, a hydroxyl group, a carboxyl group, or a cyano group, or R¹ and R² may together form a cycloalkyl group which may be substituted;
X represents the following formula (1), (2), or (3): 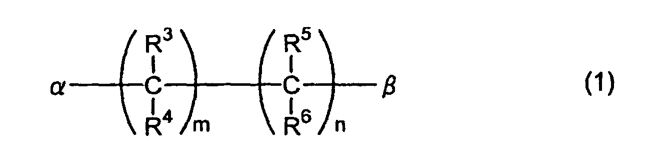 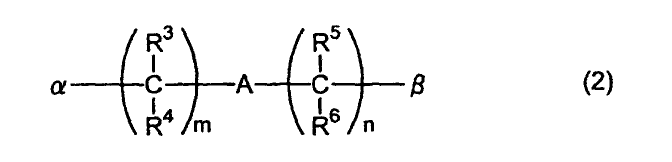 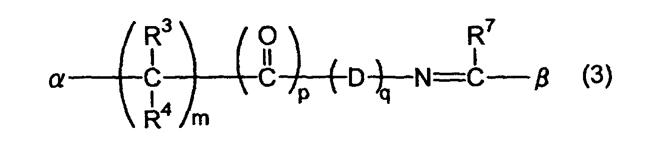 wherein R³, R⁴, R⁵, and R⁶ may be the same or different, and each represent a hydrogen atom, a lower alkyl group which may be substituted, a lower alkenyl group, a lower alkynyl group, a cycloalkyl group which may be substituted, a lower alkoxy group, a lower alkoxycarbonyl group, a lower alkylthio group, an amino group, a lower alkylamino group, a di(lower alkyl)amino group, a halogen atom, a hydroxyl group, or a cyano group, or each pair of R³ and R⁴, and R⁵ and R⁶ may together form a cycloalkyl group which may be substituted;
A represents an oxygen atom, a sulfur atom, an -S(O)- group, an -S(O)₂- group, an -NR¹²- group wherein R¹² represents a hydrogen atom or a lower alkyl group, a carbonyl group, an -NH-CO- group, a -CO-NH- group, a -C≡C- group, an -NH-CO-NH- group, an -O-CONH- group, an -HC=N- group, or 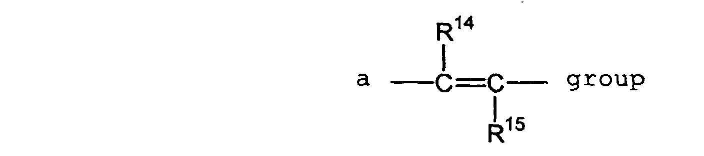 wherein R¹⁴ and R¹⁵ each represent a hydrogen atom or a lower alkyl group;
m and n each represent 0 or an integer of 1 to 3;
p and q each represent 0 or 1;
R⁷ represents a hydrogen atom or a lower alkyl group; and
D represents an oxygen atom or an -NH- group;
α binds to a Y¹ side, and β binds to an isoxazole moiety);
Y¹ represents a lower alkyl group which may be substituted, a lower alkenyl group which may be substituted, a lower alkynyl group which may be substituted, a cycloalkyl group which may be substituted, a lower cyclo alkenyl group which may be substituted, a phenyl group which may be substituted, a naphthyl group which may be substituted, a heteroaryl group which may be substituted, an aliphatic hetero ring which may be substituted, or the following formula (4): 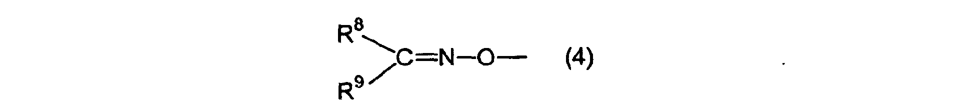 wherein R⁸ and R⁹ each represent a hydrogen atom, a lower alkyl group which may be substituted, or a phenyl group which may be substituted, or R⁸ and R⁹ may together form a cycloalkyl group which may be substituted;
Y² represents a lower alkenyl group which may be substituted, a lower alkynyl group which may be substituted, a cycloalkyl group which may be substituted, a lower cyclo alkenyl group which may be substituted, a phenyl group which may be substituted, a naphthyl group which may be substituted, a heteroaryl group which may be substituted, or an aliphatic hetero ring which may be substituted;
Z represents a group denoted by the following formula (5) or (6): 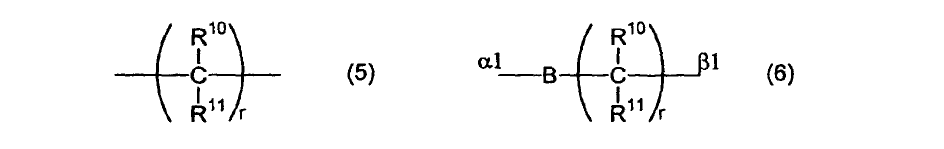 wherein R¹⁰ and R¹¹ may be the same or different, and each represent a hydrogen atom, a lower alkyl group which may be substituted, a cycloalkyl group which may be substituted, a lower alkoxy group, a lower alkoxycarbonyl group, a lower alkylthio group, an amino group, a lower alkylamino group, a di(lower alkyl)amino group, a halogen atom, a hydroxyl group, or a cyano group, or R¹⁰ and R¹¹ may together form a cycloalkyl group which may be substituted;
r represents 0 or an integer of 1 to 3; and
B represents an oxygen atom, a sulfur atom, or an -NR¹³- group wherein R¹³ represents a hydrogen atom or a lower alkyl group;
any one of α1 and β1 may be bound to a Y² side;
provided that the following (1) to (3) are excluded:
(1) a compound in which X is a single bond (i.e. the case that each of m and n is 0 in the formula (1)) and Y¹ represents a 4-hydroxy-3,5-di-tert-butylphenyl group;
(2) a compound in which R¹ and R² represent hydrogen atoms, X is a single bond (i.e. the case that each of m and n is 0 in the formula (1)) and both Y¹ and Y² represent unsubstituted phenyl groups (2-1) when Z is a single bond (i.e. the case that r is 0 in the formula (5)) or (2-2) when Z is an NH group (i.e. the case that r=0 and B represents an -NH- group in the formula (6)); and
(3) a compound in which R¹ and R² represent hydrogen atoms, Z is a single bond (i.e. the case that r is 0 in the formula (5)) and Y² represents a 4-hydroxycinnolin-3-yl group which may be substituted.

2. The substituted isoxazole alkylamine derivative according to claim 1, wherein R¹ and R² each independently represent a hydrogen atom or a lower alkyl group which may be substituted.

3. The substituted isoxazole alkylamine derivative according to claim 2, wherein Z is an oxygen atom (i.e. the case that r is 0 and B is an oxygen atom in the formula (6)), and Y² represents a phenyl group which may be substituted, a naphthyl group which may be substituted, or a cycloalkyl group which may be substituted.

4. The substituted isoxazole alkylamine derivative according to claim 2, wherein Z represents an -NR¹³- group wherein R¹³ represents a hydrogen atom or a lower alkyl group (i.e. the case that r is 0 and B is an -NR¹³- group) and Y² represents a phenyl group which may be substituted, a naphthyl group which may be substituted, or a cycloalkyl group which may be substituted.

5. The substituted isoxazole alkylamine derivative according to claim 2, wherein X and Z are single bonds (i.e. the case that m and n are 0 in the formula (1) and r is 0 in the formula (5)) and Y² represents a phenyl group which may be substituted, a naphthyl group which may be substituted, or a cycloalkyl group which may be substituted.

6. The substituted isoxazole alkylamine derivative according to claim 2, wherein X represents a group represented by the formula (1) wherein R³, R⁴, R⁵, and R⁶ may be the same or different and each represent a hydrogen atom, a lower alkyl group which may be substituted, a lower alkoxy group, a lower alkoxycarbonyl group, a halogen atom, or a cyano group, Y² represents a phenyl group which may be substituted, a naphthyl group which may be substituted, or a cycloalkyl group which may be substituted, and m + n is 1, 2, or 3.

7. The substituted isoxazole alkylamine derivative according to claim 2, wherein X represents a group represented by the formula (2) wherein R³, R⁴, R⁵, and R⁶ may be the same or different and each represent a hydrogen atom, a lower alkyl group which may be substituted, a lower alkoxy group, a lower alkoxycarbonyl group, a halogen atom, or a cyano group, Y² represents a phenyl group which may be substituted, a naphthyl group which may be substituted, or a cycloalkyl group which may be substituted, n is 1 or 2, and m + n is 1, 2, or 3.

8. The substituted isoxazole alkylamine derivative according to claim 2, wherein X represents a group represented by the formula (2) wherein R³, R⁴, R⁵, and R⁶ may be the same or different and each represent a hydrogen atom, a lower alkyl group which may be substituted, a lower alkoxy group, a lower alkoxycarbonyl group, a halogen atom, or a cyano group, Y² represents a phenyl group which may be substituted, a naphthyl group which may be substituted, or a cycloalkyl group which may be substituted, n is 0, and m is 0, 1, or 2.

9. The substituted isoxazole alkylamine derivative according to claim 2, wherein X represents a group represented by the formula (3) wherein R³ and R⁴ may be the same or different and each represent a hydrogen atom, a lower alkyl group which may be substituted, a lower alkoxy group, a halogen atom, or a cyano group, m represents 0, 1, or 2, and Y² represents a phenyl group which may be substituted, a naphthyl group which may be substituted, or a cycloalkyl group which may be substituted.

10. The substituted isoxazole alkylamine derivative according to claim 2, wherein Y¹ represents a methyl group substituted with a halogen atom, and Z is a single bond (i.e. the case that m and n are 0 in the formula (1)).

11. An agri-horticultural fungicide containing as an active ingredient a substituted isoxazole alkylamine derivative represented by the formula (IA): 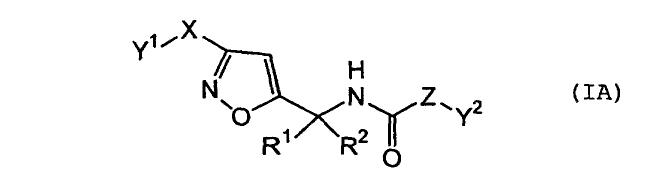 wherein R¹ and R² may be the same or different, and each represent a hydrogen atom, a lower alkyl group which may be substituted, a lower alkenyl group, a lower alkynyl group, a cycloalkyl group which may be substituted, a lower alkoxy group, a lower alkoxycarbonyl group, a lower alkylthio group, a halogen atom, a hydroxyl group, a carboxyl group, or a cyano group, or R¹ and R² may together form a cycloalkyl group which may be substituted;
X represents the following formula (1), (2), or (3): 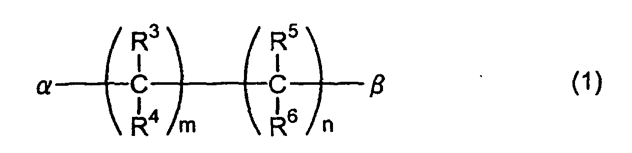 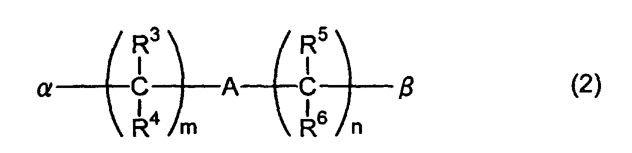 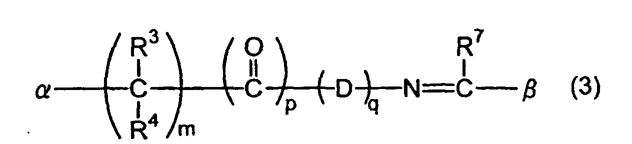 wherein R³, R⁴, R⁵, and R⁶ may be the same or different, and each represent a hydrogen atom, a lower alkyl group which may be substituted, a lower alkenyl group, a lower alkynyl group, a cycloalkyl group which may be substituted, a lower alkoxy group, a lower alkoxycarbonyl group, a lower alkylthio group, an amino group, a lower alkylamino group, a di(lower alkyl)amino group, a halogen atom, a hydroxyl group, or a cyano group, or each pair of R³ and R⁴, and R⁵ and R⁶ may together form a cycloalkyl group which may be substituted;
A represents an oxygen atom, a sulfur atom, an -S(O)- group, an -S(O)₂- group, an -NR¹²- group wherein R¹² represents a hydrogen atom or a lower alkyl group, a carbonyl group, an -NH-CO- group, a -CO-NH- group, a -C≡C- group, an -NH-CO-NH- group, an -O-CONH- group, an -HC=N- group, or 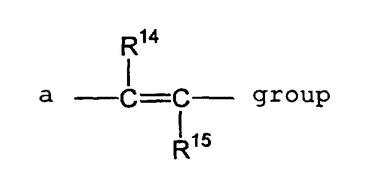 wherein R¹⁴ and R¹⁵ each represent a hydrogen atom or a lower alkyl group;
m and n each represent 0 or an integer of 1 to 3;
p and q each represent 0 or 1;
R⁷ represents a hydrogen atom or a lower alkyl group; and
D represents an oxygen atom or an -NH- group;
α binds to a Y¹ side, and β binds to an isoxazole moiety;
Y¹ represents a lower alkyl group which may be substituted, a lower alkenyl group which may be substituted, a lower alkynyl group which may be substituted, a cycloalkyl group which may be substituted, a lower cyclo alkenyl group which may be substituted, a phenyl group which may be substituted, a naphthyl group which may be substituted, a heteroaryl group which may be substituted, an aliphatic hetero ring which may be substituted, or the following formula (4): 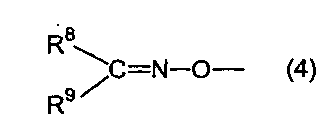 wherein R⁸ and R⁹ each represent a hydrogen atom, a lower alkyl group which may be substituted, or a phenyl group which may be substituted, or R⁸ and R⁹ may together form a cycloalkyl group which may be substituted;
Y² represents a lower alkenyl group which may be substituted, a lower alkynyl group which may be substituted, a cycloalkyl group which may be substituted, a lower cyclo alkenyl group which may be substituted, a phenyl group which may be substituted, a naphthyl group which may be substituted, a heteroaryl group which may be substituted, or an aliphatic hetero ring which may be substituted;
Z represents a group represented by the following formula (5) or (6): 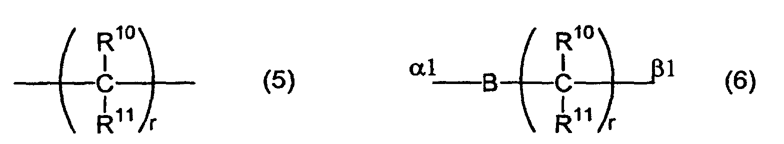 wherein R¹⁰ and R¹¹ may be the same or different, and each represent a hydrogen atom, a lower alkyl group which may be substituted, a cycloalkyl group which may be substituted, a lower alkoxy group, a lower alkoxycarbonyl group, a lower alkylthio group, an amino group, a lower alkylamino group, a di(lower alkyl)amino group, a halogen atom, a hydroxyl group, or a cyano group, or R¹⁰ and R¹¹ may together form a cycloalkyl group which may be substituted;
r represents 0 or an integer of 1 to 3; and
B represents an oxygen atom, a sulfur atom, or an -NR¹³- group wherein R¹³ represents a hydrogen atom or a lower alkyl group;
any one of α1 and β1 may be bound to a Y² side.
